# FASCICULE DE BREVET EUROPEEN

(11) **EP 1 599 167 B1**
(45) Date de publication et mention de la délivrance du brevet: **03.10.2007**
(21) Numéro de dépôt: 04714348.2
(22) Date de dépôt: 25.02.2004
(51) Int. Cl.: C07D 235/30

(54) **DERIVES DE BENZIMIDAZOLE ET D'IMIDAZO-PYRIDINE AYANT UNE AFFINITE POUR LES RECEPTEURS DES MELANOCORTINES ET LEUR UTILISATION EN TANT QUE MEDICAMENT**
BENZIMIDAZOL- UND IMIDAZOPYRIDIN-DERIVATE MIT EINER AFFINITÄT ZUR MELANOCORTIN-REZEPTOREN ZUR VERWENDUNG ALS MEDIKAMENTE
BENZIMIDAZOLE AND IMIDAZOPYRIDINE DERIVATIVES HAVING AFFINITY FOR THE MELANOCORTIN RECEPTORS AND USE THEREOF AS A MEDICAMENT

(30) Priorité: 26.02.2003 FR 0302320
(43) Date de publication de la demande: 30.11.2005
(73) Titulaire: Société de Conseils de Recherches et d'Applications Scientifiques ( S.C.R.A.S.), 75016 Paris (FR)
(72) Inventeur: POITOUT, Lydie, F-92160 ANTONY (FR); BRAULT, Valérie, F-78730 Saint-Arnoult-en-Yvelines (FR); SACKUR, Carole, F-75004 Paris (FR); ROUBERT, Pierre, F-75014 Paris (FR); PLAS, Pascale, F-92320 Châtillon (FR)
(74) Mandataire: Bourgouin, André
(86) Numéro de dépôt international: PCT/FR2004/000418
(87) Numéro de publication internationale: WO 2004/075823

(56) Documents cités:
- WO-A-01/21634

## Description

La présente demande a pour objet de nouveaux dérivés de benzimidazole et d'imidazo-pyridine. Ces produits ont une bonne affinité pour certains sous-types de récepteurs des mélanocortines, en particulier des récepteurs MC4. Ils sont particulièrement intéressants pour traiter les états pathologiques et les maladies dans lesquels un ou plusieurs récepteurs des mélanocortines sont impliqués. L'invention concerne également des compositions pharmaceutiques contenant lesdits produits et leur utilisation pour la préparation d'un médicament.

Les mélanocortines représentent un groupe de peptides qui dérivent d'un même précurseur, la proopiomélanocortine (POMC), et qui sont structurellement proches : L'hormone adrénocorticotrope (ACTH), l'hormone stimulante des mélanocytes α (α-MSH), la β-MSH et la γ-MSH (Eipper B.A. et Mains R.E., Endocr. Rev. 1980, 1, 1-27). Les mélanocortines exercent de nombreuses fonctions physiologiques. Elles stimulent la synthèse des stéroïdes par la cortico-surrénale et la synthèse d'eumelanine par les mélanocytes. Elles régulent la prise de nourriture, le métabolisme énergétique, la fonction sexuelle, la régénération neuronale, la pression sanguine et la fréquence cardiaque, ainsi que la perception de la douleur, l'apprentissage, l'attention et la mémoire. Les mélanocortines possèdent également des propriétés anti-inflammatoires et anti-pyrétiques et contrôlent la sécrétion de plusieurs glandes endocrines ou exocrines telles les glandes sébacées, lacrymales, mammaires, la prostate et le pancréas (Wikberg J.E. et al. Pharmacol. Res. 2000, 42, 393-420 ; Abdel-Malek Z.A., Cell. Mol. Life. Sci. 2001, 58, 434-441).

Les effets des mélanocortines sont médiés par une famille de récepteurs membranaires spécifiques à sept domaines transmembranaires et couplés aux protéines G. Cinq sous-types de récepteurs, nommés MC1 à MCS, ont été clonés et caractérisés à ce jour. Ces récepteurs diffèrent par leur distribution tissulaire et par l'affinité des différentes mélanocortines, les récepteurs MC2 ne reconnaissant que l'ACTH. La stimulation des récepteurs des mélanocortines active l'adénylate cyclase avec production d'AMP cyclique. Si les rôles fonctionnels spécifiques de chacun des récepteurs ne sont pas totalement élucidés, le traitement de désordres pathologiques ou de maladies peut être associé à une affinité pour certains sous-types de récepteurs. Ainsi l'activation des récepteurs MC1 a été associée au traitement des inflammations, alors que leur blocage a été associé au traitement de cancers cutanés. Le traitement des troubles de la nutrition a été associé aux récepteurs MC3 et MC4, le traitement de l'obésité par les agonistes et le traitement de la cachexie et de l'anorexie par les antagonistes. D'autres indications associées à l'activation des récepteurs MC3 et MC4 sont les troubles de l'activité sexuelle, les douleurs neuropathiques, l'anxiété, la dépression et les toxicomanies. L'activation des récepteurs MC5 a été associée au traitement de l'acné et des dermatoses.

WO 01/21634 décrit des dérivés de benzimidazole utiles comme des modulateurs des récepteurs MC3.

Les déposants ont trouvé que les nouveaux composés de formule générale (I) décrits ci-après possèdent une bonne affinité pour les récepteurs des mélanocortines. Ils agissent préférentiellement sur les récepteurs MC4. Lesdits composés, agonistes ou antagonistes des récepteurs des mélanocortines, peuvent être utilisés pour traiter les états pathologiques ou les maladies métaboliques, du système nerveux ou dermatologiques, dans lesquels un ou plusieurs récepteurs des mélanocortines sont impliqués tels que les exemples suivants : les états inflammatoires, les troubles de l'homéostasie énergétique, de la prise de nourriture, les désordres pondéraux (l'obésité, la cachexie, l'anorexie), les désordres de l'activité sexuelle (les troubles de l'érection), la douleur et plus particulièrement la douleur neuropathique. On peut également citer les troubles mentaux (l'anxiété, la dépression), les toxicomanies, les maladies de la peau (l'acné, les dermatoses, les cancers cutanés, les mélanomes). Ces composés peuvent également être utilisés pour stimuler la régénération nerveuse.

L'invention a donc pour objet des composés de formule générale (**I**) 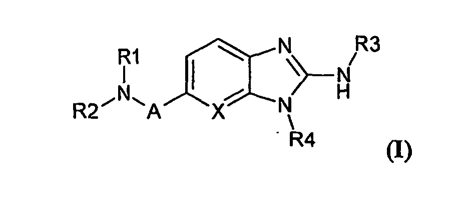 sous forme racémique, d'énantiomère ou toutes combinaisons de ces formes et dans laquelle :
A représente -CH₂- ,-C(O)-, -C(O)-C(Rₐ)(R_{b})- ;
X représente -CH- ou -N- ;
Rₐ et R_{b} représentent, indépendamment, l'atome d'hydrogène ou un radical (C₁-C₆)alkyle ;
R₁ représente l'atome d'hydrogène ; un radical (C₁-C₈)alkyle éventuellement substitué par hydroxy ou un ou plusieurs radicaux halo identiques ou différents ; (C₂-C₆)alkenyle ; ou un radical de formule -(CH₂)ₙ-X₁ ;
R₂ représente un radical (C₁-C₈)alkyle éventuellement substitué par hydroxy ou un ou plusieurs radicaux halo identiques ou différents ; (C₂-C₆)alkenyle ; ou un radical de formule -(CH₂)ₙ-X₁ ;
chaque X₁ représente indépendamment (C₁-C₆)alkoxy, (C₃-C₇)cycloalkyle, adamantyle, hétérocycloalkyle, aryle ou hétéroaryle,
   les radicaux (C₃-C₇)cycloalkyle, hétérocycloalkyle, aryle et hétéroaryle étant éventuellement substitués par un ou plusieurs substituants identiques ou différents choisis parmi : -(CH₂)_{n'}-V₁-Y₁, halo, nitro, cyano et aryle;
   V₁ représente -O-, -S- ou une liaison covalente ;
   Y₁ représente un radical (C₁-C₆)alkyle éventuellement substitué par un ou plusieurs radicaux halo identiques ou différents ;
   n représente un entier de 0 à 6 et n' un entier de 0 à 2 (étant entendu que lorsque n est égal à 0, alors X₁ ne représente pas le radical alkoxy)
ou bien R₁ et R₂ forment ensemble, avec l'atome d'azote auquel ils sont rattachés, un hétérobicycloalkyle ou un hétérocycloalkyle éventuellement substitué par un ou plusieurs substituants identiques ou différents choisis parmi : hydroxy, (C₁-C₆)alkyle éventuellement substitué par hydroxy, (C₁-C₆)alkcoxy-carbonyle, hétérocycloalkyle et -C(O)NV₁'Y₁' avec V₁' et Y₁' représentant indépendamment l'atome d'hydrogène ou un (C₁-C₆)alkyle, ; ou bien R₁ et R₂ forment ensemble un radical de formule : 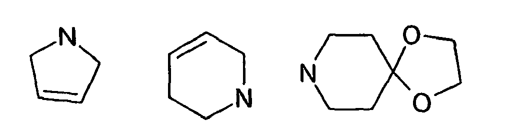
R₃ représente -Z₃, -C(R_{Z3})(R'_{Z3})-Z₃,-C(R_{Z3})(R'_{Z3})-(CH₂)ₚ-Z₃ou -C(O)-Z'₃
   R_{Z3} et R'_{Z3} représentent, indépendamment, l'atome d'hydrogène ou un radical (C₁-C₆)alkyle;
   Z₃ représente Z₃ₐ, Z_{3b}, Z_{3c}, Z_{3d}, ou Z₃ₑ;
   Z₃ₐ représente un radical (C₁-C₆)alkyle ;
   Z_{3b} représente un radical (C₁-C₆)alkoxy, (C₁-C₆)alkylthio, (C₁-C₆)alkylamino ou di((C₁-C₆)alkyl)amino;
   Z_{3c} représente un radical aryle ou hétéroaryle ;
   Z_{3d} représente un radical (C₁-C₆)alkoxy-carbonyle, amino-carbonyle, (C₁-C₆)alkylamino-carbonyle, di((C₁-C₆)alkyl)amino-carbonyle, (C₁-C₆)alkyl-C(O)-NH- , (C₃-C₇)cycloalkyle, hétérocycloalkyle ;
   les radicaux (C₃-C₇) cycloalkyle et hétérocycloalkyle étant éventuellement substitués par un ou plusieurs substituants identiques ou différents choisis parmi : halo, nitro, (C₁-C₆)alkoxy éventuellement substitué par un ou plusieurs radicaux halo identiques ou différents, (C₁-C₆)alkyle éventuellement substitué par un ou plusieurs radicaux halo identiques ou différents, (C₁-C₆)alkyl-carbonyle, (C₁-C₆)alkoxy-carbonyle, amino-carbonyle, (C₁-C₆)alkylamino-carbonyle, di((C₁-C₆)alkyl)amino-carbonyle et oxy,
   les radicaux aryle et hétéroaryle étant éventuellement substitués par un ou plusieurs substituants identiques ou différents choisis parmi : halo, cyano, nitro, azido, oxy, (C₁-C₆)alkoxy-carbonyl-(C₁-C₆)alkenyle, (C₁-C₆)alkylamino-carbonyl-(C₁-C₆)alkenyle, -SO₂-NR₃₁R₃₂, hétérocycloalkyle, hétéroaryle ou -(CH₂)_{p'}-V₃-Y₃;
   R₃₁ et R₃₂ forment ensemble, avec l'atome d'azote sur lequel ils sont rattachés, un hétérocycloalkyle ;
   V₃ représente -O-, -S-, -C(O)-, -C(O)-O-, -O-C(O)-, -SO₂- -SO₂NH-, -NR'₃-SO₂-, -NR'₃-, -NR'₃-C(O)-, -C(O)-NR'₃-, -NH-C(O)-NR'₃- ou une liaison covalente ;
   Y₃ représente l'atome d'hydrogène ; un radical (C₁-C₆)alkyle éventuellement substitué par un ou plusieurs radicaux halo identiques ou différents ; un radical aryle éventuellement substitué par un ou plusieurs substituants identiques ou différents choisis parmi : halo, nitro, (C₁-C₆)alkyle et (C₁-C₆)alkoxy ; ou un radical aryl-(C₁-C₆)alkyle éventuellement substitué par un ou plusieurs substituants identiques ou différents choisis parmi : halo, nitro, (C₁-C₆)alkyle et (C₁-C₆)alkoxy;
   Z₃ₑ représente un radical de formule 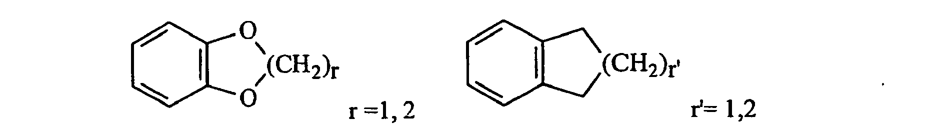 Z'₃ représente un radical aryle éventuellement substitué par un ou plusieurs substituants identiques ou différents choisis parmi : halo, nitro et -(CH₂)_{p"}-V'₃-Y'₃;
   V'₃ représente -O-, -C(O)-, -C(O)-O-, -C(O)-NR'₃, -NH-C(O)-NR'₃ ou une liaison covalente ;
   Y'₃ représente l'atome d'hydrogène ou un radical (C₁-C₆)alkyle éventuellement substitué par un ou plusieurs radicaux halo identiques ou différents ;
   R'₃ représente l'atome d'hydrogène, un radical (C₁-C₆)alkyle ou (C₁-C₆)alkoxy ;
   p représente un entier de 1 à 4 ; p'et p" représentent, indépendamment, un entier de 0 à 4 ;
R₄ représente un radical de formule-(CH₂)ₛ-R'₄ ;
R'₄ représente le radical guanidino ; un hétérocycloalkyle contenant au moins un atome d'azote et éventuellement substitué par (C₁-C₆)alkyle ou aralkyle ; un hétéroaryle contenant au moins un atome d'azote et éventuellement substitué par (C₁-C₆)alkyle ; ou un radical de formule -NW₄W'₄
   W₄ représente l'atome d'hydrogène ou (C₁-C₈)alkyle ;
   W'₄ représente un radical de formule -(CH₂)_{s'}-Z₄ ;
   Z₄ représente l'atome d'hydrogène, (C₁-C₈)alkyle éventuellement substitué par un ou plusieurs substituants identiques ou différents choisis parmi : (C₁-C₆)alkoxy, (C₁-C₆)alkylthio et hydroxy ; (C₂-C₆)alkényle ; (C₃-C₇)cycloalkyle éventuellement substitué par un ou plusieurs substituants (C₁-C₆)alkyle identiques ou différents ; cyclohexène ; hétéroaryle et aryle ;
   les radicaux aryle et hétéroaryle étant éventuellement substitués par un ou plusieurs radicaux identiques ou différents choisis de formule -(CH₂)_{s"}-V₄-Y₄, hydroxy, halo, nitro et cyano ;
   V₄ représente -O-, -S-, -NH-C(O)-, -NV'₄ ou une liaison covalente ;
   Y₄ représente un atome d'hydrogène ou un radical (C₁-C₆)alkyle éventuellement substitué par un ou plusieurs radicaux halo identiques ou différents ;
   V₄' représente un atome d'hydrogène ou un (C₁-C₆)alkyle ;
   s" représente un entier de 0 à 4 ;
ou bien Z₄ représente un radical de formule 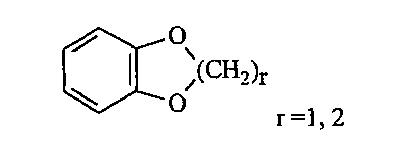
s et s' représentent, indépendamment, un entier de 0 à 6 ;
et i) lorsque R₃ représente -C(O)-Z'₃ et R₄ représente un radical de formule-(CH₂)ₛ-NW₄W'₄ et W₄ et W'₄ représentent, indépendamment, l'atome d'hydrogène ou le radical (C₁-C₆)alkyle, alors -(CH₂)ₛ ne représente ni le radical éthylène ni le radical -(CH₂)-CH((C₁-C₄)alkyle)- et ii) lorsque R₃ représente -Z_{3c} et Z_{3c} représente un radical phényle ou naphtyle, alors phényle et naphtyle ne sont pas susbtitués par cyano ; et étant entendu que lorsque R₃ représente -Z_{3d} alors Z_{3d} ne représente qu'un radical (C₃-C₇)cycloalkyle ou hétérocycloalkyle ;ou un sel pharmaceutiquement acceptable de ces derniers.

Dans les définitions indiquées ci-dessus, l'expression halo représente le radical fluoro, chloro, bromo ou iodo, de préférence chloro, fluoro ou bromo: L'expression alkyle (lorsqu'il n'est pas donné plus de précision), représente de préférence un radical alkyle ayant de 1 à 6 atomes de carbone, linéaire ou ramifié, tels que les radicaux méthyle, éthyle, propyle, isopropyle, butyle, isobutyle, *sec*-butyle et *tert*-butyle, pentyle ou amyle, isopentyle, néopentyle, 2,2-diméthyl-propyle, hexyle, isohexyle ou 1,2,2-triméthyl-propyle. Le terme (C₁-C₈)alkyle désigne un radical alkyle ayant de 1 à 8 atomes de carbone, linéaire ou ramifié, tels les radicaux contenant de 1 à 6 atomes de carbone tels que définis ci-dessus mais également heptyle, octyle, 1,1,2,2-tétraméthyl-propyle, 1,1,3,3-tétraméthyl-butyle. Le terme hydroxyalkyle désigne les radicaux dans lesquels le radical alkyle est tel que défini ci-dessus comme par exemple hydroxyméthyle, hydroxyéthyle. Par l'expression alkyle substitué par hydroxy, il faut comprendre toute chaîne alkyle linéaire ou ramifiée, contenant un radical hydroxy positionnée le long de la chaîne ; ainsi pour une chaîne contenant 3 atomes de carbone et un radical hydroxy, on peut donner comme exemple HO-(CH₂)₃-, CH₃-CH(OH)-CH₂- et CH₃-CH₂-CH(OH)-.

Par alkényle, lorsqu'il n'est pas donné plus de précision, on entend un radical alkyle linéaire ou ramifié comptant de 1 à 6 atomes de carbone et présentant au moins une insaturation (double liaison), comme par exemple vinyle, allyle, propényle, butènyle ou pentènyle.

Le terme alkcoxy désigne les radicaux dans lesquels le radical alkyle est tel que défini ci-dessus comme par exemple les radicaux méthoxy, éthoxy, propyloxy ou isopropyloxy mais également butoxy linéaire, secondaire ou tertiaire, pentyloxy. Le terme alkoxy-carbonyle désigne de préférence les radicaux dans lesquels le radical alkoxy est tel que défini ci-dessus comme par exemple méthoxycarbonyle, éthoxycarbonyle. Le terme alkylthio désigne les radicaux dans lesquels le radical alkyle est tel que défini ci-dessus comme par exemple méthylthio, éthylthio. Le terme guanidino représente le radical -NHC(=NH)NH₂.

Le terme (C₃-C₇)cycloalkyle désigne un système monocyclique carboné saturé comprenant de 3 à 7 atomes de carbone, et de préférence les cycles cyclopropyle, cyclobutyle, cyclopentyle, cyclohexyle ou cycloheptyle. L'expression hétérocycloalkyle désigne un système saturé monocyclique ou bicyclique condensé contenant de 2 à 9 atomes de carbone et au moins un hétéroatome. Ce radical peut contenir plusieurs hétéroatomes identiques ou différents. De préférence, les hétéroatomes sont choisis parmi l'oxygène, le soufre ou l'azote. Comme exemple d'hétérocycloalkyle, on peut citer les cycles contenant au moins un atome d'azote tels que pyrrolidine, imidazolidine, pyrrazolidine, isothiazolidine, thiazolidine, isoxazolidine, oxazolidine, pipéridine, pipérazine, azépane (azacycloheptane), azacyclooctane, diazépane, morpholine, décahydroisoquinoline (ou décahydroquinoline) mais également les cycles ne contenant aucun atome d'azote tels tétrahydrofurane ou tétrahydrothiophène. Comme illustration de cycloalkyle ou hétérocycloalkyle substitué par oxy, on peut citer les comme exemple pyrrolidinone et imidazolidinone.

Le terme hétérobicycloalkyle désigne un système bicyclique hydrocarboné saturé non condensé contenant de 5 à 8 atomes de carbone et au moins un hétéroatome choisi parmi l'azote, l'oxygène et le soufre. Comme exemple d'hétérobicycloalkyle, on peut citer les aza-bicycloheptane et aza-bicyclooctane tels que 7-aza-bicyclo[2,2,1]heptane, 2-aza-bicyclo[2,2,2]octane ou 6-aza-bicyclo[3,2,1]octane.

L'expression aryle représente un radical aromatique, constitué d'un cycle ou de cycles condensés, comme par exemple le radical phényle, naphtyle, fluorényle ou anthryle. L'expression hétéroaryle désigne un radical aromatique, constitué d'un cycle ou de cycles condensés, avec au moins un cycle contenant un ou plusieurs hétéroatomes identiques ou différents choisis parmi le soufre, l'azote ou l'oxygène. Comme exemple de radical hétéroaryle, on peut citer les radicaux contenant au moins un atome d'azote tels que pyrrolyle, imidazolyle, pyrazolyle, isothiazolyle, thiazolyle, isoxazolyle, oxazolyle, triazolyle, thiadiazolyle, pyridyle, pyrazinyle, pyrimidyle, quinolyle, isoquinolyle, quinoxalinyle, indolyle, dihydroindolyle, benzoxadiazoyle, carbazolyle, phénoxazinyle mais également les radicaux ne contenant pas d'atome d'azote tels que thiényle, benzothiényle, furyle, benzofuryle, dibenzofuryle, dihydrobenzofuryle, dibenzothiènyle, thioxanthènyle, ou pyranyle. Le terme aralkyle (arylalkyle) désigne de préférence les radicaux dans lesquels les radicaux aryle et alkyle sont tels que définis ci-dessus comme par exemple benzyle ou phenéthyle. Comme illustration de radical aryle ou hétéroaryle substitué par oxy, on peut citer par exemple fluorènone, l'acridone, xanthènone, benzothiényle-dione, anthraquinone, thioxanthène, benzocoumarine.

Dans la présente demande également, le radical -(CH₂)ᵢ- (i entier pouvant représenter n, n', p, p', p", s, s' et s" tels que définis ci-dessus), représente une chaîne hydrocarbonée, linéaire ou ramifiée, de i atomes de carbone. Ainsi le radical -(CH₂)₃- peut représenter -CH₂-CH₂-CH₂- mais également -CH(CH₃)-CH₂-, -CH₂-CH(CH₃)- ou -C(CH₃)₂-.

Selon la présente demande également, lorsqu'un radical a pour formule -B-D-E avec D représentant par exemple -C(O)-NH-, cela signifie que l'atome de carbone de -C(O)-NH- est lié à B et l'atome d'azote à E.

De préférence, l'invention concerne des composés de formule 1 telle que définie ci-dessus et caractérisés en ce que X représente -CH- ; ou un sel pharmaceutiquement acceptable de ces derniers.

De manière préférentielle, l'invention concerne des composés de formule I telle que définie ci-dessus, caractérisés en ce que X représente -CH- et A représente -CH₂, et plus particulièrement
R₁ et R₂ représentent, indépendamment, un radical (C₁-C₈)alkyle ;
R₃ représente -Z_{3c}, -C(R_{Z3})(R'_{Z3})-Z_{3c}, -C(R_{Z3})(R'_{Z3})-Z_{3d}, -C(R_{Z3})(R'_{Z3})-(CH₂)ₚ-Z_{3d} ;
R₄ représente un radical de formule-(CH₂)ₛ-R'₄ ;
R'₄ représente un hétérocycloalkyle contenant au moins un atome d'azote et éventuellement substitué par (C₁-C₆)alkyle ; ou un radical de formule -NW₄W'₄ ;
W₄ représente l'atome d'hydrogène ou (C₁-C₈)alkyle ;
W'₄ représente un radical de formule -(CH₂)_{s'}-Z₄ ;
Z₄ représente l'atome d'hydrogène ;
s représente un entier de 2 à 4 ; s' représente un entier de 0 à 4 ;
et de préférence
l'hétérocycloalkyle que représente R'₄ est le cycle pipéridine ;
R_{Z3} et R'_{Z3} représentent l'atome d'hydrogène ;
Z_{3c} représente le radical thiényle, furyle ou phényle,
le radical phényle étant substitué par un ou plusieurs substituants identiques ou différents choisis parmi : halo et -(CH₂)_{p'}-V₃-Y₃ ;
V₃ représente -O-, -C(O)-, -C(O)-O-, -C(O)-NR'₃- ou une liaison covalente ;
R'₃ représente l'atome d'hydrogène ou un radical (C₁-C₆)alkyle ;
Y₃ représente l'atome d'hydrogène ; un radical (C₁-C₆)alkyle éventuellement substitué par un ou plusieurs radicaux halo identiques ou différents ;
Z_{3d} représente le radical (C₁-C₆)alkoxy-carbonyle ou hétérocycloalkyle , et de préférence l'hétérocycloalkyle est l'imidazolidine ; ou un sel pharmaceutiquement acceptable de ces derniers.

De manière préférentielle, l'invention concerne également des composés de formule I telle que définie ci-dessus, caractérisés en ce que X représente -CH- et A représente -C(O)-C(Rₐ)(R_{b})- avec Rₐ et R_{b} qui représentent le radical méthyle; et plus particulièrement
R₁ et R₂ représentent, indépendamment, un radical (C₁-C₈)alkyle ;
R₃ représente -Z_{3c}, -C(R_{Z3})(R'_{Z3})-Z_{3c}, -C(R_{Z3})(R'_{Z3})-Z_{3d} ou -C(R_{Z3})(R'_{Z3})-(CH₂)ₚ-Z_{3d} ;
R₄ représente un radical de formule-(CH₂)ₛ-R'₄ ;
R'₄ représente un hétérocycloalkyle contenant au moins un atome d'azote et éventuellement substitué par (C₁-C₆)alkyle ; ou un radical de formule -NW₄W'₄ ;
W₄ représente l'atome d'hydrogène ou (C₁-C₈)alkyle ;
W'₄ représente un radical de formule -(CH₂)_{s'}-Z₄ ;
Z₄ représente l'atome d'hydrogène, le radical phényle ou un hétéroaryle ;
s représente un entier de 2 à 4 ; s' représente un entier de 0 à 4 ;
et de préférence
R_{Z3} et R'_{Z3} représentent, indépendamment, l'atome d'hydrogène ;
Z_{3c} représente un radical thiényle éventuellement substitué par (C₁-C₆)alkoxy-carbonyle ; ou phényle substitué par un ou plusieurs substituants identiques ou différents choisis parmi : halo, nitro ou -(CH₂)_{p'}-V₃-Y₃;
V₃ représente -O-, -C(O)-, -C(O)-O-, -C(O)-NR'₃- ou une liaison covalente;
Y₃ représente l'atome d'hydrogène ; un radical (C₁-C₆)alkyle éventuellement substitué par un ou plusieurs radicaux halo identiques ou différents ;
R'₃ représente l'atome d'hydrogène ;
Z_{3d} représente un radical (C₁-C₆)alkoxy-carbonyle ;
l'hétérocycloalkyle que représente R'₄ est la pipéridine ;
l'hétéroaryle que représente Z₄ est la pyridine ; ou un sel pharmaceutiquement acceptable de ces derniers.

De manière préférentielle, l'invention concerne également des composés de formule I telle que définie ci-dessus, caractérisés en ce que X représente -CH- et A représente -C(O)-,
et plus particulièrement R₃ représente -C(O)-Z'₃
R₁ et R₂ représentent, indépendamment, un radical (C₁-C₈)alkyle ;
Z'₃ représente un radical phényle éventuellement substitué par un ou plusieurs substituants identiques ou différents choisis parmi : halo, nitro et -(CH₂)_{p"}-V'₃-Y'₃
V'₃ représente -O-, -C(O)-O- ou une liaison covalente ;
Y'₃ représente l'atome d'hydrogène ou un radical (C₁-C₆)alkyle ;
p" représente l'entier 0 ;
R₄ représente un radical de formule-(CH₂)ₛ-R'₄ et R'₄ qui représente un radical de formule -NW₄W'₄
W₄ représente l'atome d'hydrogène ou (C₁-C₈)alkyle ;
W'₄ représente un radical de formule -(CH₂)_{s'}-Z₄ et Z₄ qui représente l'atome d'hydrogène ;
s représente un entier de 2 à 4 ; s' représente un entier de 0 à 4 ;
ou un sel phannaceutiquement acceptable de ces derniers.

De manière préférentielle, l'invention concerne également des composés de formule I telle que définie ci-dessus, caractérisés en ce que X représente -CH-, A représente -C(O)-, et
R₁ représente un atome d'hydrogène, un radical (C₁-C₈)alkyle éventuellement substitué par hydroxy, (C₂-C₆)alkenyle ou un radical de formule -(CH₂)ₙ-X₁ ;
R₂ représente un radical (C₁-C₈)alkyle éventuellement substitué par hydroxy, (C₂-C₆)alkenyle ou un radical de formule -(CH₂)ₙ-X₁ ;
chaque X₁ représente indépendamment (C₁-C₆)alkoxy, (C₃-C₇)cycloalkyle , aryle ou hétéroaryle,
le radical aryle étant éventuellement substitué par un ou plusieurs substituants identiques ou différents choisis parmi : -(CH₂)_{n'}-V₁-Y₁, halo ;
V₁ représente -O- ou une liaison covalente ;
Y₁ représente un radical (C₁-C₆)alkyle éventuellement substitué par un ou plusieurs radicaux halo identiques ou différents ; ou aryle ;
ou bien R₁ et R₂ forment ensemble, avec l'atome d'azote auquel ils sont rattachés, un hétérocycloalkyle éventuellement substitué par un ou plusieurs substituants identiques ou différents choisis parmi : hydroxy, (C₁-C₆)alkyle éventuellement substitué par hydroxy, (C₁-C₆)alkoxy-carbonyle, hétérocycloalkyle et -C(O)NV₁'Y₁' avec V₁' et Y₁' représentant indépendamment l'atome d'hydrogène ou un (C₁-C₆)alkyle,
ou bien R₁ et R₂ forment ensemble un radical de formule : 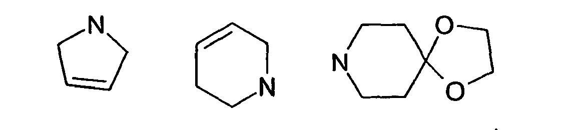 R₃ représente -Z₃, -C(R_{Z3})(R'_{Z3})-Z₃ ou -C(R_{Z3})(R'_{Z3})-(CH₂)ₚ Z₃ ;
R₄ représente un radical de formule-(CH₂)ₛ-R'₄ ;
R'₄ représente un hétérocycloalkyle contenant au moins un atome d'azote et éventuellement substitué par (C₁-C₆)alkyle ou aralkyle ; un hétéroaryle contenant au moins un atome d'azote et éventuellement substitué par (C₁-C₆)alkyle; ou un radical de formule -NW₄W'₄
W₄ représente l'atome d'hydrogène ou (C₁-C₈)alkyle;
W'₄ représente un radical de formule -(CH₂)_{s'}-Z₄ ;
Z₄ représente l'atome d'hydrogène, (C₃-C₇)cycloalkyle ou aryle;
s représente un entier de 0 à 5 ; s' représente un entier de 0 à 4 ;
et plus particulièrement caractérisés en ce qu'ils comportent au moins une des caractéristiques suivantes :
■ le radical (C₃-C₇)cycloalkyle que représent X₁ est le cyclopropyle ;
■ le radical aryle que représente X₁ le radical phényle ;
■ le radical hétéroaryle que représente X₁ est la pyridine ;
■ l'hétérocycloalkyle que forment ensemble R₁ et R₂ avec l'atome d'azote auquel ils sont rattachés, est choisi parmi : pyrrolidine, pipéridine, azépane, azacyclooctane, morpholine, pipérazine et décahydroisoquinoline ;
■ le radical hétérocycloalkyle que représente R'₄ , éventuellement substitué par C₁-C₆)alkyle ou benzyle, est choisi parmi : pyrrolidinyle, pipéridinyle, morpholinyle ou pipérazinyle ;
■ le radical hétéroaryle que représente R'₄ est le radical imidazolyle ;
■ le cycloalkyle que représente Z₄ est choisi parmi : cyclopropyle, cyclobutyle, cyclopentyle, cyclohexyle ou cycloheptyle ;
■ l'aryle que représente Z₄ est le phényle ; ou un sel pharmaceutiquement acceptable de ce dernier.

De manière très préférentielle, l'invention concerne des composés de formule 1. telle que définie ci-dessus, caractérisés en ce que R₄ représente un radical de formule-(CH₂)ₛ-R'₄ avec R'₄ qui représente le radical pyrrolidinyle ou pipéridinyle ; ou un radical de formule -NW₄W'₄
W₄ qui représente l'atome d'hydrogène ou (C₁-C₈)alkyle ;
W'₄ représente un radical de formule -(CH₂)_{s'}-Z₄ avec Z₄ qui représente l'atome d'hydrogène ;
s représente un entier de 2 à 4 ; s' représente un entier de 0 à 4 ; ou un sel pharmaceutiquement acceptable de ces derniers.

De manière très préférentielle également, l'invention concerne des composés de formule I telle que définie ci-dessus, caractérisé en ce que R₁ et R₂ représentent, indépendamment, un radical (C₁-C₈)alkyle ; ou un sel pharmaceutiquement acceptable de ces derniers.

De manière préférentielle, l'invention concerne également des composés de formule I telle que définie ci-dessus, caractérisés en ce que X représente -CH-, A représente -C(O)-, et
R₃ représente -Z₃ et Z₃ représente Z_{3c}, Z_{3d} ou Z₃ₑ ;
Z_{3d} représente un radical (C₃-C₇)cycloalkyle ou hétérocycloalkyle ;
et plus particulièrement
Z_{3c} représente un radical hétéroaryle choisi parmi thiényle, furyle, indolyle, dihydroindolyle, pyridyle, benzothiényle et benzofuryle ; ou un radical aryle choisi parmi phényle, napthyle et fluorènyle ;
le radical hétéroaryle étant éventuellement substitué par un ou plusieurs substituants identiques ou différents choisis parmi : (C₁-C₆)alkyl-carbonyle et (C₁-C₆)alkoxy-carbonyle ;
le radical aryle étant éventuellement substitué par un ou plusieurs substituants identiques ou différents choisis parmi : halo, cyano, nitro, azido,(C₁-C₆)alkoxy-carbonyl-(C₁-C₆)alkenyle, oxy, -SO₂-NR₃₁R₃₂, hétérocycloalkyle, hétéroaryle, ou -(CH₂)_{p'}-V₃-Y₃;
R₃₁ et R₃₂ forment ensemble, avec l'atome d'azote sur lequel ils sont rattachés, le cycle pipéridine ;
V₃ représente -O-, -S-, -C(O)-, -C(O)-O-, -SO₂- -SO₂NH-, -NR'₃-, -NR'₃-C(O)-, -C(O)-NR'₃-, -NH-C(O)-NR'₃- ou une liaison covalente ;
Y₃ représente l'atome d'hydrogène; un radical (C₁-C₆)alkyle éventuellement substitué par un ou plusieurs radicaux halo identiques ou différents ; phényle ; ou benzyle ;
R'₃ représente l'atome d'hydrogène, un radical (C₁-C₆)alkyle ou (C₁-C₆)alkoxy ;
Z_{3d} représente le radical cyclopropyle, cyclohexyle ou pipéridinyle, chacun pouvant être substitué par un radical (C₁-C₆)alkoxy-carbonyle ; ou un sel pharmaceutiquement acceptable de ces derniers.

De manière préférentielle, l'invention concerne également des composés de formule I telle que définie ci-dessus, caractérisés en ce que X représente -CH-, A représente -C(O)-, et
R₃ représente -Z₃ et _{Z3} représente Z_{3c}, Z_{3d} ou Z₃ₑ;
Z_{3d} représente un radical (C₃-C₇)cycloalkyle ou hétérocycloalkyle ;
et plus particulièrement
Z_{3c} représente un radical hétéroaryle choisi parmi thiényle, indolyle et benzothiényle ; ou un radical aryle choisi parmi phényle et napthyle ;
le radical hétéroaryle étant éventuellement substitué par un ou plusieurs radicaux oxy;
le radical aryle étant éventuellement substitué par un ou plusieurs substituants identiques ou différents choisis parmi : halo, nitro, hétéroaryle ou -(CH₂)_{p'}-V₃-Y₃;
V₃ représente -O-, -S-, -C(O)-, -C(O)-O-, -SO₂-, -SO₂NH-, -NR'₃-C(O)-, -C(O)-NR'₃-, -NH-C(O)-NR'₃- ou une liaison covalente ;
Y₃ représente l'atome d'hydrogène ; un radical (C₁-C₆)alkyle éventuellement substitué par un ou plusieurs radicaux halo identiques ou différents ; un radical phényle ; ou un radical benzyle ;
R'₃ représente l'atome d'hydrogène, un radical (C₁-C₆)alkyle ou (C₁-C₆)alkoxy ;
Z_{3d} représente le radical cyclopropyle ou pipéridinyle, chacun éventuellement substitué par (C₁-C₆)alkoxy-carbonyle ;
et de préférence.
Z₃ représente Z_{3c} ou Z₃ₑ;
Z_{3c} représente un phényle étant éventuellement substitué par un ou plusieurs substituants identiques ou différents choisis parmi nitro et -(CH₂)_{p'}-V₃-Y₃;
V₃ représente -O-, -S-, -C(O)-, -C(O)-O-, -SO₂-, -SO₂NH-, -NR'₃-C(O)-, -C(O)-NR'₃- ou une liaison covalente ;
Y₃ représente l'atome d'hydrogène ; un radical (C₁-C₆)alkyle ; un radical phényle ; ou un radical benzyle ;
R'₃ représente l'atome d'hydrogène ;
Z₃ₑ représente 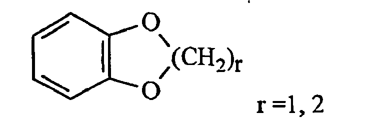 ou un sel pharmaceutiquement acceptable de ces derniers.

De manière préférentielle, l'invention concerne également des composés de formule I telle que définie ci-dessus, caractérisés en ce que X représente -CH-, A représente -C(O)-, R₃ représente -C(R_{Z3})(R'_{Z3})-Z₃ et Z₃ représente Z_{3b}, Z_{3c}, Z_{3d} ou Z₃ₑ; ou un sel pharmaceutiquement acceptable de ces derniers.

De manière très préférentielle, l'invention concerne également des composés de formule I telle que définie ci-dessus, caractérisés en ce que X représente -CH-, A représente -C(O)-, et
R₃ représente -C(R_{Z3})(R'_{Z3})-Z₃ et Z₃ représente Z_{3b} ou Z_{3c} ;
R_{Z3} et R'_{Z3} représentent l'atome d'hydrogène;
et plus particulièrement
Z_{3b} représente un radical (C₁-C₆)alkoxy ;
Z_{3c} représente un radical hétéroaryle choisi parmi thiényle, furyle, pyridyle, benzothiényle et dihydrobenzofuryle ; ou un radical aryle choisi parmi phényle et napthyle,
le radical aryle étant éventuellement substitué par un ou plusieurs substituants identiques ou différents choisis parmi : halo ou -(CH₂)_{p'}-V₃-Y₃ ;
V₃ représente -O-, -S-, -C(O)-, -C(O)-O-, -SO₂-, -SO₂NH-, -NR'₃-C(O)-, -C(O)-NR'₃-,
Y₃ représente l'atome d'hydrogène ; un radical (C₁-C₆)alkyle éventuellement substitué par un ou plusieurs radicaux halo identiques ou différents;
R'₃ représente l'atome d'hydrogène;
ou un sel pharmaceutiquement acceptable de ces derniers.

De manière très préférentielle, l'invention concerne des composés de formule I telle que définie ci-dessus, caractérisés en ce que X représente -CH-, A représente -C(O)-, et
R₃ représente -C(R_{Z3})(R'_{Z3})-Z₃ et Z₃ représente Z_{3b} ou Z_{3c} ;
R_{Z3} et R'_{Z3} représentent l'atome d'hydrogène ;
et plus particulièrement
Z_{3b} représente un radical (C₁-C₆)alkoxy ;
Z_{3c} représente un radical hétéroaryle choisi parmi thiényle, furyle, dihydrobenzofuryle; ou un radical phényle ;
le radical phényle étant éventuellement substitué par un ou plusieurs substituants identiques ou différents choisis parmi : nitro ou -(CH₂)_{p'}-V₃-Y₃ ;
V₃ représente -O-, -S-, -C(O)-, -C(O)-O-, -SO₂-, -SO₂NH-, -C(O)-NR'₃-,
Y₃ représente l'atome d'hydrogène ; ou un radical (C₁-C₆)alkyle éventuellement substitué par un ou plusieurs radicaux halo identiques ou différents ;
R'₃ représente l'atome d'hydrogène ;
et de préférence
Z₃ représente Z_{3c};
Z_{3c} représente un radical furyle ou phényle,
le radical phényle étant éventuellement substitué par un ou plusieurs substituants identiques ou différents de formule -(CH₂)_{p'}-V₃-Y₃;
V₃ représente -O-, -S-, -C(O)-, -C(O)-O-, -SO₂-, -SO₂NH- -C(O)-NR'₃-,
Y₃ représente l'atome d'hydrogène ; ou un radical (C₁-C₆)alkyle éventuellement substitué par un ou plusieurs radicaux halo identiques ou différents;
R'₃ représente l'atome d'hydrogène;
ou un sel pharmaceutiquement acceptable de ces derniers.

De manière très préférentielle, l'invention concerne également des composés de formule I telle que définie ci-dessus, caractérisés en ce que X représente -CH-, A représente -C(O)-, et
R₃ représente -C(R_{Z3})(R'_{Z3})-Z₃ et Z₃ représente Z_{3d} ou Z₃ₑ ;
R_{Z3} et R'_{Z3} représentent l'atome d'hydrogène ou (C₁-C₆)alkyle;
Z_{3d} représente un radical (C₁-C₆)alkoxy-carbonyle, (C₃-C₇)cycloalkyle ou hétérocycloalkyle ;
Z₃ₑ représente 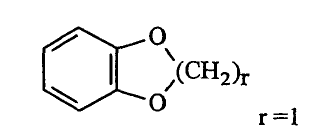 et plus particulièrement
Z_{3d} représente un radical (C₁-C₆)alkoxy-carbonyle, cyclohexyle ou un tétrahydrofuranyle ; ou un sel pharmaceutiquement acceptable de ces derniers.

De manière très préférentielle, l'invention concerne également des composés de formule 1 telle que définie ci-dessus, caractérisés en ce que X représente -CH-, A représente -C(O)-, et
R₃ représente -C(R_{Z3})(R'_{Z3})-Z₃ et Z₃ représente Z_{3d} ou Z₃ₑ ;
Z_{3d} représente un radical (C₁-C₆)alkoxy-carbonyle ;
Z₃ₑ représente 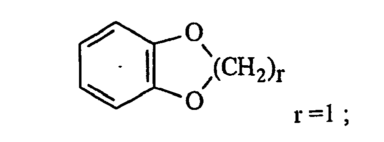 et de préférence Z₃ représente Z₃ₑ 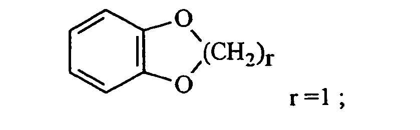 ou un sel pharmaceutiquement acceptable de ces derniers.

De manière préférentielle, l'invention concerne également des composés de formule I telle que définie ci-dessus, caractérisés en ce que X représente -CH-, A représente -C(O)-, R₃ représente -C(R_{Z3})(R'_{Z3})-(CH₂)ₚ-Z₃ et Z₃ représente Z_{3b}, Z_{3c} ou Z_{3d} ; ou un sel pharmaceutiquement acceptable de ces derniers.

De manière très préférentielle, l'invention concerne des composés de formule I telle que définie ci-dessus, caractérisés en ce que X représente -CH-, A représente -C(O)-, et
R₃ représente -C(R_{Z3})(R'_{Z3})-(CH₂)ₚ-Z₃ et Z₃ représente Z_{3b} ;
et plus particulièrement
R_{Z3} et R'_{Z3} représentent, indépendamment, l'atome d'hydrogène ou un radical (C,-C₆)alkyle ;
Z_{3b} représente un radical (C₁-C₆)alkoxy, (C₁-C₆)alkylthio ou di((C₁-C₆)alkyl)amino ; ou un sel pharmaceutiquement acceptable de ces derniers.

De manière très préférentielle, l'invention concerne également des composés de formule I telle que définie ci-dessus, caractérisés en ce que X représente -CH-, A représente -C(O)-, et
R₃ représente -C(R_{Z3})(R'_{Z3})-(CH₂)ₚ-Z₃ et Z₃ représente Z_{3b} ;
et plus particulièrement
R_{Z3} et R'_{Z3} représentent, indépendamment, l'atome d'hydrogène ou un radical (C₁-C₆)alkyle ;
Z_{3b} représente un radical (C₁-C₆)alkoxy ou (C₁-C₆)alkylthio; ou un sel pharmaceutiquement acceptable de ces derniers.

De manière très préférentielle, l'invention concerne également des composés de formule I telle que définie ci-dessus, caractérisés en ce que X représente -CH-, A représente -C(O)-, et
R₃ représente -C(R_{Z3})(R'_{Z3})-(CH₂)ₚZ₃ et Z₃ représente Z_{3c} ou Z_{3d} ;
et plus particulièrement
R_{Z3} et R'_{Z3} représentent, indépendamment, l'atome d'hydrogène ou un radical (C₁-C₆)alkyle ;
Z_{3c} représente un radical indolyle ou phényle;
le radical phényle étant éventuellement substitué par un ou plusieurs substituants identiques ou différents choisi parmi : halo et -(CH₂)_{p'}-V₃-Y₃;
V₃ représente -SO₂NH- ,
Y₃ représente l'atome d'hydrogène ; ou un radical (C₁-C₆)alkyle;
Z_{3d} représente un radical (C₁-C₆)alkoxy-carbonyle, amino-carbonyle, (C₁-C₆)alkyl-amino-carbonyle, (C₁-C₆)alkyl-C(O)-NH-, ou hétérocycloalkyle éventuellement substitué par oxy, et de préférence pipéridinyle, morpholinyle, pyrrolidine ou imidazolidinyle ; ou un sel pharmaceutiquement acceptable de ces derniers.

De manière très préférentielle, l'invention concerne également des composés de formule I telle que définie ci-dessus, caractérisés en ce que X représente -CH-, A représente -C(O)-, et
R₃ représente -C(R_{Z3})(R'_{Z3})-(CH₂)ₚ-Z₃ et Z₃ représente Z_{3c} ou Z_{3d} ;
et plus particulièrement
Z₃ représente Z_{3d} ;
R_{Z3} et R'_{Z3} représentent, indépendamment, l'atome d'hydrogène ou un radical (C₁-C₆)alkyle ;
Z_{3d} représente un radical (C₁-C₆)alkoxy-carbonyle, amino-carbonyle, (C₁-C₆)alkylamino-carbonyle, (C₁-C₆)alkyl-C(O)-NH- ou hétérocycloalkyle, et de préférence pyrrolidine ou imidazolidine, éventuellement substitué par oxy ; ou un sel pharmaceutiquement acceptable de ces derniers.

Dans la présente demande, le symbole -> * correspond au point de rattachement du radical. Lorsque le site de rattachement n'est pas précisé sur le radical, cela signifie que le rattachement s'effectue sur un des sites disponibles de ce radical pour un tel rattachement.

Suivant les définitions des groupes variables A, X, R₁, R₂, R₃ et R₄, les composés selon l'invention peuvent être préparés en phase liquide selon les différentes procédures A à G décrites ci-dessous.

### A. Préparation selon le schéma réactionnel A :

Les composés de formule (I) selon l'invention dans laquelle A représente -C(O)-, peuvent être préparés selon le schéma A suivant : 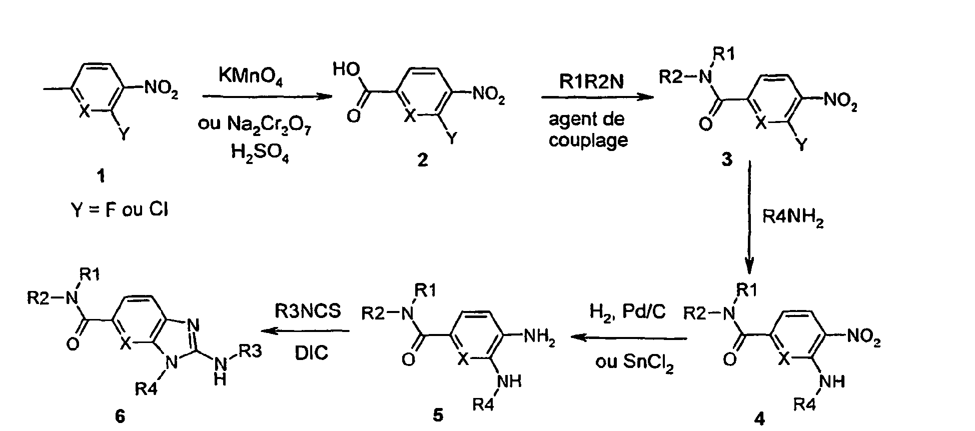 Comme décrit dans le schéma A, le dérivé méthylé (1) (pour X = C composé commercial ; pour X = N composé préparé selon la procédure de Baumgarten et al, J. Am. Chem. Soc, 1952, 74, 3828-3831, à partir du 6-méthyl-3-nitro-pyridin2-amine) peut être oxydé en acide carboxylique (2) par une solution aqueuse de permanganate de potassium à une température de 100° C pendant 3 à 6 heures (selon procédure de Schmelkes et al, J. Am, Chem. Soc, 1944, 1631), ou par une solution aqueuse de dichromate de sodium en présence d'acide sulfurique à une température de 20-90° C pendant 1 à 3 heures (selon procédure de Howes et al, European J. Med. Chem, 1999, 34, 225-234). L'acide carboxylique (2) peut être couplé avec une amine primaire ou secondaire en présence d'un agent de couplage tel que le diisopropylcarbodiimide (DIC), le dicyclohexylcarbodiimide (DCC), le chlorhydrate de 1-3-dimethylaminopropyl)-3-éthylcarbodiimide (EDC) ou le carbonyldiimidazole (CDI) avec ou sans du 1-hydroxybenzotriazole (HOBt) dans un solvant organique inerte tel que du chlorure de méthylène, tétrahydrofuranne ou diméthylformamide à température ambiante pendant 3 à 24 heures pour conduire à l'amide correspondante (3). Le traitement du dérivé fluoré ou chloré (3) par une amine primaire en présence d'une base inorganique telle que du carbonate de césium ou de potassium dans un solvant organique inerte tel que le diméthylformamide ou l'acétonitrile à une température de 20-100° C pendant 2 à 48 heures conduit au dérivé (4). La fonction nitro du composé (4) est réduite par traitement avec du chlorure d'étain dihydrate dans un solvant inerte tel que l'acétate d'éthyle ou le diméthylformamide à une température de 60-80° C pendant 3 à 15 heures, ou par hydrogénation catalytique en présence de palladium sur charbon 10 % dans un solvant inerte tel que le méthanol, éthanol, acétate d'éthyle ou un mélange de ces solvants, à une température de 18-25° C, pendant 2 à 8 heures pour conduire à la dianiline (5). Le dérivé (5) est ensuite traité par un isothiocyanate en présence d'un agent de couplage supporté sur une résine ou non tel que le diisopropylcarbodiimide ou dicyclohexylcarbodiimide ou la résine *N*-méthylcyclohexylcarbodiimide *N*-méthyl polystyrène dans un solvant inerte tel que le tétrahydrofuranne, chlorure de méthylène, ou chloroforme à une température de 20-70° C pendant 2 à 72 heures pour conduire au dérivé (6). Alternativement, le dérivé (5) peut être traité par un isothiocyanate dans un solvant inerte tel que le tétrahydrofuranne, chlorure de méthylène, chloroforme ou éthanol à une température de 20-80° C pendant 1-16 heures puis la thiourée résultante peut être traitée par de l'iodure de méthyle ou de l'oxyde de mercure (II) jaune en présence d'une quantité catalytique de soufre dans un solvant polaire tel que le méthanol ou l'éthanol pendant 2 à 24 heures à une température de 20-80° C pour conduire à (6). Le composé (6) peut être isolé soit par chromatographie éclair sur gel de silice, soit par addition au mélange réactionnel d'un réactif nucléophile supporté sur un polymère comme par exemple une résine aminométhylpolystyrène et/ou d'un réactif électrophile supporté sur un polymère comme par exemple la résine méthylisothiocyanate-polystyrène, suivie d'une filtration et de l'évaporation du filtrat.

### Exemple A1 : dichlorhydrate de 4-[(1-(3-aminopropyl)-6-{[bis(3-méthylbutyl) amino]carbonyl}-1H-benzimidazol-2-yl)amino]benzoate de méthyle

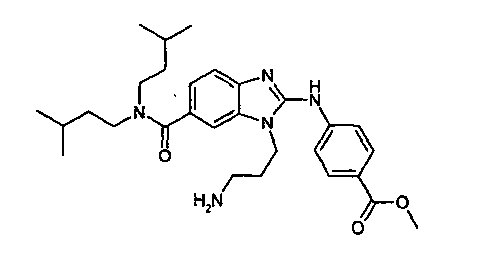

### Etape 1 : acide 3-fluoro-4-nitrobenzoique

Un mélange de 3-fluoro-4-nitrotoluène (10 g, 1 eq) et de permanganate de potassium (25,5 g, 2,5 eq) dans l'eau (1 L) est chauffé au reflux pendant 6 heures puis refroidi à température ambiante. Le mélange est filtré sur célite et la phase aqueuse est lavée deux fois à l'éther diéthylique (2 x 300 ml). La phase aqueuse est acidifiée, à 0° C, avec une solution d'acide chlorhydrique concentrée puis concentrée sous pression réduite à 40° C jusqu'à un volume d'environ 300 ml. Le précipité formé est filtré puis lavé à l'éther de pétrole et séché pour donner le composé attendu sous forme d'un solide blanc (6,9 g ; 58 % rendement).

RMN (¹H, 400 MHz, DMSO-*d₆*) : δ 7,93 (m, 2H), 8,25 (m, 1H), 13,95 (m, 1H).

### Etape 2 : 3-fluoro-N,N-bis(3-méthylbutyl)-4-nitrobenzamide

A l'acide 3-fluoro-4-nitrobenzoïque (3,8 g, 1 eq) en solution dans le THF anhydre (30 ml) sont successivement additionnés le chlorhydrate de 1-(3-diméthylaminopropyl)-3-éthylcarbodiimide (EDC) (4,4 g, 1,1 eq) en solution dans le chloroforme (25 ml) et le 1-hydroxybenzotriazole (HOBt) (3,05 g, 1,1 eq) en solution dans le THF (40 ml). Le mélange est agité 1 heure à une température d'environ 20° C puis la diisoamylamine (3,6 g, 1,1 eq) en solution dans le THF (30 ml) est additionnée. Après 16 heures d'agitation à une température d'environ 20° C, le mélange réactionnel est concentré sous pression réduite à 40° C. Le résidu est repris par du dichlorométhane (200 ml) et de l'eau (70 ml). Après décantation et extractions, les phases organiques combinées sont lavées avec de la saumure, séchées sur Na₂SO₄ puis concentrées sous pression réduite à 40° C. La purification du composé par chromatographie éclair sur gel de silice (éluant : heptane / acétate d'éthyle 9 :1) donne le composé attendu sous forme d'une huile jaune (4,3 g ; 65 % rendement).
SM/CL : MM calculée = 324,4 ; m/z = 325,2 (MH+)
RMN (¹H, 400 MHz, DMSO-*d₆*) : δ 0,69 (m, 6H), 0,93 (m, 6H), 1,35-1,60 (m, 6H), 3,09 (m, 2H), 3,41 (m, 2H), 7,38 (d, 1H), 7,63 (d, 1H), 8,21 (t, 1H).

### Etape 3 : tert-butyl 3-[(5-{[bis(3-méthylbutyl)amino]carbonyl}-2-nitrophényl)amino] propylcarbamate

Un mélange de 3-fluoro-N,N bis(3-méthylbutyl)-4-nitrobenzamide (1,6 g, 1 eq), de N Boc-1,3-diaminopropane (0,9 g, 1,2 eq) et de carbonate de potassium (1,35 g, 2 eq) dans l'acétonitrile (80 ml) est chauffé au reflux pendant 5 heures puis concentré sous pression réduite à 40° C. Le résidu est repris par du dichlorométhane (100 ml) et de l'eau (40 ml). Après décantation et extractions, les phases organiques combinées sont lavées avec de la saumure, séchées sur Na₂SO₄ puis concentrées sous pression réduite à 40° C. La purification du résidu par chromatographie éclair sur gel de silice (éluant : heptane/acétate d'éthyle 8:2 à 6:4) donne le composé attendu sous forme d'une huile jaune (2,2 g ; 96 % rendement).
SM/CL : MM calculée = 478,6; m/z = 479,2 (MH+)
RMN (¹H, 400 MHz, DMSO-*d₆*) : δ 0,68 (m, 6H), 0,92 (m, 6H), 1,36 (s, 9H), 1,31-1,69 (m, 8H), 3,0 (m, 2H), 3,09 (m, 2H), 3,38 (m, 4H), 6,53 (d, 1H), 6,88 (m, 2H), 8,10 (d, 1H), 8,26 (m,1H).

### Etape 4 : tert-butyl 3-[(2-amino-5-{[bis(3-méthylbutyl)amino]carbonyl}phényl)amino] propylcarbamate

Dans un autoclave sont additionnés le *tert*-butyl 3-[(5-{[bis (3-méthylbutyl)amino]carbonyl}-2-nitrophényl)amino]propylcarbamate (1,65 g) en solution dans un mélange d'acétate d'éthyle/ éthanol 2 :1 (130 ml), et le palladium sur charbon 10 % (165 mg). Après 3 heures d'agitation sous atmosphère d'hydrogène (3 bars) à une température d'environ 20° C, le catalyseur est éliminé par filtration sur célite et le filtrat est concentré sous pression réduite à 40° C pour donner le composé attendu sous forme d'une huile (1,35 g ; 89 % rendement).
SM/CL, : MM calculée = 448,6 ; m/z = 449,2 (MH+)
RMN (¹H, 400 MHz, DMSO-*d₆*) : δ 0,81 (m, 12H), 1,37 (s, 9H), 1,32-1,53 (m, 6H), 1,70 (m, 2H), 3,0 (m, 4H), 3,26 (m, 4H), 4,47 (m, 1H), 4,79 (s, 2H), 6,35-6,51 (m, 3H), 6,85 (m, 1H).

### Etape 5 : 4-[(6-{[bis(3-méthylbutyl)amino]carbonyl}-1-{3-[(tert-butoxycarbonyl) amino] propyl}-1H benzimidazol-2-yl)amino]benzoate de méthyle

A une solution de *tert*-butyl-3-[(2-amino-5-{[bis(3-méthylbutyl)amino]carbonyl} phényl)amino]propylcarbamate (500 mg, 1 eq) dans le tétrahydrofuranne (30 ml) sont successivement additionnés le 4-méthoxycarbonylphényl isothiocyanate (327 mg, 1,5 eq) et la résine *N*-méthylcyclohexylcarbodiimide-*N*-méthyl-polystyrène (acquise auprès de Novabiochem ; charge 1,9 mmol/g ; 1,75 g, 3 eq). Le mélange est chauffé à reflux pendant 17 heures puis refroidi à température ambiante et additionné de résine aminométhylpolystyrène (acquise auprès de Novabiochem, 2 eq). Après 4 heures d'agitation à température ambiante, le mélange est filtré sur fritté et le filtrat est concentré sous pression réduite à 40° C. La purification du résidu par chromatographie éclair sur gel de silice (éluant : heptane /acétate d'éthyle 1 : 1) donne le composé attendu sous forme d'un solide blanc (409 mg ; 60 % rendement).
SM/CL : MM calculée = 607,8; m/z = 608,1 (MH+)
RMN (¹H, 400 MHz, DMSO-*d₆*) : δ 0,65-0,90 (m, 12H), 1,36 (s, 9H), 1,31-1,44 (m, 6H), 1,81 (m, 2H), 3,0 (m, 2H), 3,26-3,39 (m, 4H), 3,82 (s, 3H), 4,29 (m, 2H), 6,95 (m, 1H), 7,04 (d, 1H), 7,36 (s, 1H), 7,44 (d, 1H), 7,94 (AB, 2H), 8,02 (AB, 2H), 9,34 (s, 1H).

### Etape 6 : dichlorhydrate de 4-[(1-(3-aminopropyl)-6-{[bis(3-méthylbutyl)amino] carbonyl}-1H-benzimidazol-2-yl)amino]benzoate de méthyle

A une solution de 4-[(6-{[bis(3-méthylbutyl)amino]carbonyl}-1-{3-[(*tert-*butoxycarbonyl)amino]propyl}-1*H*-benzimidazol-2-yl)amino]benzoate de méthyle (180 mg) dans l'acétate d'éthyle (2 ml) est additionnée une solution d'acide chlorhydrique dans le dioxane (4N, 2 ml). Après 1 heure d'agitation à une température voisine de 20° C, le mélange est concentré sous pression réduite à 40° C. Le solide obtenu est lavé à l'éther éthylique et séché (165 mg ; 96 % rendement).
SM/CL : MM calculée = 507,7 ; m/z = 508,3 (MH+)
RMN (¹H, 400 MHz, DMSO-*d₆*): δ 0,63-0,98 (m, 12H), 1,45 (m, 6H), 2,08 (m, 2H), 2,98 (m, 2H), 3,12-3,45 (m, 4H), 3,85 (s, 3H), 4,59 (m, 2H), 7,20 (d, 1H), 7,46 (d, 1H), 7,67 (s, 1H), 7,90 (m, 2H), 8,01-8,07 (m, 5H), 11,08 (m, 1H).

### Exemple A2: dichlorhydrate de 2-[(4-acétylphényl)amino]-N,N-bis(3-méthylbutyl)-1-(3-pipéridin-1-ylpropyl)-1H-benzimidazole-6-carboxamide

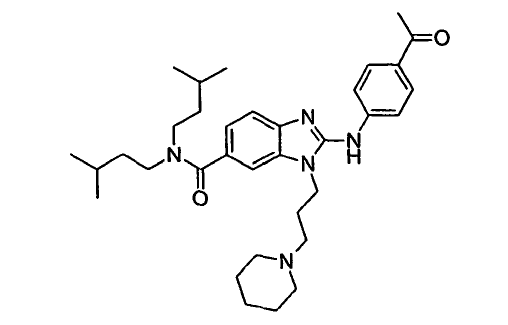

### Etape 1 : N,N bis(3-méthylbutyl)-4-nitro-3-[(3-pipéridin-1-ylpropyl)amino]benzamide

Un mélange de 3-fluoro-*N,N*-bis(3-méthylbutyl)-4-nitrobenzamide (430 mg, 1 eq, préparé selon l'exemple A1), de 3-pipéridino-propylamine (212 mg, 1,1 eq) et de carbonate de potassium (365 mg, 2 eq) dans l'acétonitrile (10 ml) est chauffé au reflux pendant 3 heures puis concentré sous pression réduite à 40° C. Le résidu est repris par du dichlorométhane (50 ml) et de l'eau (20 ml). Après décantation et extractions, les phases organiques combinées sont lavées avec de la saumure, séchées sur Na₂SO₄ puis concentrées sous pression réduite à 40°C. La purification du résidu par chromatographie éclair sur gel de silice (éluant : acétate d'éthyle 100 %) donne le composé attendu sous forme d'une huile jaune (460 mg ; 78 % rendement).
SM/CL : MM calculée = 446,6 ; m/z = 447,3 (MH+)
RMN (¹H, 400 MHz, DMSO-*d₆*) : δ 0,68 (d, 6H), 0,92 (d, 6H), 1,31-1,69 (m, 12H), 1,74 (m, 2H), 2,32 (m, 6H), 3,10 (m, 2H), 3,38 (m, 4H), 6,53 (d, 1H), 6,91 (m, 1H), 8,09 (d, 1 H), 8,44 (t,1H).

### Etape 2 : dichlorhydrate de 2-[(4-acétylphényl)amino]-N,N bis(3-méthylbutyl)-1-(3-pipéridine-1-ylpropyl)-1H benzimidazole-6-carboxamide

Dans un tube à hémolyse placé dans un autoclave sont additionnés le *N,N-*bis(3-méthylbutyl)-4-nitro-3-[(3-pipéridin-1-yl-propyl)amino]benzamide (44 mg) en solution dans un mélange d'acétate d'éthyle/ éthanol 2 :1 (1,5 ml), et le palladium sur charbon 10 % (5 mg). Après 3 heures d'agitation sous atmosphère d'hydrogène (3 bars) à une température d'environ 20° C, le catalyseur est éliminé par filtration sur célite et le filtrat est concentré sous pression réduite à 40° C. A l'aniline ainsi obtenue, en solution dans le tétrahydrofuranne (2 ml) sont successivement additionnés le 4-acétylphényl isothiocyanate (27 mg, 1,5 eq) et la résine *N*-méthylcyclohexylcarbodiimide-*N*-méthyl-polystyrène (acquise auprès de Novabiochem ; charge 1,9 mmol/g ; 158 mg, 3 eq). Le mélange est chauffé à reflux pendant 18 heures puis refroidi à température ambiante et additionné de résine aminométhylpolystyrène (acquise auprès de Novabiochem, 2 eq). Après 4 heures d'agitation à température ambiante, le mélange est filtré sur fritté et le filtrat est concentré sous pression réduite à 40° C. La purification du résidu par chromatographie éclair sur gel de silice (éluant : dichlorométhane 100 % à dichlorométhane/méthanol 9 : 1) donne le composé attendu sous forme de base. Le sel de chlorhydrate correspondant est formé par addition d'une solution d'acide chlorhydrique IN dans l'éther. Le précipité obtenu est filtré et séché pour donner le composé dichlorhydrate attendu.
SM/CL : MM calculée = 559,8 ; m/z = 560,3 (MH+)
RMN (¹H, 400 MHz, DMSO-*d₆*) : δ 0,68 (m, 6H), 0,94 (m, 6H), 1,31-1,56 (m, 6H), 1,57-1,90 (m, 6H), 2,28 (m, 2H), 2,60 (s, 3H), 2,86 (m, 2H), 3,21 (m, 4H), 3,40 (m, 4H), 4,62 (t, 2H), 7,24 (AB, 1H), 7,47 (AB, 1H), 7,76 (s, 1H), 7,81 (m, 2H), 8,07 (m, 2H), 10,40 (s, 1H), 11,64 (m, 1H).

### Exemple A3 : dichlorhydrate de 2-(cyclohexylamino)-1-[3-(diméthylamino)propyl]-N,N-bis(3-méthylbutyl)-1H-benzimidazole-6-carboxamide

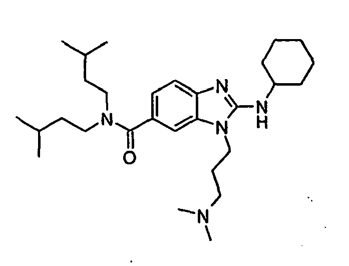

### Etape 1 : 3-{[3-(diméthylamino)propyl]amino}-N,N-bis(3-méthylbutyl)-4-nitrobenzamide

Un mélange de 3-fluoro-*N,N*-bis(3-méthylbutyl)-4-nitrobenzamide (2,5 g, 1 eq, préparé selon l'exemple A1), de 3-diéthylamino-propylamine (877 mg, 1,1 eq) et de carbonate de potassium (2,13 g, 2 eq) dans l'acétonitrile (80 ml) est chauffé au reflux pendant 5 heures puis concentré sous pression réduite à 40° C. Le résidu est repris par du dichlorométhane (130 ml) et de l'eau (50 ml). Après décantation et extractions, les phases organiques combinées sont lavées avec de la saumure, séchées sur Na₂SO₄ puis concentrées sous pression réduite à 40° C. La purification du résidu par chromatographie éclair sur gel de silice (éluant: dichlorométhane / méthanol 9 :1) donne le composé attendu sous forme d'une huile jaune (2,1g mg ; 68 % rendement).
SM/CL : MM calculée = 406,6 ; m/z = 407,3 (MH+)
RMN (¹H, 400 MHz, DMSO-*d₆*) : *δ* 0,68 (d, 6H), 0,92 (d, 6H), 1,31-1,51 (m, 5H), 1,59 (m, 1H), 1,74 (m, 2H), 2,14 (s, 6H), 2,31 (t, 2H), 3,11 (m, 2H), 3,39 (m, 4H), 6,53 (d, 1H), 6,90 (s, 1H), 8,09 (d, 1H), 8,57 (t,1H).

### Etape 2 : dichlorhydrate de 2-(cyclohexylamino)-1-[3-(diméthylamino)propyl]-N,N-bis(3-méthylbutyl)-1H-benzimidazole-6-carboxamide

Dans un tube à hémolyse placé dans un autoclave sont additionnés le 3- {[3-(diméthylamino)propyl]amino}-*N,N*-bis(3-méthylbutyl)-4-nitrobenzamide (81 mg) en solution dans un mélange d'acétate d'éthyle/ éthanol 2:1 (4 ml), et le palladium sur charbon 10 % (8 mg). Après 3 heures d'agitation sous atmosphère d'hydrogène (3 bars) à une température d'environ 20° C, le catalyseur est éliminé par filtration sur célite et le filtrat est concentré sous pression réduite à 40° C. A l'aniline ainsi obtenue, en solution dans le tétrahydrofuranne (2 ml) sont successivement additionnés le cyclohexyl isothiocyanate (58 mg; 2 eq) Le mélange est chauffé au reflux pendant 3 heures puis refroidi à température ambiante et concentré sous pression réduite. A la thiourée ainsi formée en solution dans l'éthanol (3 ml) sont successivement additionnés l'oxyde de mercure (II) jaune (87 mg, 2 eq) et le soufre (1,4 mg). Le mélange est chauffé 17 heures au reflux puis refroidi à température ambiante et filtré sur papier microfibre. Le filtrat est concentré sous pression réduite. La purification du résidu par chromatographie éclair sur gel de silice (éluant : dichlorométhane 100 % à dichlorométhane/méthanol 9 :1) donne le composé attendu sous forme de base. Le sel de chlorhydrate correspondant est formé par addition d'une solution d'acide chlorhydrique 1N dans l'éther. Le précipité obtenu est filtré et séché pour donner le composé dichlorhydrate attendu (87 mg, 78 % rendement).
SM/CL : MM calculée = 483,7 ; m/z = 484,4 (MH+)
RMN (¹H, 400 MHz, DMSO-*d₆*): *δ* 0,58-1,03 (m, 12H), 1,18 (m, 1H), 1,30-1,71 (m, 11H), 1,80 (m, 2H), 2,01 (m, 4H), 2,73 (s, 6H), 3,14 (m, 4H), 3,25 (m, 2H), 3,71 (m, 1H), 4,32 (m, 2H), 7,16 (m, 1H), 7,39 (m, 1H), 7,54 (m, 1H), 8,42 (m, 1H), 10,40 (m, 1H), 13,41 (m, 1H).

### Préparation d'isothiocyanates non commerciaux :

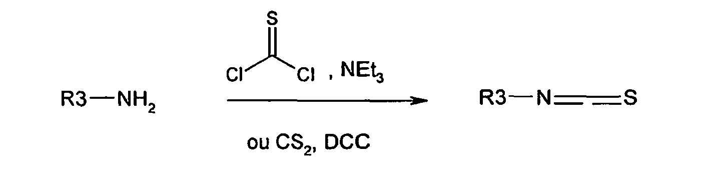

Une amine primaire peut être convertie en isothiocyanate, par traitement avec du thiophosgène en présence d'une base tertiaire telle que la triéthylamine, dans un solvant aprotique tel que le dichlorométhane ou le tétrahydrofuranne, à une température de 0-20° C pendant 0,3 à 2 heures, ou alternativement par traitement avec du disulfure de carbone et du cyclohexylcarbodiimide supporté sur une résine ou non dans un solvant aprotique tel que le dichlorométhane ou le tétrahydrofuranne, à une température de 0-70° C pendant 0,3 à 15 heures.

### N-(4-isothiocyanatophényl)acétamide

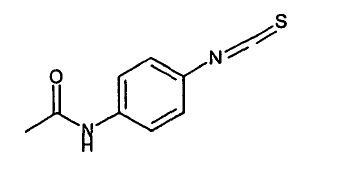

A une solution refroidie à 0° C, de *N*-(4-aminophényl)acétamide (1 g, 1 eq) et de triéthylamine (2,8 ml, 3 eq) dans le tétrahydrofuranne (130 ml) est additionné goutte à goutte le thiophosgène (0,56 ml, 1,1 eq). Le mélange est agité 30 min à 0° C puis le bain froid est retiré et l'agitation est poursuivie 30 min supplémentaires. Le mélange est additionné d'eau (70 ml) et d'éther diéthylique (150 ml). Après décantation et extractions, les phases organiques sont réunies, lavées avec de la saumure, séchées sur Na₂SO₄ puis concentrées sous pression réduite à 40° C. Le solide obtenu est recristallisé dans un mélange dichlorométhane / éther de pétrole (0,95 g ; 75 % rendement).
RMN (¹H, 400 MHz, DMSO-*d₆*) : δ 2,04 (s, 3H), 7,35 (AB, 2H), 7,63 (AB, 2H), 10,14 (s, 1H).

Les isothiocyanates suivants ont été préparés selon la même procédure que celle décrite pour le *N*-(4-isothiocyanatophényl)acétamide :

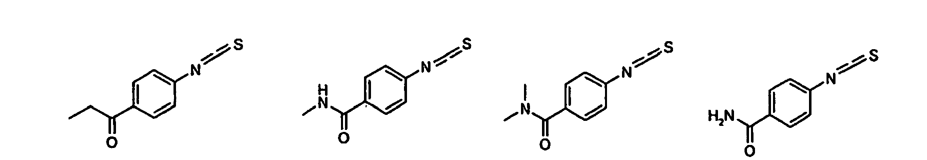

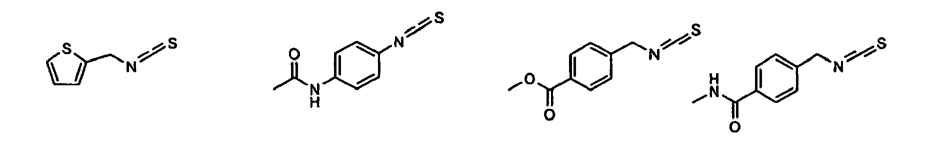

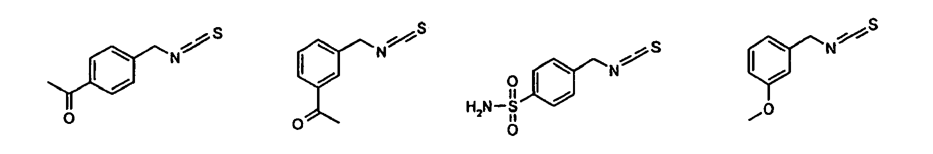

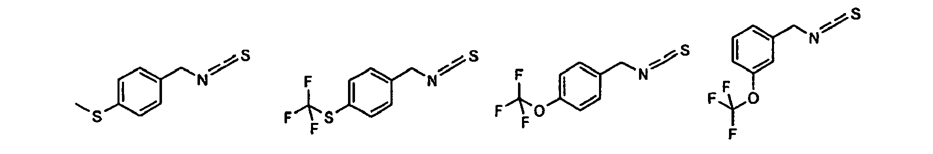

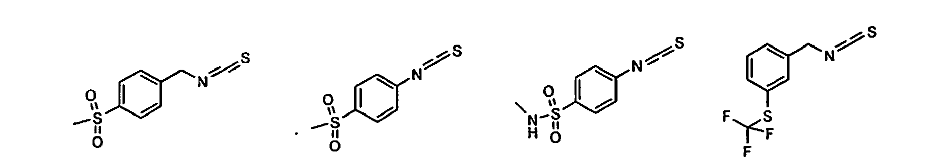

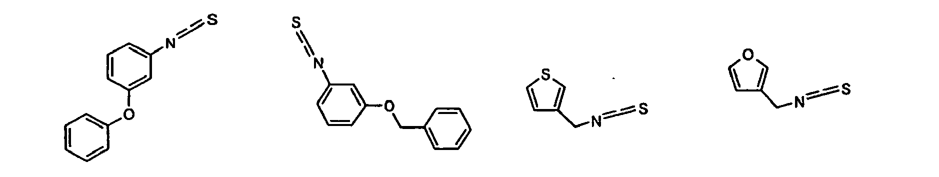

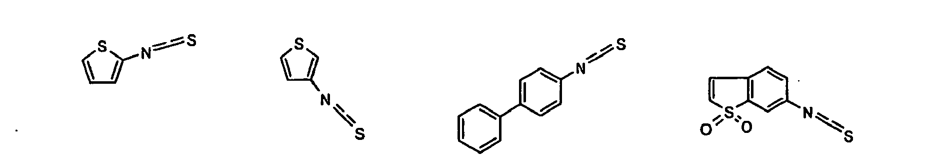

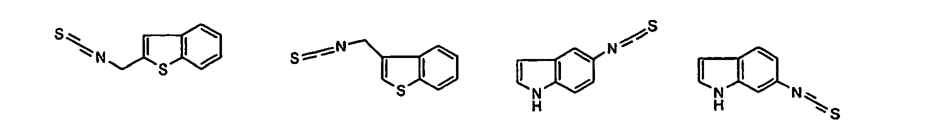

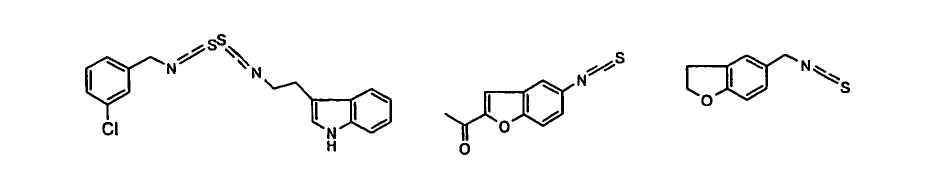

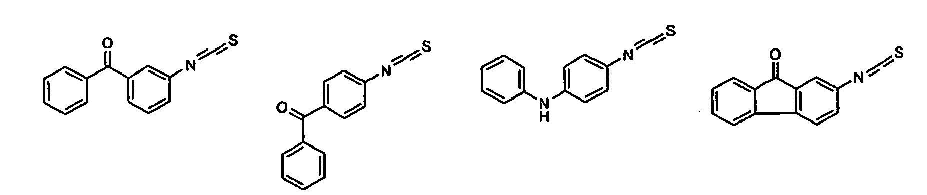

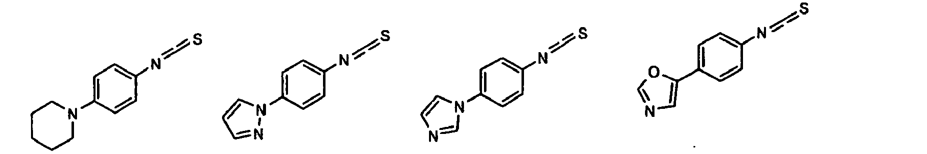

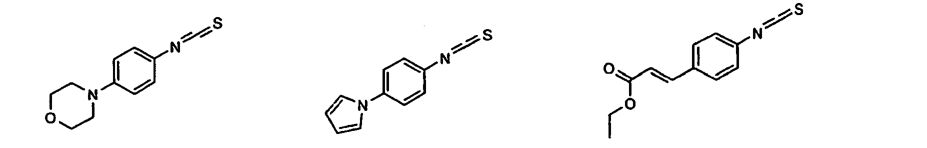

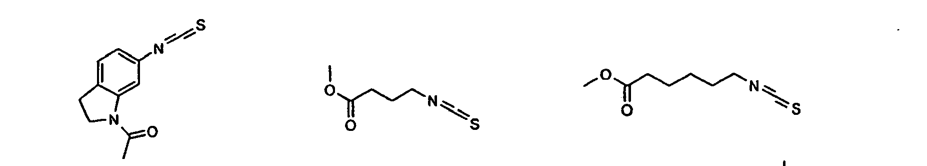

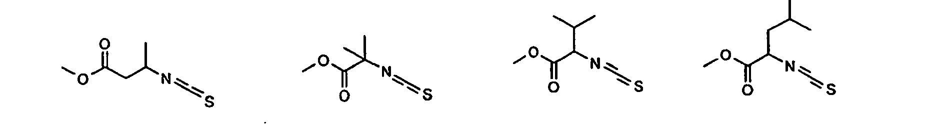

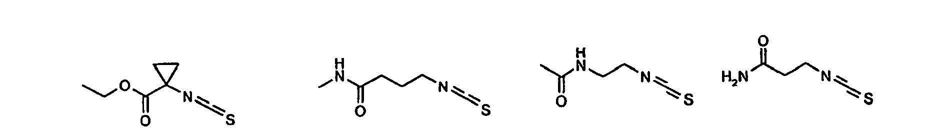

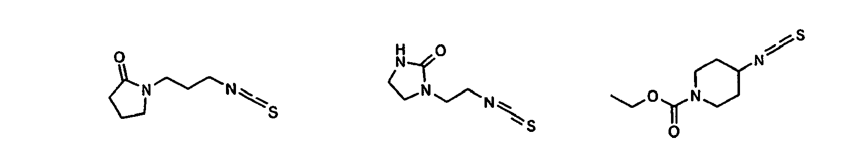

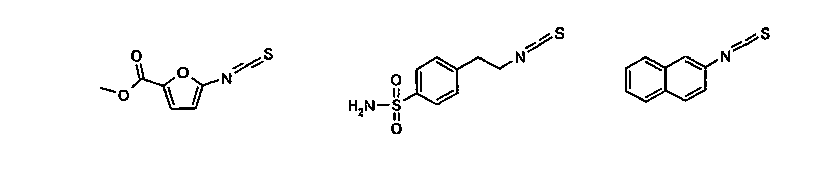

### Préparation de N-(4-isothiocyanatophényl)-N-méthoxyurée

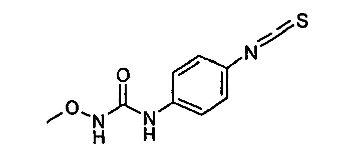

A une solution refroidie à 0° C de *tert*-butyl 4-aminophénylcarbamate (1,04 g) dans le dichlorométhane anhydre (100 ml) est additionné le carbonyl-di-imidazole (1,62 g, 2 éq). Le mélange est ramené à une température de 20° C et est agité à cette température pendant 15 heures. Au milieu réactionnel refroidi à 0° C sont successivement additionnés la triéthylamine (7 ml, 10 éq) suivie du chlorhydrate de *O*-méthylhydroxylamine (4,2 g, 10 éq). Après 3 heures d'agitation à une température voisine de 20° C, le mélange est additionné d'eau saturée en hydrogénocarbonate de sodium et de chloroforme. Après décantation et extractions, les phases organiques réunies sont lavées avec de la saumure, séchées sur Na₂SO₄ puis concentrées sous pression réduite à 40° C pour conduire au *tert-*butyl 4-{[(methoxyamino)carbonyl]amino}phenylcarbamate (1,33 g). Une suspension de ce dérivé dans l'acétate d'éthyle est traversée par un flux d'acide chlorhydrique gazeux jusqu'à ce que la réaction soit totale. Le précipité obtenu est filtré puis lavé à l'éther diéthylique et séché pour conduire au chlorhydrate de *N*-(4-aminophényl)-*N*'-méthoxyurée (1 g).

A une solution refroidie à 0° C, de chlorhydrate de *N*-(4-aminophényl)-*N'*-méthoxyurée (1 g) et de triéthylamine (3,2 ml, 5 éq) dans le tétrahydofuranne (90 ml) est additionné goutte à goutte le thiophosgène (0,38 ml, 1,1 éq). Le mélange est agité 15 min à 0° C puis est additionné d'eau et d'éther diéthylique. Après décantation et extractions, les phases organiques sont réunies, lavées avec de la saumure, séchées sur Na₂SO₄ puis concentrées sous pression réduite à 40° C. La purification par chromatographie éclair sur gel de silice (éluant : heptane / acétate d'éthyle 7 :3 à 3 :7) donne le composé attendu (630 mg ; 62 % rendement).
RMN (¹H, 400 MHz, DMSO-*d₆*) : δ 3,61 (s, 3H), 7,34 (AB, 2H), 7,67 (AB, 2H), 9,11 (s, 1H), 9,65 (s, 1H).

### Préparation d'acyl-isothiocyanates non commerciaux:

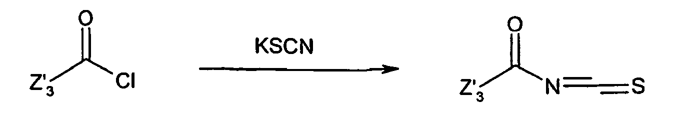

Les acyl-isothiocyanates peuvent être préparés à partir des chlorures d'acides correspondants par traitement avec du thiocyanate de potassium dans un solvant aprotique tel que l'acétonitrile à une température de 0-60° C pendant 0,2-5 heures.

### 4-isothiocyanatocarbonylbenzoate de méthyle :

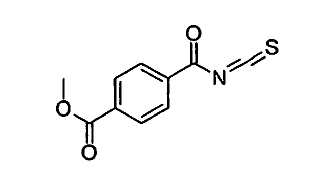

A une solution de 4-chlorocarbonylbenzoate de méthyle (2 g) dans l'acétonitrile (30 ml) est additionné le thiocyanate de potassium (1,08 g). Après 1 heure d'agitation à environ 20° C, le mélange est filtré et le filtrat est concentré sous pression réduite à 40° C. Le solide obtenu est purifié par chromatographie éclair sur gel de silice (éluant : heptane / acétate d'éthyle 1 :1) pour donner le composé attendu (2,1 g ; 95 % rendement).
RMN (¹H, 400 MHz, DMSO-*d₆*) : δ 3,88 (s, 3H), 8,0 (m, 4H).

Les isothiocyanates suivants ont été préparés selon la même procédure que celle décrite pour le 4-isothiocyanatocarbonylbenzoate de méthyle :

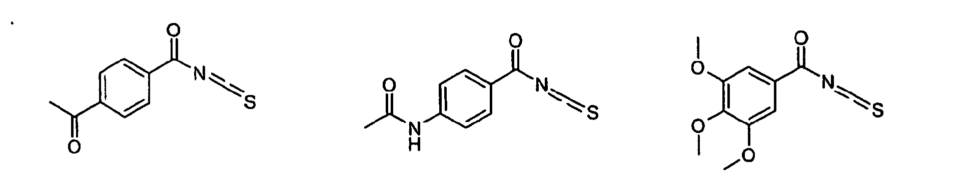

Selon le schéma réactionnel A et de façon analogue à la procédure décrite pour la synthèse du dichlorhydrate de 4-[(1-(3-aminopropyl)-6-{[bis(3-méthylbutyl) amino]carbonyl}-1*H*-benzimidazol-2-yl)amino] benzoate de méthyle, du dichlorhydrate de 2-[(4-acétylphényl)amino]-*N,N*-bis(3-méthylbutyl)-1-(3-pipéridin-1-ylpropyl)-1*H-*benzimidazole-6-carboxamide ou du dichlorhydrate de 2-(cyclohexylamino)-1-[3-(diméthylamino)propyl]-*N,N*-bis(3-méthylbutyl)-1*H*-benzimidazole-6-carboxamide, les composés suivants ont été préparés : 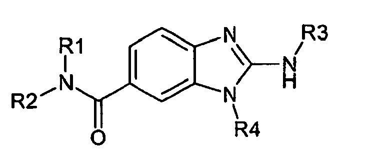 dans lesquels R₁R₂N représente l'un des radicaux ci-après : 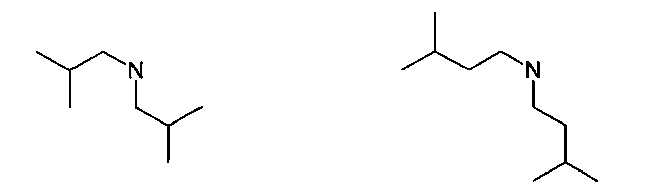 et R₃ représente l'un des radicaux ci-après : 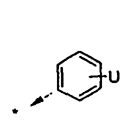 1 ou plusieurs substitutions choisies parmi: U = H, F, Cl, Br. I, NO₂, N₃, OMe, OEt. SMe, Me, Et, iPr, tBu, CF₃, OCF₃, SCF₃, C(O)OMe, C(O)OEt, C(O)Me. C(O)Et, C(O)Ph, NHC(O)Me, C(O)NHMe, C(O)N(Me)₂, C(O)NH₂, NHC(O)NHMe. NHC(O)NHOMe, S(O)₂M S(O)₂NH₂, S(O)₂NHMe, S(O)₂pipéridine, NHphényl, phényl, phénoxy, benzyloxy 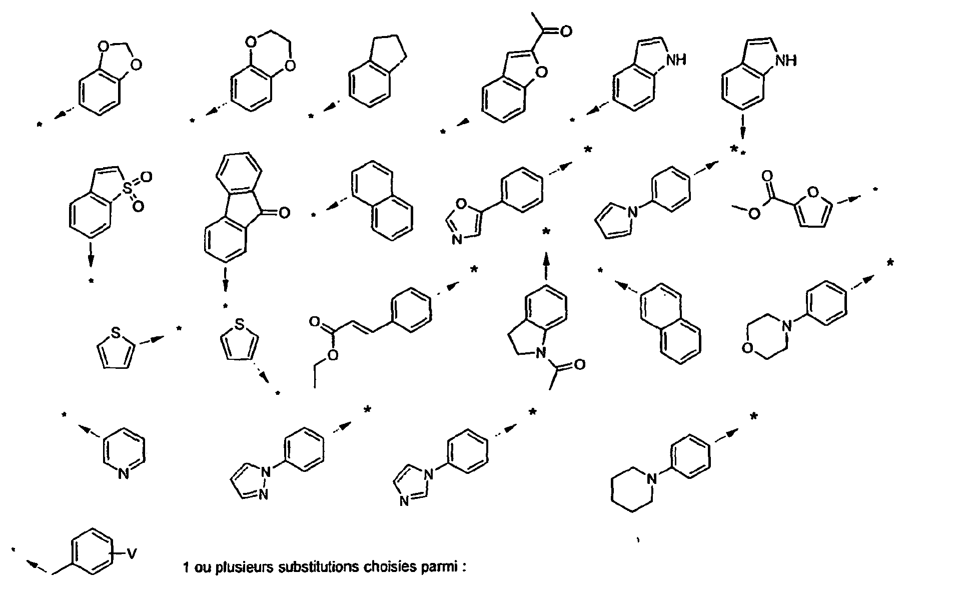 V = H, F, O, Br, I, NO₂, OMe, SMe, Me, Et, iPr, CF₃, OCF₃, SCF₃, C(O)OMe, C(O)OEt, C(O)Me, C(O)Et, C(O)NHMe, S(O)₂Me, S(O)₂N- 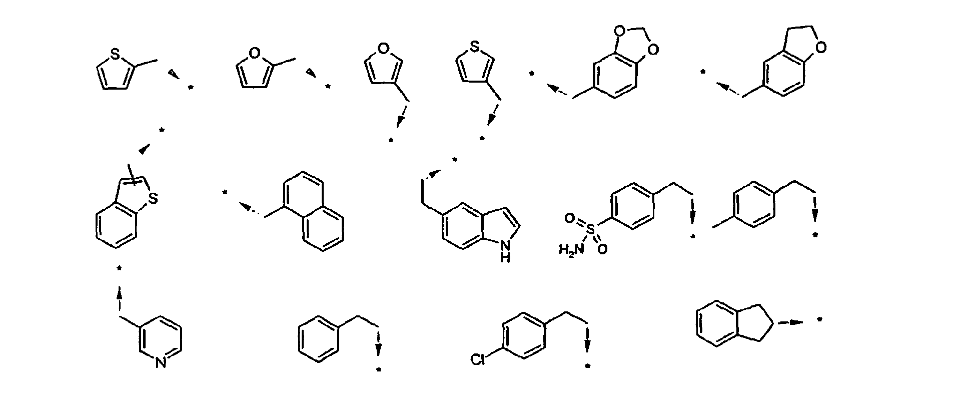 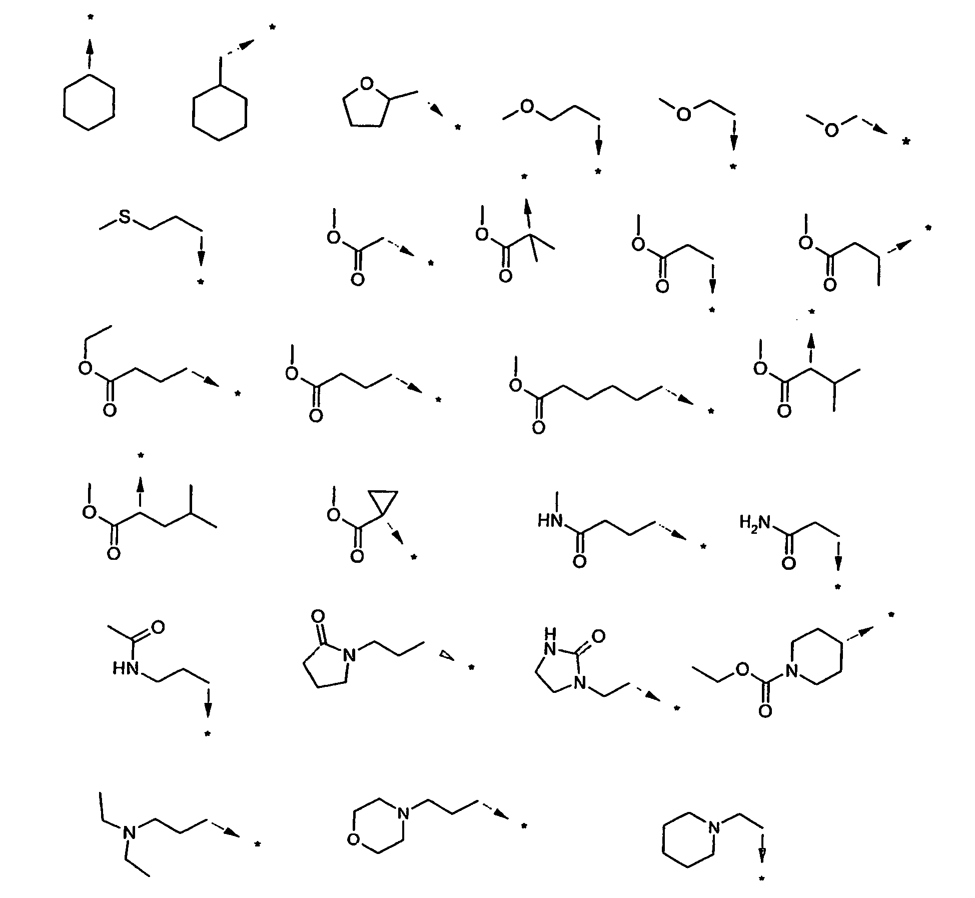 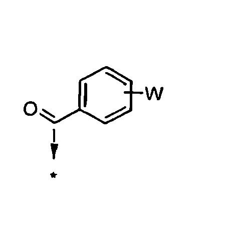 W = H, F, Cl, Br, NO₂, Me, OMe, OEt, CF₃, OCF₃, tBu, C(O)Me, C(O)OMe, C(O)NHMe et R₄ représente l'un des radicaux ci-après :

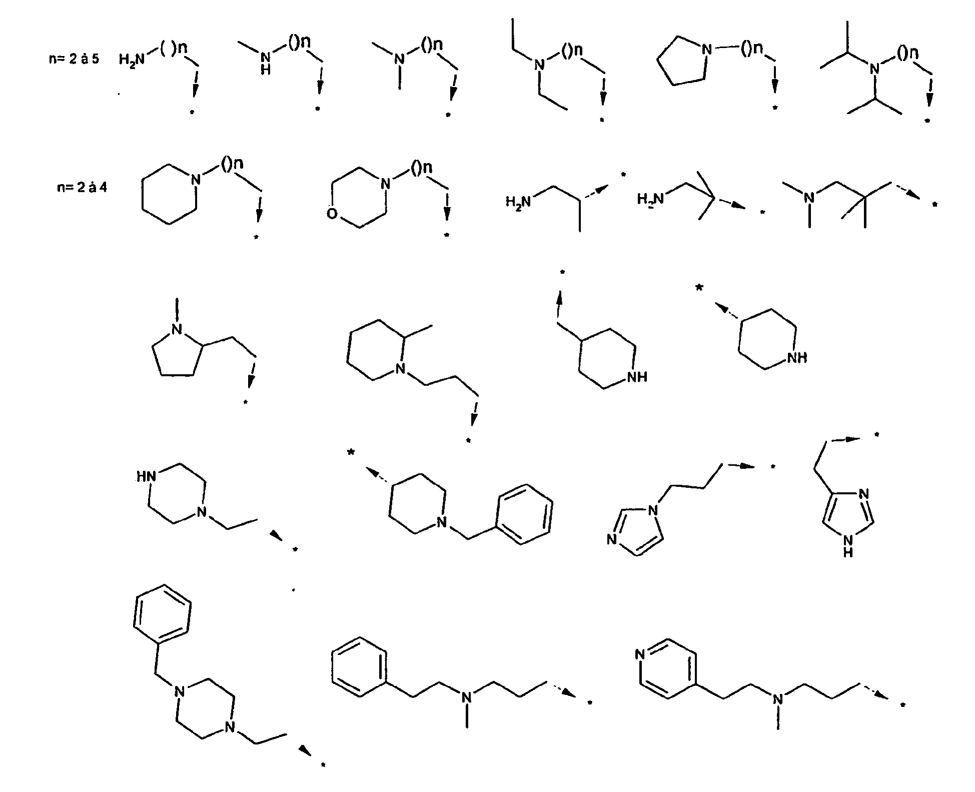

### B. Préparation selon le schéma réactionnel B:

Les composés de formule (I) selon l'invention dans laquelle A représente -C(O)-, peuvent être également préparés selon le schéma B suivant :

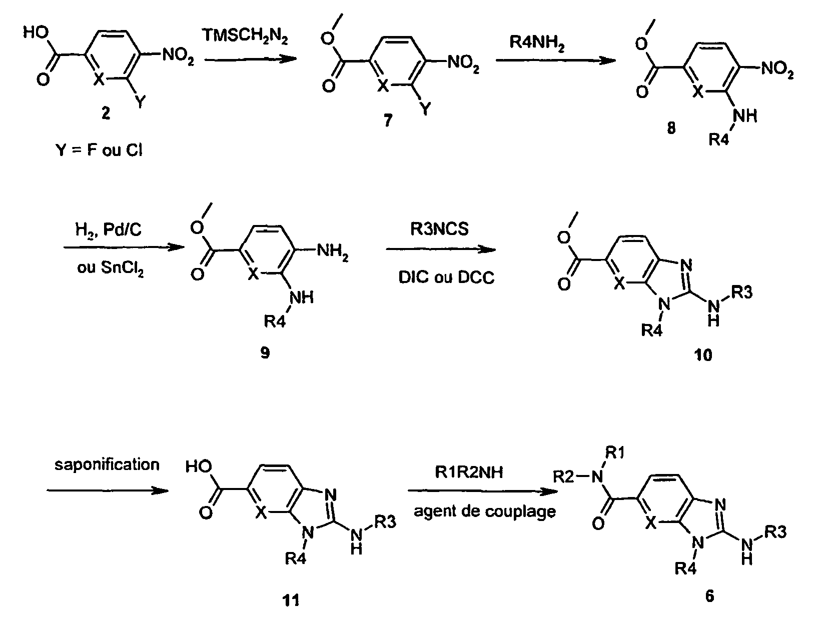

Comme décrit dans le schéma B, l'acide carboxylique (2) peut être converti en ester méthylique (7) soit par traitement avec une solution de triméthylsilyl-diazométhane à une température de 0-20° C, soit par formation d'un sel de carboxylate à l'aide d'une base inorganique telle que l'hydroxyde de lithium dihydrate ou le carbonate de césium, à température ambiante pendant 30 min à 2 heures, dans un solvant organique inerte tel que le tétrahydrofuranne, suivi de l'addition de diméthylsulfate à température ambiante et agitation à reflux pendant 5 à 15 heures. Le dérivé fluoré ou chloré (7) peut-être traité par une amine primaire en présence d'une base inorganique telle que le carbonate de césium ou de potassium dans un solvant organique inerte tel que le diméthylformamide ou l'acétonitrile à une température de 20-100° C pendant 2 à 48 heures pour conduire au dérivé (8). La fonction nitro du composé (8) peut-être réduite par traitement avec du chlorure d'étain dihydrate dans un solvant inerte telle que l'acétate d'éthyle ou le diméthylformamide, à une température de 60-80° C pendant 3 à 15 heures, ou par hydrogénation catalytique en présence de palladium sur charbon 10 % dans un solvant inerte tel que le méthanol, l'éthanol, l'acétate d'éthyle ou un mélange de ces solvants, à une température de 18-25° C, pendant 2 à 8 heures, pour conduire à la dianiline (9). Le dérivé (9) est ensuite traité par un isothiocyanate en présence d'un agent de couplage tel que la diisopropylcarbodiimide ou la dicyclohexylcarbodiimide dans un solvant inerte tel que le tétrahydrofuranne, le chlorure de méthylène ou le chloroforme à une température de 20-70° C pendant 2 à 72 heures pour conduire au dérivé (10). Alternativement, le dérivé (9) peut être traité par un isothiocyanate dans un solvant inerte tel que le tétrahydrofuranne, chlorure de méthylène, chloroforme ou éthanol à une température de 20-80° C pendant 1-16 heures puis la thiourée résultante peut être traitée par de l'iodure de méthyle ou de l'oxyde de mercure (II) jaune en présence d'une quantité catalytique de soufre dans un solvant polaire tel que le méthanol ou l'éthanol pendant 2 à 24 heures à une température de 20-80° C pour conduire à (10). L'ester méthylique (10) peut ensuite être saponifié en présence d'une base inorganique telle que l'hydroxyde de lithium dihydrate dans un mélange de solvants polaires tels que l'eau et le tétrahydrofuranne à une température de 20 à 70° C pendant 3 à 17 heures. L'acide carboxylique résultant (11) peut être couplé avec une amine primaire ou secondaire en présence d'un agent de couplage tel que le diisopropylcarbodiimide (DIC), le dicyclohexylcarbodiimide (DCC), le chlorhydrate de 1-(3-dimethylaminopropyl)-3-éthylcarbodiimide (EDC) ou le carbonyldiimidazole (CDI), avec ou sans du 1-hydroxybenzotriazole (HOBt) dans un solvant organique inerte tel que du chlorure de méthylène, tétrahydrofuranne ou diméthylformamide à température ambiante pendant 3 à 24 heures pour conduire à l'amide correspondante (6) qui peut être isolée, soit par chromatographie éclair sur gel de silice, soit par addition au mélange réactionnel d'un réactif nucléophile supporté sur un polymère comme par exemple une résine aminométhylpolystyrène et d'un réactif électrophile supporté sur un polymère comme par exemple la résine méthylisothiocyanate-polystyrène, suivie d'une filtration et de l'évaporation du filtrat.

### Exemple B1: dichlorhydrate de 1-(3-aminopropyl)-6-(pipéridin-1-ylcarbonyl)-N-(3,4,5-triméthoxyphényl)-1H-benzimidazol-2-amine

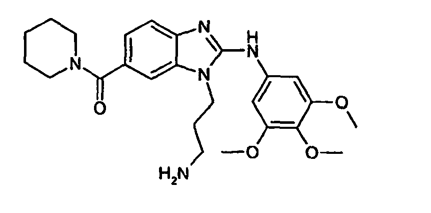

### Etape 1 : 3-fluoro-4-nitrobenzoate de méthyle

A une solution d'acide 3-fluoro-4-nitrobenzoique (4,7 g, 1 eq) dans le méthanol (70 ml) est additionnée lentement une solution de triméthylsilyldiazométhane (2M dans l'hexane, 50 ml, 4 eq) jusqu'à ce que le dégagement gazeux cesse. L'excès de triméthylsilyldiazométhane est consommé par addition goutte à goutte d'acide acétique jusqu'à décoloration de la solution. Le mélange est alors concentré sous pression réduite à une température d'environ 40° C. Le résidu est additionné d'eau (200 ml) et de dichlorométhane (300 ml). Après décantation et extractions, les phases organiques combinées sont lavées avec de la saumure, séchées sur Na₂SO₄ puis concentrées sous pression réduite à 40° C. Le solide obtenu est lavé à l'éther de pétrole et séché (4,4 g ; 87 % rendement).
RMN (¹H, 400 MHz, CDCl₃) : δ 4,0 (s, 3H), 7,97 (m, 2H), 8,11 (d, 1H).

### Etape 2 : 3-({ 3-[(tert-butoxycarbonyl)amino]propyl } amino)-4-nitrobenzoate de méthyle

Un mélange de 3-fluoro-4-nitrobenzoate de méthyle (5,8 g, 1 eq), de *N*-Boc-1,3-diaminopropane (5,75 g, 1,1 eq) et de carbonate de potassium (8,04 g, 2 eq) dans l'acétonitrile (200 ml) est chauffé au reflux pendant 2 heures puis concentré sous pression réduite à 40° C. Le résidu est repris par du dichlorométhane (200 ml) et de l'eau (100 ml). Après décantation et extractions, les phases organiques combinées sont lavées avec de la saumure, séchées sur Na₂SO₄ puis concentrées sous pression réduite à 40° C. Le solide obtenu est lavé à l'éther de pétrole et séché (10,2 g ; 99 % rendement).
RMN (¹H, 400 MHz, CDCl₃) : δ 1,45 (s, 9H), 1,95 (m, 2H), 3,30 (m, 2H), 3,44 (m, 2H), 3,95 (s, 3H), 4,67 (m, 1H), 7,25 (m,1H), 7,55 (s, 1H), 8,04 (m, 1H), 8,22 (m,1H).

### Etape 3: 4-amino-3-({3-[(tert-butoxycarbonyl)amino]propyl}amino)benzoate de méthyle

Dans un autoclave sont additionnés le 3-({3-[(*tert*-butoxycarbonyl)amino]propyl} amino)-4-nitrobenzoate de méthyle (10,2 g) en solution dans un mélange d'acétate d'éthyle/ méthanol 3 :1 (300 ml), et le palladium sur charbon 10 % (1,02 g). Après 4 heures d'agitation sous atmosphère d'hydrogène (3 bars) à une température d'environ 20° C, le catalyseur est éliminé par filtration sur célite et le filtrat est concentré sous pression réduite à 40° C pour donner le composé attendu sous forme d'une huile (7,75 g; 83 % rendement).
SM/CL : MM calculée = 323,4 ; m/z = 324,2 (MH+)
RMN (¹H, 400 MHz, CDCl₃) : 1,45 (s, 9H), 1,85 (m, 2H), 3,24 (m, 2H), 3,30 (m, 2H), 3,86 (m, 5H), 4,68 (m, 1H), 6,68 (d, 1H), 7,34 (s, 1H), 7,45 (d, 1H).

### Etape 4 : 1-{3-[(tert-butoxycarbonyl)amino]propyl}-2-[(3,4,5-triméthoxyphényl) amino] -1H-benzimidazole-6-carboxylate de méthyle

A une solution de 4-amino-3-({3-[(*tert*-butoxycarbonyl)amino]propyl}amino) benzoate de méthyle (7,75 g, 1 eq) dans le tétrahydrofuranne (130 ml) sont successivement additionnés le 3,4,5-triméthoxyphényl isothiocyanate (6,6 g, 1,2 eq) et le diisopropylcarbodiimide (9,1 g, 3 eq). Le mélange est chauffé à reflux pendant 16 heures puis refroidi à température ambiante et concentré sous pression réduite à 40° C. Le résidu obtenu est additionné d'eau (100 ml) et de dichlorométhane (200 ml). Après décantation et extractions, les phases organiques combinées sont lavées avec de la saumure, séchées sur Na₂SO₄ puis concentrées sous pression réduite à 40° C. La purification du résidu par chromatographie éclair sur gel de silice (éluant : heptane / acétate d'éthyle 8 :2 à 3 :7) donne le composé attendu sous forme d'un solide qui est lavé à l'éther (4,4 g ; 36 % rendement).
SM/CL : MM calculée = 514,5 ; m/z = 515,3 (MH+)
RMN (¹H, 400 MHz, CDCl₃) : 1,54 (s, 9H), 2,11 (m, 2H), 3,26 (m, 2H), 3,83 (m, 3H), 3,90 (s, 3H), 3,93 (s, 6H), 4,22 (m, 2H), 5,03 (m, 1H), 7,23 (s, 2H), 7,53 (d, 1H), 7,90 (s, 1H), 7,92 (d, 1H), 9,12 (m, 1H).

### Etape 5 : acide 1-{3-[(tert-butoxycarbonyl)amino]propyl}-2-[(3,4,5-triméthoxyphényl) amino]-1H-benzimidazole-6-carboxylique

A une solution de 1-{3-[(tert-butoxycarbonyl)amino]propyl}-2-[(3,4,5-triméthoxyphényl)amino]-1H-benzimidazole-6-carboxylate de méthyle (4,4 g, 1 eq) dans un mélange de tétrahydrofuranne (40 ml) et d'eau (30 ml) est additionné l'hydroxyde de lithium (2,18 g, 6 eq). Le mélange est chauffé à reflux pendant 18 heures puis refroidi à température ambiante et concentré sous pression réduite à 40° C. Le résidu est additionné de dichlorométhane (150 ml) et d'eau (100 ml). Le mélange est acidifié par addition d'acide acétique jusqu'à pH 5. Après décantation et extractions, les phases organiques combinées sont séchées sur sulfate de sodium et concentrées sous pression réduite. Le solide obtenu est lavé à l'éther éthylique (3,95 g ; 93%).
SM/CL : MM calculée = 500,5 ; m/z = 501,3 (MH+)
RMN (¹H, 400 MHz, DMSO-*d₆*) : 1,37 (s, 9H), 1,83 (m, 2H), 3,03 (m, 2H), 3,61 (s, 3H), 3,80 (s, 6H), 4,27 (m, 2H), 7,0 (m, 1H), 7,31(s, 2H), 7,35 (d, 1H), 7,71 (d, 1H), 7,87 (s, 1 H), 8,97 (s, 1H).

### Etape 6 : dichlorhydrate de 1-(3-aminopropyl)-6-(piperidin-1-ylcarbonyl)-N-(3,4,5-triméthoxyphényl)-1H-benzimidazol-2-amine

A une solution d'acide 1-{3-[(*tert*-butoxycarbonyl)amino]propyl}-2-[(3,4,5-trimétlioxyphényl) amino]-1*H* benzimidazole-6-carboxylique (50 mg, 1 eq) dans le tétrahydrofuranne (0,45 ml) et le diméthylformamide (0,05 ml) est additionnée une solution de carbonyldiimidazole (CDI) (18 mg, 1,1 eq) dans le chloroforme (0,2 ml). Le mélange est agité 16 heures à une température voisine de 20° C puis une solution de pipéridine (17 mg, 2 eq) dans le tétrahydrofuranne (0,2 ml) est additionnée. Après 18 heures d'agitation à une température voisine de 20° C, le mélange est dilué par du dichlorométhane (3 ml) et additionné de résine aminométhylpolystyrène (2 eq), résine TBD-méthyl-polystyrène (2 eq) et résine méthylisothiocyanate-polystyrène (4 eq). Après 6 heures d'agitation à environ 20° C, le mélange est filtré et le filtrat est concentré sous pression réduite à 40° C. Le résidu obtenu est dissout dans l'acétate d'éthyle (0,5 ml) et additionné d'une solution d'acide chlorhydrique (1N dans l'éther diéthylique, 3 ml). Après 1 heure d'agitation à une température voisine de 20° C, le précipité obtenu est filtré et séché pour donner le composé attendu (35 mg, 65 %).
SM/CL: MM calculée = 467,56 ; m/z = 467,9 (MH+)
RMN (¹H, 400 MHz, DMSO-*d₆*) : 1,48-1,63 (m, 6H), 2,05 (m, 2H), 2,90 (m,2H), 3,50 (m, 4H), 3,65 (s, 3H), 3,79 (s, 6H), 4,45 (m, 2H), 7,10-7,60 (m, 5H), 7,54 (m, 1H), 7,94 (m, 3H), 8,41 (m, 1H), 14,3 (m, 1H).

Selon le schéma réactionnel B et de façon analogue à la procédure décrite pour la synthèse du dichlorhydrate de 1-(3-aminopropyl)-6-(piperidin-1-ylcarbonyl)-*N*-(3,4,5-trimethoxyphenyl)-1*H*-benzimidazol-2-amine, les composés suivants ont été préparés : 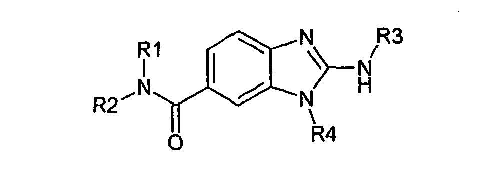 dans lesquels R₁R₂N représente l'un des radicaux ci-après : 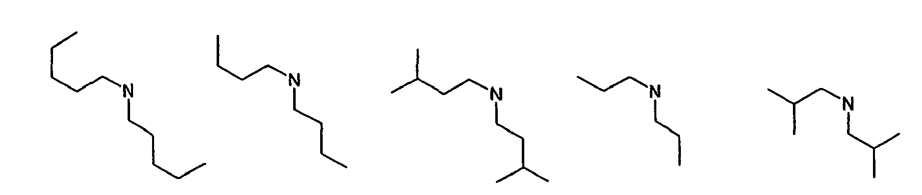 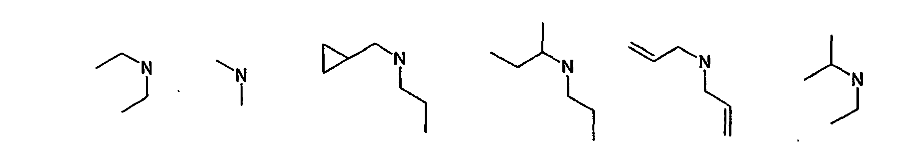 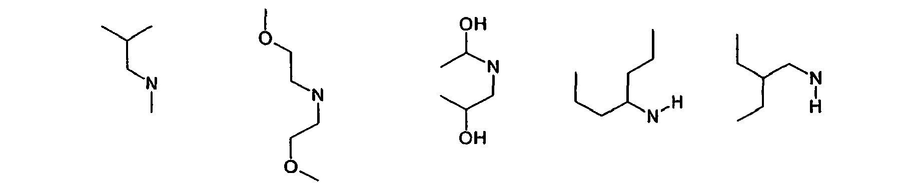 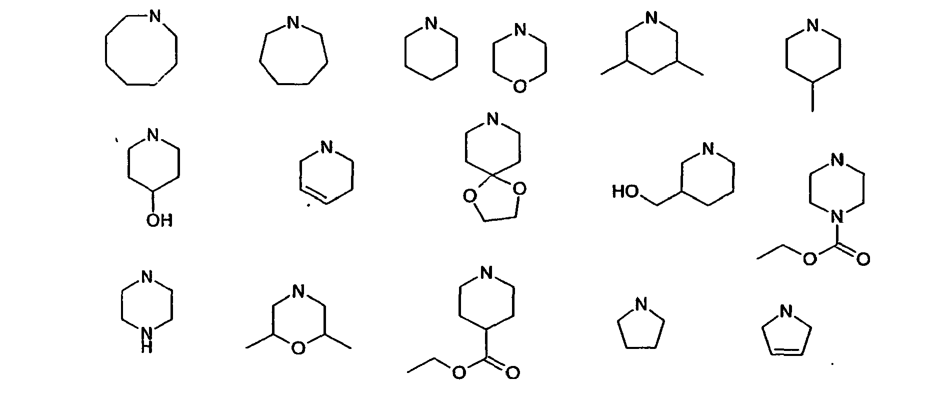 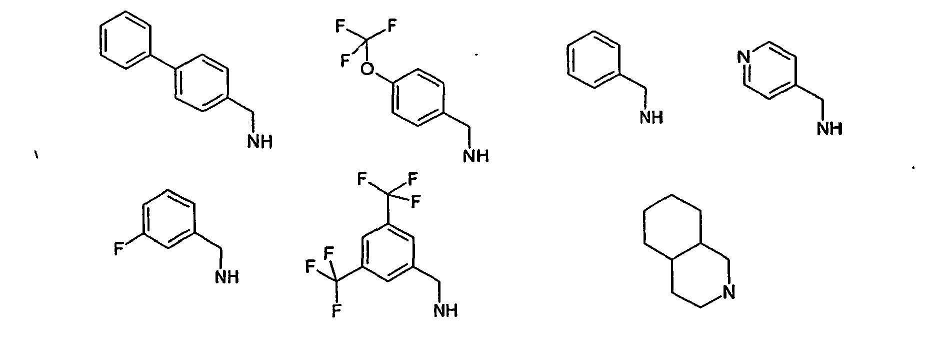 R₃ représente le radical ci-après : 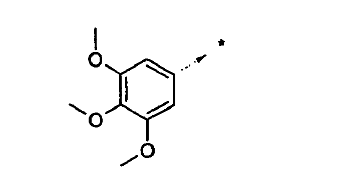 et R₄ représente l'un des radicaux ci-après :

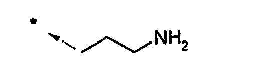

### C. Préparation selon le schéma réactionnel C :

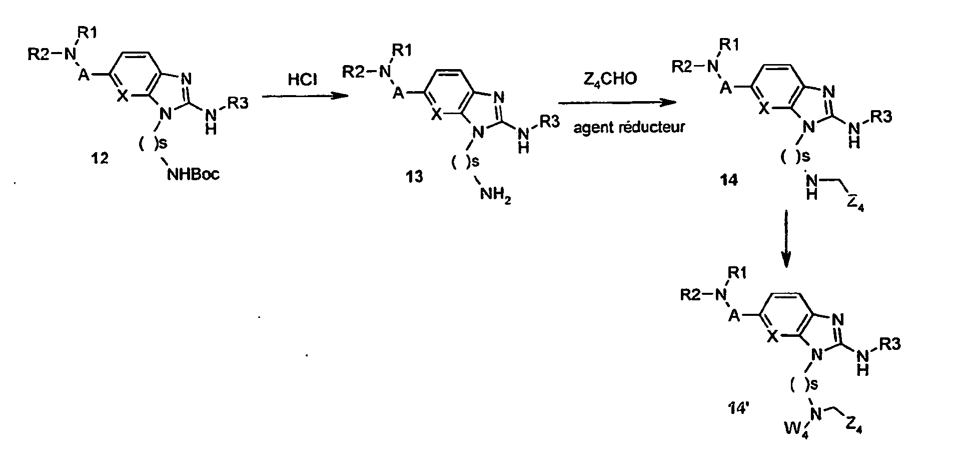

Comme décrit dans le schéma C, le dérivé (12), préparé selon les schémas réactionnels A ou B peut être traité par un acide organique ou inorganique comme l'acide trifluoroacétique ou l'acide chlorhydrique (aqueux ou sous forme gazeuse) dans un solvant aprotique tel que le dichlorométhane, l'éther diéthylique ou l'acétate d'éthyle à une température de 0-20° C pendant 0,5 à 5 heures, pour conduire à l'amine (13). L'amine (13) peut réagir avec un aldéhyde dans un solvant protique ou aprotique, tel que le dichlorométhane, tétrahydrofuranne ou le méthanol, pendant 1 à 15 heures à une température de 0-50° C. L'imine résultante est ensuite réduite *in situ* par un agent réducteur supporté sur une résine ou non, de préférence le triacétoxyborohydrure de sodium, le cyanoborohydrure de sodium ou borohydrure supporté sur une résine, avec ou sans la présence d'un acide tel que l'acide acétique, à une température de 20 à 50° C pendant une durée de 0,2 à 5 heures, pour conduire au composé (14). L'amine secondaire (14) peut éventuellement subir une seconde amination réductrice dans les mêmes conditions opératoires que celles décrites précédemment pour conduire à l'amine tertiaire (14').

### Exemple C1 : dichlorhydrate de N,N-diisobutyl-1-[3-(néopentylamino)propyl]-2-[(3,4,5-triméthoxyphényl)amino]-1H-benzimidazole-6-carboxamide

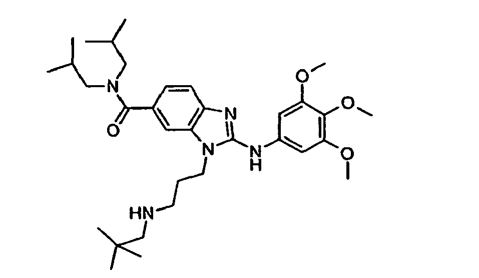

### Etape 1 : 1-(3-aminopropyl)-N,N diisobutyl-2-[(3,4,5-triméthoxyphényl)amino]-1H-benzimidazole-6-carboxamide

Une solution de 3-{6-[(diisobutylamino)carbonyl]-2-[(3,4,5-triméthoxyphényl) amino]-1*H*-benzimidazol-1-yl}propylcarbamate de *tert*-butyle (350 mg; préparé selon le schéma A) dans l'acétate d'éthyle (30 ml), refroidie à 0° C, est traversée par un flux de HCl sec jusqu'à ce que la CCM (éluant : acétate d'éthyle 100 %) montre la disparition totale du produit de départ. Le mélange est alors concentré sous pression réduite à 40° C. Le solide obtenu est trituré à l'éther éthylique puis filtré, lavé au dichlorométhane et séché. Le dichlorhydrate obtenu est repris dans du dichlorométhane et de l'eau saturée en hydrogénocarbonate de sodium. Après décantation et extractions, les phases organiques réunies sont lavées avec de la saumure, séchées sur Na₂SO₄ puis concentrées sous pression réduite à 40° C pour donner le composé attendu sous forme de base libre (275 mg ; 94 % rendement).
SM/CL : MM calculée = 511,6 ; m/z = 512,3 (MH+)
RMN (¹H, 400 MHz, DMSO-*d₆*) :δ 0,82 (m, 12H), 1,87 (m, 4H), 2,58 (m, 2H), 3,21 (m, 4H), 3,62 (s, 3H), 3,78 (s, 6H), 4,25 (t, 2H), 7,0 (AB, 1H), 7,20 (s, 2H), 7,26 (s, 1H), 7,34 (AB, 1H).

### Etape 2 : dichlorhydrate de N,N-diisobutyl-1-[3-(néopentylamino)propyl]-2-[(3,4,5-triméthoxyphényl)amino]-1H-benzimidazole-6-carboxamide

Une solution de 1-(3-aminopropyl)-*N,N* diisobutyl-2-[(3,4,5-triméthoxyphényl)amino]-1*H*-benzimidazole-6-carboxamide (100 mg, 1 eq) et de triméthylacetaldehyde (25 mg, 1,5 eq) dans le dichlorométhane (1 ml) est agitée 4 heures à une température voisine de 20° C. Le mélange est dilué avec du méthanol (1 ml) puis additionné de triacétoxyborohydrure de sodium (41 mg, 2 eq). Après 1 heure à une température voisine de 20° C, le mélange est additionné de dichlorométhane (20 ml) et d'eau saturée en hydrogénocarbonate de sodium (10 ml). Après décantation et extractions, les phases organiques réunies sont lavées avec de la saumure, séchées sur Na₂SO₄ puis concentrées sous pression réduite à 40° C. La purification du résidu par chromatographie éclair sur gel de silice (éluant : dichlorométhane 100 % à dichlorométhane/méthanol 9 :1) donne le composé attendu sous forme de base. Le sel de chlorhydrate correspondant est formé par addition d'une solution d'acide chlorhydrique 1N dans l'éther. Le précipité obtenu est filtré et séché pour donner le composé dichlorhydrate attendu (83 mg, 65 % rendement).
SM/CL : MM calculée = 581,8 ; m/z = 582,3 (MH+)
RMN (¹H, 400 MHz, DMSO-*d₆*) : δ 0,67 (m, 6H), 0,95 (m, 6H), 0,99 (s, 9H), 1,82 (m, 1H), 2,06 (m, 1H), 2,27 (m, 2H), 2,71 (m, 2H), 3,10 (m, 4H), 3,28 (m, 2H), 3,70 (s, 3H), 3,81 (s, 6H), 4,58 (t, 2H), 6,99 (m, 2H), 7,22 (AB, 1H), 7,41 (AB, 1H), 7,69 (s, 1H), 8,72 (m, 2H), 11,42 (m, 1H), 13,02 (m, 1H).

### Préparation selon le schéma réactionnel C' :

Les composés (14) pour lesquels s=3 peuvent également être préparés selon le schéma C' suivant :

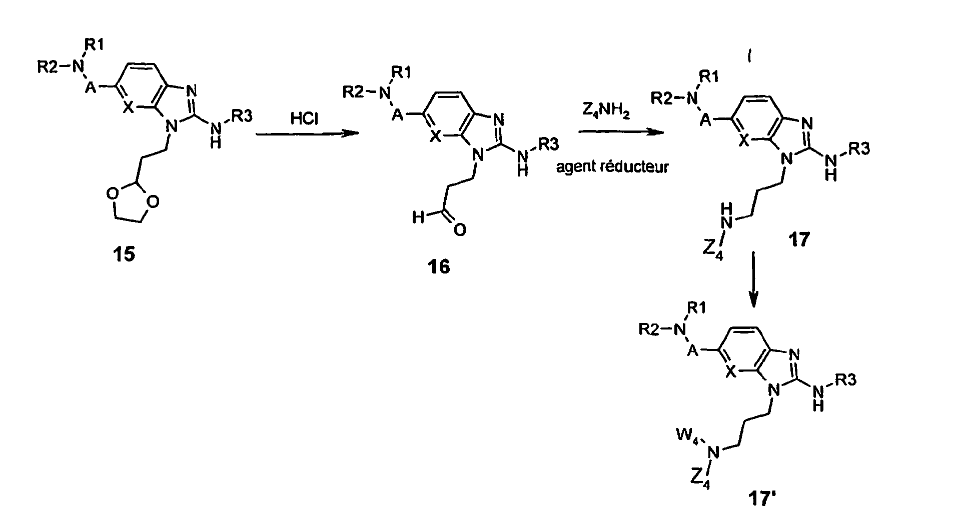

Comme décrit dans le schéma C', le dérivé (15) préparé selon le schéma réactionnel A peut être traité soit par un acide organique tel que le tosylate de pyridinium ou l'acide paratoluènesulfonique dans un solvant aprotique tel que l'acétone en présence d'eau, à une température de 20-70° C pendant 2 à 12 heures, soit par un acide inorganique tel que le chlorure d'hydrogène aqueux dans un solvant aprotique tel que le tétrahydrofuranne à une température de 0-20° C pendant 6 à 18 heures pour conduire au composé (16). L'aldéhyde (16) peut ensuite être traité par une amine dans un solvant protique ou aprotique tel que le dichlorométhane, le tétrahydrofuranne ou le méthanol pendant 1 à 18 heures à une température de 20° C. L'imine résultante est ensuite réduite *in situ* par un agent réducteur, de préférence le triacétoxyborohydrure de sodium ou le cyanoborohydrure de sodium, en présence ou non d'un acide tel que l'acide acétique, à une température de 20-50° C pendant une durée de 0,2 à 6 heures, pour conduire au composé (17). L'amine secondaire (17) peut éventuellement subir une seconde amination réductrice dans les mêmes conditions opératoires que celles décrites précédemment pour conduire à l'amine tertiaire (17').

### Exemple C1' : dichlorhydrate de 2-[(4-acétylphényl)amino]-1-{3-[cyclohexylmethyl amino]propyl}-N,N-bis(3-méthylbutyl)-1H-benzimidazole-6-carboxamide

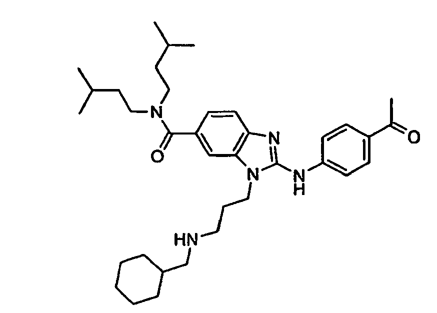

### Etape 1 : 3-{[2-(1,3-dioxolan-2-yl)éthyl]amino}-N,N-bis(3-méthylbutyl)-4-nitrobenzamide

Un mélange de 3-fluoro-*N*,*N*-bis(3-méthylbutyl)-4-nitrobenzamide préparé selon l'exemple A1 (1,86 g, 1 eq), de 2-(2-aminoéthyl)-1,3-dioxolane (0,8 g, 1,2 eq) et de carbonate de potassium (1,58 g, 2 eq) dans l'acétonitrile (150 ml) est chauffé au reflux pendant 3 heures puis concentré sous pression réduite à 40° C. Le résidu est repris par du dichlorométhane (150 ml) et de l'eau (60 ml). Après décantation et extractions, les phases organiques combinées sont lavées avec de la saumure, séchées sur Na₂SO₄ puis concentrées sous pression réduite à 40° C. La purification du résidu par chromatographie éclair sur gel de silice (éluant : heptane /acétate d'éthyle 8 :2 à 7 :3) donne le composé attendu sous forme d'une huile jaune orangée (2,4 g ; 98 % rendement).
SM/CL : MM calculée = 421,5 ; m/z = 422,2 (MH+)
RMN (¹H, 400 MHz, DMSO-*d₆*) : δ 0,68 (d, 6H), 0,92 (d, 6H), 1,31-1,50 (m, 5H), 1,61 (m, 1H), 1,97 (m, 2H), 3,10 (m, 2H), 3,37-3,48 (m, 4H), 3,80 (m, 2H), 3,91 (m, 2H), 4,94 (t, 1H), 6,55 (d, 1H), 6,89 (s, 1H), 8,10 (d, 1H), 8,39 (t, 1H).

### Etape 2 : 4-amino-3-{[2-(1,3-dioxolan-2-yl)éthyl]amino}-N,N-bis(3-méthylbutyl) benzamide

Dans un autoclave sont additionnés le 3-{[2-(1,3-dioxolan-2-yl)éthyl]amino}-*N*,*N-*bis(3-méthylbutyl)-4-nitrobenzamide (2,4 g) en solution dans un mélange d'acétate d'éthyle/ méthanol 2:1 (100 ml), et le palladium sur charbon 10 % (240 mg). Après 4 heures d'agitation sous atmosphère d'hydrogène (3 bars) à une température d'environ 20° C, le catalyseur est éliminé par filtration sur célite et le filtrat est concentré sous pression réduite à 40° C pour donner le composé attendu sous forme d'une huile (2,02 g; 89 % rendement).
SM/CL : MM calculée = 391,5 ; m/z = 392,2 (MH+)
RMN (¹H, 400 MHz, DMSO-*d₆*): δ 0,80 (m, 12H), 1,40 (m, 6H), 1,90 (m, 2H), 3,10 (m, 2H), 3,29 (m, 4H), 3,77 (m; 2H), 3,90 (m, 2H), 4,54 (m, 1H), 4,78 (s, 2H), 4,93 (t, 1H), 6.36-6,52 (m, 1H).

### Etape 3: 2-[(4-acétylphényl)amino]-1-[2-(1,3-dioxolan-2-yl)éthyl]-N,N-bis(3-méthylbutyl)-1H-benzimidazole-6-carboxamide

A une solution de 4-amino-3-{[2-(1,3-dioxolan-2-yl)éthyl]amino}-*N*,*N-*bis(3-méthylbutyl)benzamide (2 g, 1 eq) dans le tétrahydrofuranne (50 ml) sont successivement additionnés le 4-acétylphényl isothiocyanate (1,1 g, 1,2 eq) et le diisopropylcarbodiimide (1,95 g, 3 eq). Le mélange est chauffé à reflux pendant 18 heures puis refroidi à température ambiante et concentré sous pression réduite à 40° C. Le résidu obtenu est additionné d'eau (100 ml) et de dichlorométhane (200 ml). Après décantation et extractions, les phases organiques combinées sont lavées avec de la saumure, séchées sur Na₂SO₄ puis concentrées sous pression réduite à 40° C. La purification du résidu par chromatographie éclair sur gel de silice (éluant : heptane / acétate d'éthyle 4 :6) donne le composé attendu sous forme d'une mousse blanche (1,8 g ; 66 % rendement).
SM/CL : MM calculée = 534,7 ; m/z = 535,3 (MH+)
RMN (¹H, 400 MHz, DMSO-*d₆*) : *δ* 0,80 (m, 12H), 1,44 (m, 6H), 2,01 (m, 2H), 2,52 (s, 3H), 3,30 (t, 4H), 3,72 (t, 2H), 3,85 (m, 2H), 4,39 (t, 2H), 4,83 (t, 1H), 7,05 (AB, 1H), 7,30 (s, 1H), 7,44 (AB, 1H), 7,96 (s, 4H), 9,37 (s, 1H).

### Etape 4 : 2-[(4-acétylphenyl)amino]-N,N bis(3-méthylbutyl)-1-(3-oxopropyl)-1H-benzimidazole-6-carboxamide

Une solution de 2-[(4-acétylphényl)amino]-1-[2-(1,3-dioxolan-2-yl)éthyl]-*N*,*N-*bis(3-méthylbutyl)-1*H*-benzimidazole-6-carboxamide (900 mg) dans un mélange de tétrahydrofuranne (30 ml) et d'acide chlorhydrique aqueux (3N, 40 ml) est agitée 18 heures à une température voisine de 20° C puis concentrée sous pression réduite à 40° C. La phase aqueuse restante est additionnée de dichlorométhane (100 ml). Après décantation et extractions, les phases organiques combinées sont lavées avec de la saumure, séchées sur Na₂SO₄ puis concentrées sous pression réduite à 40° C pour donner le composé attendu sous forme d'une mousse beige (820 mg, 99 % rendement).
SM/CL : MM calculée = 490,6 ; m/z = 491,3 (MH+)
RMN (¹H, 400 MHz, DMSO-*d₆*) : δ 0,68-0,99 (m, 12H), 1,35 (m, 6H), 2,39 (m, 2H), 2,64 (s, 3H), 3,10-3,49 (m, 4H), 3,72 (m, 2H), 4,15 (m, 1H), 4,50 (m, 1H), 5,54 (s, 1H), 7,27 (AB, 1H), 7,39 (AB, 1H), 7,64 (s, 1H), 7,82 (AB, 1H), 8,15 (AB, 1H).

### Etape 5 : dichlorhydrate de 2-[(4-acétylphényl)amino]-1-{3-[(cyclohexylméthyl) amino]propyl}-N,N-bis(3-méthylbutyl)-1H-benzimidazole-6-carboxamide

Une solution de 2-[(4-acétylphényl)amino]-*N,N-*bis(3-méthylbutyl)-1-(3-oxopropyl)-1*H*-benzimidazole-6-carboxamide (100 mg, 1 eq) et d'aminométhylcyclohexane (46 mg, 2 eq) est agitée 4 heures à une température voisine de 20° C. Le mélange est dilué avec du méthanol (1 ml) puis additionné de triacétoxyborohydrure de sodium (86 mg, 2 eq) et de quelques gouttes d'acide acétique jusqu'à pH 5. Après 1 heure à une température voisine de 20° C, le mélange est additionné de dichlorométhane (20 ml) et d'eau saturée en hydrogénocarbonate de sodium (10 ml). Après décantation et extractions, les phases organiques réunies sont lavées avec de la saumure, séchées sur Na₂SO₄ puis concentrées sous pression réduite à 40° C. La purification du résidu par chromatographie éclair sur gel de silice (éluant: dichlorométhane 100% à dichlorométhane / méthanol 9 : 1) donne le composé attendu sous forme de base. Le sel de chlorhydrate correspondant est formé par addition d'une solution d'acide chlorhydrique IN dans l'éther. Le précipité obtenu est filtré et séché pour donner le composé dichlorhydrate attendu (83 mg, 62 % rendement).
SM/CL MM calculée = 587,8 ; m/z = 588,3 (MH+)
RMN (¹H, 400 MHz, DMSO-*d₆*) : δ 0,68-0,99 (m, 14H), 1,19 (m, 3H), 1,29-1,82 (m, 12H), 2,59 (s, 3H), 2,73 (m, 2H), 3,07 (t, 2H), 3,21 (m, 2H), 3,43 (m, 2H), 4,64 (t, 2H), 7,24 (AB, 1H), 7,47 (AB, 1H), 7,37 (s, 1H), 7,84 (d, 2H), 8,06 (d, 2H), 8,89 (m, 2H), 11,42 (m, 1 H).

Selon le schéma réactionnel C ou C' et de façon analogue à la procédure décrite pour la synthèse du dichlorhydrate de *N*,*N*-diisobutyl-1-[3-(néopentylamino)propyl]-2-[(3,4,5-triméthoxyphényl)amino]-1*H*-benzimidazole-6-carboxamide ou du dichlorhydrate de 2-[(4-acétylphényl)amino]-1-{3-[(cyclohexylméthyl) amino]propyl}-*N*,*N*-bis(3-méthyl butyl)-1*H*-benzimidazole-6-carboxamide, les composés suivants ont été préparés : 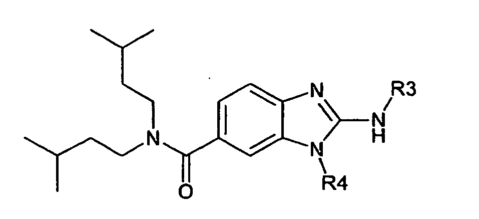 dans lesquels R₃ représente l'un des radicaux ci-après : 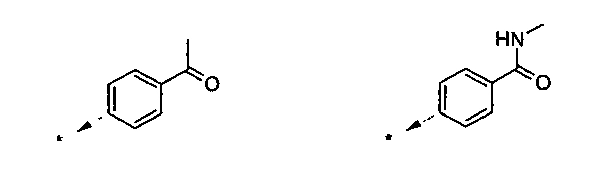 et R₄ représente l'un des radicaux ci-après :

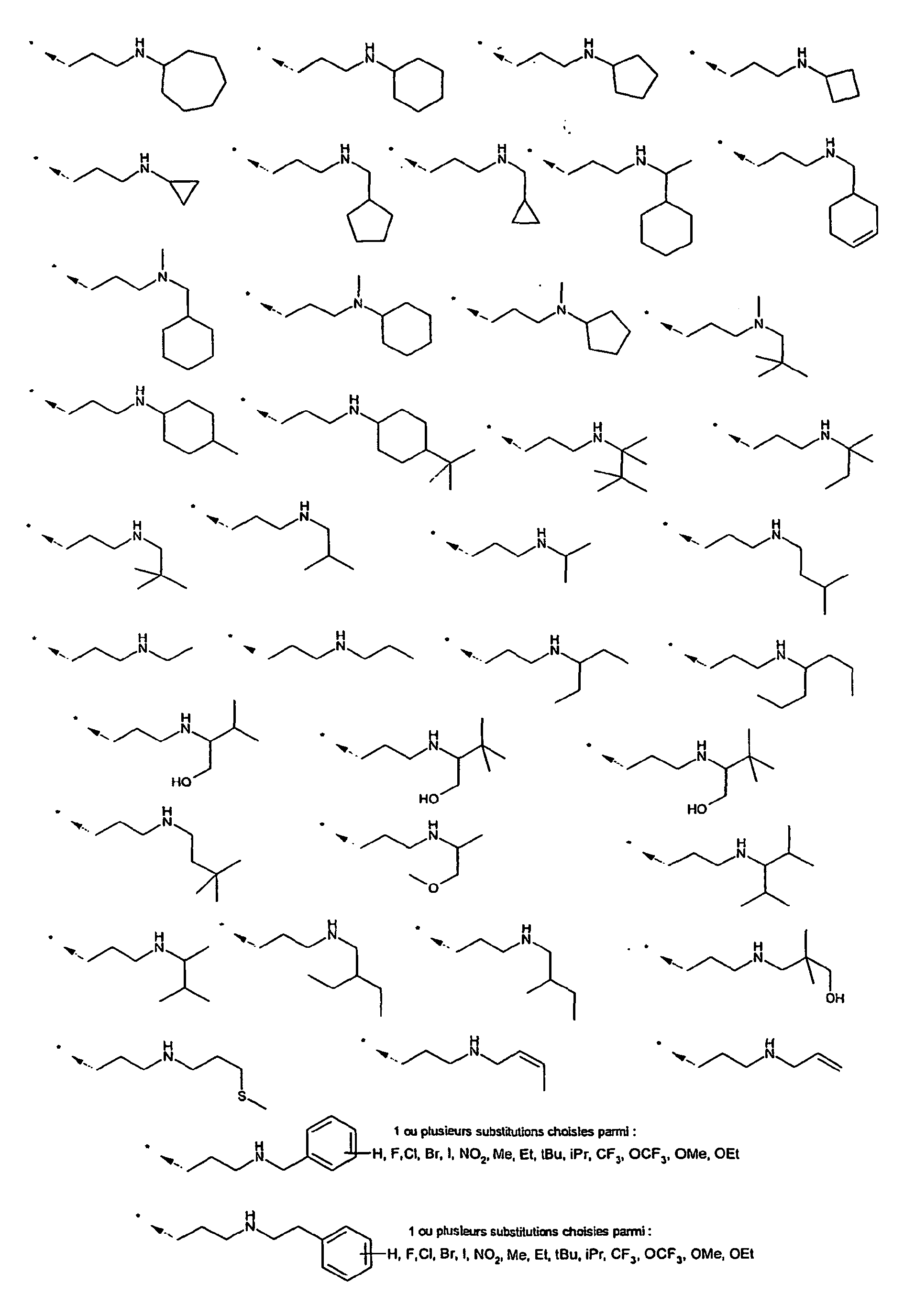

### D. Préparation selon le schéma réactionnel D :

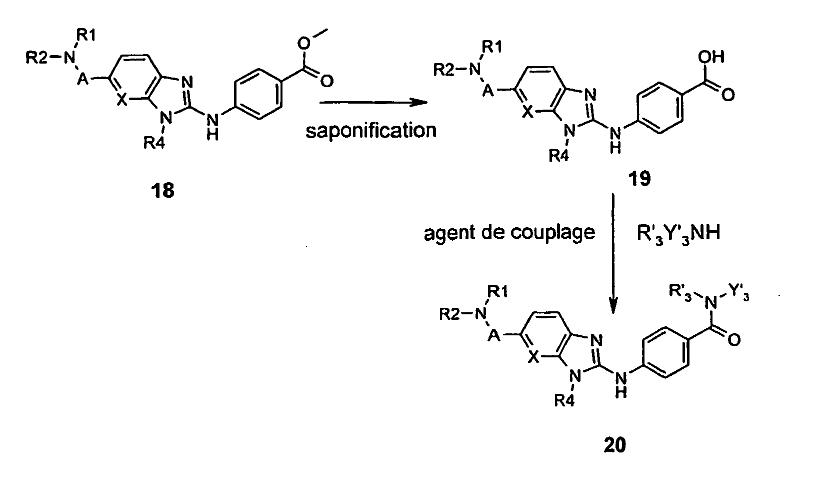

Comme décrit dans le schéma D, le dérivé (18) préparé selon le schéma réactionnel A, peut être saponifié en présence d'une base inorganique telle que l'hydroxyde de lithium dihydrate dans un mélange de solvants polaires tels que l'eau et le tétrahydrofuranne à une température de 20 à 70° C pendant 3 à 17 heures. L'acide carboxylique résultant (19) peut être couplé avec une amine primaire ou secondaire en présence d'un agent de couplage tel que le diisopropylcarbodiimide (DIC), le dicyclohexylcarbodiimide (DCC), le chlorhydrate de 1-(3-dimethylaminopropyl)-3-éthylcarbodiimide (EDC) ou le carbonyldiimidazole (CDI), avec ou sans du 1-hydroxybenzotriazole (HOBt) dans un solvant organique inerte tel que du chlorure de méthylène, tétrahydrofuranne ou diméthylformamide à température ambiante pendant 3 à 24 heures pour conduire au composé (20).

### Exemple D1 : dichlorhydrate de 1-(3-aminopropyl)-2-({4-[(méthylamino)carbonyl] phényl} amino)-N,N-bis(3-méthylbutyl)-1H-benzimidazole-6-carboxamide

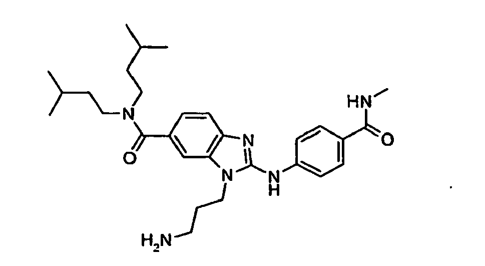

### Etape 1 : acide 4-[(6-{[bis(3-méthylbutyl)amino]carbonyl}-1-{3-[(tert-butoxycarbonyl) amino] propyl}-1H-benzimidazol-2-yl)amino]benzoique

Au 4-[(6- {[bis(3-méthylbutyl)amino]carbonyl}-1-{3-[(*tert*-butoxycarbonyl)amino] propyl}-1*H*-benzimidazol-2-yl)amino]benzoate de méthyle préparé selon le schéma réactionnel A, exemple A1, (405 mg, 1 eq) dans un mélange de tétrahydrofuranne (4 ml) et d'eau (3 ml) est additionné l'hydroxyde de lithium (141 mg, 5 eq). Le mélange est chauffé à reflux pendant 18 heures puis refroidi à température ambiante et concentré sous pression réduite à 40° C. Le résidu est additionné de dichlorométhane (50 ml) et d'eau (20 ml). Le mélange est acidifié par addition d'acide acétique jusqu'à pH 5. Après décantation et extractions, les phases organiques combinées sont séchées sur sulfate de sodium et concentrées sous pression réduite. Le solide obtenu est repris dans de l'éther éthylique pour donner le composé attendu (309 mg ; 79 %).
SM/CL : MM calculée= 593,8 ; m/z = 594,3 (MH+)
RMN (¹H, 400 MHz, DMSO-*d₆*) : 0,77 (m, 12H), 1,22-1,55 (m, 6H), 1,36 (s, 9H), 1,83 (m, 2H), 3,03 (m, 2H), 3,33 (m, 4H), 4,28 (m, 2H), 6,95- 7,90 (m, 8H), 9,24 (s, 1H).

### Etape 2: dichlorhydrate de 1-(3-aminopropyl)-2-({4-[(méthylamino)carbonyl] phényl}amino)-N,N-bis(3-méthylbutyl)-1H-benzimidazole-6-carboxamide

A l'acide 4-[(6-{[bis(3-méthylbutyl)amino]carbonyl}-1-{3-[(*tert*-butoxycarbonyl) amino]propyl}-1*H*-benzimidazol-2-yl)amino]benzoique (50 mg, 1 eq) dans le tétrahydrofuranne anhydre (1 ml) sont successivement additionnées une solution de chlorhydrate de l-(3-diméthylaminopropyl)-3-éthylcarbodiimide (EDC) (18 mg, 1,1 eq) dans le chloroforme (1 ml) et une solution de 1-hydroxybenzoh-iazole (HOBt) (13 mg, 1,1 eq) dans le tétrahydrofuranne (1 ml). Le mélange est agité 1 heure à une température voisine de 20° C puis la méthylamine est additionnée (2M dans le THF ; 0,86 ml, 2 eq). Après 17 heures d'agitation à une température voisine de 20° C, le mélange est dilué par du dichlorométhane (3 ml) et additionné de résine aminométhylpolystyrène (2 eq), résine TBD-méthyl polystyrène (2 eq) et résine méthylisothiocyanate-polystyrène (4 eq). Après 6 heures d'agitation à environ 20° C, le mélange est filtré et le filtrat est concentré sous pression réduite à 40° C. Le résidu obtenu est dissous dans du dichlorométhane (3 ml) et lavé avec une solution aqueuse saturée en hydrogénocarbonate de sodium. La phase organique est séchée sur sulfate de sodium et concentrée à une température voisine de 40° C. Le résidu obtenu est dissous dans l'acétate d'éthyle (0,5 ml) et additionné d'une solution d'acide chlorhydrique (4N dans le dioxanne, 2 ml). Après 1 heure d'agitation à une température voisine de 20° C, le précipité obtenu est filtré et séché pour donner le composé attendu (29 mg ; 60 % rendement).
SM/CL : MM calculée = 506,7 ; m/z = 507,2 (MH+)
RMN (¹H, 400 MHz, DMSO-*d₆*) : 0,78 (m, 12H), 1,46 (m, 6H), 2,0 (m, 2H), 2,77 (d, 3H), 2,89 (m, 2H), 3,33 (m, 4H), 4,45 (m, 2H), 7,07 (d, 1H), 7,43 (d, 1H), 7,48 (s, 1H), 7,84 (m, 5H), 7,97 (m, 2H), 8,28 (m, 2H), 9,49 (m, 1H).

Selon le schéma réactionnel D et de façon analogue à la procédure décrite pour la synthèse du dichlorhydrate de 1-(3-aminopropyl)-2-({4-[(méthylamino)carbonyl] phényl}amino)-*N,N*-bis(3-méthylbutyl)-1*H*-benzimidazole-6-carboxamide, les composés suivants ont été préparés 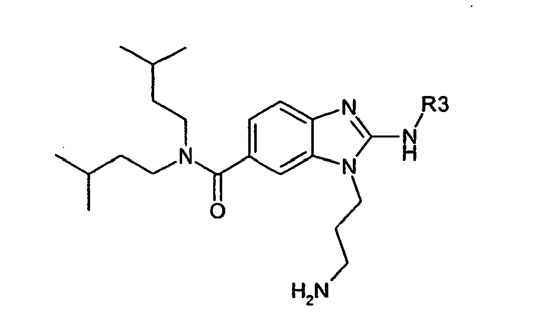 dans lesquels R₃ représente l'un des radicaux ci-après :

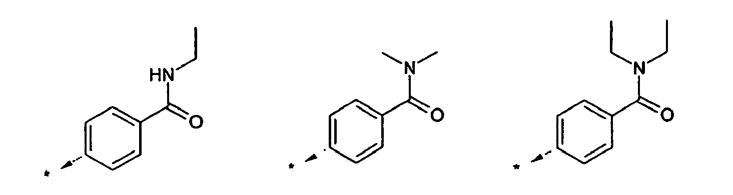

### E. Préparation selon le schéma réactionnel E :

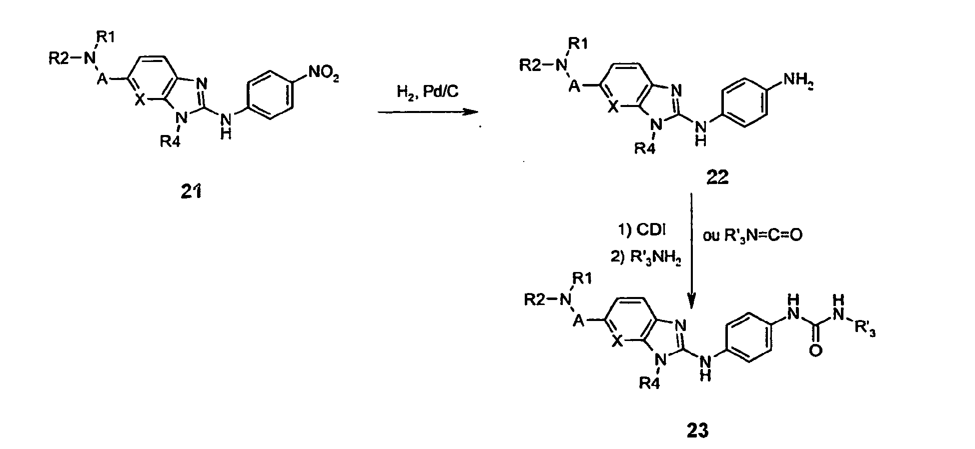

Comme décrit dans le schéma E, le dérivé (21) préparé selon le schéma réactionnel A, peut être réduit par traitement avec du chlorure d'étain dihydrate dans un solvant inerte tel que l'acétate d'éthyle ou le diméthylformamide, à une température de 60-80° C pendant 3 à 15 heures, ou par hydrogénation catalytique en présence de palladium sur charbon 10 % dans un solvant inerte tel que le méthanol, l'éthanol, l'acétate d'éthyle ou un mélange dé ces solvants, à une température de 18-25° C, pendant 2 à 8 heures, pour conduire à l'aniline (22). Le composé 23 peut-être traité par un isocyanate dans un solvant aprotique tel que le dichlorométhane ou le tetrahydrofuranne à une température de 20-60° C pendant 2 à 24 heures ou alternativement par du carbonyldiimidazole (CDI) dans un solvant aprotique tel que le dichlorométhane ou le tétrahydrofuranne à une température de 0-60° C pendant 6 à 24 heures, suivie d'une amine primaire à une température de 20-60° C pendant 2 à 24 heures, pour conduire à l'urée (23).

### Exemple E1 : 1-(3-aminopropyl)-2-[(4-{[(méthylamino)carbonyl]amino} phényl)amino]-N,N-bis(3-méthylbutyl)-1H-benzimidazole-6-carboxamide

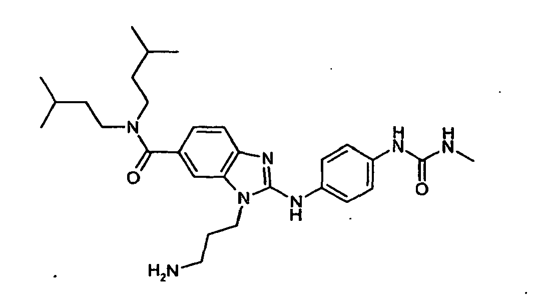

### Etape 1 : tert-butyl 3-{6-{[bis(3-méthylbutyl)amino]carbonyl}-2-[(4-nitrophényl) amino]-1H-benzimidazol-1-yl}propylcarbamate

A une solution de *tert*-butyl-3-[(2-amino-5-{[bis(3-méthylbutyl)amino]carbonyl} phényl)amino] propylcarbamate préparé selon l'exemple A1 (500 mg, 1 eq) dans le tétrahydrofuranne (30 ml) sont successivement additionnés le 4-nitrophényl isothiocyanate (305 mg, 1,5 eq) et la résine *N*-méthylcyclohexylcarbodiimide-*N-*méthyl-polystyrène (acquise auprès de Novabiochem ; charge 1,9 mmol/g; 1,75 g, 3 eq). Le mélange est chauffé à reflux pendant 18 heures puis refroidi à température ambiante et filtré sur fritté. Le filtrat est concentré sous pression réduite à 40° C. La purification du résidu par chromatographie éclair sur gel de silice (éluant : heptane / acétate d'éthyle 7 :3 à 2 :8) donne le composé attendu sous forme d'un solide jaune (584 mg ; 88 % rendement).
SM/CL : MM calculée = 594,7 ; m/z = 595,3 (MH+)
RMN (¹H, 400 MHz, DMSO-*d₆*) : δ 0,64-0,95 (m, 12H), 1,36 (s, 9H), 1,31-1,65 (m, 6H), 1,82 (m, 2H), 3,0 (m, 2H), 3,15-3,39 (m, 4H), 4,32 (t, 2H), 6,95 (m, 1H), 7,05 (d, 1H), 7,40 (s, 1H), 7,48 (d, 1H), 8,11 (AB, 2H), 8,26 (AB, 2H), 9,71 (s, 1H).

### Etape 2 : tert-butyl 3-(2-[(4-aminophényl)amino]-6-{[bis(3-méthylbutyl)amino] carbonyl}-1H-benzimidazol-1-yl)propylcarbamate

Dans un autoclave sont additionnés le *tert*-butyl 3-{6-{[bis (3-méthylbutyl)amino]carbonyl}-2-[(4-nitrophényl)amino]-1*H*-benzimidazol-1-yl} propylcarbamate (580 mg) en solution dans un mélange d'acétate d'éthyle / méthanol 3 :1 (40 ml), et le palladium sur charbon 10 % (58 mg). Après 15 heures d'agitation sous atmosphère d'hydrogène (3 bars) à une température d'environ 20° C, le catalyseur est éliminé par filtration sur célite et le filtrat est concentré sous pression réduite à 40° C pour donner le composé attendu sous forme d'une mousse (480 mg ; 87 % rendement).
SM/CL : MM calculée = 564,7 ; m/z = 565,3 (MH+)
RMN (¹H, 400 MHz, DMSO-*d₆*) : δ 0,86 (m, 12H), 1,37 (s, 9H), 1,31-1,58 (m, 6H), 1,79 (m, 2H), 3,01 (m, 2H), 3,15-3,39 (m, 4H), 4,15 (t, 2H), 4,80 (m, 2H), 6,56 (m, 2H), 6,94 (d, 2H), 7,21 (AB, 2H), 7,40 (AB, 2H), 8,45 (s, 1H).

### Etape 3 : dichlorhydrate de tert-butyl 3-{6-{[bis(3-méthylbutyl)amino]carbonyl}-2-[(4-{[(méthylamino)carbonyl]amino}phényl)amino]-1H-benzimidazol-1-yl} propyl carbamate

A une solution de *tert*-butyl 3-(2-[(4-aminophényl)amino]-6-{[bis (3-méthylbutyl)amino]carbonyl}- 1*H*-benzimidazol-1-yl)propylcarbamate (50 mg, 1 eq) dans le dichlorométhane (2 ml) est additionnée une solution de carbonyldiimidazole (CDI) (29 mg, 2 eq) dans le dichlorométhane (2 ml). Le mélange est agité 18 heures à une température voisine de 20° C puis la méthylamine est additionnée (2M dans le THF, 0,440 ml, 10 eq). Le mélange est agité 4 heures à à une température voisine de 20° C puis concentré sous pression réduite à 40° C. Le résidu est repris par du dichlorométhane (7 ml) et une solution aqueuse saturée en hydrogénocarbonate de sodium (3 ml). Après décantation et extractions les phases organiques réunies sont lavées avec de la saumure puis concentrées sous pression réduite à 40° C. La purification du résidu par chromatographie éclair sur gel de silice (éluant : heptane / acétate d'éthyle 1 :1 à 1 :9) donne un composé sous forme de mousse qui est dissous dans l'acétate d'éthyle (0,5 ml). Une solution d'acide chlorhydrique (2N dans l'éther diéthylique, 2 ml) est additionnée et le mélange est agité 1 heure à une température voisine de 20° C puis le précipité obtenu est filtré et séché pour donner le composé attendu (28 mg, 55 % rendement).
SM/CL : MM calculée = 521,7 ; m/z = 522,3 (MH+)

Selon le schéma réactionnel E et de façon analogue à la procédure décrite pour la synthèse du dichlorhydrate de *tert*-butyl 3-{6-{[bis(3-méthylbutyl)amino]carbonyl}-2-[(4-{[(méthylamino)carbonyl]amino} phényl)amino]-1*H*-benzimidazol-1-yl} propyl carbamate, les composés suivants ont été préparés : 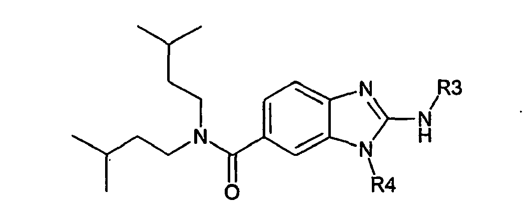 dans lesquels R₃ représente l'un des radicaux ci-après : 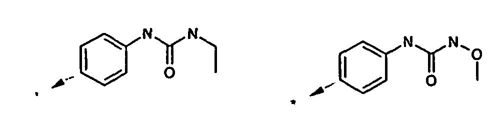 et R₄ représente l'un des radicaux ci-après :

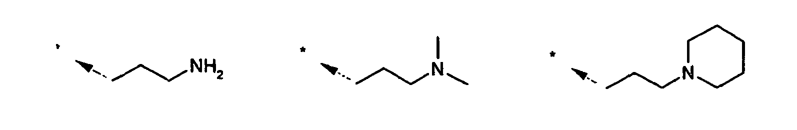

### F. Préparation selon le schéma réactionnel F :

Les composés de formule (I) selon l'invention dans laquelle A représente -CH₂-, peuvent être préparés selon les schémas F et F' suivants :

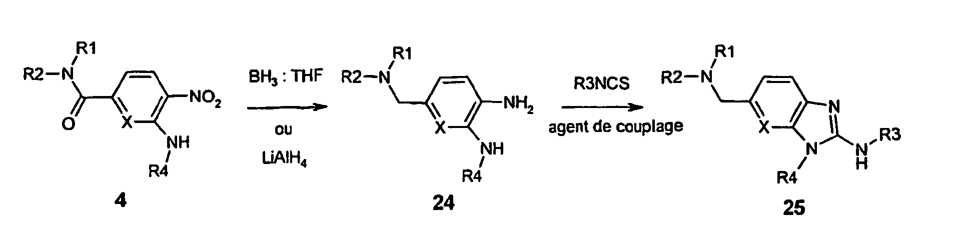

Comme décrit dans le schéma F, le dérivé (4) préparé selon le schéma réactionnel A, peut être réduit en composé (24) à l'aide de borane ou d'hydrure de lithium aluminium dans un solvant aprotique tel que le tétrahydrofuranne ou l'éther diéthylique à une température de 0 à 70° C, pendant 18 à 24 heures. La dianiline (24) peut être ensuite traitée par un isothiocyanate en présence d'un agent de couplage supporté sur une résine ou non tel que le diisopropylcarbodiimide ou dicyclohexylcarbodiimide ou la résine *N*-méthylcyclohexylcarbodimide *N*-méthyl polystyrène dans un solvant inerte tel que le tétrahydrofuranne, chlorure de méthylène, ou chloroforme à une température de 20-70°C pendant 2 à 72 heures pour conduire au dérivé (25).

### Préparation selon le schéma réactionnel F' :

Les composés (25) peuvent également être préparés selon le schéma F' suivant :

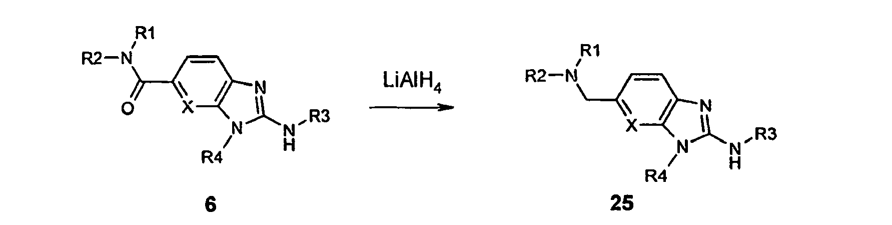

Comme décrit dans le schéma F', l'amide (6) préparée selon les schémas réactionnels A ou B, peut être réduite en amine correspondante (25) à l'aide de borane ou d'hydrure de lithium aluminium dans un solvant aprotique tel que le tétrahydrofuranne ou l'éther diéthylique à une température de 0 à 70° C, pendant 1 à 6 heures.

### Exemple F'1: chlorhydrate de 6-{[bis(3-méthylbutyl)amino]méthyl}-1-[3-(diméthylamino) propyl]-N-(4-méthoxyphényl)-1H-benzimidazol-2-amine

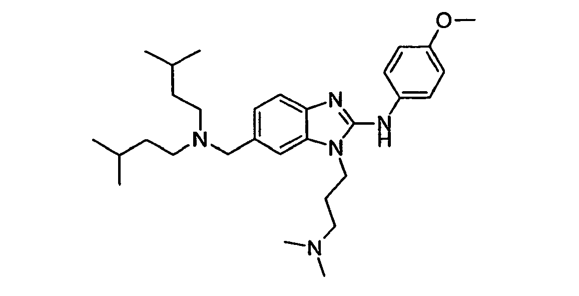

A une solution refroidie à 0° C de 1-[3-(diméthylamino)propyl]-2-[(4-méthoxyphényl) amino]-*N,N*-bis(3-méthylbutyl)-1*H*-benzimidazole-6-carboxamide préparé selon le schéma réactionnel A (80 mg, 1 eq) dans le tétrahydrofuranne anhydre (2 ml), est additionnée goutte à goutte une solution d'hydrure de lithium aluminium (0,785 ml ;1 M dans le THF). Le mélange est ramené à une température de 20° C puis chauffé à 60° C pendant 3 heures. Après refroidissement à 0° C, le milieu réactionnel est hydrolysé. Après addition d'acétate d'éthyle, décantation et extractions, les phases organiques réunies sont lavées avec de la saumure, séchées sur sulfate de sodium et concentrées sous pression réduite. La purification par chromatograhie éclair sur gel de silice (éluant : dichlorométhane 100 % à dichlorométhane/méthanol 9 :1) donne le composé attendu sous forme de base. Le sel de chlorhydrate correspondant est formé par addition d'une solution d'acide chlorhydrique 1N dans l'éther diéthylique (61 mg ; 55 % rendement).
SM/CL : MM calculée = 493,7 ; m/z = 494,4 (MH+)
RMN (¹H, 400 MHz, DMSO-*d₆*) : δ 0, 83 (m, 12H), 1,50-1,72 (m, 6H), 2,29 (m, 2H), 2,78 (m, 6H), 2,99 (m, 4H), 3,30 (m, 2H), 3,80 (s, 3H), 4,41 (m, 4H), 6,90-8,50 (m, 7H), 10,5 (m, 1H), 10,85 (m, 1H), 12,9 (m, 1H).

Les composés suivants ont été préparés selon les schémas réactionnels F ou F' : 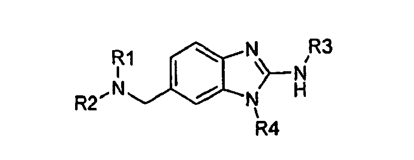 dans lesquels R₁R₂N représente l'un des radicaux ci-après : 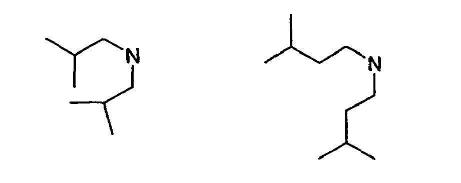 dans lesquels R₃ représente l'un des radicaux ci-après : 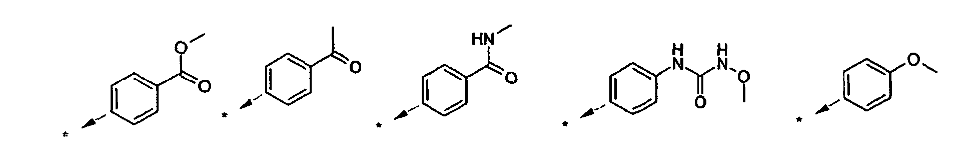 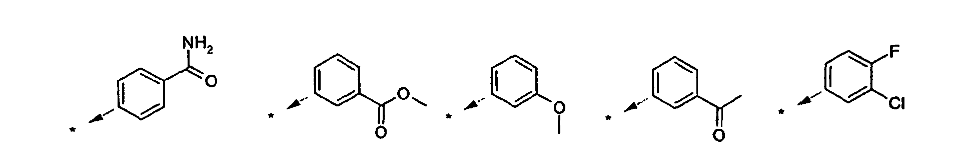 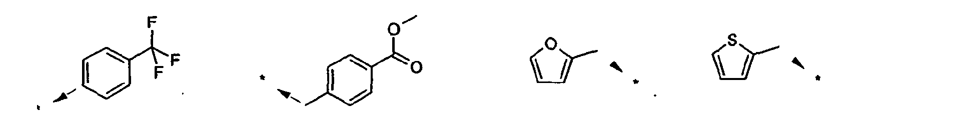 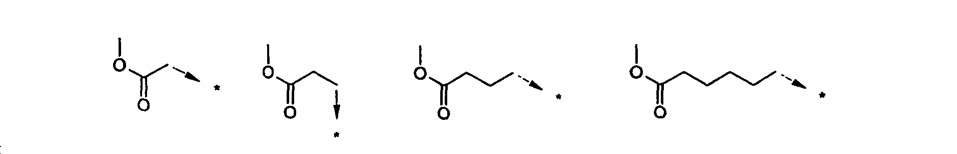 et R₄ représente l'un des radicaux ci-après :

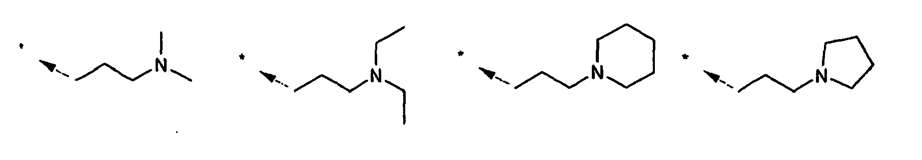

### G. Préparation selon le schéma réactionnel G :

Les composés de formule (I) selon l'invention dans laquelle A représente -C(O)-C(Rₐ)(R_{b})-, peuvent être préparés selon le schéma G suivant :

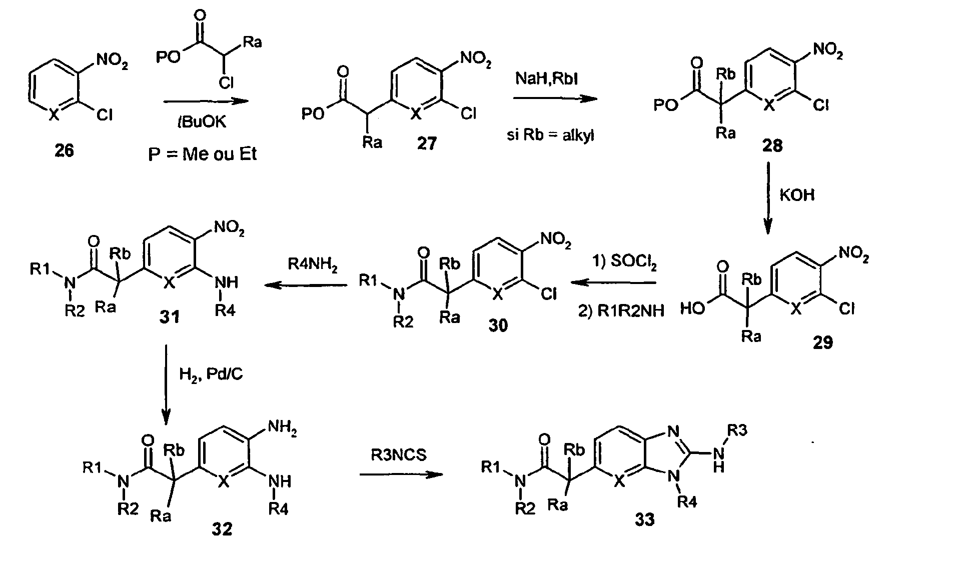

Comme décrit dans le schéma G, le dérivé (26) peut être alkylé en présence d'une base forte telle que le *tert*butylate de potassium, par un dérivé α-chloroester, dans un solvant aprotique polaire tel que le diméthylformamide à une température de 0-20° C pendant 0,5-2 heures, pour conduire au composé (27). Le dérivé (27) peut-être éventuellement alkylé en présence d'une base forte telle que l'hydrure de sodium et d'un agent alkylant tel qu'un iodure d'alkyle dans un solvant aprotique tel que le diméthylformamide à une température de 0-20° C pendant 1-4 heures, pour conduire au composé (28). L'ester (28) peut-être saponifié en présence d'une base inorganique telle que l'hydroxyde de lithium ou potassium dans un mélange de solvants polaires tels que l'eau et le méthanol à une température de 20-80° C pendant 1-6 heures. L'acide carboxylique résultant (29) peut être couplé avec une amine primaire ou secondaire en présence d'un agent de couplage tel que le diisopropylcarbodiimide (DIC), le dicyclohexylcarbodiimide (DCC), le chlorhydrate de 1-(3-dimethylaminopropyl)-3-éthylcarbodümide (EDC) ou le carbonyldiimidazole (CDI), avec ou sans du 1-hydroxybenzotriazole (HOBt) dans un solvant organique inerte tel que du chlorure de méthylène, tétrahydrofuranne ou diméthylformamide à température ambiante pendant 3 à 24 heures. Alternativement l'acide (29) peut-être traité avec du chlorure de thionyle ou d'oxalyle dans un solvant aprotique tel que le dichlorométhane ou toluène à une température de 40-60° C pendant 2-16 heures puis le chlorure d'acide ainsi obtenu peut réagir avec une amine primaire ou secondaire, en présence d'une base tertiaire telle que la triéthylamine, la diisopropyléthylamine dans un solvant aprotique tel que le dichlorométhane ou tétrahydrofuranne à une température de 0-20° C pendant 0,5-4 heures pour conduire à l'amide (30). Le traitement du dérivé fluoré ou chloré (30) par une amine primaire en présence d'une base inorganique telle que du carbonate de césium ou de potassium dans un solvant organique inerte tel que le diméthylformamide ou l'acétonitrile à une température de 20-100° C pendant 2 à 48 heures conduit au dérivé (31). La fonction nitro du composé (31) est réduite par traitement avec du chlorure d'étain dihydrate dans un solvant inerte tel que l'acétate d'éthyle ou le diméthylformamide à une température de 60-80° C pendant 3 à 15 heures, ou par hydrogénation catalytique en présence de palladium sur charbon 10 % dans un solvant inerte tel que le méthanol, éthanol, acétate d'éthyle ou un mélange de ces solvants, à une température de 18-25° C, pendant 2 à 8 heures pour conduire à la dianiline (32). Le dérivé (32) est ensuite traité par un isothiocyanate en présence d'un agent de couplage supporté sur une résine ou non tel que le diisopropylcarbodiimide ou dicyclohexylcarbodiimide ou la résine *N*-méthylcyclohexylcarbodiimide *N-*méthyl polystyrène dans un solvant inerte tel que le tétrahydrofuranne, chlorure de méthylène, ou chloroforme à une température de 20-70° C pendant 2 à 72 heures pour conduire au dérivé (33). Alternativement, le dérivé (32) peut être traité par un isothiocyanate dans un solvant inerte tel que le tétrahydrofuranne, chlorure de méthylène, chloroforme ou éthanol à une température de 20-80° C pendant 1-16 heures puis la thiourée résultante peut être traitée par de l'iodure de méthyle ou de l'oxyde de mercure (II) jaune en présence d'une quantité catalytique de soufre dans un solvant polaire tel que le méthanol ou l'éthanol pendant 2 à 24 heures à une température de 20-80° C pour conduire à (33). Le composé (6) peut être isolé soit par chromatographie éclair sur gel de silice, soit par addition au mélange réactionnel d'un réactif nucléophile supporté sur un polymère comme par exemple une résine aminométhylpolystyrène et / ou d'un réactif électrophile supporté sur un polymère comme par exemple la résine méthylisothiocyanate-polystyrène, suivie d'une filtration et de l'évaporation du filtrat.

### Exemple G1 : dichlorhydrate de 2-{2-[(4-acétylphényl)amino]-1-[3-(diméthylamino) propyl]-1H-benzimidazol-6-yl}-N,N-diisobutyl-2-méthylpropanamide

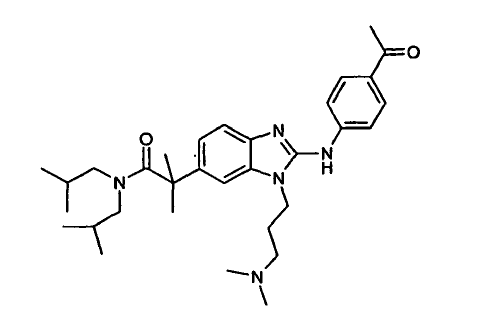

### Etape 1 : 2-(3-chloro-4-nitrophényl)propanoate d'éthyle

A une solution de DMF (80 ml) refroidie à 0° C, est additionné le *tert*-butylate de potassium (11,22 g, 2 eq). Une solution de 1-chloro-2-nitrobenzene (7,87 g, 1 eq) et de 2-chloropropanoate d'éthyle (7 ml, 1,1 eq) est additionnée goutte à goutte en 45 min au mélange en maintenant la température réactionnelle inférieure à 5° C. A la fin de l'addition, l'agitation est maintenue 2 heures à 0° C puis le mélange est hydrolysé à cette température par une solution d'acide chlorhydrique IN et additionné d'acétate d'éthyle. Après décantation et extractions, les phases organiques réunies sont lavées avec de la saumure, séchées sur Na₂SO₄ et concentrées sous pression réduite. La purification par chromatograhie éclair sur gel de silice (éluant : heptane / dichlorométhane 8 :2 à 6 :4) donne le composé attendu sous forme d'une huile jaune (8,28 g ; 64 % rendement).
RMN (¹H, 400 MHz, DMSO-*d₆*) : δ 1,14 (t, 3H), 1,42 (d, 3H), 3,99 (q, 1H), 4,08 (m, 2H), 7,52 (AB,1H), 7,71 (s,1H), 8,05 (AB, 1H).

### Etape 2 : 2-(3-chloro-4-nitrophényl)-2-méthylpropanoate d'éthyle

A une suspension d'hydrure de sodium (60 % dans l'huile, 2,4 g, 1,1 eq) dans le DMF (15 ml), refroidie à 0° C, est additionnée goutte à goutte une solution de 2-(3-chloro-4-nitrophényl)propanoate d'éthyle (14,1 g). Après 1 heure d'agitation à cette température, une solution d'iodure de méthyle (3,72 ml, 1,1 eq) dans le DMF (40 ml) est additionnée goutte à goutte au mélange. L'agitation est poursuivie 3 heures à température ambiante. Le milieu réactionnel est refroidi à 0° C puis additionné d'acétate d'éthyle, d'eau saturée en hydrogénocarbonate de sodium goutte à goutte, puis d'eau. Après décantation et extractions, les phases organiques réunies sont lavées avec de la saumure, séchées sur Na₂SO₄ et concentrées sous pression réduite pour donner le composé attendu sous forme d'une huile qui cristallise. Les cristaux sont lavés à l'heptane et séchés (13,8g ; 94 % rendement).
RMN (¹H, 400 MHz, DMSO-*d₆*) : δ 1,12 (t, 3H), 1,54 (s, 6H), 4,09 (q, 1H), 7,50 (AB, 1H), 7,66 (s, 1H), 8,04 (AB, 1H).

### Etape 3 : acide 2-(3-chloro-4-nitrophényl)-2-méthylpropanoique

A une solution de 2-(3-chloro-4-nitrophényl)-2-méthylpropanoate d'éthyle (1 g) dans le méthanol (20 ml) est additionnée à une température voisine de 20° C, une solution d'hydroxyde de potassium 2N (18 ml). Le mélange est ensuite chauffé à 80° C pendant 1,5 heures puis refroidi à température ambiante. Le méthanol est évaporé par concentration du mélange sous pression réduite. La phase aqueuse restante est lavée au dichlorométhane puis refroidie à 0° C et acidifiée par de l'acide acétique. Après addition de dichlorométhane, décantation et extractions, les phases organiques réunies sont lavées avec de la saumure, séchées sur Na₂SO₄ et concentrées sous pression réduite pour donner le composé attendu sous forme d'une huile qui cristallise (852 mg, 95 % rendement).
RMN (¹H, 400 MHz, DMSO-*d₆*) : δ 1,52 (s, 6H), 7,53 (AB, 1H), 7,66 (s, 1H), 8,04 (AB, 1H), 12,72 (s, 1H).

### Etape 4 : 2-(3-chloro-4-nitrophényl)-N,N-diisobutyl-2-méthylpropanamide

A une solution d'acide 2-(3-chloro-4-nitrophényl)-2-méthylpropanoique (500 mg) dans le dichlorométhane (1 ml) est additionné le chlorure de thionyle (0,54 ml, 4 eq). Le mélange est chauffé au reflux pendant 16 heures puis refroidi à température ambiante. Le solvant est évaporé sous pression réduite à 40° C (co-évaporation avec du toluène). A une solution du chlorure d'acide ainsi obtenu dans le dichlorométhane (1 ml), refroidie à 0° C, sont successivement additionnées la diisopropyléthylamine (0,42 ml, 1,2 eq) et la diisobutylamine (0,36 ml, 1 eq). A la fin de l'addition, l'agitation est poursuivie 3 heures à température ambiante puis le mélange est concentré sous pression réduite à 40° C. Le résidu est dissous dans l'éther éthylique et la phase organique est lavée successivement avec de la soude IN, une solution saturée en hydrogénocarbonate de sodium, de la saumure puis séchée sur Na₂SO₄ et concentrée sous pression réduite à 40° C. La purification par chromatographie éclair sur gel de silice (éluant : heptane acétate d'éthyle 8 :2 à 7 :3) donne le composé attendu sous forme d'une huile qui cristallise (0,585g ; 82 % rendement).
SM/CL : MM calculée = 354,9 ; m/z = 355,2 (MH+)
RMN (¹H, 400 MHz, CDCl₃) : δ 0,58 (d, 6H), 0,90 (d, 6H), 1,58 (m, 6H), 1,74 (m, 1H), 1,95 (m, 1H), 2,65 (d, 2H), 3,27 (d, 2H), 7,30 (AB, 1H), 7,44 (s,1H), 7,91 (AB, 1H).

### Etape 5 : 2-(3-{[3-(diméthylamino)propyl]amino}-4-nitrophényl)-N,N düsobutyl-2-méthylpropanamide

Un mélange de 2-(3-chloro-4-nitrophényl)-*N*,*N* diisobutyl-2-méthylpropanamide (78 mg, 1 eq), de 3-diméthylaminopropylamine (45 mg, 2 eq) et de carbonate de potassium (62 mg, 2 eq) dans le DMF (2 ml) est chauffé au reflux pendant 3 heures puis refroidi à température ambiante. Le résidu est repris par de l'acétate d'éthyle (20 ml) et de l'eau (8 ml). Après décantation et extractions, les phases organiques combinées sont lavées avec de la saumure, séchées sur Na₂SO₄ puis concentrées sous pression réduite à 40° C. La purification du résidu par chromatographie éclair sur gel de silice (éluant : dichlorométhane 100 % à dichlorométhane/méthanol 8 :2 ) donne le composé attendu sous forme d'une huile jaune (44 mg ; 48 % rendement).
SM/CL : MM calculée = 420,6 ; m/z = 421,3 (MH+)
RMN (¹H, 400 MHz, CDCl₃) : δ 0,60 (d, 6H), 0,90 (d, 6H), 1,57 (m, 6H), 1,75 (m, 1H), 1,88 (m, 2H), 1,97 (m, 1H), 2,28 (s, 6H), 2,45 (t, 1H), 2,75 (d, 2H), 3,26 (d, 2H), 3,34 (m, 2H), 6,57 (m, 1H), 6,68 (s, 1H), 8,15 (m, 1H), 8,49 (m, 1H).

### Etape 6 : 2-(4-amino-3-{[3-(diméthylamino)propyl]amino}phényl)-N,N-diisobutyl-2-méthylpropanamide

Dans un autoclave sont additionnés le 2-(3-{[3-(diméthylamino)propyl]amino}-4-nitrophényl)-*N*,*N*-diisobutyl-2-méthylpropanamide (44 mg) en solution dans un mélange d'acétate d'éthyle/ éthanol 2 :1 (3 ml), et le palladium sur charbon 10 % (5 mg). Après 4 heures d'agitation sous atmosphère d'hydrogène (3 bars) à une température d'environ 20° C, le catalyseur est éliminé par filtration sur célite et le filtrat est concentré sous pression réduite à 40° C pour donner le composé attendu sous forme d'une huile (39 mg ; 95 % rendement).
SM/CL : MM calculée = 390,6 ; m/z = 391,3 (MH+)
RMN (¹H, 400 MHz, DMSO-*d₆*) : δ0,49 (m, 6H), 0,81 (m, 6H), 1,36 (s, 6H), 1,65 (m, 1H), 1,72 (m, 2H), 1,87 (m, 1H), 2,20 (s, 6H), 2,39 (t, 2H), 2,81 (m, 2H), 2,97 (t, 2H), 3,11 (m, 2H), 4,56 (m, 2H), 6,18 (s, 1H), 6,30 (AB,1H), 6,48 (AB, 1H).

### Etape 7 : dichlorhydrate de 2-{2-[(4-acétylphényl)amino]-1-[3-(diméthylamino)propyl]-1H-benzimidazol-6-yl}-N,N-diisobutyl-2-méthylpropanamide

A une solution de 2-(4-amino-3-{[3-(diméthylamino)propyl]amino}phényl)-*N*,*N-*diisobutyl-2-méthylpropanamide (39 mg, 1 eq) dans le tétrahydrofuranne (2 ml) sont successivement additionnés le 4-acétylphényl isothiocyanate (14 mg, 1,2 eq) et la résine *N*-méthylcyclohexylcarbodiimide-*N*-méthyl-polystyrène (acquise auprès de Novabiochem ; charge 1,9 mmol/g ; 210 mg, 4 eq). Le mélange est chauffé à reflux pendant 17 heures puis refroidi à température ambiante et filtré. Le filtrat est concentré sous pression réduite à 40° C. La purification du résidu par chromatographie éclair sur gel de silice (éluant : dichlorométhane 100 % à dichlorométhane/méthanol 9:1) donne le composé attendu sous forme de base (409 mg ; 60 % rendement). Le sel de chlorhydrate correspondant est formé par addition d'une solution d'acide chlorhydrique 1N dans l'éther. Le précipité obtenu est filtré et séché pour donner le composé dichlorhydrate attendu (51 mg, 85 % rendement).
SM/CL : MM calculée = 533,7 ; m/z = 534,4 (MH+)
RMN (¹H, 400 MHz, DMSO-*d₆*): δ0,40 (m, 6H), 0,82 (m, 6H), 1,53 (s, 6H), 1,64 (m, 1H), 1,89 (m, 1H), 2,21 (m, 2H), 2,59 (s, 3H), 2,75 (m, 8H), 3,15 (m, 2H), 3,25 (m, 2H), 4,60 (t, 2H), 7,10 (AB, 1H), 7,41 (AB, 1H), 7,56 (s, 1H), 7,82 (m, 2H), 8,05 (m, 2H), 10,79 (m, 1H), 11,4 (m, 1H).

Selon le schéma réactionnel G, les composés suivants ont été préparés : 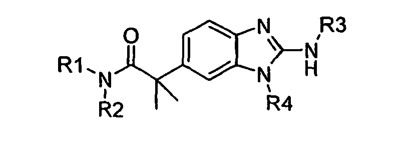 dans lesquels R₁R₂N représente l'un des radicaux ci-après : 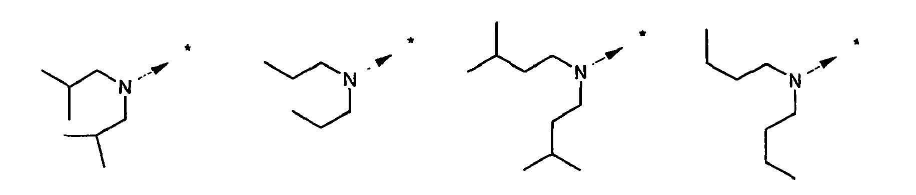 R₃ représente l'un des radicaux ci-après : 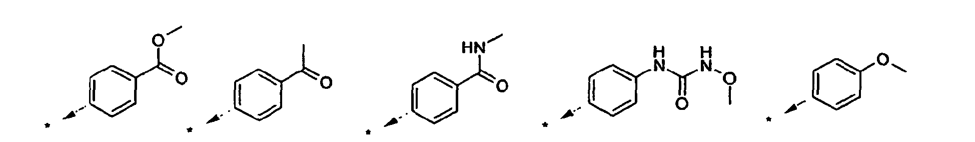 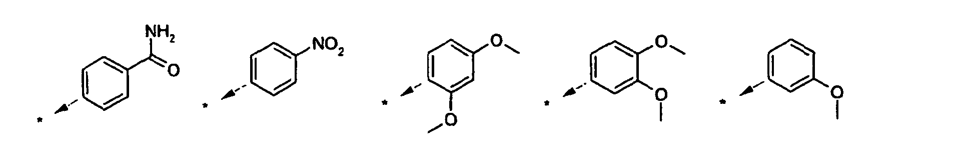 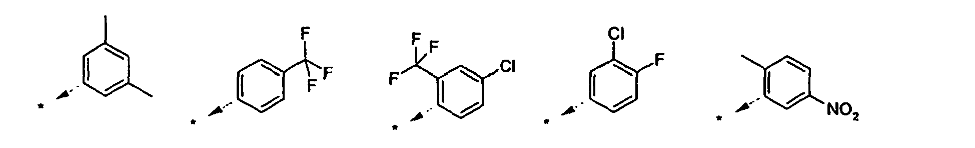 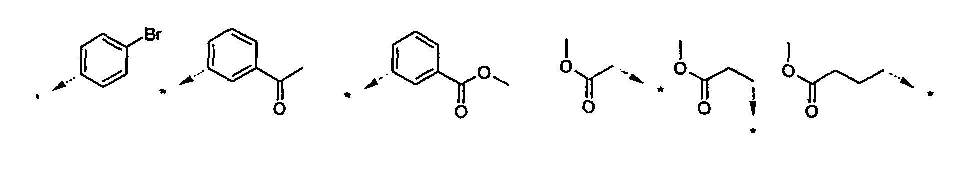 et R₄ représente l'un des radicaux ci-après :

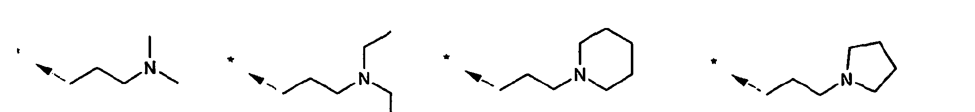

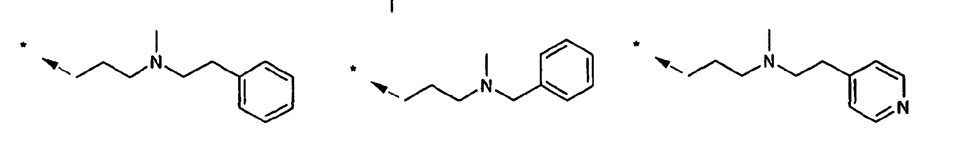

L'invention a également pour objet un procédé de préparation d'un composé de formule (I) tel que défini ci-dessus, caractérisé en ce que l'on traite le composé de formule générale : 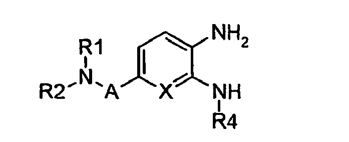 dans laquelle A, X, R₁, R₂, R₄ ont la signification indiquée ci-dessus, par un isothiocyanate de formule générale R₃N=C=S dans lequel R₃ a la signification indiquée ci-dessus, en présence d'un agent de couplage ou d'oxyde de mercure (II) jaune en présence de soufre, pendant une durée de 3 à 48 heures, dans un solvant protique ou aprotique, à une température de 50 à 80°C.

L'agent de couplage peut être supporté tel que la résine N-méthylcyclohexylcarbodiimide N-méthyl polystyrène ou non supporté tel que diisopropylcarbodiimide, diéthylcarbodiimide ou dicyclohexylcarbodiimide. On peut utiliser un solvant protique tel que le méthanol ou l'éthanol ou aprotique tel que le tétrahydrofuranne ou l'acétonitrile.

Les composés 1 de la présente invention possèdent d'intéressantes propriétés pharmacologiques. C'est ainsi que l'on a découvert que les composés I de la présente invention possèdent une bonne affinité pour certains sous-types de récepteurs des mélanocortines, en particulier des récepteurs MC4.

Les composés de la présente invention peuvent ainsi être utilisés dans différentes applications thérapeutiques. Ils peuvent avantageusement être utilisés pour traiter les états pathologiques ou les maladies dans lesquels un ou plusieurs récepteurs des mélanocortines sont impliqués tels que les états inflammatoires, les désordres pondéraux (l'obésité, la cachexie, l'anorexie), les désordres de l'activité sexuelle (les troubles de l'érection), la douleur, mais également les troubles mentaux (l'anxiété, la dépression), les toxicomanies, les maladies de la peau (l'acné, les dermatoses, les mélanomes). On trouvera ci-après, dans la partie expérimentale, une illustration des propriétés pharmacologiques des composés de l'invention.

La présente demande a également pour objet, des compositions pharmaceutiques contenant, à titre de principe actif, au moins un produit de formule I telle que définie ci-dessus, ainsi que les sels pharmaceutiquement acceptables dudit produit de formule I, en association avec un support pharmaceutiquement acceptable.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", Int. J. Pharm. (1986), 33, 201-217.

La présente demande a également pour objet l'utilisation des composés selon la présente invention, pour la préparation d'un médicament pour le traitement des désordres pondéraux tels que l'obésité la cachexie et plus particulièrement la cachexie des pathologies cancéreuses, cachexie du sida, cachexie de la personne âgée, cachexie cardiaque, cachexie rénale, cachexie de l'arthrite rhumatoïde, et l'anorexie, le traitement de la douleur et plus particulièrement la douleur neuropathique, des troubles mentaux tels que l'anxiété et la dépression, des désordres de l'activité sexuelle tels que les troubles de l'érection.

La composition pharmaceutique peut être sous forme d'un solide, par exemple, des poudres, des granules, des comprimés, des gélules ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

La présente invention a également pour objet l'utilisation d'un composé de formule générale **(I')** 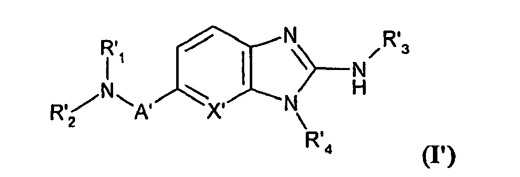 sous forme racémique, d'énantiomère ou toutes combinaisons de ces formes et dans laquelle :
A' représente -CH₂-,-C(O)-, -C(O)-C(Rₐ)(R_{b})-;
X' représente -CH- ;
Rₐ et R_{b} représentent, indépendamment, l'atome d'hydrogène ou un radical (C₁-C₆)alkyle ;
R'₁ représente l'atome d'hydrogène ; un radical (C₁-C₈)alkyle éventuellement substitué par hydroxy ou un ou plusieurs radicaux halo identiques ou différents ; (C₂-C₆)alkenyle ; ou un radical de formule -(CH₂)ₙ-X₁ ;
R'₂ représente un radical (C₁-C₈)alkyle éventuellement substitué par hydroxy ou un ou plusieurs radicaux halo identiques ou différents ; (C₂-C₆)alkenyle ; ou un radical de formule -(CH₂)ₙ-X₁ ;
chaque X₁ représente indépendamment (C₁-C₆)alkoxy, (C₃-C₇)cycloalkyle, adamantyle, hétérocycloalkyle, aryle ou hétéroaryle,
   les radicaux (C₃-C₇)cycloalkyle, hétérocycloalkyle, aryle et hétéroaryle étant éventuellement substitués par un ou plusieurs substituants identiques ou différents choisis parmi : -(CH₂)_{n'}-V₁-Y₁, halo, nitro, cyano et aryle ;
   V₁ représente -O-, -S- ou une liaison covalente ;
   Y₁ représente un radical (C₁-C₆)alkyle éventuellement substitué par un ou plusieurs radicaux halo identiques ou différents ;
   n représente un entier de 0 à 6 et n' un entier de 0 à 2 (étant entendu que lorsque n est égal à 0, alors X₁ ne représente pas le radical alkoxy)
ou bien R₁ et R₂ forment ensemble, avec l'atome d'azote auquel ils sont rattachés, un hétérobicycloalkyle ou un hétérocycloalkyle éventuellement substitué par un ou plusieurs substituants identiques ou différents choisis parmi : hydroxy, (C₁-C₆)alkyle éventuellement substitué par hydroxy, (C₁-C₆)alkoxy-carbonyle, hétérocycloalkyle et - C(O)NV₁'Y₁' avec V₁' et Y₁' représentant indépendamment l'atome d'hydrogène ou un (C₁-C₆)alkyle ; ou bien R₁ et R₂ forment ensemble un radical de formule : 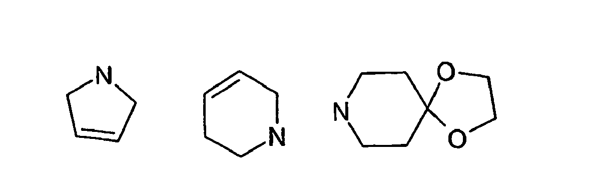
R'₃ représente -Z₃, C(R_{Z3})(R'_{Z3})-Z₃, -C(R_{Z3})(R'₃)-(CH₂)ₚ-Z₃ ou -C(O)-Z'₃
   R_{Z3} et R'_{Z3} représentent, indépendamment, l'atome d'hydrogène ou un radical (C₁-C₆)alkyle ;
   Z₃ représente Z₃ₐ, Z_{3b}, Z_{3c}, Z_{3d}, ou Z₃ₑ;
   Z₃ₐ représente un radical (C₁-C₆)alkyle ;
   Z_{3b} représente un radical (C₁-C₆)alkoxy, (C₁-C₆)alkylthio, (C₁-C₆)alkylarnino ou di((C₁-C₆)alkyl)amino ;
   Z_{3c} représente un radical aryle ou hétéroaryle;
   Z_{3d} représente un radical (C₁-C₆)alkoxy-carbonyle, amino-carbonyle, (C₁-C₆)alkylamino-carbonyle, di((C₁-C₆)alkyl)amino-carbonyle, (C₁-C₆)alkyl-C(O)-NH- , (C₃-C₇)cycloalkyle, hétérocycloalkyle ;
   les radicaux (C₃-C₇) cycloalkyle et hétérocycloalkyle étant éventuellement substitués par un ou plusieurs substituants identiques ou différents choisis parmi : halo, nitro, (C₁-C₆)alkoxy éventuellement substitué par un ou plusieurs radicaux halo identiques ou différents, (C₁-C₆)alkyle éventuellement susbtitué par un ou plusieurs radicaux halo identiques ou différents, (C₁-C₆)alkyl-carbonyle, (C₁-C₆)alkoxy-carbonyle, amino-carbonyle, (C₁-C₆)alkylamino-carbonyle, di((C₁-C₆)alkyl)amino-carbonyle et oxy,
   les radicaux aryle et hétéroaryle étant éventuellement substitués par un ou plusieurs substituants identiques ou différents choisis parmi : halo, cyano, nitro, azido, oxy, (C₁-C₆)alkoxy-carbonyl-(C₁-C₆)alkenyle, (C₁-C₆)alkylamino-carbonyl-(C₁-C₆)alkenyle, -SO₂NR₃₁R₃₂, hétérocycloalkyle, hétéroaryle ou -(CH₂)_{p'}-V₃-Y₃;
   R₃₁ et R₃₂ forment ensemble, avec l'atome d'azote sur lequel ils sont rattachés, un hétérocycloalkyle ;
   V₃ représente -O-, -S-, -C(O)-, -C(O)-O-, -O-C(O)-, -SO₂-, -SO₂ -NR'₃-SO₂-, -NR'₃-, -NR'₃-C(O)-, -C(O)-NR'₃-, -NH-C(O)-NR'₃- ou une liaison covalente ;
   Y₃ représente l'atome d'hydrogène ; un radical (C₁-C₆)alkyle éventuellement substitué par un ou plusieurs radicaux halo identiques ou différents; un radical aryle éventuellement substitué par un ou plusieurs substituants identiques ou différents choisis parmi : halo, nitro, (C₁-C₆)allcyle et (C₁-C₆)alkoxy ; ou un radical aryl-(C₁-C₆)alkyle éventuellement substitué par un ou plusieurs substituants identiques ou différents choisis parmi : halo, nitro, (C₁-C₆)alkyle et (C₁-C6)alkoxy ;
   Z₃ₑ représente un radical de formule 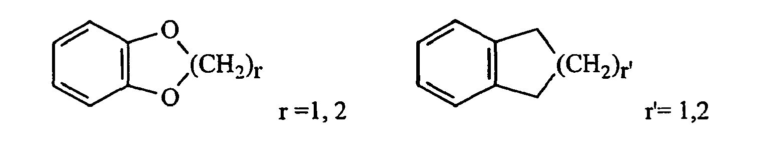 Z'₃ représente un radical aryle éventuellement substitué par un ou plusieurs substituants identiques ou différents choisis parmi : halo, nitro et -(CH₂)_{p"}-V'₃-Y'₃ ;
   V'₃ représente -O-, -C(O)-, -C(O)-O-, -C(O)-NR'₃, -NH-C(O)-NR'₃ ou une liaison covalente ;
   Y'₃ représente l'atome d'hydrogène ou un radical (C₁-C₆)alkyle éventuellement substitué par un ou plusieurs radicaux halo identiques ou différents ;
   R'₃ représente l'atome d'hydrogène, un radical (C₁-C₆)alkyle ou (C₁-C₆)alkoxy ;
   p représente un entier de 1 à 4 ; p'et p" représentent, indépendamment, un entier de 0 à 4;
R₄ représente un radical de formule-(CH₂)ₛ-R'₄ ;
R'₄ représente le radical guanidino ; un hétérocycloalkyle contenant au moins un atome d'azote et éventuellement substitué par (C₁-C₆)alkyle ou aralkyle ; un hétéroaryle contenant au moins un atome d'azote et éventuellement substitué par (C₁-C₆)alkyle ; ou un radical de formule -NW₄W'₄
   W₄ représente l'atome d'hydrogène ou (C₁-C₈)alkyle ;
   W'₄ représente un radical de formule -(CH₂)_{s'}-Z₄ ;
   Z₄ représente l'atome d'hydrogène, (C₁-C₈)alkyle éventuellement substitué par un ou plusieurs substituants identiques ou différents choisis parmi : (C₁-C₆)alkoxy, (C₁-C₆)alkylthio et hydroxy ; (C₂-C₆)alkényle ; (C₃-C₇)cycloalkyle éventuellement substitué par un ou plusieurs substituants (C₁-C₆)alkyle identiques ou différents ; cyclohexène ; hétéroaryle et aryle ;
   les radicaux aryle et hétéroaryle étant éventuellement substitués par un ou plusieurs radicaux identiques ou différents choisis de formule -(CH₂)_{s"}-V₄-Y₄, hydroxy, halo, nitro et cyano ;
   V₄ représente -O-, -S-, -NH-C(O)-, -NV'₄ ou une liaison covalente ;
   Y₄ représente un atome d'hydrogène ou un radical (C₁-C₆)alkyle éventuellement substitué par un ou plusieurs radicaux halo identiques ou différents ;
   V₄' représente un atome d'hydrogène ou un (C₁-C₆)alkyle
   s" représente un entier de 0 à 4 ;
ou bien Z₄ représente un radical de formule 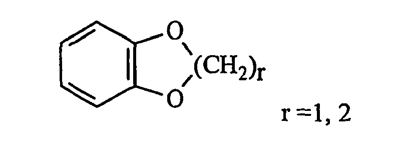
s et s' représentent, indépendamment, un entier de 0 à 6 ; ou un sel pharmaceutiquement acceptable de ce dernier ;
pour la préparation d'un médicament pour le traitement des désordres pondéraux, des troubles mentaux, de la douleur, ou des désordres de l'activité sexuelle.

La présente invention a plus particulièrement pour objet l'utilisation d'un composé de formule générale (I') telle que définie ci-dessus, caractérisé en ce que
R₁ et R₂ représentent, indépendamment, un radical (C₁-C₈)alkyle
R₃ représente Z_{3c} et Z_{3c} représente un radical phényle ou naphtyle, chacun substitué au moins par cyano ;
R₄ représente un radical de formule-(CH₂)ₛ-R'₄ avec R'₄ qui représente le radical pyrrolidinyle ou pipéridinyle ; ou un radical de formule -NW₄W'₄ ;
W₄ qui représente l'atome d'hydrogène ou (C₁-C₈)alkyle ;
W'₄ représente un radical de formule -(CH₂)_{s'}-Z₄ avec Z₄ qui représente l'atome d'hydrogène ;
s représente un entier de 2 à 4 ; s' représente un entier de 0 à 4 ;
ou un sel pharmaceutiquement acceptable de ces derniers.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau, additionnés à des huiles ou des graisses pharmaceutiquement acceptables. Les compositions liquides stériles peuvent être utilisées pour les injections intramusculaires, intrapéritonéales ou sous-cutanées et les compositions stériles peuvent également être administrées par intraveineuse.

Tous les termes techniques et scientifiques utilisés dans le présent texte ont la signification connue de l'homme de l'art. Par ailleurs, tous les brevets (ou demandes de brevet) ainsi que les autres références bibliographiques sont incorporés par référence.

### Partie expérimentale :

Les composés selon l'invention obtenus selon les procédures des exemples A, B, C, C', D, E, F, F' et G précédemment décrites, sont rassemblés dans le tableau ci-dessous.

Les composés sont caractérisés par leur temps de rétention (tr) et leur pic moléculaire déterminé par spectrométrie de masse (MH+).

Pour la spectrométrie de masse, un spectromètre de masse simple quadripôle (Micromass, modèle Platform) equipé d'une source *electrospray* est utilisé avec une résolution de 0,8 Da à 50 % de vallée. Un calibrage est effectué mensuellement entre les masses 80 et 1000 Da à l'aide d'un mélange calibrant d'iodure de sodium et d'iodure de rubidium en solution dans un mélange isopropanol/eau (1/1 Vol.).

Pour la chromatographie liquide, un système Waters incluant un dégazeur en ligne, une pompe quaternaire Waters 600, un injecteur plaque Gilson 233 et un détecteur UV Waters PAD 996, est utilisé.

Les conditions d'élution employées sont les suivantes :
Eluant : A eau + 0.04 % acide trifluoroacétique ; **B** acétonitrile

| **T (min)** | **A%** | **B%** |
|---|---|---|
| 1 | 95 | 5 |
| 8,5 | | 95 |
| 10,5 | 5 | 95 |
| 10,6 | 95 | 5 |
| 14,9 | 95 | 5 |
| 15,0 | 95 | 5 |

Débit : 1 ml/min ; Injection : 10 µl ; Colonne : Uptisphere ODS 3 µm 75*4,6 mm i.d.

Ces exemples sont présentés pour illustrer les procédures ci-dessus et ne doivent en aucun cas être considérés comme une limite à la portée de l'invention.

| **Exemples** | **Structures moléculaires** | **[M+H]+** | **tr (min)** |
|---|---|---|---|
| 1 | 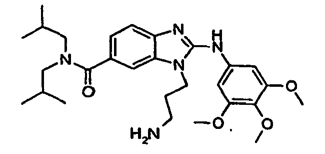 | 512,3 | 7,8 |
| 2 | 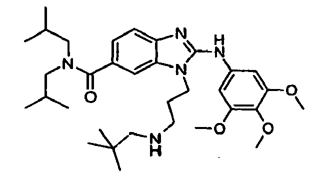 | 582,3 | 8,0 |
| 3 | 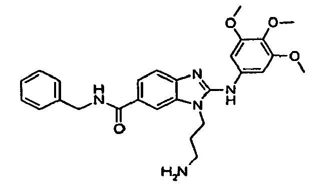 | 490,5 | 7,5 |
| 4 | 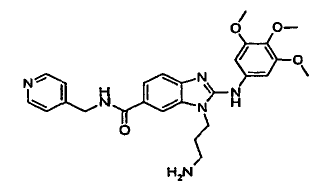 | 491,4 | 6,9 |
| 5 | 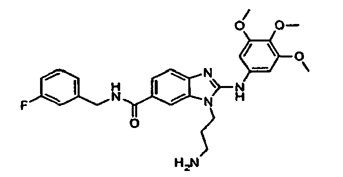 | 508,4 | 7,6 |
| 6 | 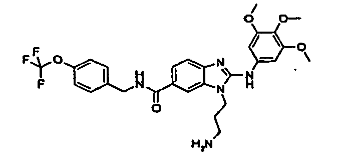 | 574,4 | 8,0 |
| 7 | 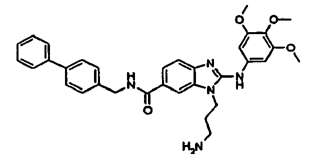 | 566,4 | 8,1 |
| 8 | 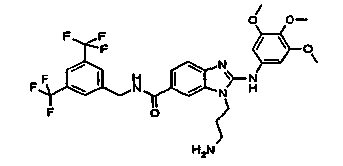 | 626,4 | 8,2 |
| 9 | 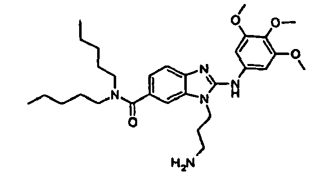 | 540,3 | 8,2 |
| 10 | 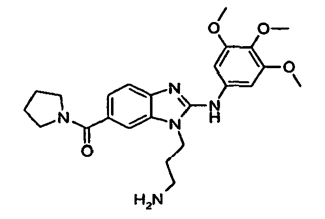 | 454,3 | 7,2 |
| 11 | 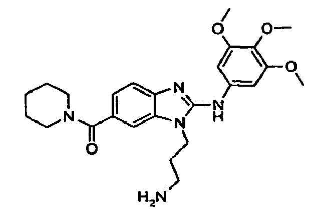 | 467,9 | 7,3 |
| 12 | 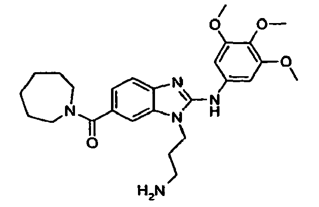 | 482,3 | 7,4 |
| 13 | 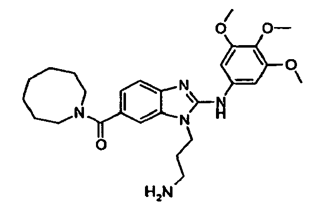 | 496,1 | 7,5 |
| 14 | 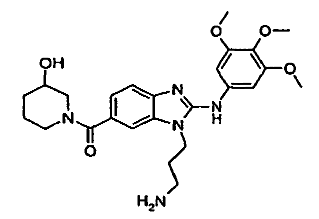 | 484,5 | 7,0 |
| 15 | 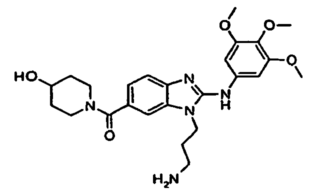 | 484,3 | 7,0 |
| 16 | 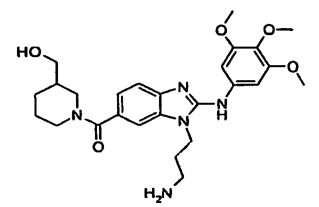 | 498,3 | 7,1 |
| 17 | 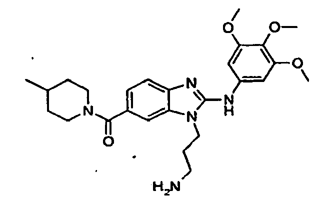 | 482,3 | 7,5 |
| 18 | 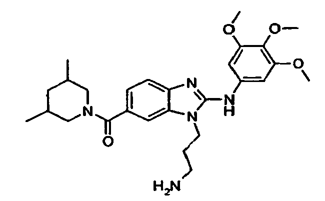 | 496,2 | 7,6 |
| 19 | 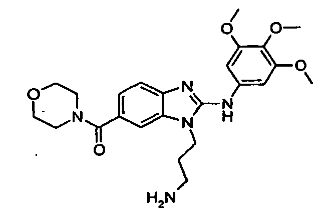 | 470,2 | 7,0 |
| 20 | 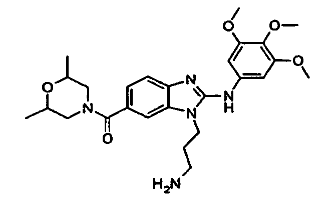 | 498,3 | 7,2 |
| 21 | 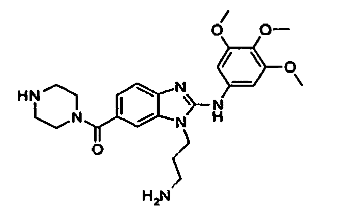 | 469,1 | 6,2 |
| 22 | 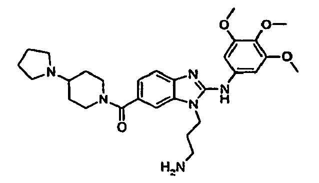 | 537,1 | 6,8 |
| 23 | 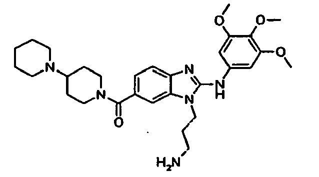 | 551,0 | 6,9 |
| 24 | 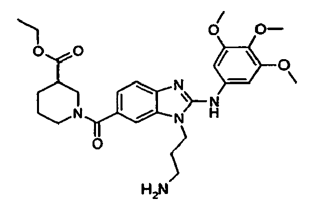 | 540,2 | 7,4 |
| 25 | 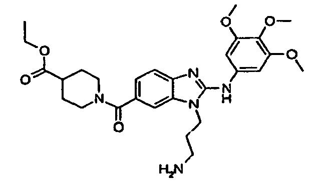 | 540,0 | 7,4 |
| 26 | 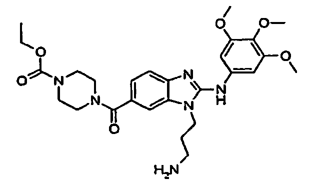 | 541,0 | 7,3 |
| 27 | 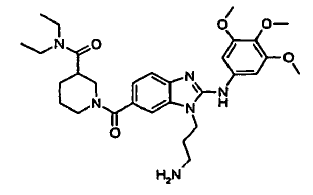 | 566,9 | 7,3 |
| 28 | 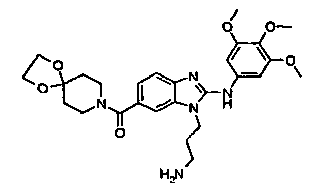 | 526,0 | 7,2 |
| 29 | 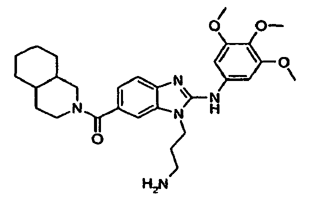 | 522,3 | 7,7 |
| 30 | 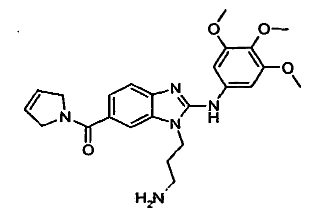 | 452,2 | 7,2 |
| 31 | 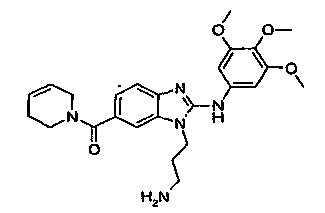 | 466,1 | 7,3 |
| 32 | 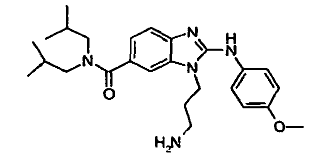 | 452,3 | 7,8 |
| 33 | 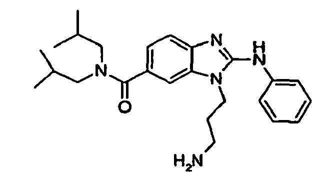 | 422,3 | 7,7 |
| 34 | 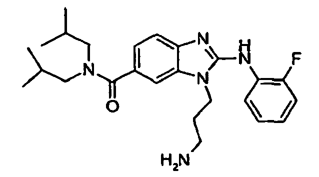 | 440,3 | 7,8 |
| 35 | 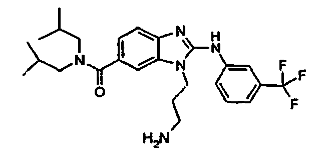 | 490,3 | 8,3 |
| 36 | 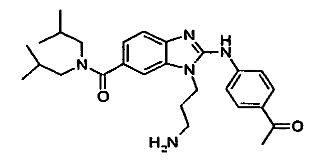 | 464,3 | 8,0 |
| 37 | 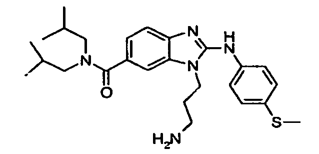 | 468,3 | 7,9 |
| 38 | 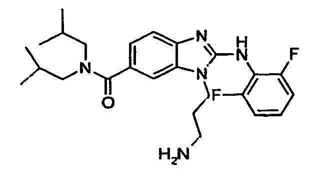 | 458,3 | 8,0 |
| 39 | 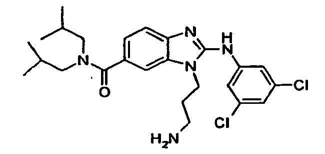 | 490,3 | 8,9 |
| 40 | 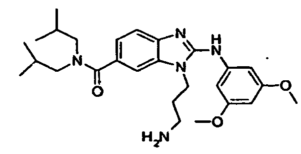 | 482,3 | 7,9 |
| 41 | 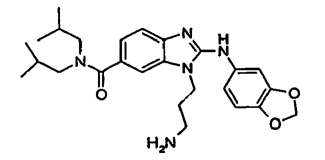 | 466,3 | 7,8 |
| 42 | 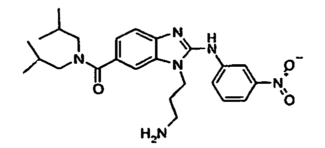 | 467,3 | 8,3 |
| 43 | 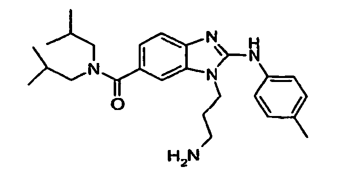 | 436,3 | 7,8 |
| 44 | 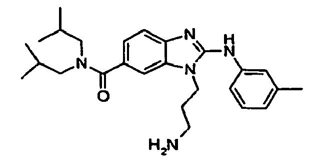 | 436,3 | 7,8 |
| 45 | 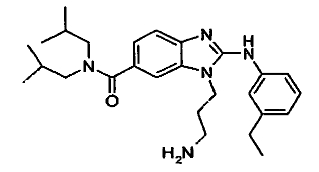 | 450,3 | 8,0 |
| 46 | 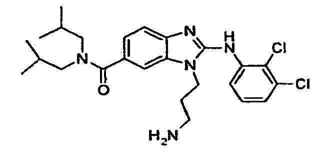 | 490,3 | 8,2 |
| 47 | 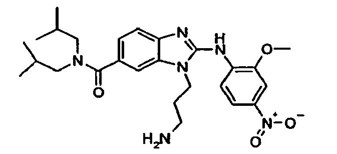 | 497,3 | 8,5 |
| 48 | 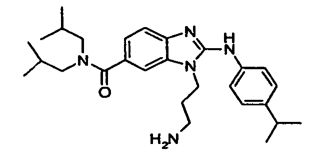 | 464,3 | 8,1 |
| 49 | 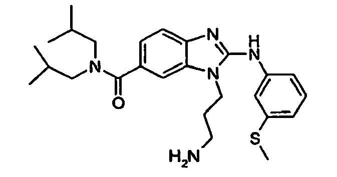 | 468,3 | 8,0 |
| 50 | 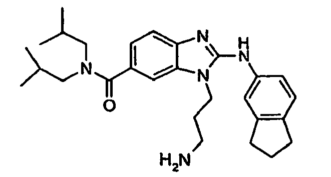 | 462,4 | 8,0 |
| 51 | 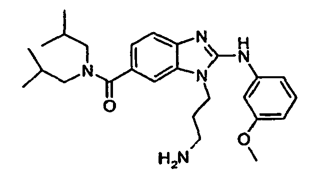 | 452,4 | 7,8 |
| 52 | 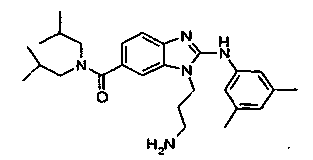 | 450,4 | 8,0 |
| 53 | 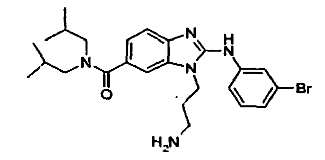 | 500,2 | 8,2 |
| 54 | 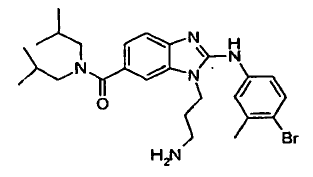 | 514,3 | 8,2 |
| 55 | 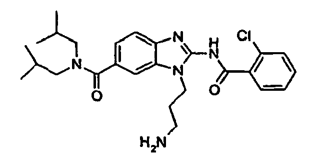 | 484,2 | 8,7 |
| 56 | 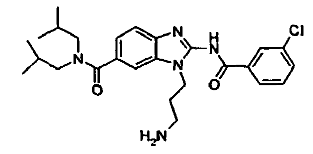 | 484,2 | 9,1 |
| 57 | 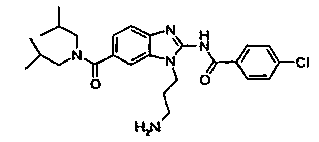 | 484,2 | 9,0 |
| 58 | 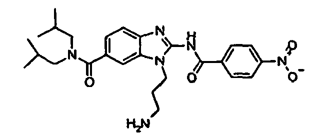 | 495,3 | 8,9 |
| 59 | 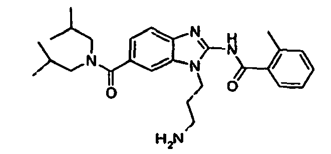 | 464,3 | 8,7 |
| 60 | 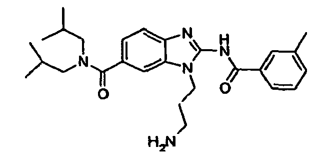 | 464,3 | 8,9 |
| 61 | 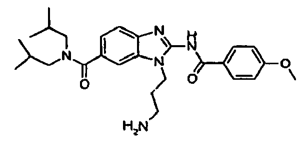 | 480,3 | 8,7 |
| 62 | 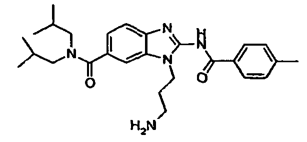 | 464,3 | 8,9 |
| 63 | 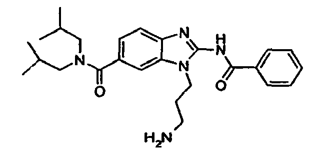 | 450,3 | 8,7 |
| 64 | 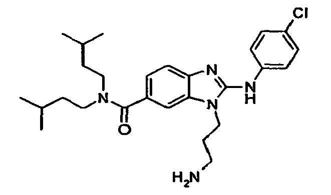 | 484,2 | 8,4 |
| 65 | 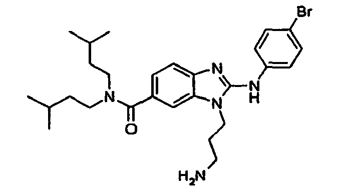 | 528,2 | 8,5 |
| 66 | 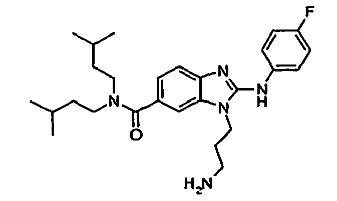 | 468,3 | 8,2 |
| 67 | 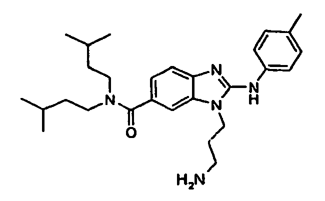 | 464,3 | 8,1 |
| 68 | 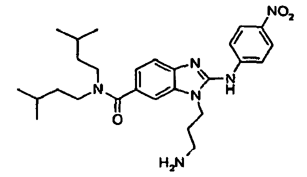 | 495,3 | 9,1 |
| 69 | 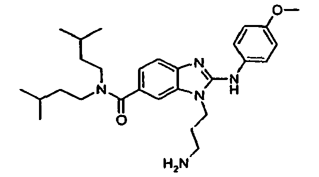 | 480,3 | 8,1 |
| 70 | 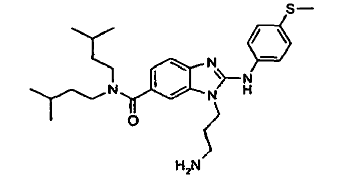 | 496,3 | 8,3 |
| 71 | 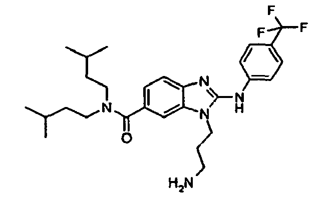 | 518,2 | 9,0 |
| 72 | 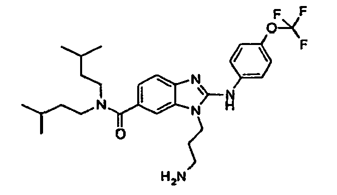 | 534,2 | 8,7 |
| 73 | 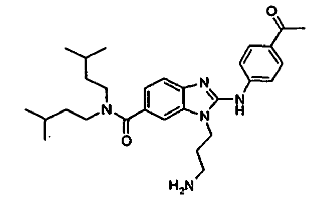 | 492,3 | 8,4 |
| 74 | 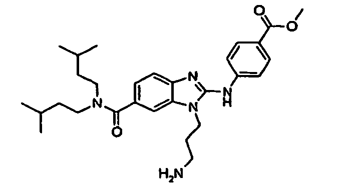 | 508,3 | 8,5 |
| 75 | 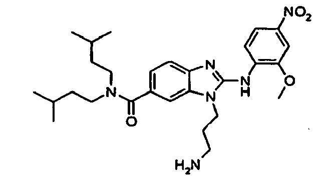 | 525,3 | 8,9 |
| 76 | 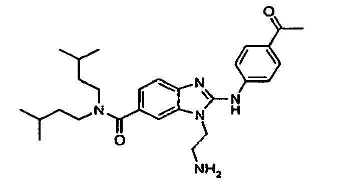 | 478,2 | 8,5 |
| 77 | 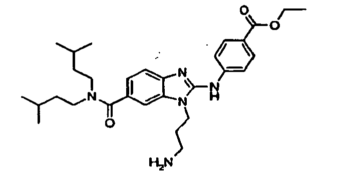 | 522,2 | 8,6 |
| 78 | 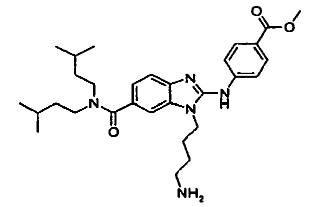 | 522,2 | 8,4 |
| 79 | 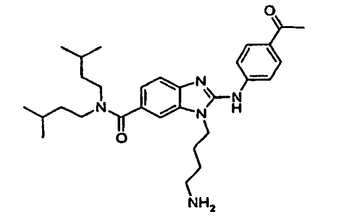 | 506,2 | 8,2 |
| 80 | 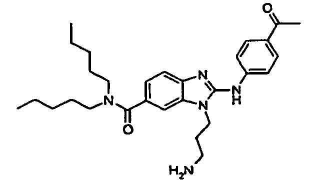 | 492,2 | 8,5 |
| 81 | 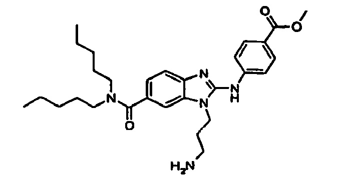 | 508,2 | 8,6 |
| 82 | 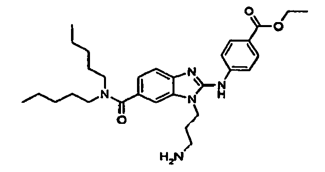 | 522,3 | 8,8 |
| 83 | 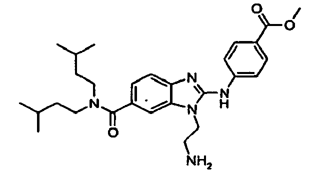 | 494,2 | 8,7 |
| 84 | 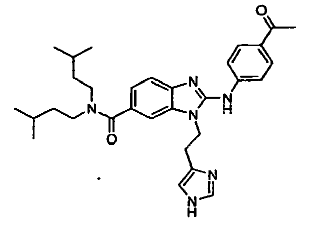 | 529,3 | 8,5 |
| 85 | 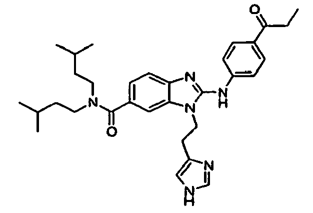 | 543,3 | 8,7 |
| 86 | 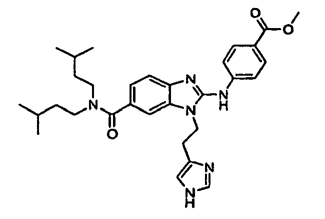 | 545,3 | 8,6 |
| 87 | 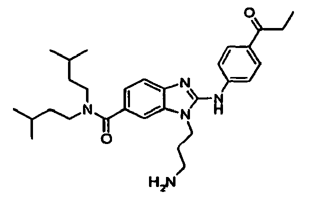 | 506,3 | 8,5 |
| 88 | 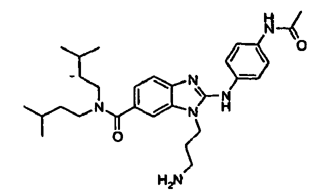 | 507,3 | 7,9 |
| 89 | 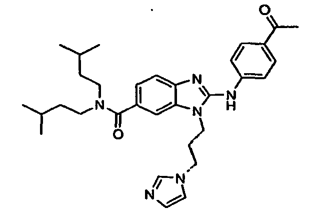 | 543,3 | 8,5 |
| 90 | 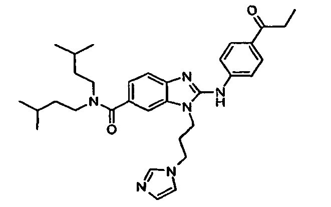 | 557,3 | 8,7 |
| 91 | 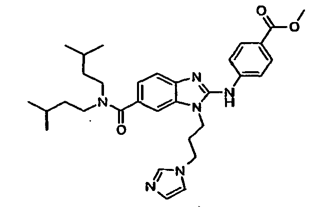 | 559,3 | 8,7 |
| 92 | 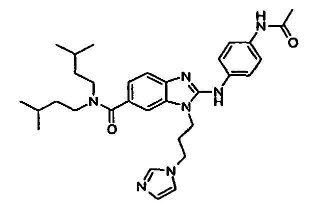 | 558,3 | 8,0 |
| 93 | 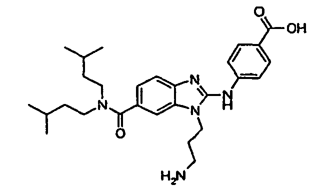 | 494,3 | 8,1 |
| 94 | 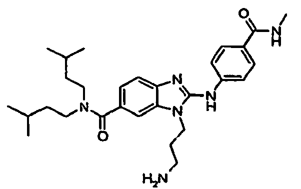 | 507,2 | 8,0 |
| 95 | 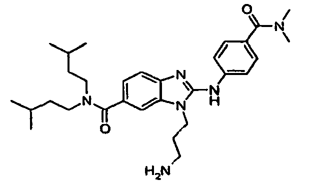 | 521,2 | 8,0 |
| 96 | 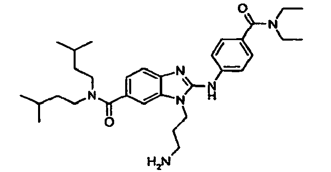 | 549,3 | 8,2 |
| 97 | 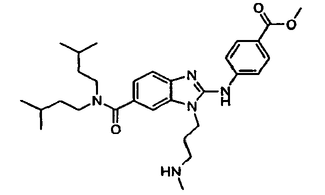 | 522,3 | 8,6 |
| 98 | 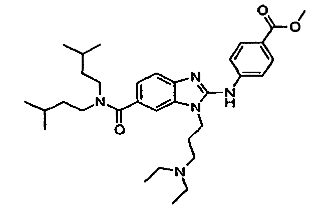 | 564,3 | 8,9 |
| 99 | 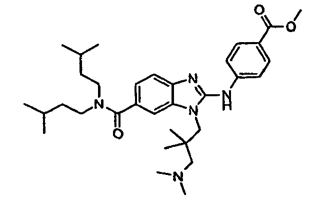 | 564,3 | 9,1 |
| 100 | 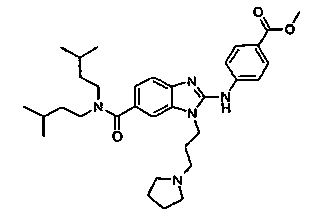 | 562,3 | 8,8 |
| 101 | 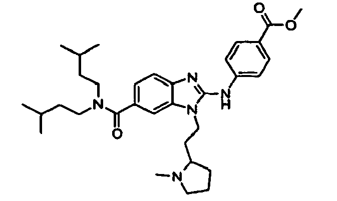 | 562,3 | 8,8 |
| 102 | 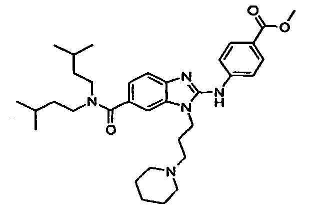 | 576,3 | 8,9 |
| 103 | 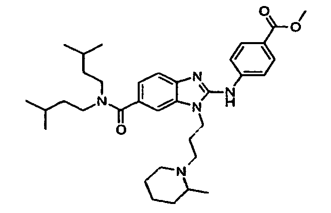 | 590,3 | 9,0 |
| 104 | 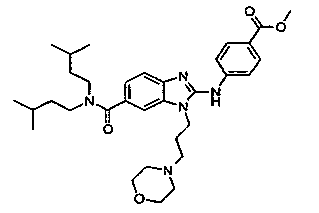 | 578,3 | 8,7 |
| 105 | 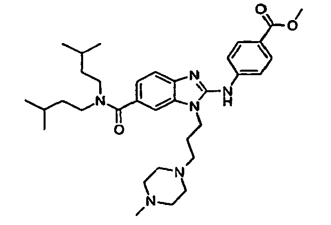 | 591,3 | 8,5 |
| 106 | 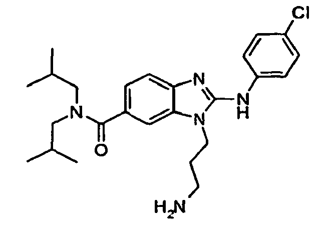 | 456,2 | 8,0 |
| 107 | 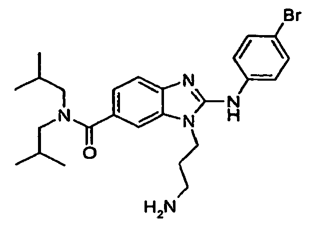 | 500,1 | 8,0 |
| 108 | 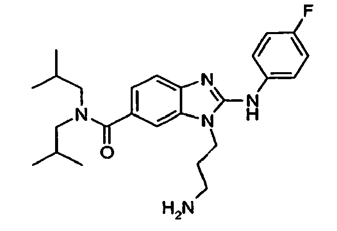 | 440,2 | 7,7 |
| 109 | 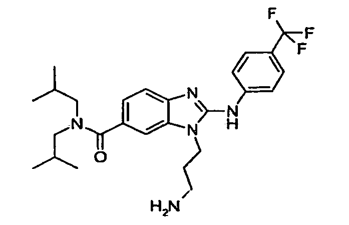 | 490,2 | 8,4 |
| 110 | 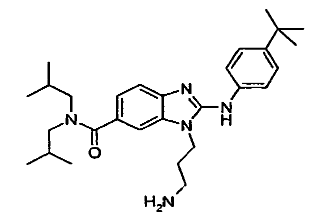 | 478,3 | 8,2 |
| 111 | 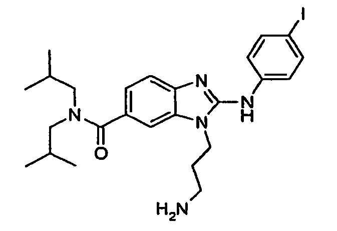 | 548,1 | 8,1 |
| 112 | 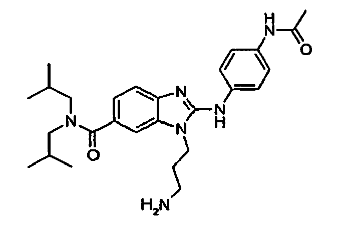 | 479,2 | 7,6 |
| 113 | 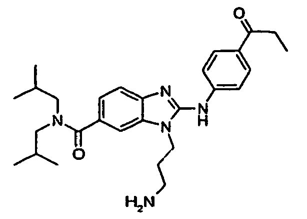 | 478,2 | 8,1 |
| 114 | 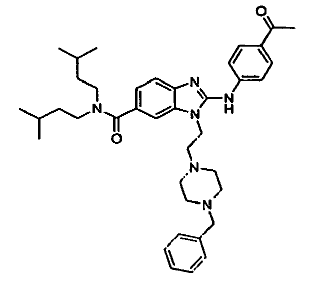 | 637,3 | 8,8 |
| 115 | 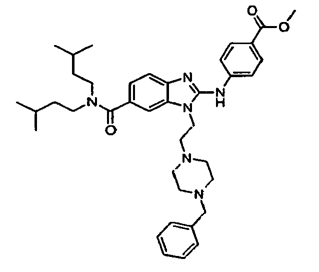 | 653,3 | 9,0 |
| 116 | 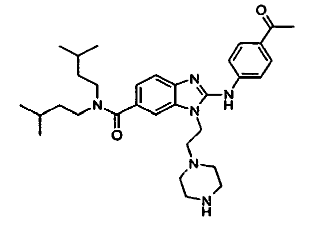 | 547,3 | 8,8 |
| 117 | 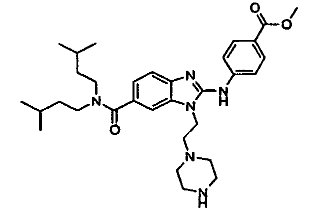 | 563,3 | 9,0 |
| 118 | 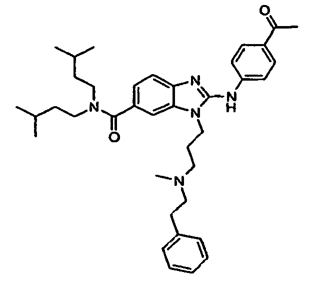 | 610,4 | 9,1 |
| 119 | 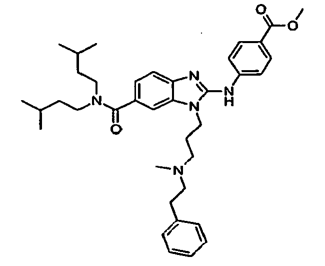 | 626,4 | 9,3 |
| 120 | 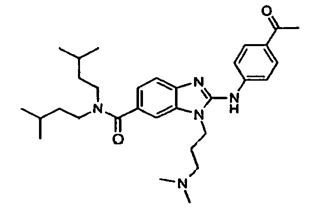 | 520,3 | 8,5 |
| 121 | 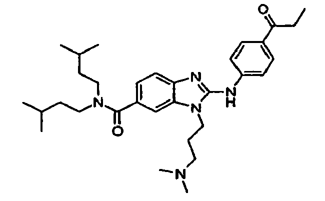 | 534,3 | 8,7 |
| 122 | 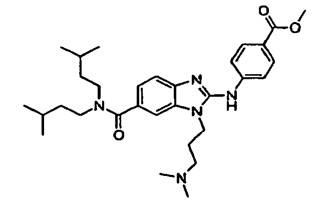 | 536,3 | 8,6 |
| 123 | 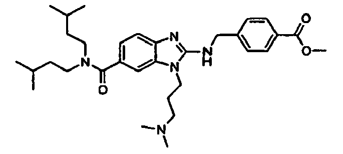 | 550,3 | 8,2 |
| 124 | 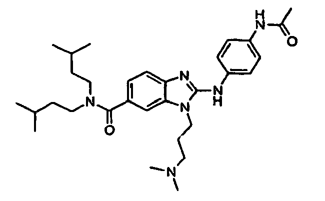 | 535,3 | 8,0 |
| 125 | 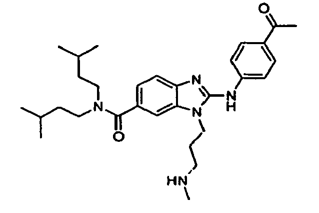 | 506,3 | 8,4 |
| 126 | 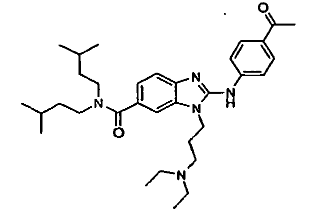 | 548,3 | 8,6 |
| 127 | 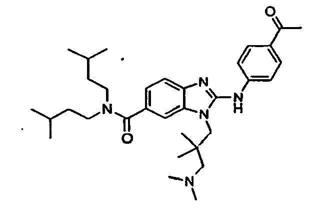 | 548,3 | 8,8 |
| 128 | 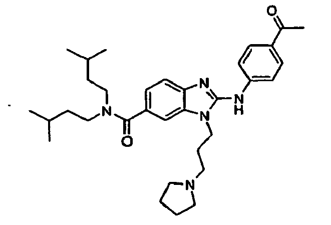 | 546,3 | 8,6 |
| 129 | 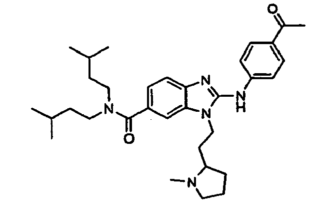 | 546,3 | 8,6 |
| 130 | 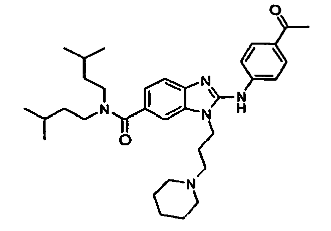 | 560,3 | 8,6 |
| 131 | 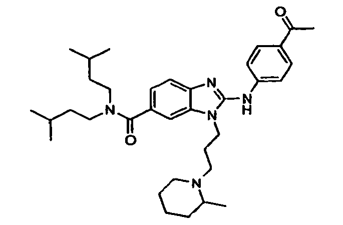 | 574,3 | 8,7 |
| 132 | 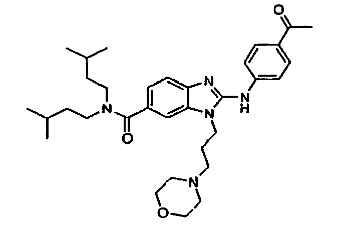 | 562,3 | 8,5 |
| 133 | 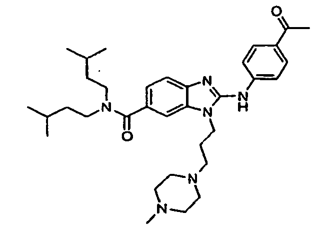 | 575,3 | 8,3 |
| 134 | 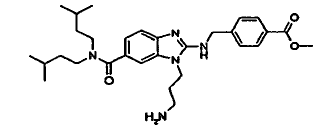 | 522,3 | 8,2 |
| 135 | 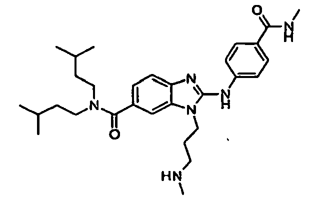 | 521,2 | 8,1 |
| 136 | 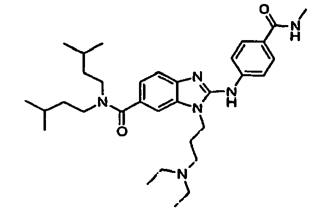 | 563,2 | 8,2 |
| 137 | 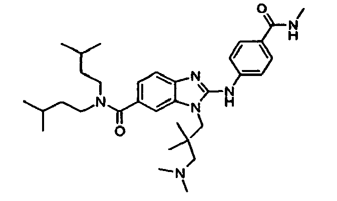 | 563,2 | 8,3 |
| 138 | 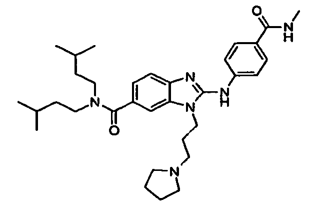 | 561,2 | 8,2 |
| 139 | 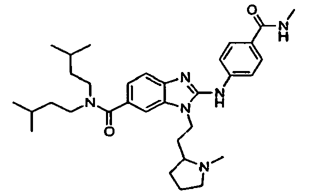 | 561,2 | 8,2 |
| 140 | 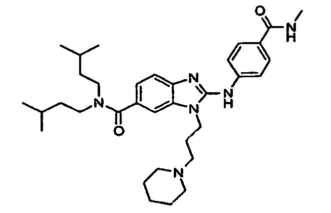 | 575,2 | 8,2 |
| 141 | 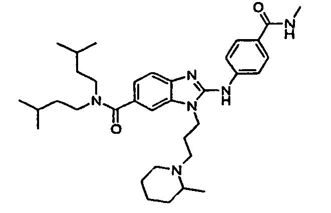 | 589,2 | 8,3 |
| 142 | 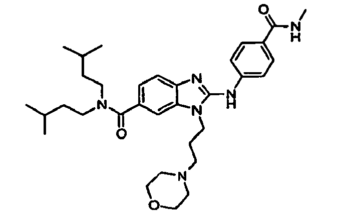 | 577,2 | 8,1 |
| 143 | 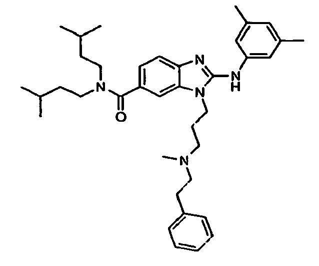 | 594,4 | 8,8 |
| 144 | 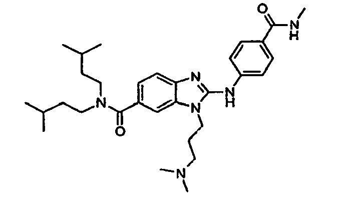 | 535,4 | 8,0 |
| 145 | 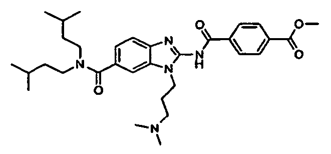 | 564,4 | 9,5 |
| 146 | 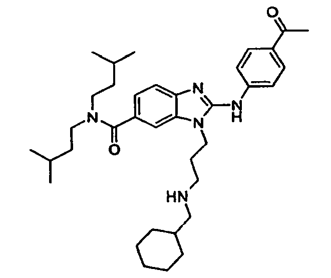 | 588,4 | 9,2 |
| 147 | 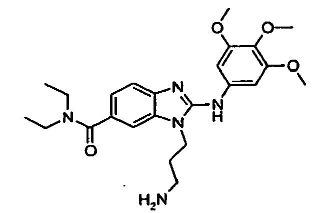 | 456,2 | 7,2 |
| 148 | 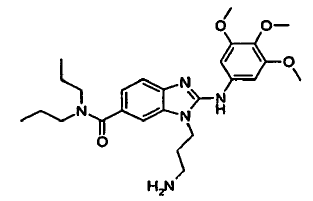 | 484,3 | 7,5 |
| 149 | 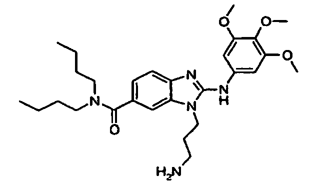 | 512,2 | 7,8 |
| 150 | 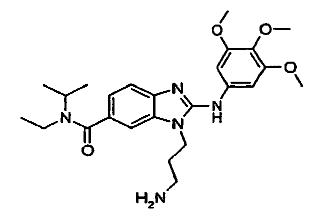 | 470,3 | 7,3 |
| 151 | 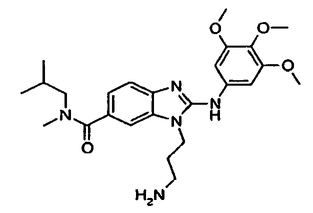 | 470,2 | 7,4 |
| 152 | 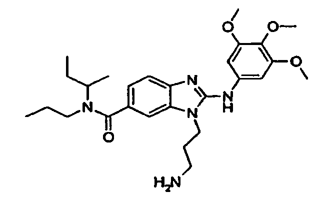 | 498,3 | 7,6 |
| 153 | 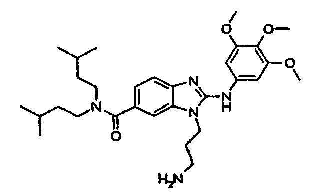 | 540,2 | 8,1 |
| 154 | 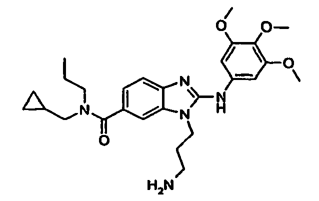 | 496,3 | 7,6 |
| 155 | 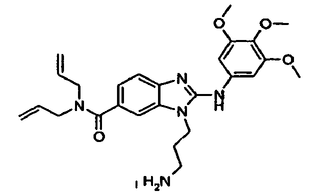 | 480,3 | 7,4 |
| 156 | 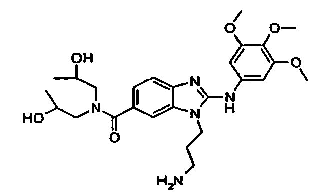 | 516,0 | 7,0 |
| 157 | 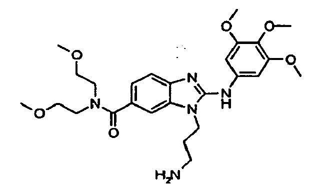 | 516,2 | 7,2 |
| 158 | 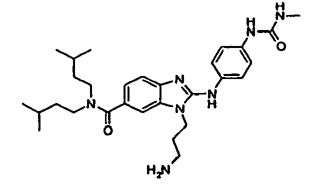 | 522,3 | 7,9 |
| 159 | 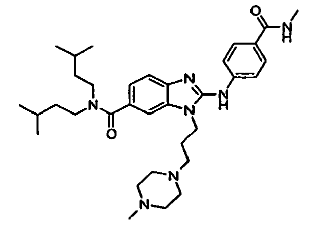 | 590,2 | 8,0 |
| 160 | 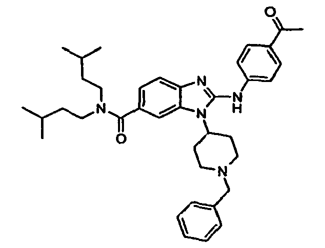 | 608,5 | 9,2 |
| 161 | 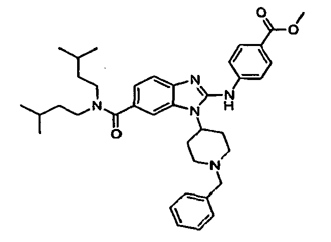 | 624,5 | 9,4 |
| 162 | 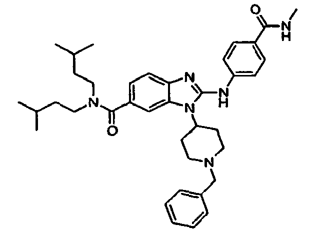 | 623,5 | 8,5 |
| 163 | 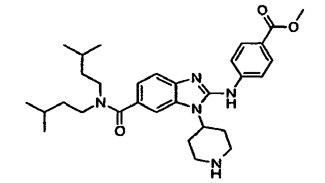 | 534,3 | 8,7 |
| 164 | 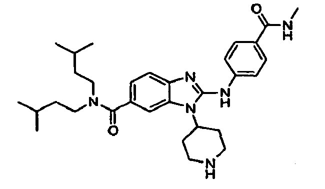 | 533,3 | 8,0 |
| 165 | 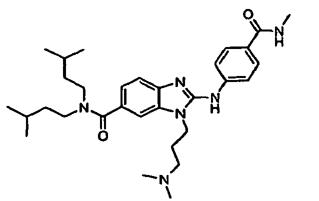 | 535,4 | 8,0 |
| 166 | 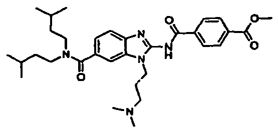 | 564,4 | 9,5 |
| 167 | 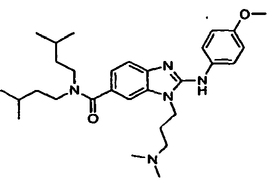 | 508,2 | 8,2 |
| 168 | 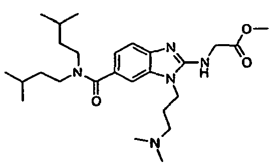 | 474,3 | 8,0 |
| 169 | 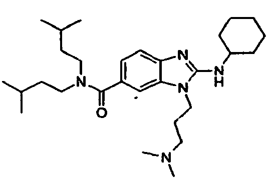 | 484,4 | 8,2 |
| 170 | 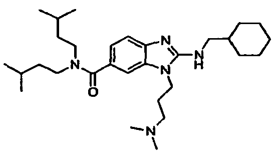 | 498,4 | 8,4 |
| 171 | 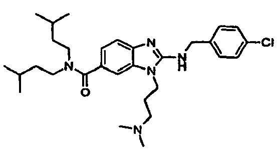 | 526,3 | 8,4 |
| 172 | 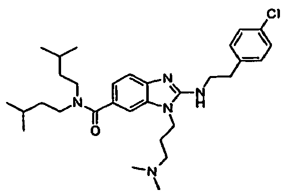 | 540,3 | 8,5 |
| 173 | 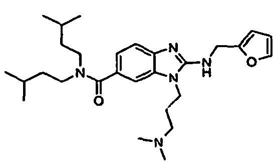 | 482,3 | 8,1 |
| 174 | 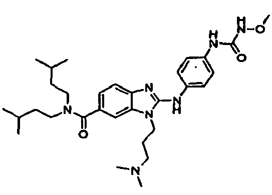 | 566,4 | 8,1 |
| 175 | 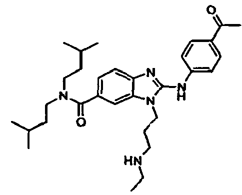 | 520,2 | 8,6 |
| 176 | 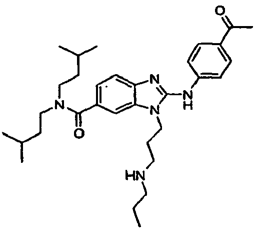 | 534,2 | 8,7 |
| 177 | 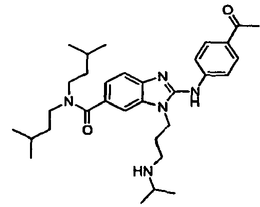 | 534,2 | 8,7 |
| 178 | 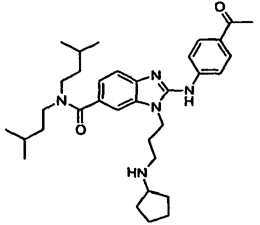 | 560,2 | 8,9 |
| 179 | 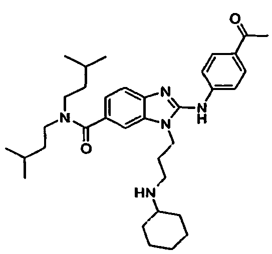 | 574,2 | 9,0 |
| 180 | 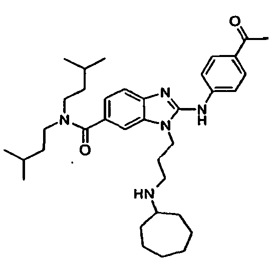 | 588,2 | 9,1 |
| 181 | 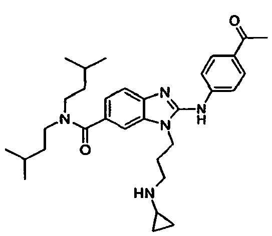 | 532,2 | 8,6 |
| 182 | 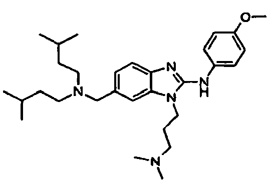 | 494,4 | 7,5 |
| 183 | 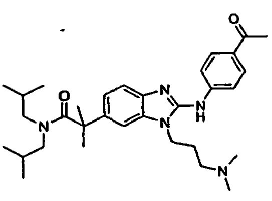 | 534,4 | 8,1 |
| 184 | 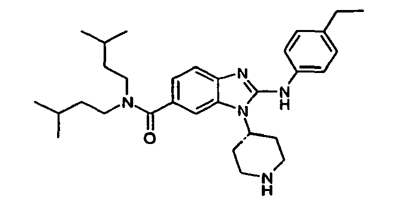 | 504,4 | 8,4 |
| 185 | 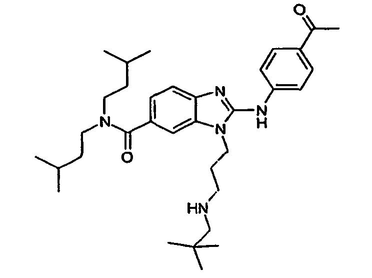 | 562,2 | 8,9 |
| 186 | 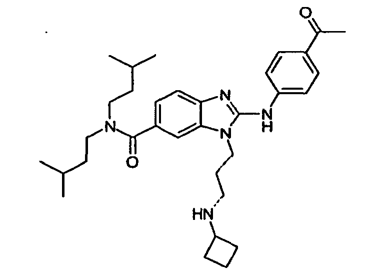 | 546,2 | 8,7 |
| 187 | 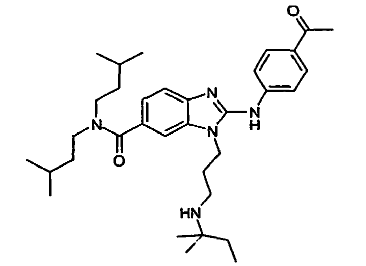 | 562,2 | 8,9 |
| 188 | 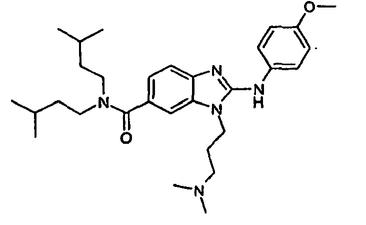 | 508,2 | 8,2 |
| 189 | 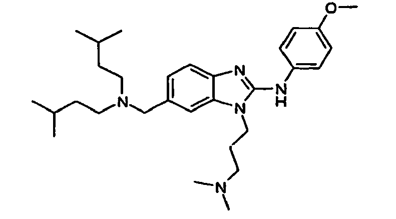 | 494,4 | 7,5 |
| 190 | 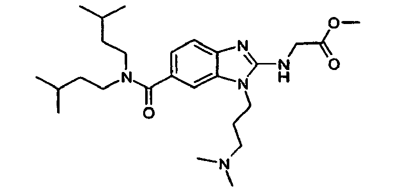 | 474,3 | 8,0 |
| 191 | 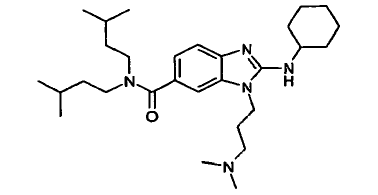 | 484,4 | 8,2 |
| 192 | 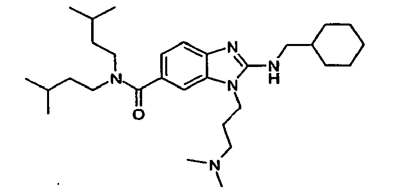 | 498,4 | 8,4 |
| 193 | 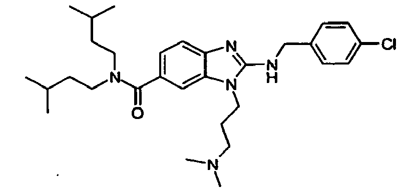 | 526,3 | 8,4 |
| 194 | 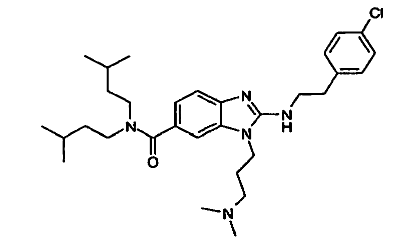 | 540,3 | 8,5 |
| 195 | 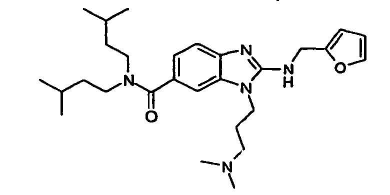 | 482,3 | 8,1 |
| 196 | 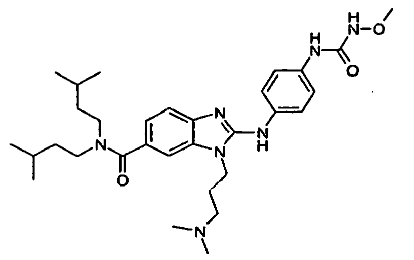 | 566,4 | 8,1 |
| 197 | 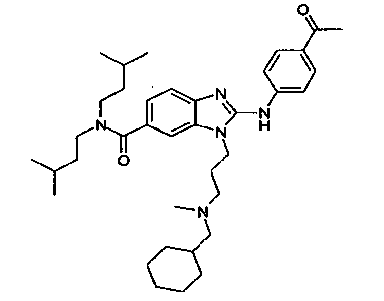 | 602,3 | 9,2 |
| 198 | 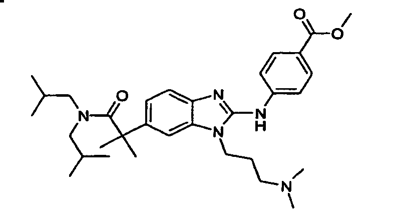 | 550,3 | 8,1 |
| 199 | 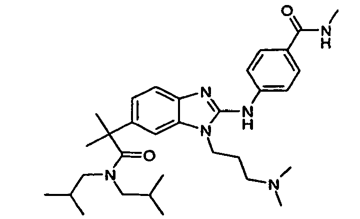 | 549,3 | 7,8 |
| 200 | 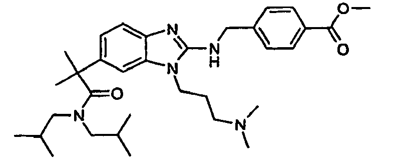 | 564,4 | 8,1 |
| 201 | 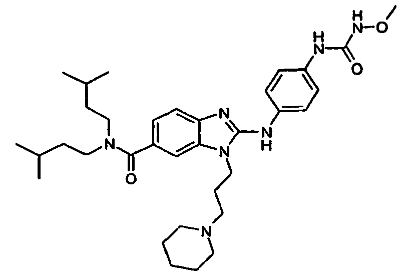 | 606,4 | 8,2 |
| 203 | 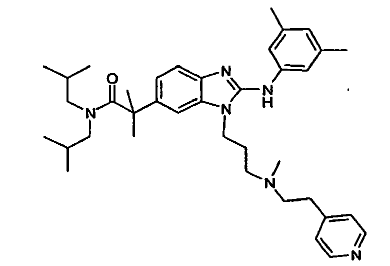 | 611,4 | 7,4 |
| 204 | 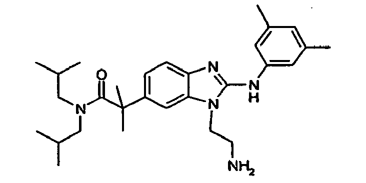 | 47S,4 | 7,5 |
| 205 | 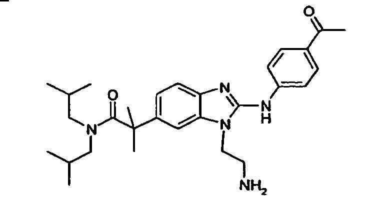 | 492,4 | 7,5 |
| 206 | 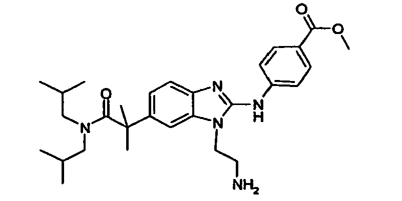 | 508,3 | 7,6 |
| 207 | 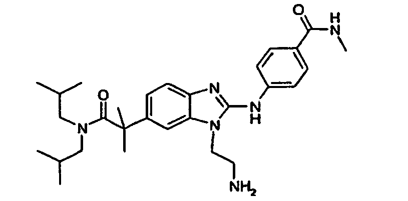 | 507,3 | 7,3 |
| 208 | 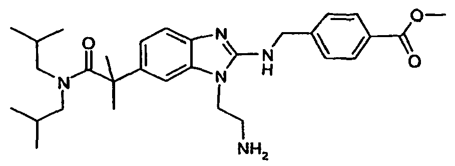 | 522,3 | 7,4 |
| 209 | 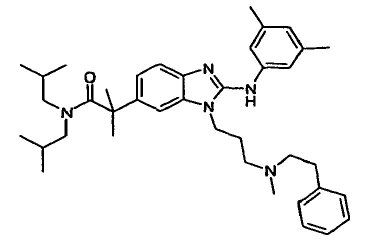 | 610,3 | 8,7 |
| 210 | 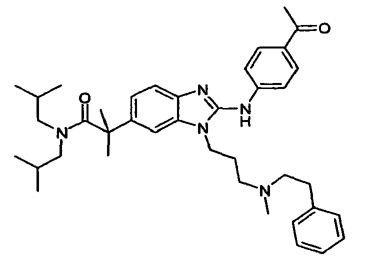 | 624,4 | 8,7 |
| 211 | 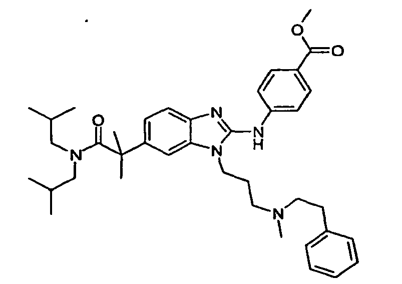 | 640,4 | 8,8 |
| 212 | 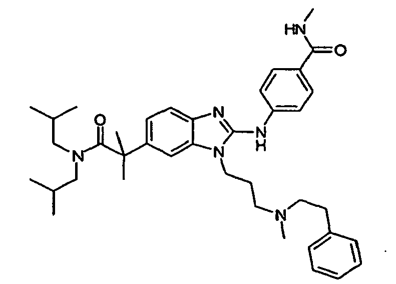 | 639,3 | 8,3 |
| 213 | 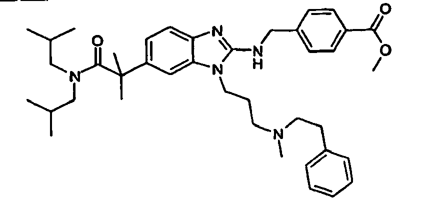 | 654,4 | 8,5 |
| 214 | 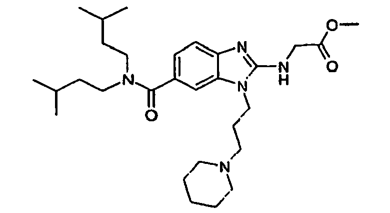 | 514,4 | 8,2 |
| 215 | 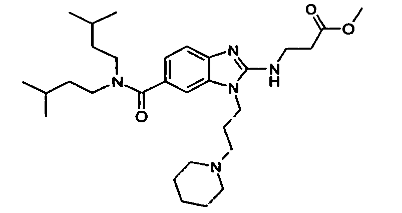 | 528,4 | 8,2 |
| 216 | 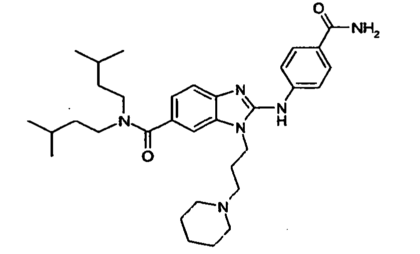 | 560,4 | 8,2 |
| 217 | 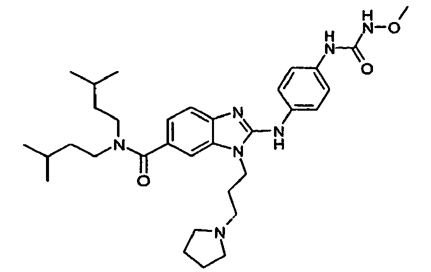 | 592,4 | 8,1 |
| 218 | 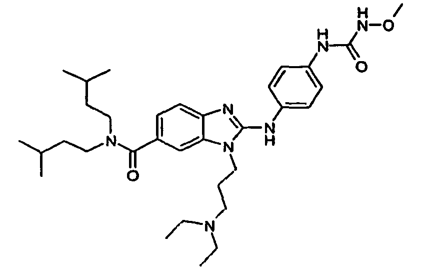 | 594,3 | 8,1 |
| 219 | 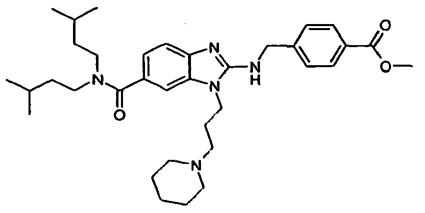 | 590,4 | 8,4 |
| 220 | 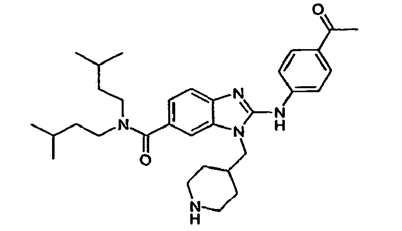 | 532,3 | 8,4 |
| 221 | 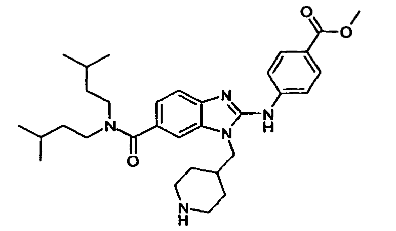 | 548,2 | 8,6 |
| 222 | 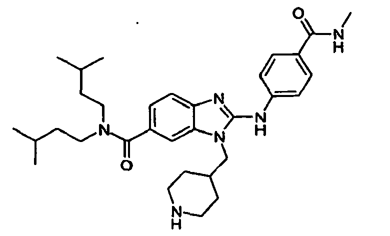 | 547,4 | 8,0 |
| 223 | 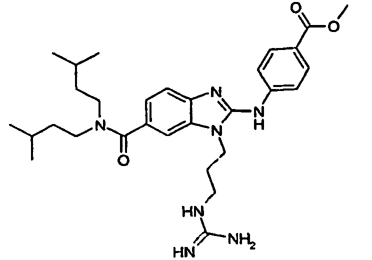 | 550,4 | 8,5 |
| 224 | 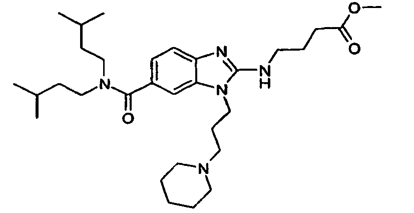 | 542,4 | 8,2 |
| 225 | 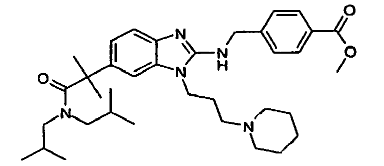 | 604,5 | 8,3 |
| 226 | 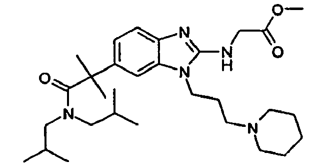 | 528,4 | 7,9 |
| 227 | 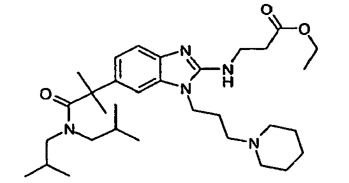 | 556,5 | 8,1 |
| 228 | 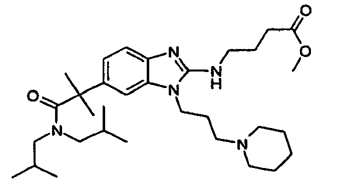 | 556,5 | 8,0 |
| 229 | 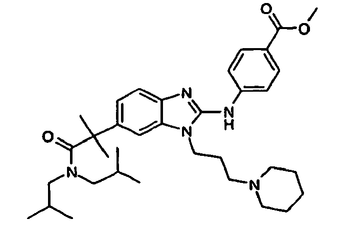 | 590,4 | 8,4 |
| 230 | 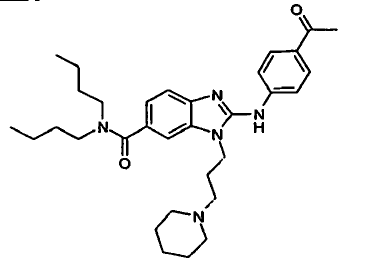 | 532,4 | 8,6 |
| 231 | 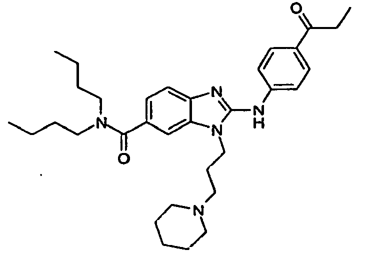 | 546,4 | 8,6 |
| 232 | 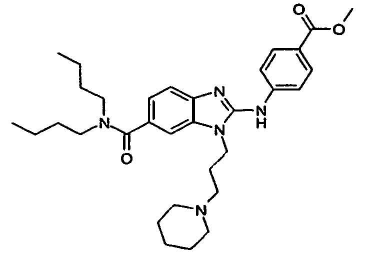 | 548,3 | 8,5 |
| 233 | 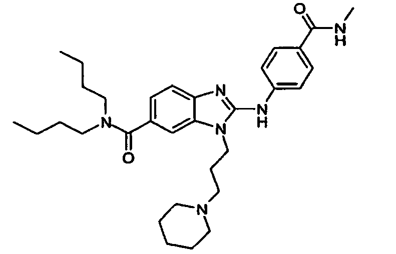 | 547,4 | 7,9 |
| 234 | 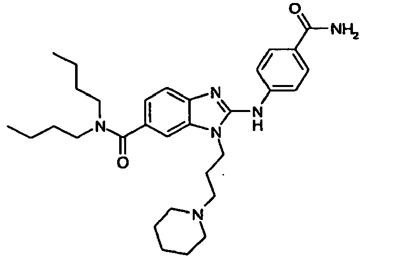 | 533,4 | 7,9 |
| 235 | 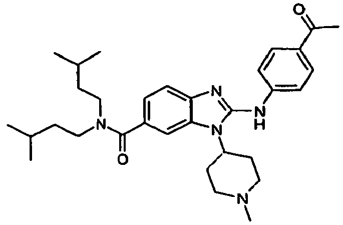 | 532,5 | 8,7 |
| 236 | 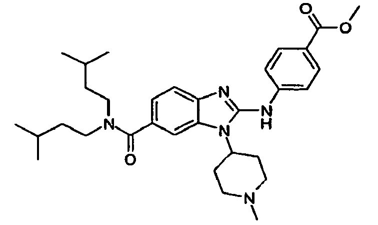 | 548,4 | 8,8 |
| 237 | 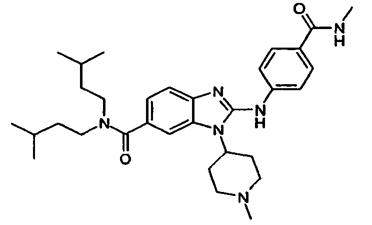 | 547,5 | 8,1 |
| 238 | 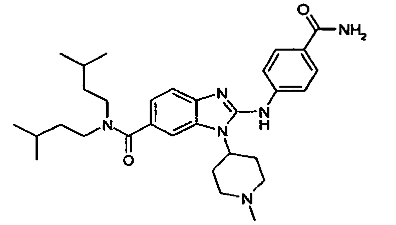 | 533,4 | 8,0 |
| 239 | 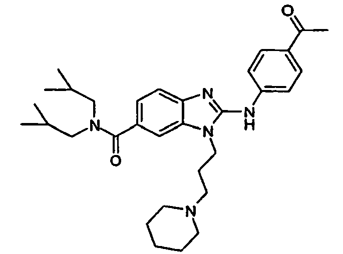 | 532,4 | 8,1 |
| 240 | 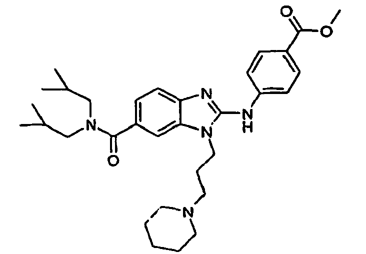 | 548,4 | 8,3 |
| 241 | 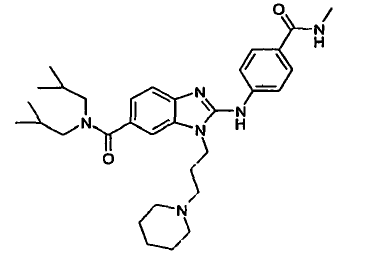 | 547,4 | 7,7 |
| 242 | 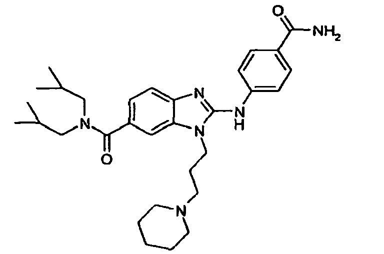 | 533,4 | 7,7 |
| 243 | 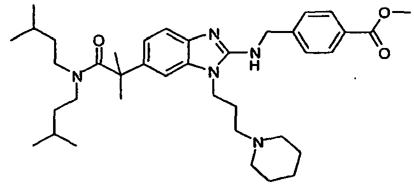 | 632,5 | 8,3 |
| 244 | 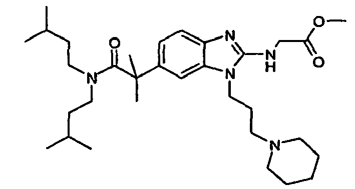 | 556,4 | 8,1 |
| 245 | 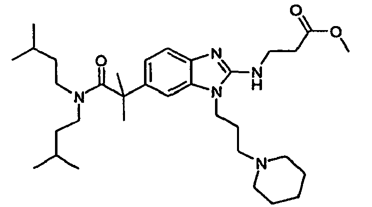 | 570,4 | 8,1 |
| 246 | 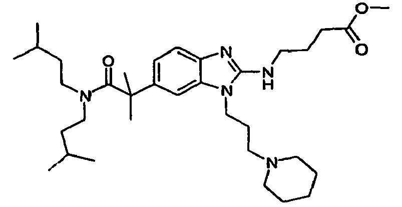 | 584,5 | 8,2 |
| 247 | 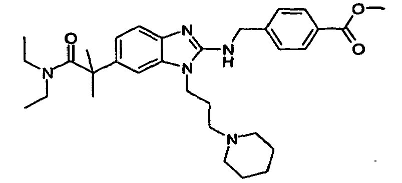 | 548,4 | 7,5 |
| 248 | 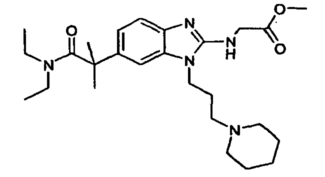 | 472,4 | 7,2 |
| 249 | 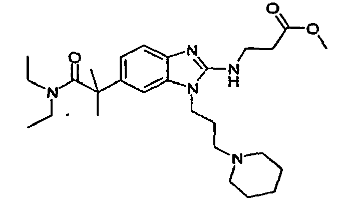 | 486,4 | 7,3 |
| 250 | 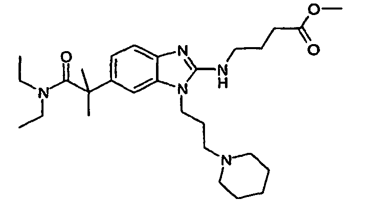 | 500,4 | 7,3 |
| 251 | 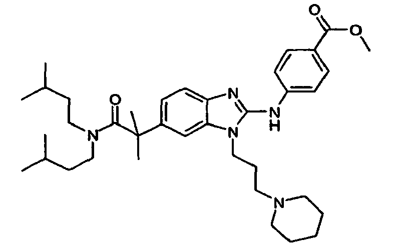 | 618,5 | 8,7 |
| 252 | 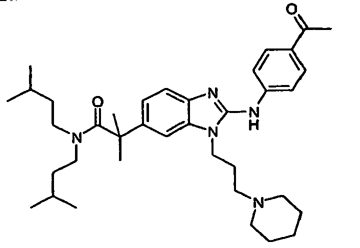 | 602,5 | 8,6 |
| 253 | 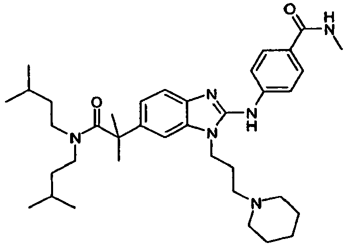 | 617,5 | 8,1 |
| 254 | 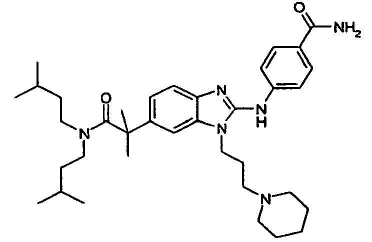 | 603,5 | 8,1 |
| 255 | 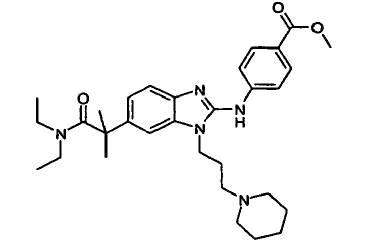 | 534,4 | 7,6 |
| 256 | 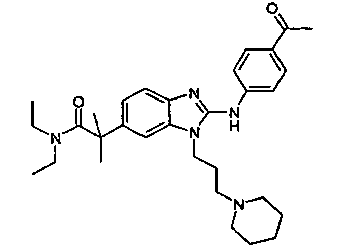 | 518,4 | 7,5 |
| 257 | 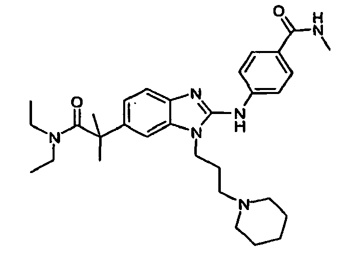 | 533,4 | 7,3 |
| 258 | 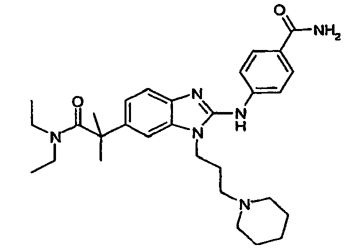 | 519,4 | 7,2 |
| 259 | 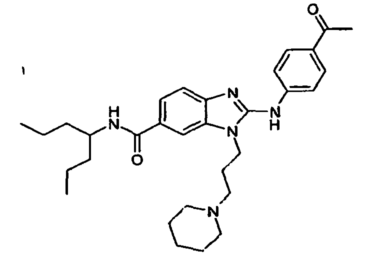 | 518,4 | 8,1 |
| 260 | 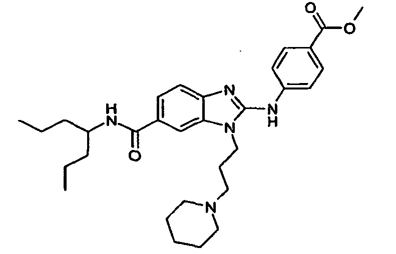 | 534,4 | 8,2 |
| 261 | 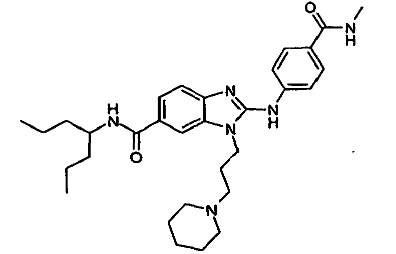 | 533,4 | 7,7 |
| 262 | 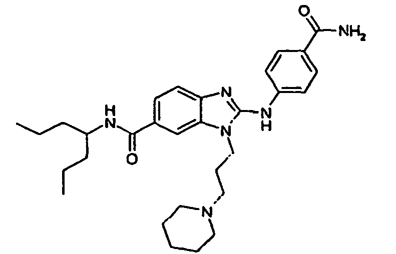 | 519,4 | 7,7 |
| 263 | 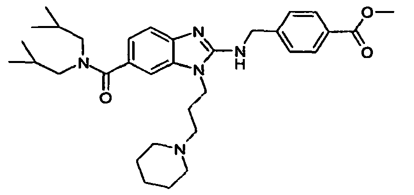 | 562,4 | 7,8 |
| 264 | 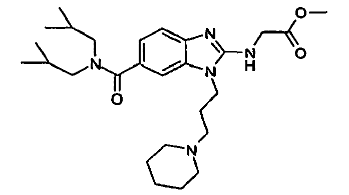 | 486,3 | 7,6 |
| 265 | 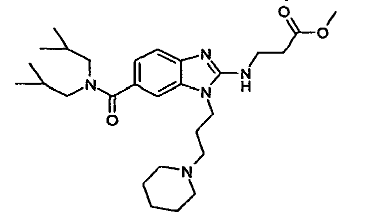 | 500,4 | 7,6 |
| 266 | 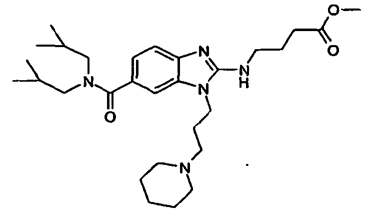 | 514,4 | 7,7 |
| 267 | 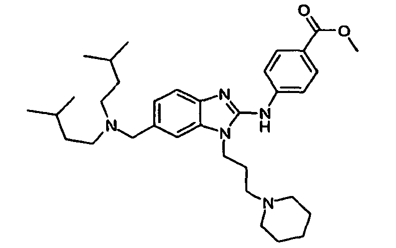 | 562,4 | 8,2 |
| 268 | 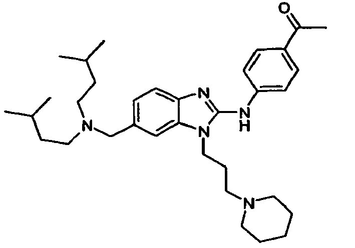 | 546,4 | 8,1 |
| 269 | 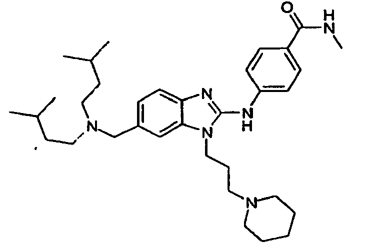 | 561,4 | 7,8 |
| 270 | 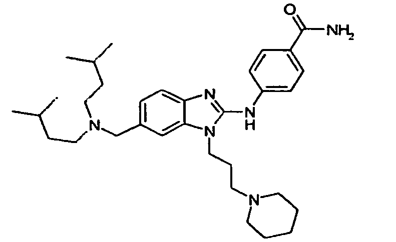 | 547,4 | 7,8 |
| 271 | 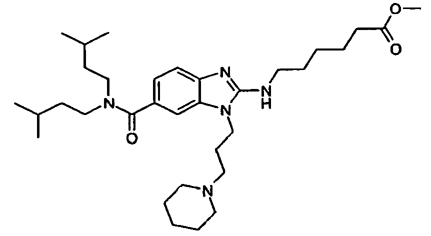 | 570,4 | 8,7 |
| 272 | 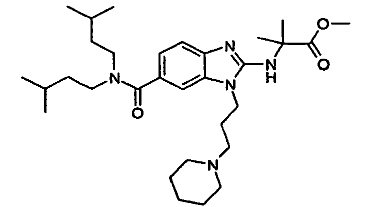 | 542,4 | 8,7 |
| 273 | 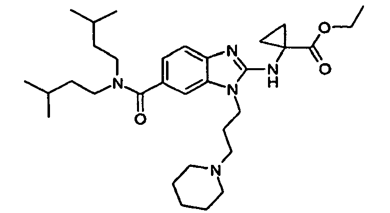 | 554,4 | 8,6 |
| 274 | 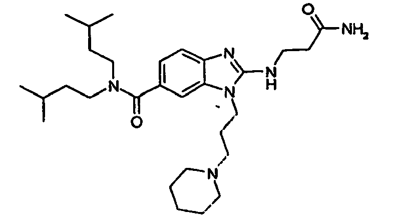 | 513,4 | 8,3 |
| 275 | 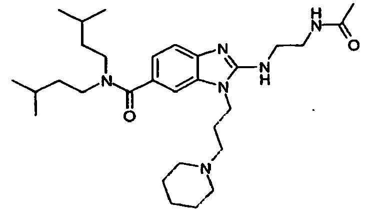 | 527,4 | 8,4 |
| 276 | 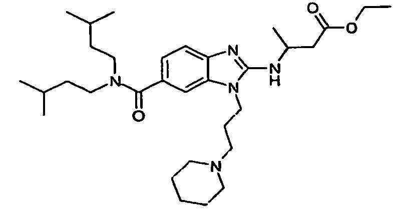 | 556,4 | 8,6 |
| 277 | 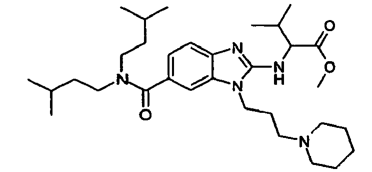 | 556,4 | 8,8 |
| 278 | 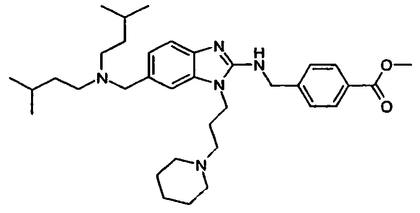 | 576,4 | 7,9 |
| 279 | 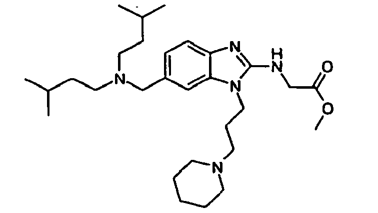 | 500,4 | 7,6 |
| 280 | 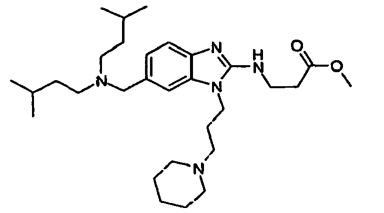 | 514,4 | 7,6 |
| 281 | 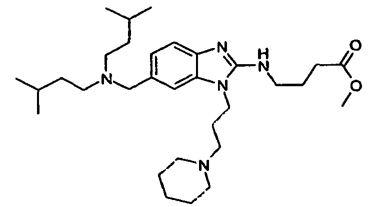 | 528,5 | 7,7 |
| 282 | 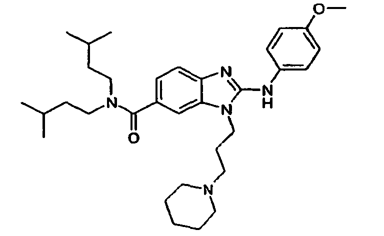 | 548,4 | 8,7 |
| 283 | 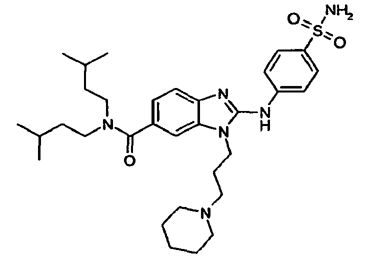 | 597,4 | 8,9 |
| 284 | 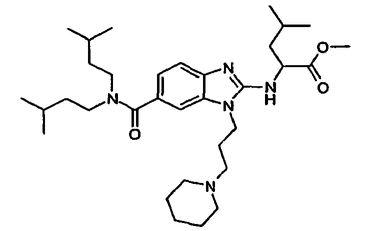 | 570,4 | 8,9 |
| 285 | 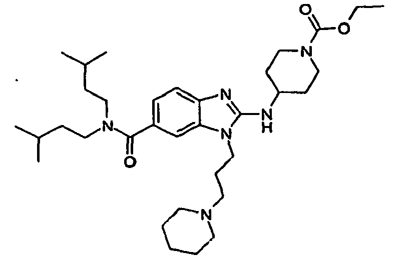 | 597,4 | 8,7 |
| 286 | 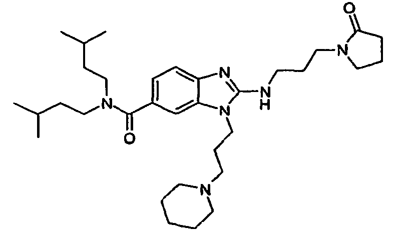 | 567,4 | 8,5 |
| 287 | 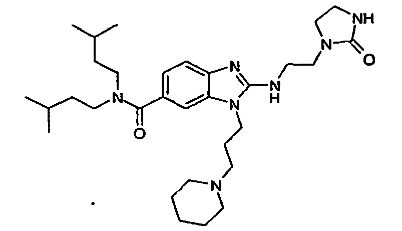 | 554,4 | 8,4 |
| 288 | 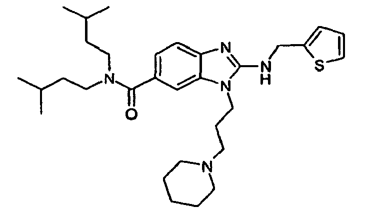 | 538,3 | 8,7 |
| 289 | 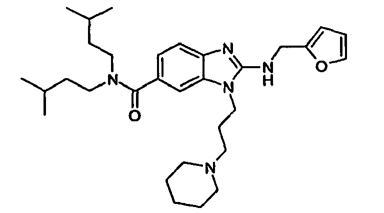 | 522,4 | 8,6 |
| 290 | 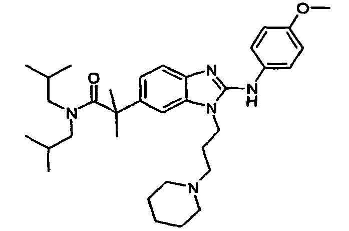 | 562,4 | 8,5 |
| 291 | 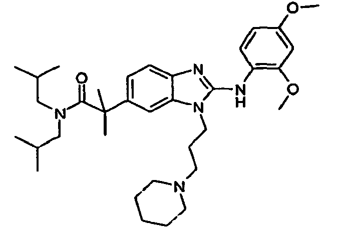 | 592,4 | 8,6 |
| 292 | 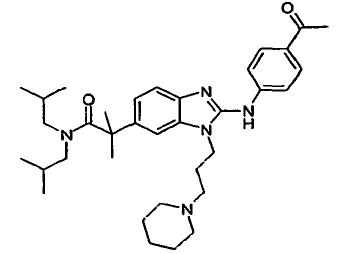 | 574,4 | 8,6 |
| 293 | 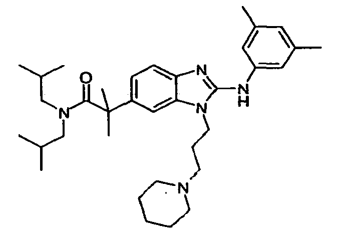 | 560,4 | 8,7 |
| 294 | 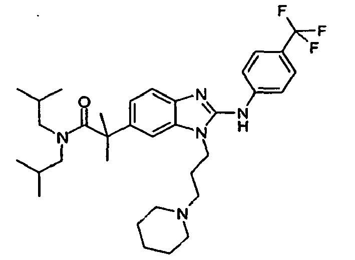 | 600,3 | 9,2 |
| 295 | 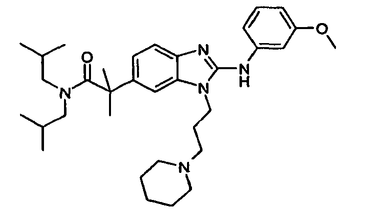 | 562,4 | 8,5 |
| 296 | 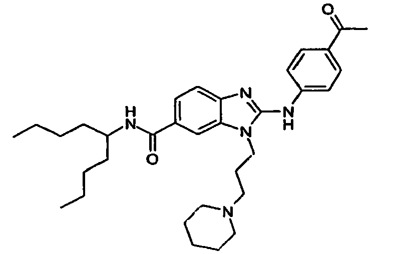 | 546,3 | 8,9 |
| 297 | 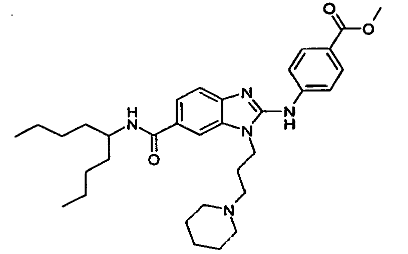 | 562,3 | 9,1 |
| 298 | 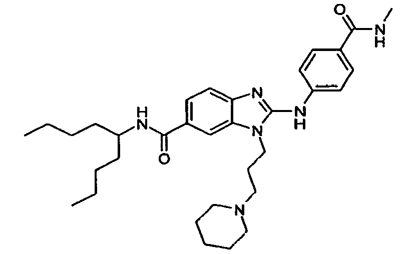 | 561,3 | 8,5 |
| 299 | 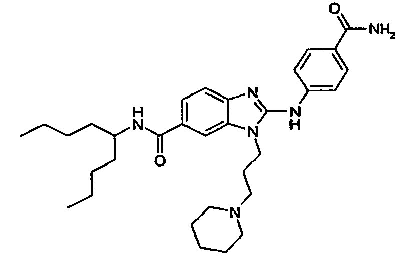 | 547,3 | 8,5 |
| 300 | 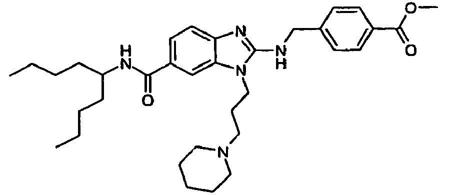 | 576,4 | 8,6 |
| 301 | 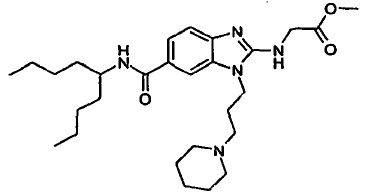 | 500,4 | 8,4 |
| 302 | 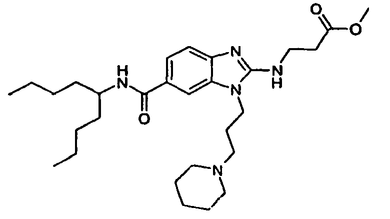 | 514,4 | 8,4 |
| 303 | 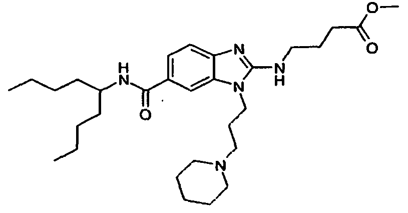 | 528,4 | 8,4 |
| 304 | 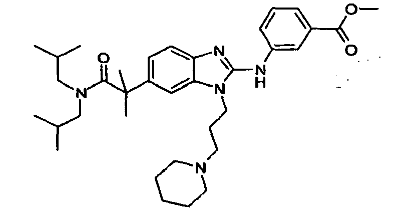 | 590,4 | 8,5 |
| 305 | 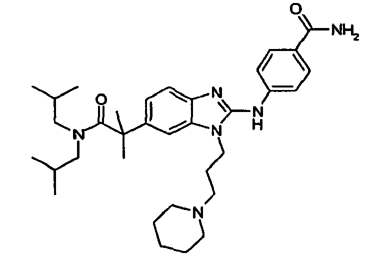 | 575,4 | 8,1 |
| 306 | 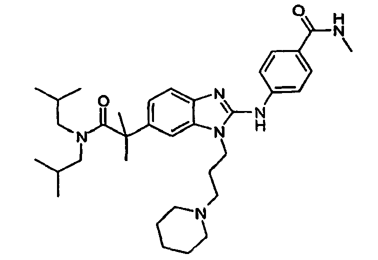 | 589,4 | 8,2 |
| 307 | 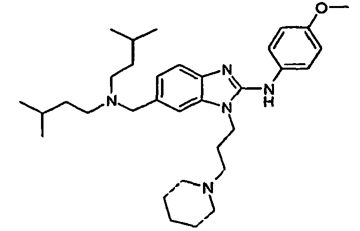 | 534,5 | 7,7 |
| 308 | 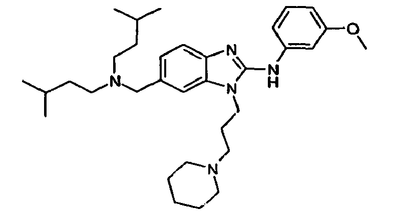 | 534,5 | 7,8 |
| 309 | 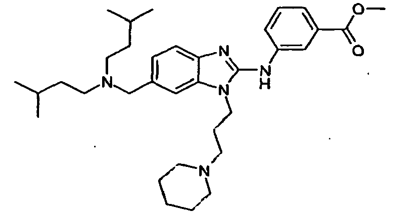 | 562,5 | 7,9 |
| 310 | 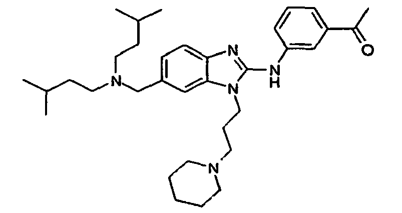 | 546,5 | 7,8 |
| 311 | 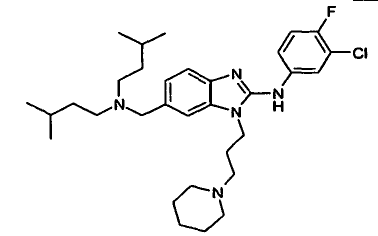 | 556,4 | 8,2 |
| 312 | 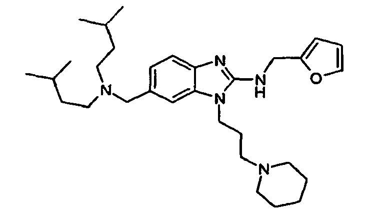 | 508,5 | 7,7 |
| 313 | 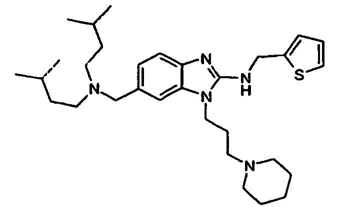 | 524,3 | 7,5 |
| 314 | 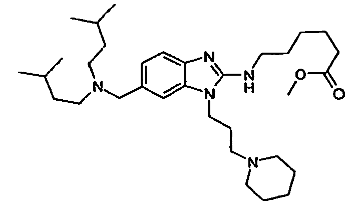 | 556,4 | 7,5 |
| 315 | 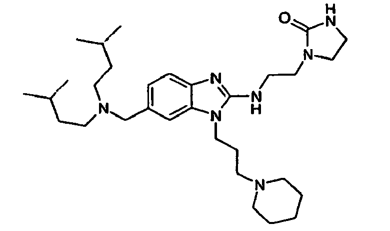 | 540,3 | 7,5 |
| 316 | 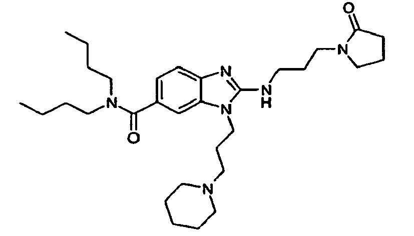 | 539,5 | 7,9 |
| 317 | 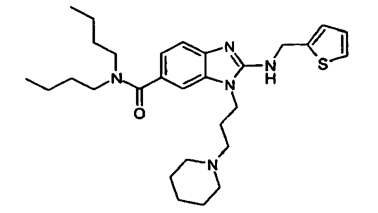 | 510,4 | 8,1 |
| 318 | 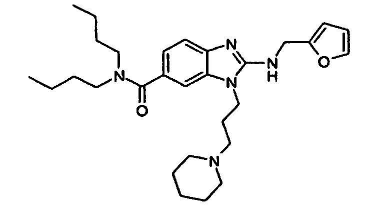 | 494,4 | 8,0 |
| 319 | 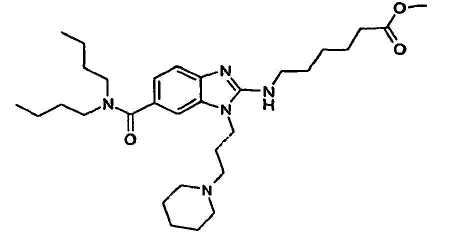 | 542,4 | 8,1 |
| 320 | 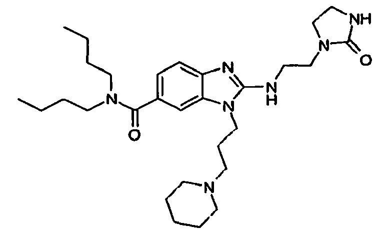 | 526,4 | 7,8 |
| 321 | 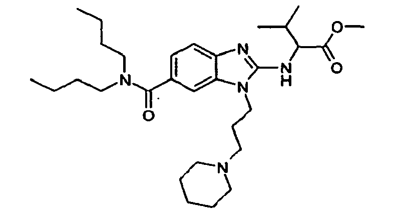 | 528,4 | 8,3 |
| 322 | 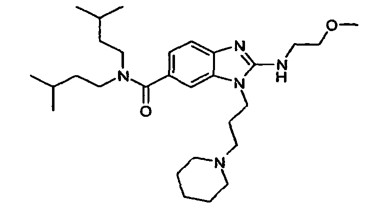 | 500,4 | 8,4 |
| 323 | 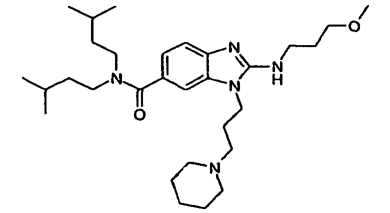 | 514,4 | 8,4 |
| 324 | 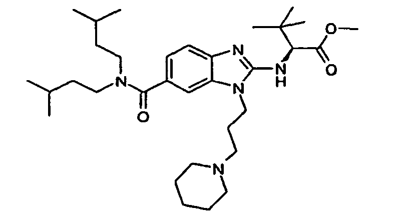 | 570,4 | 8,8 |
| 325 | 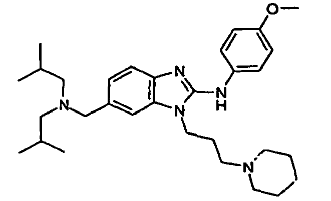 | 506,4 | 7,3 |
| 326 | 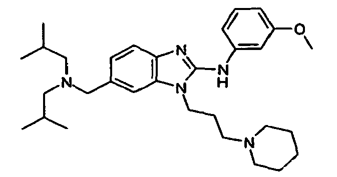 | 506,4 | 7,3 |
| 327 | 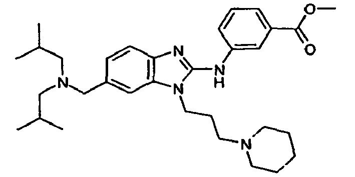 | 534,4 | 7,4 |
| 328 | 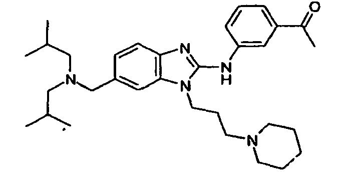 | 518,4 | 7,3 |
| 329 | 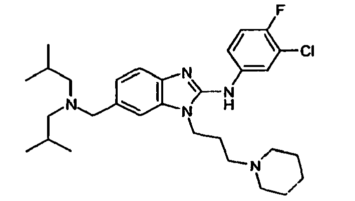 | 528,3 | 7,6 |
| 330 | 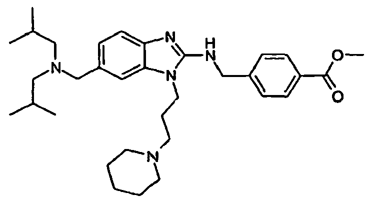 | 548,3 | 7,3 |
| 331 | 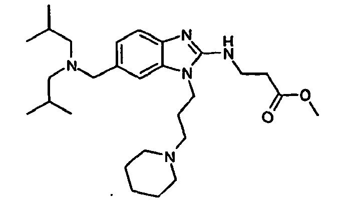 | 486,3 | 7,0 |
| 332 | 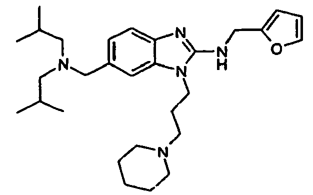 | 480,4 | 7,1 |
| 333 | 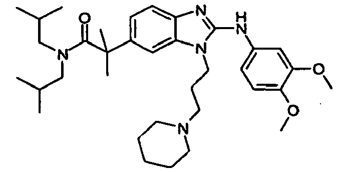 | 592,4 | 8,1 |
| 334 | 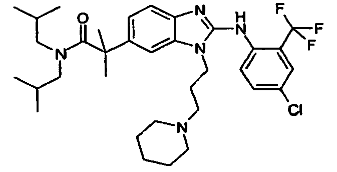 | 634,3 | 9,2 |
| 335 | 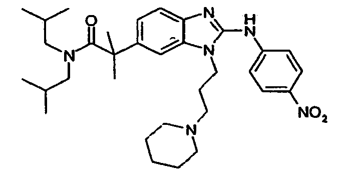 | 577,4 | 9,0 |
| 336 | 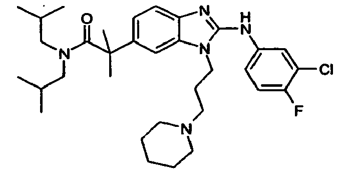 | 584,3 | 8,4 |
| 337 | 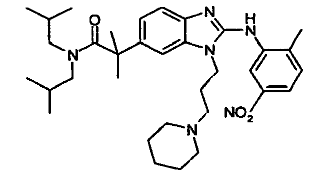 | 591,4 | 8,3 |
| 338 | 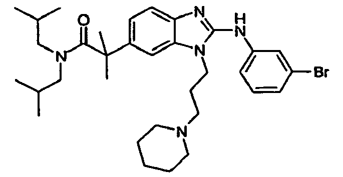 | 610,3 | 8,4 |
| 339 | 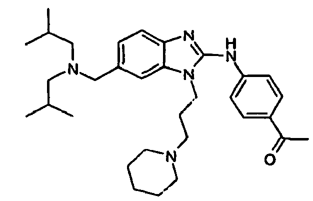 | 518,4 | 7,4 |
| 340 | 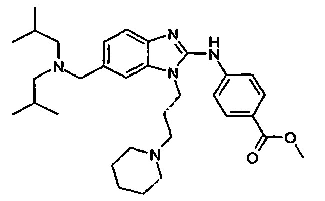 | 534,3 | 7,5 |
| 341 | 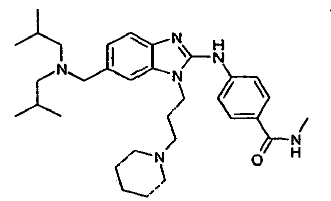 | 533,3 | 7,2 |
| 342 | 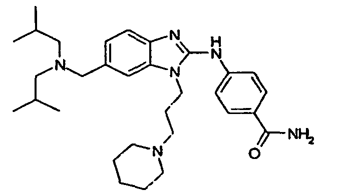 | 519,3 | 7,1 |
| 343 | 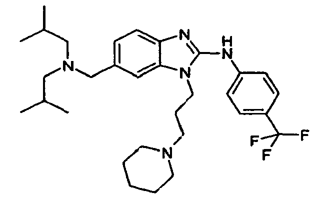 | 544,3 | 7,8 |
| 344 | 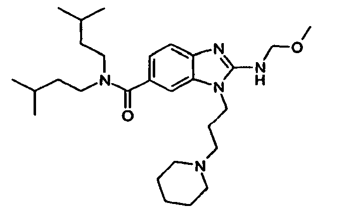 | 486,3 | 8,1 |
| 345 | 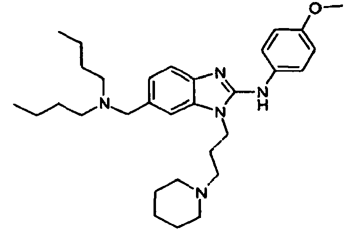 | 506,5 | 7,3 |
| 346 | 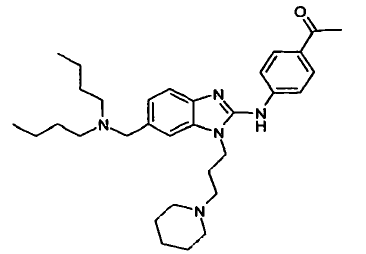 | 518,5 | 7,5 |
| 347 | 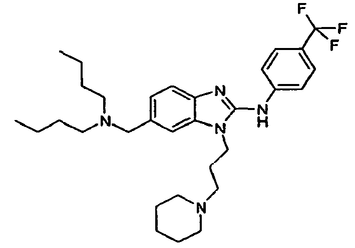 | 544,4 | 7,9 |
| 348 | 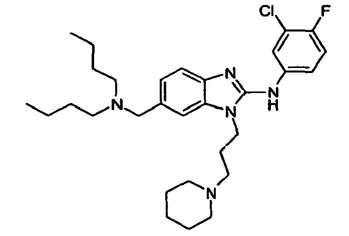 | 528,4 | 7,7 |
| 349 | 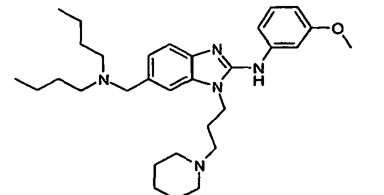 | 506,5 | 7,4 |
| 350 | 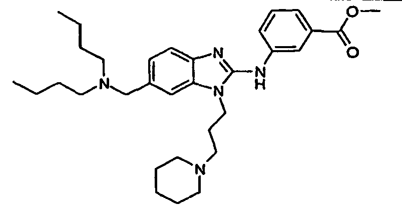 | 534,4 | 7,4 |
| 351 | 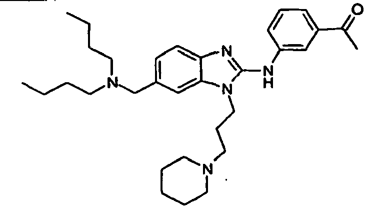 | 518,4 | 7,4 |
| 352 | 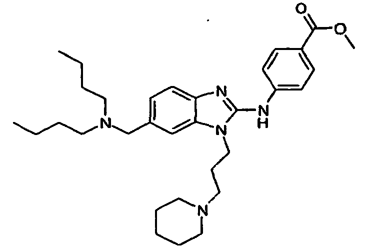 | 534,4 | 7,6 |
| 353 | 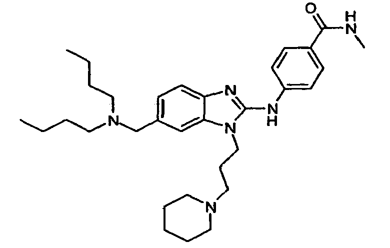 | 533,5 | 7,3 |
| 354 | 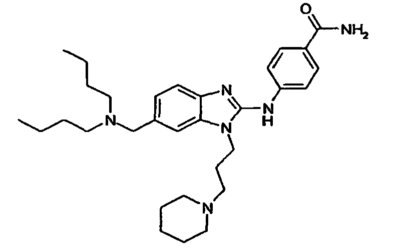 | 519,4 | 7,3 |
| 355 | 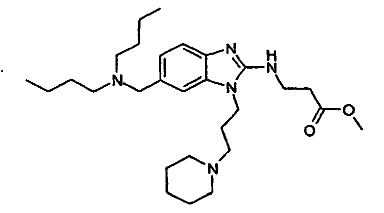 | 486,4 | 7,2 |
| 356 | 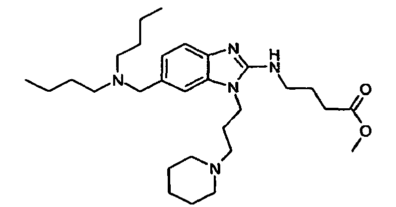 | 500,4 | 7,2 |
| 357 | 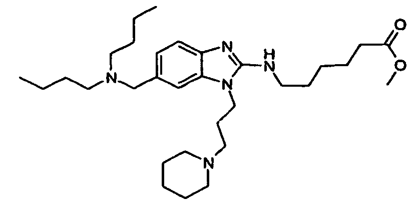 | 528,5 | 7,3 |
| 358 | 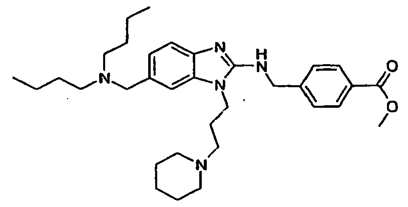 | 548,4 | 7,4 |
| 359 | 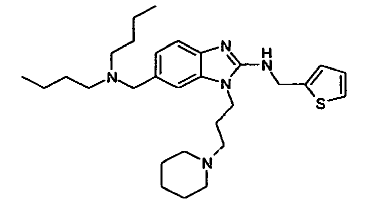 | 496,4 | 7,3 |
| 360 | 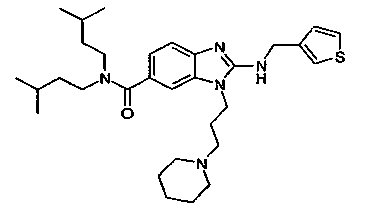 | 538,5 | 8,3 |
| 361 | 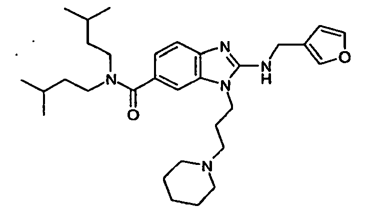 | 522,5 | 8,2 |
| 362 | 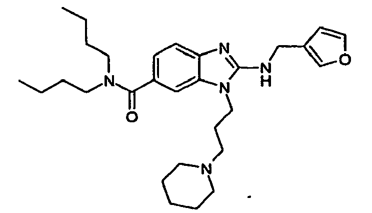 | 494,5 | 8,0 |
| 363 | 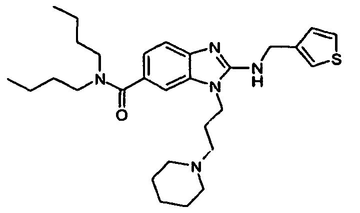 | 510,5 | 8,1 |
| 364 | 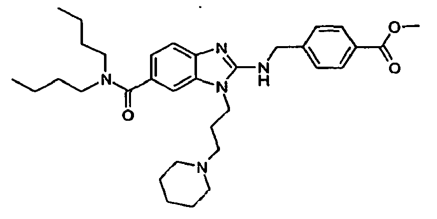 | 562,5 | 8,1 |
| 365 | 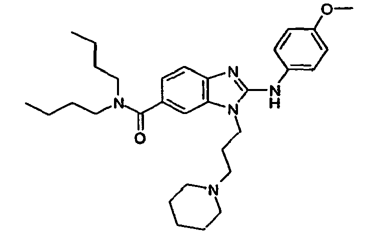 | 520,5 | 8,1 |
| 366 | 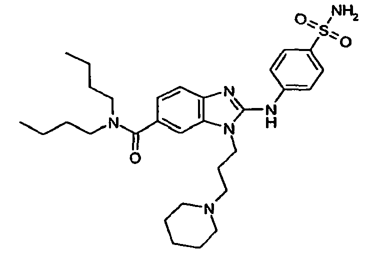 | 569,4 | 8,2 |
| 367 | 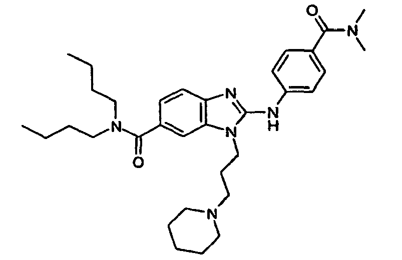 | 561,5 | 7,9 |
| 368 | 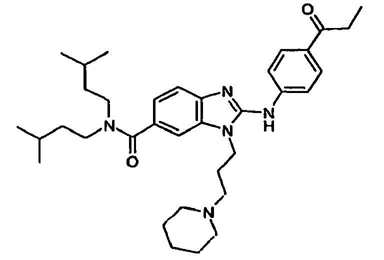 | 574,5 | 9,0 |
| 369 | 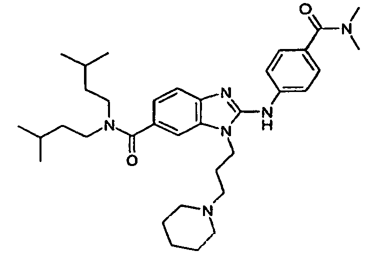 | 589,6 | 8,3 |
| 370 | 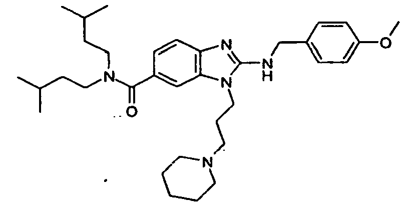 | 562,5 | 8,4 |
| 371 | 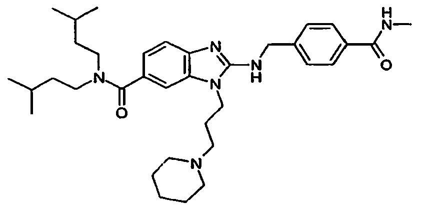 | 589,6 | 8,1 |
| 372 | 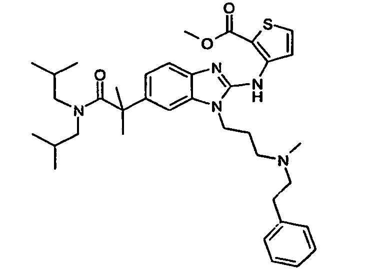 | 646,4 | 9,9 |
| 373 | 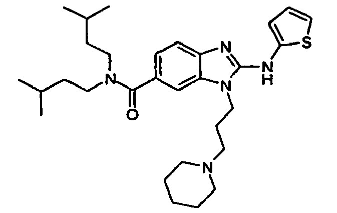 | 524,4 | 8,7 |
| 374 | 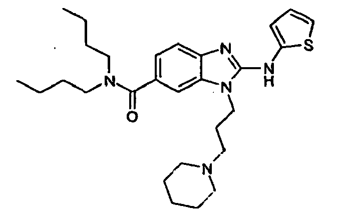 | 496,4 | 8,3 |
| 375 | 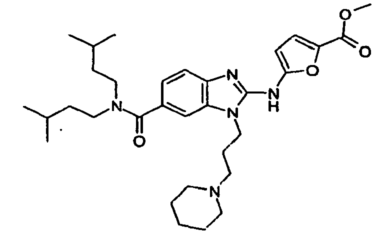 | 566,4 | 9,9 |
| 376 | 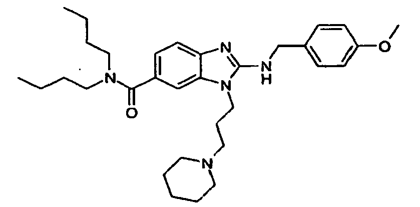 | 534,4 | 8,2 |
| 377 | 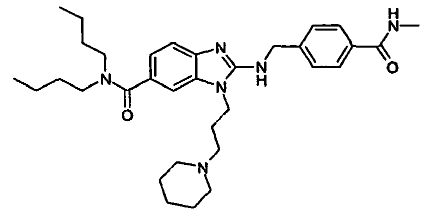 | 561,4 | 7,9 |
| 378 | 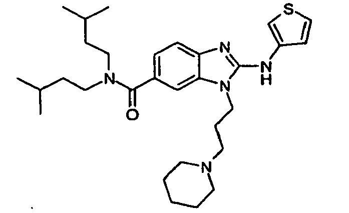 | 524,4 | 8,4 |
| 379 | 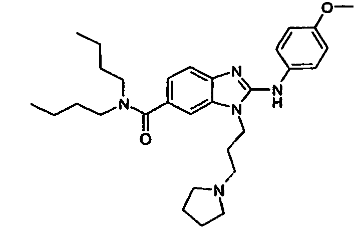 | 506,4 | 8,1 |
| 380 | 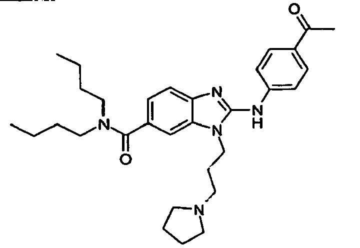 | 518,4 | 8,4 |
| 381 | 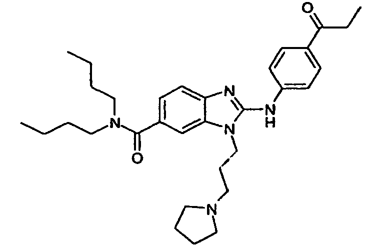 | 532,4 | 8,6 |
| 382 | 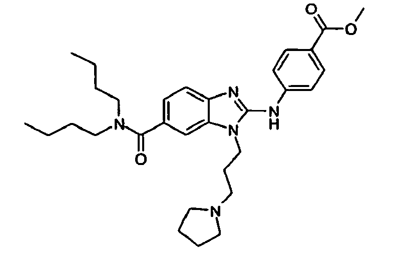 | 534,4 | 8,5 |
| 383 | 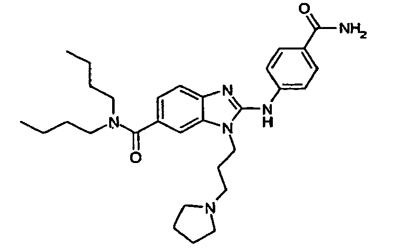 | 519,4 | 7,9 |
| 384 | 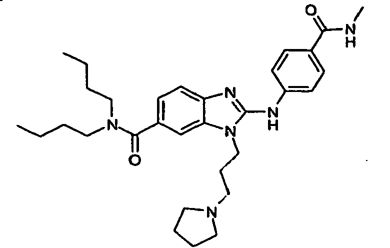 | 533,4 | 7,9 |
| 385 | 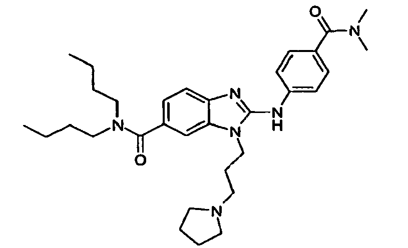 | 547,4 | 7,9 |
| 386 | 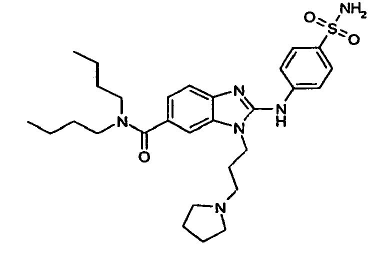 | 555,3 | 8,2 |
| 387 | 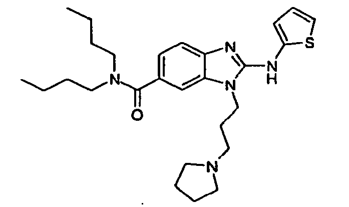 | 482,5 | 8,2 |
| 388 | 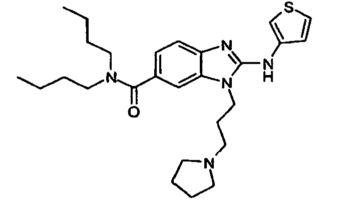 | 482,3 | 8,0 |
| 389 | 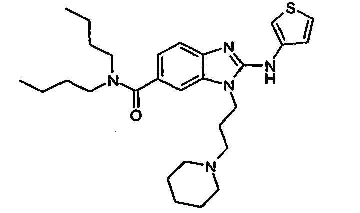 | 496,4 | 8,1 |
| 390 | 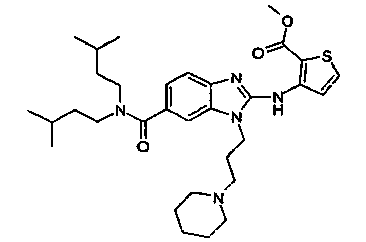 | 582,4 | 10,3 |
| 391 | 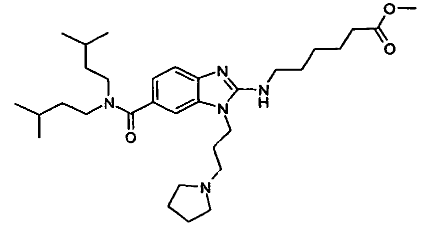 | 556,5 | 8,4 |
| 392 | 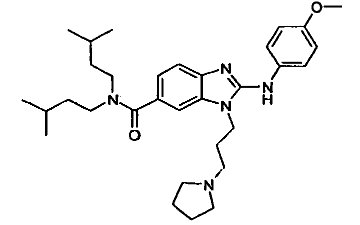 | 534,4 | 8,4 |
| 393 | 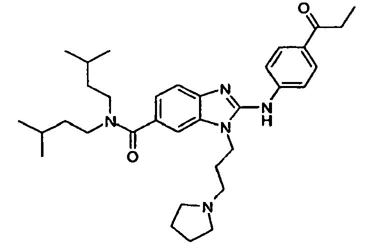 | 560,4 | 9,0 |
| 394 | 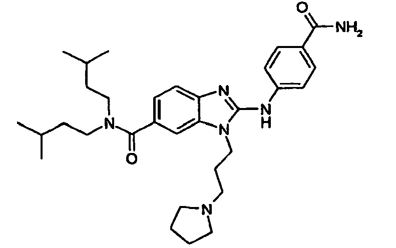 | 547,4 | 8,2 |
| 395 | 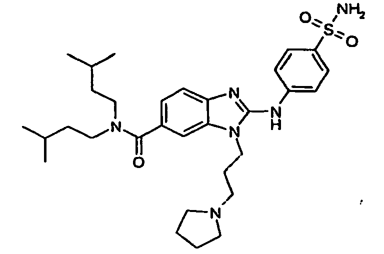 | 583,4 | 8,5 |
| 396 | 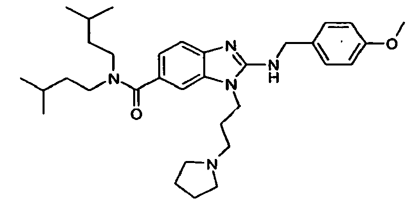 | 548,4 | 8,4 |
| 397 | 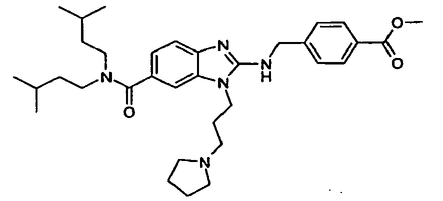 | 576,4 | 8,4 |
| 398 | 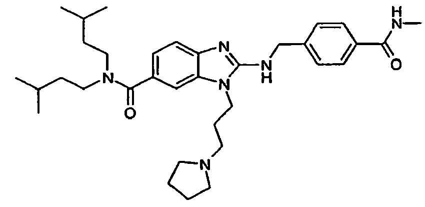 | 575,5 | 8,1 |
| 399 | 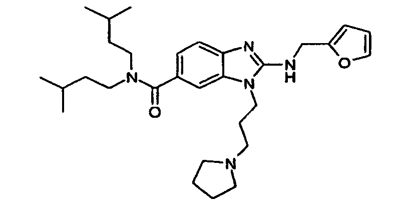 | 508,4 | 8,3 |
| 400 | 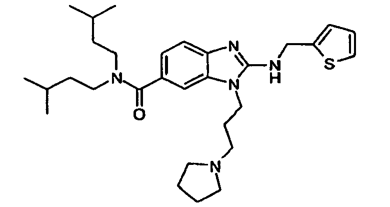 | 524,4 | 8,4 |
| 401 | 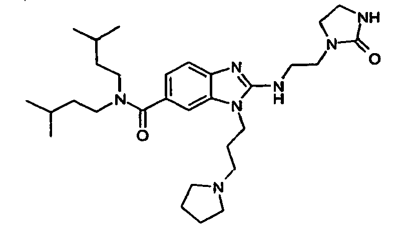 | 540,4 | 8,1 |
| 402 | 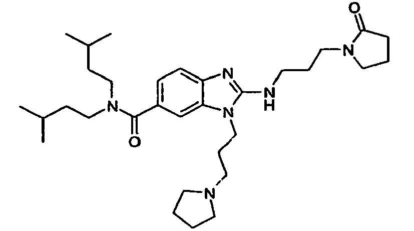 | 553,5 | 8,1 |
| 403 | 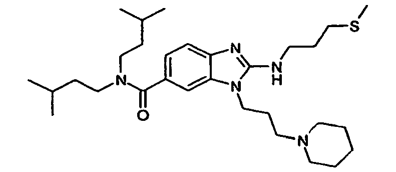 | 530,4 | 8,4 |
| 404 | 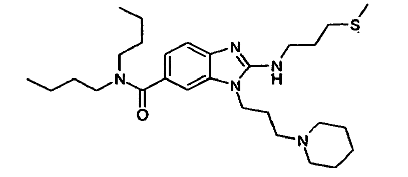 | 502,4 | 8,1 |
| 405 | 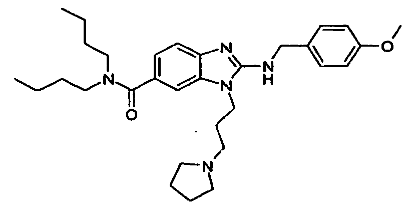 | 520,4 | 8,2 |
| 406 | 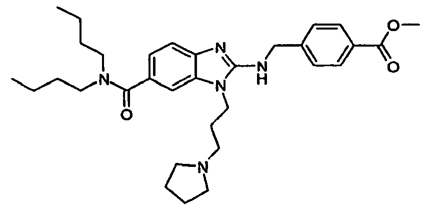 | 548,4 | 8,2 |
| 407 | 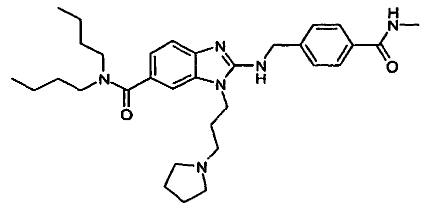 | 547,4 | 7,9 |
| 408 | 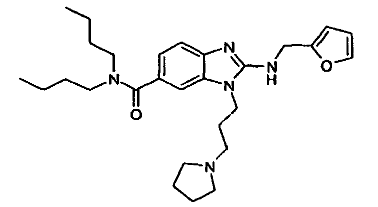 | 480,4 | 8,0 |
| 409 | 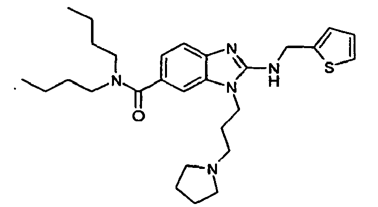 | 496,4 | 8,1 |
| 410 | 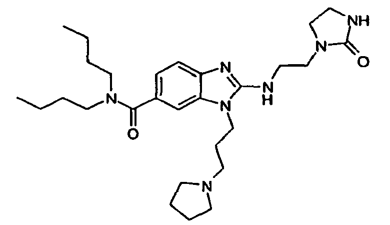 | 512,4 | 7,8 |
| 411 | 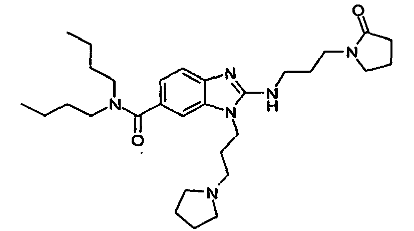 | 525,4 | 7,9 |
| 412 | 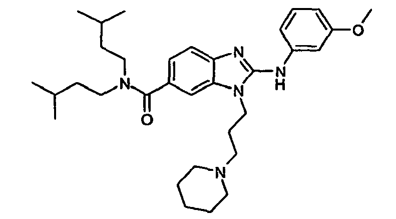 | 548,4 | 8,6 |
| 413 | 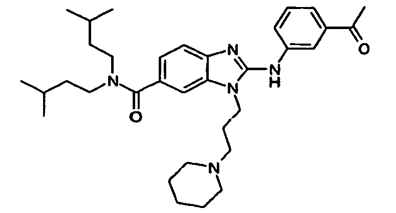 | 560,4 | 8,6 |
| 414 | 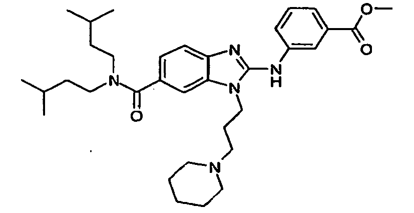 | 576,4 | 8,8 |
| 415 | 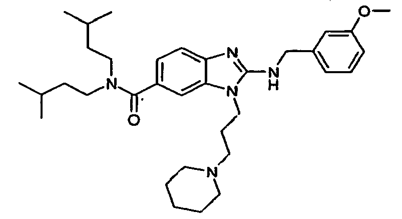 | 562,4 | 8,5 |
| 416 | 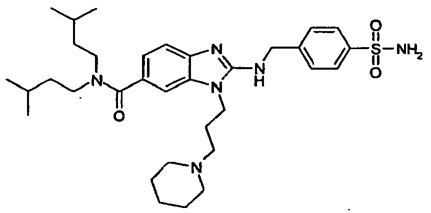 | 611,4 | 8,3 |
| 417 | 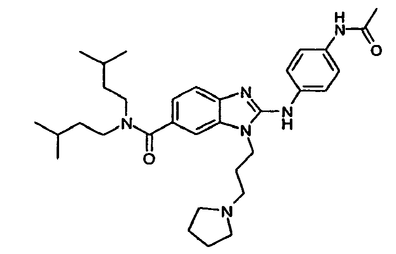 | 561,4 | 8,2 |
| 418 | 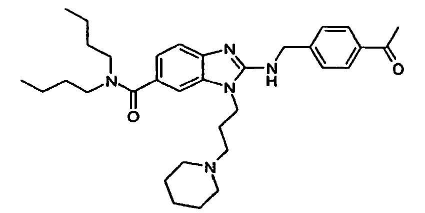 | 546,6 | 8,1 |
| 419 | 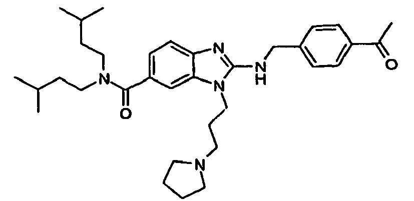 | 560,6 | 8,3 |
| 420 | 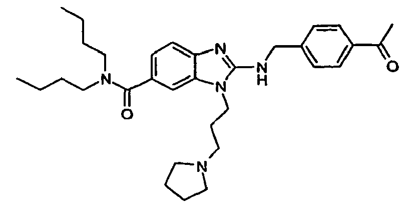 | 532,6 | 8,0 |
| 421 | 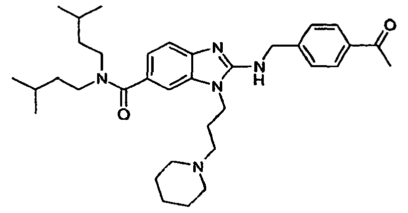 | 574,4 | 8,3 |
| 422 | 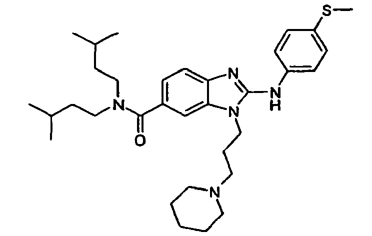 | 564,6 | 8,7 |
| 423 | 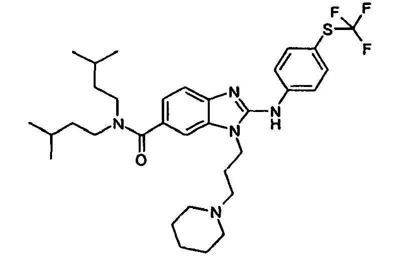 | 618,6 | 9,9 |
| 424 | 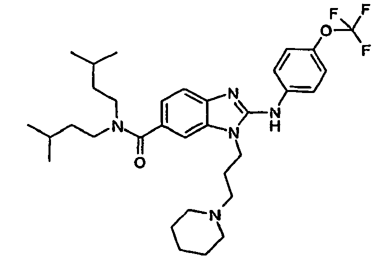 | 602,6 | 9,1 |
| 425 | 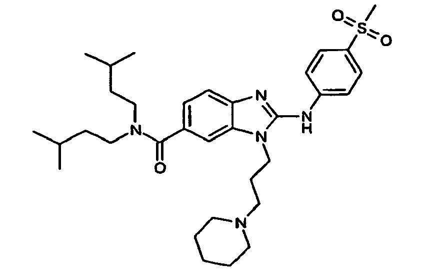 | 596,6 | 8,9 |
| 426 | 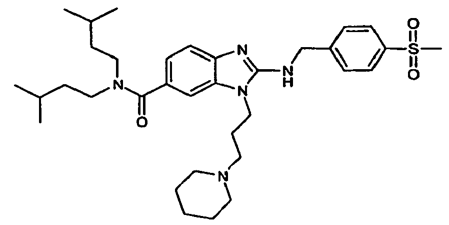 | 610,7 | 8,3 |
| 427 | 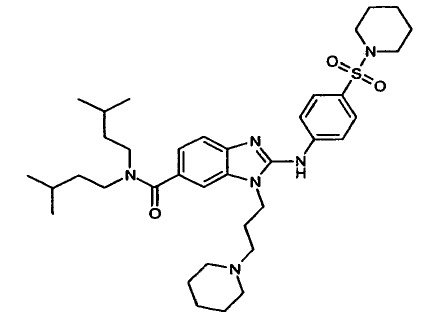 | 665,7 | 9,6 |
| 428 | 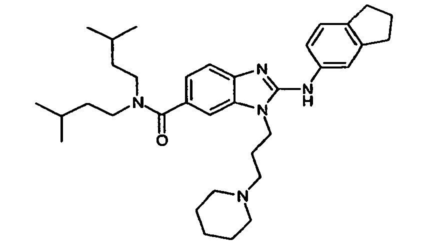 | 558,7 | 8,7 |
| 429 | 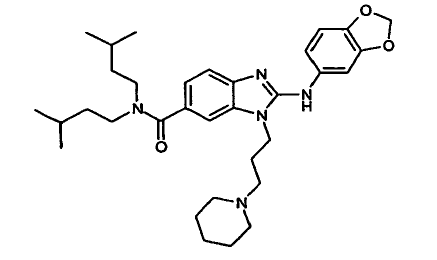 | 562,6 | 8,4 |
| 430 | 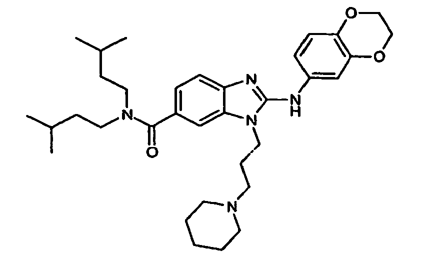 | 576,6 | 8,5 |
| 431 | 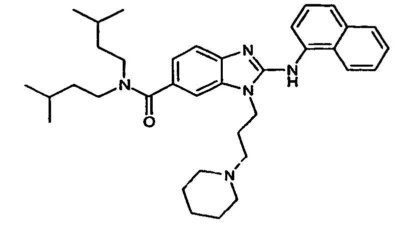 | 568,6 | 8,6 |
| 432 | 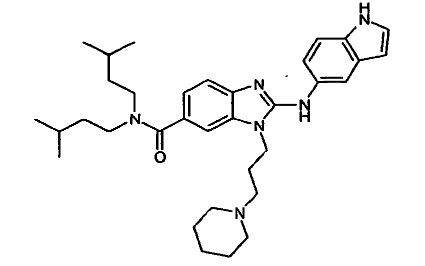 | 557,6 | 8,5 |
| 433 | 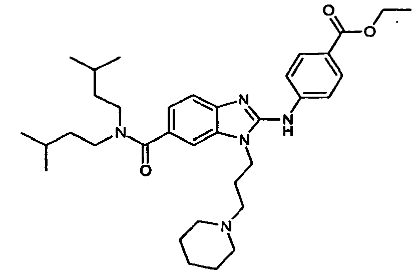 | 590,3 | 9,3 |
| 434 | 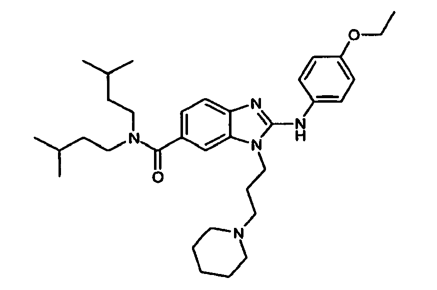 | 562,3 | 8,6 |
| 435 | 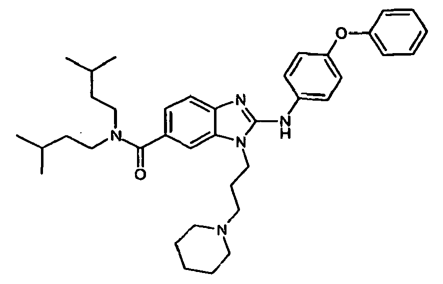 | 610,3 | 9,0 |
| 436 | 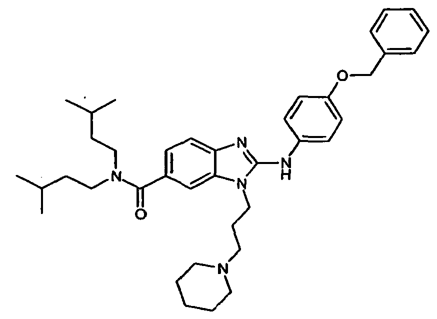 | 624,4 | 9,0 |
| 437 | 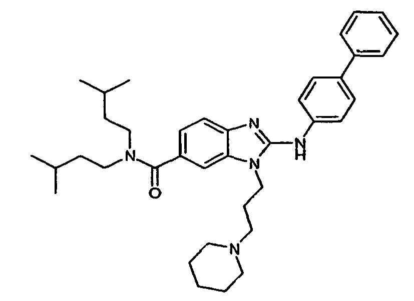 | 594,3 | 9,1 |
| 438 | 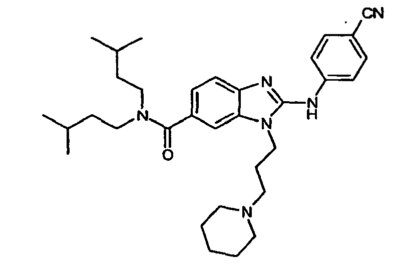 | 543,3 | 9,3 |
| 439 | 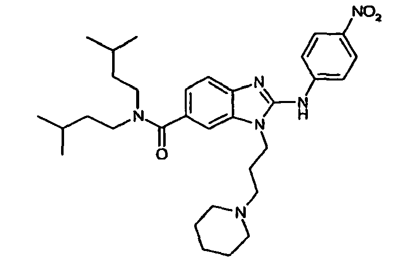 | 563,2 | 9,7 |
| 440 | 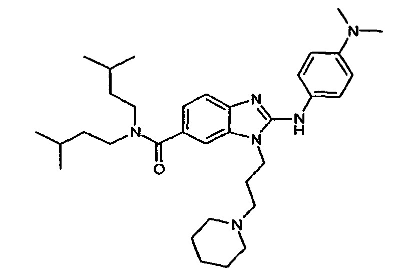 | 561,3 | 8,4 |
| 441 | 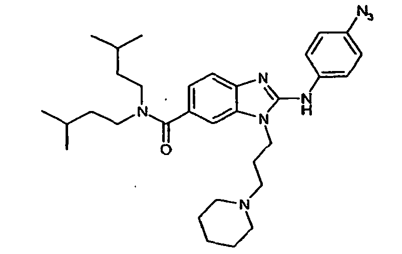 | 559,3 | 8,8 |
| 442 | 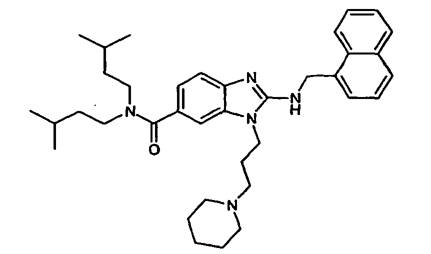 | 582,3 | 8,8 |
| 443 | 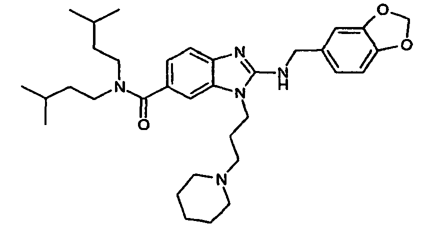 | 576,2 | 8,5 |
| 444 | 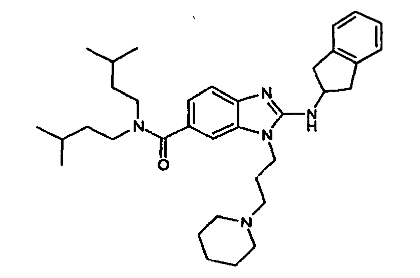 | 558,3 | 8,6 |
| 445 | 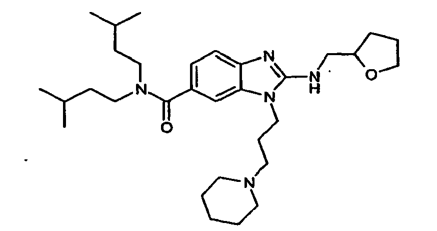 | 526,3 | 8,3 |
| 446 | 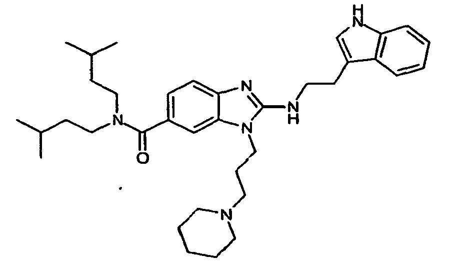 | 585,3 | 8,6 |
| 447 | 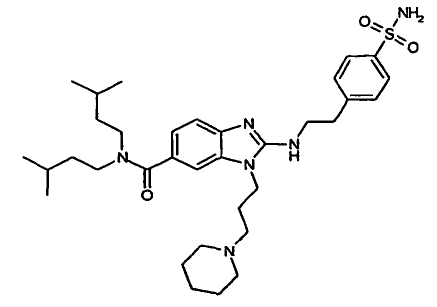 | 625,4 | 8,3 |
| 448 | 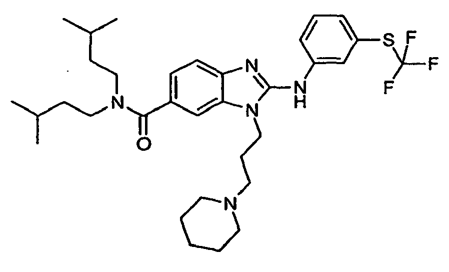 | 618,4 | 9,6 |
| 449 | 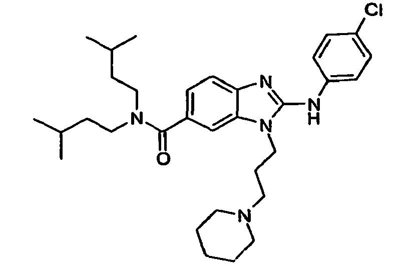 | 552,4 | 8,8 |
| 450 | 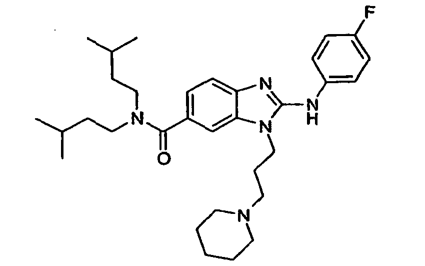 | 536,4 | 8,5 |
| 451 | 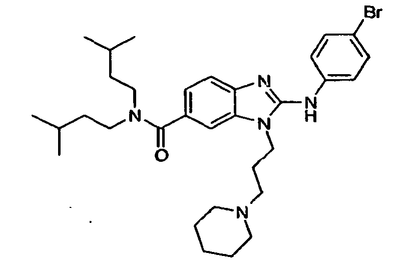 | 596,3 | 8,9 |
| 452 | 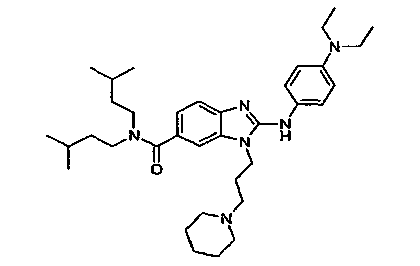 | 589,5 | 8,2 |
| 453 | 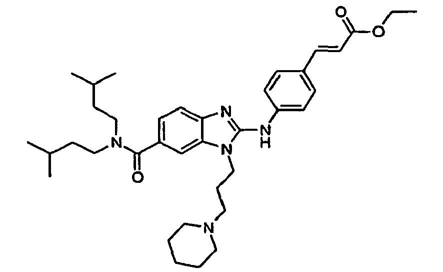 | 616,5 | 9,1 |
| 454 | 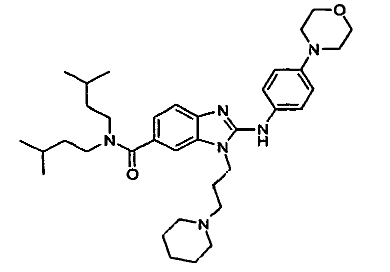 | 603,5 | 8,3 |
| 455 | 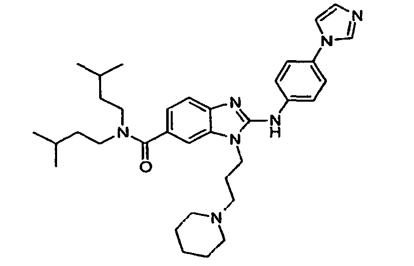 | 584,5 | 8,0 |
| 456 | 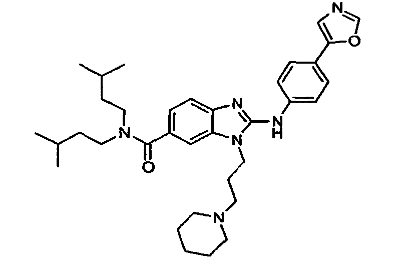 | 585,4 | 8,6 |
| 457 | 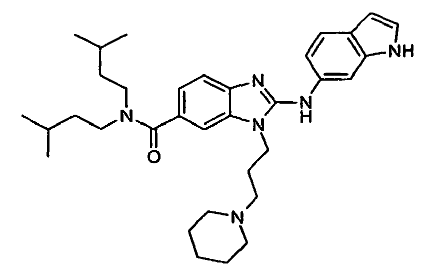 | 557,4 | 8,5 |
| 458 | 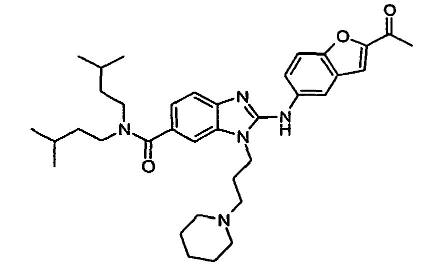 | 600,4 | 8,5 |
| 459 | 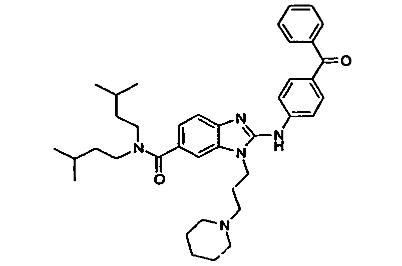 | 622,4 | 9,1 |
| 460 | 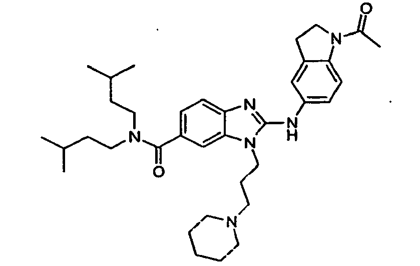 | 601,4 | 8,7 |
| 461 | 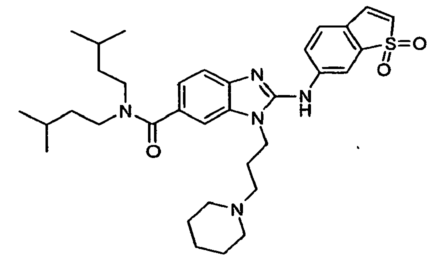 | 623,4 | 9,0 |
| 462 | 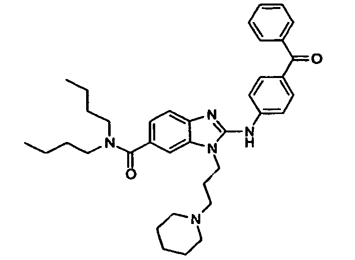 | 594,4 | 8,6 |
| 463 | 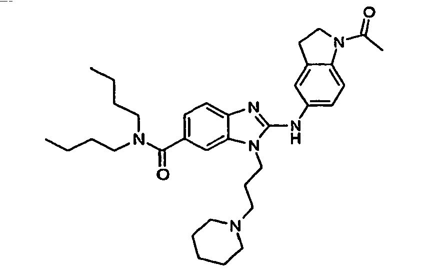 | 573,4 | 8,3 |
| 464 | 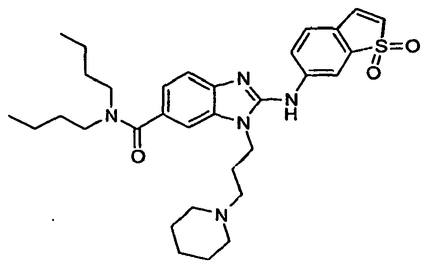 | 595,3 | 8,7 |
| 465 | 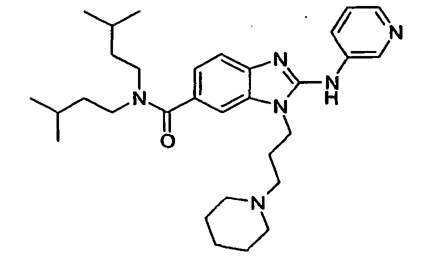 | 519,4 | 8,1 |
| 466 | 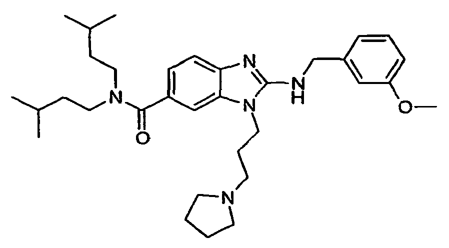 | 548,4 | 8,4 |
| 467 | 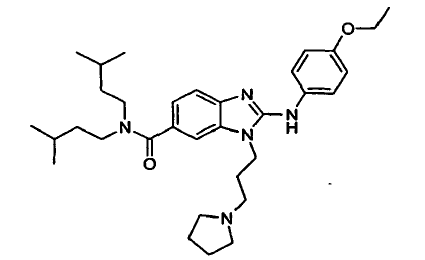 | 548,4 | 8,5 |
| 468 | 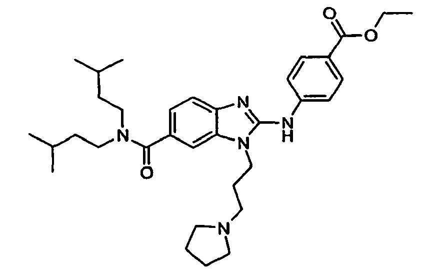 | 576,4 | 9,1 |
| 469 | 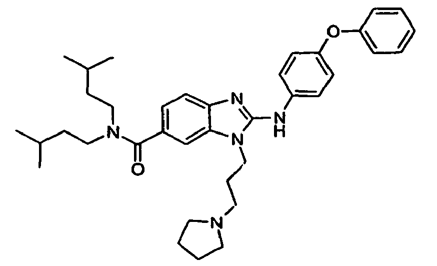 | 596,4 | 8,9 |
| 470 | 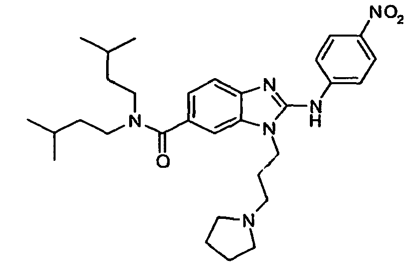 | 549,3 | 9,5 |
| 471 | 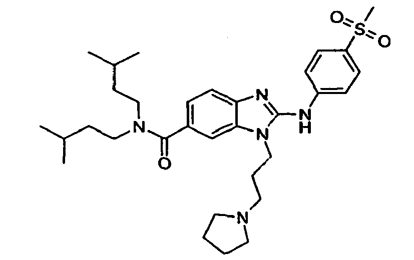 | 582,3 | 8,8 |
| 472 | 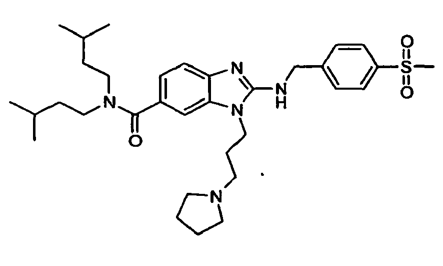 | 596,4 | 8,2 |
| 473 | 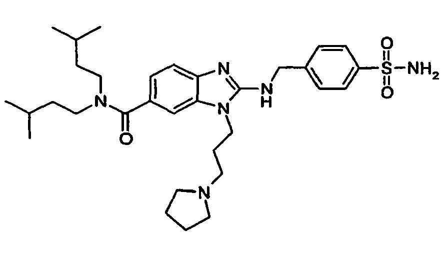 | 597,4 | 8,2 |
| 474 | 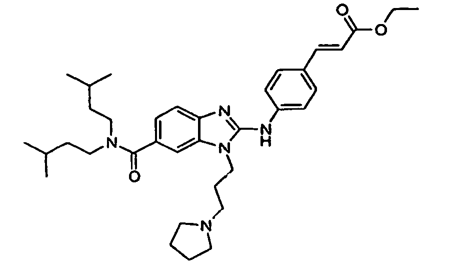 | 602,4 | 9,0 |
| 475 | 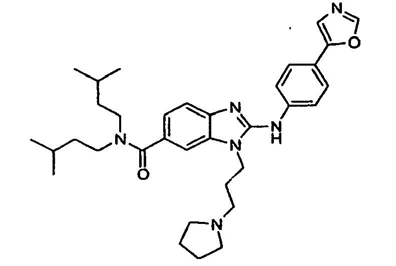 | 571,4 | 8,5 |
| 476 | 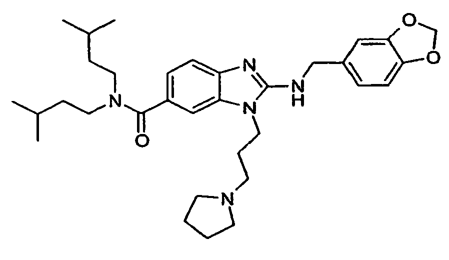 | 562,4 | 8,4 |
| 477 | 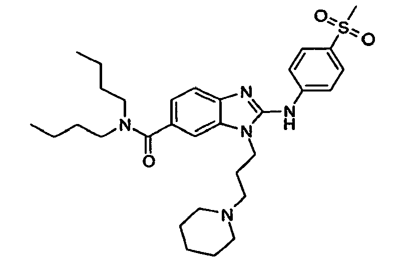 | 568,3 | 8,3 |
| 478 | 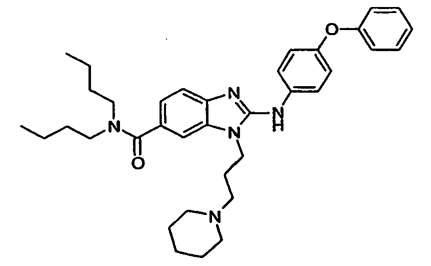 | 582,3 | 8,5 |
| 479 | 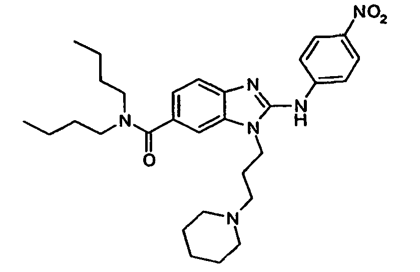 | 535,4 | 9,1 |
| 480 | 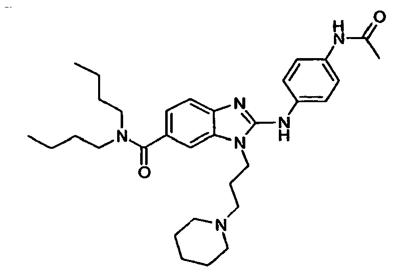 | 547,3 | 7,8 |
| 481 | 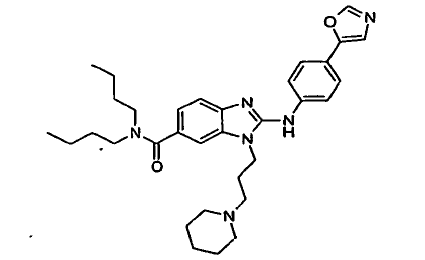 | 557,4 | 8,2 |
| 482 | 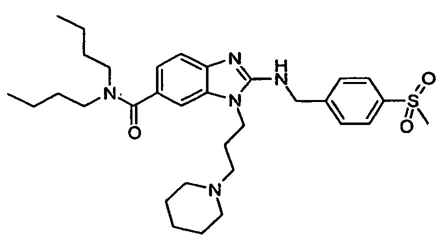 | 582,3 | 7,9 |
| 483 | 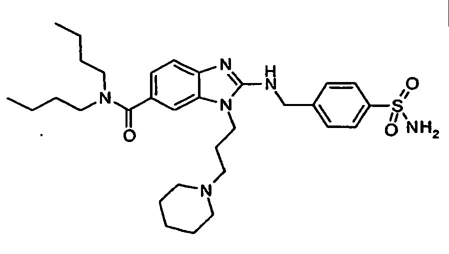 | 583,3 | 7,8 |
| 484 | 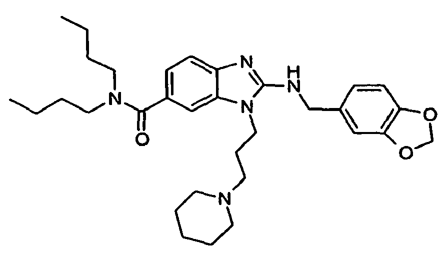 | 548,4 | 8,2 |
| 485 | 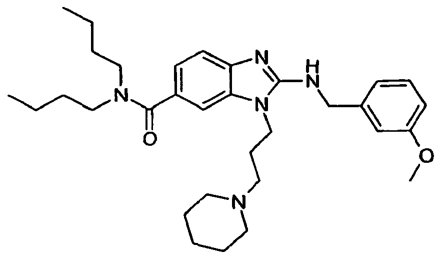 | 534,4 | 8,2 |
| 486 | 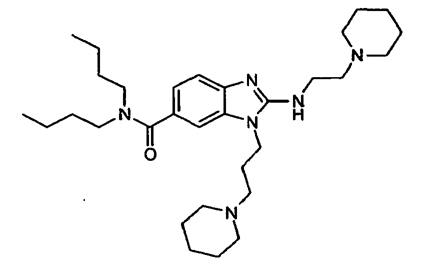 | 525,4 | 7,6 |
| 487 | 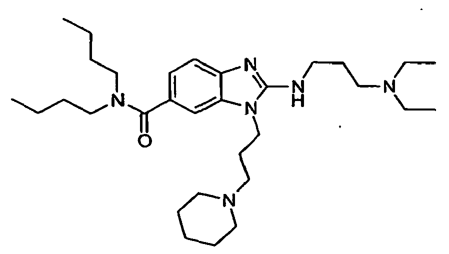 | 527,5 | 7,5 |
| 488 | 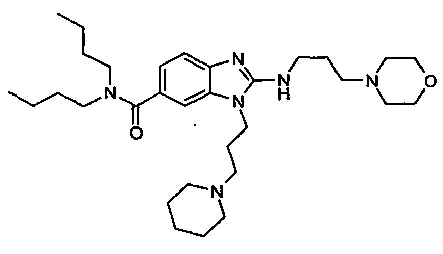 | 541,4 | 7,5 |
| 489 | 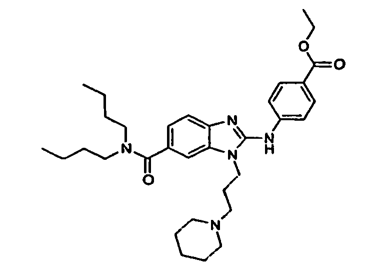 | 562,4 | 8,7 |
| 490 | 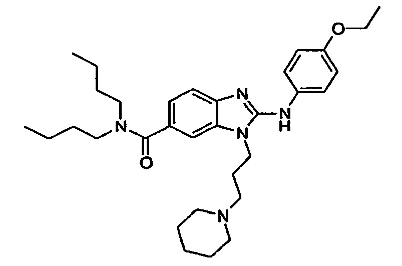 | 534,4 | 8,2 |
| 491 | 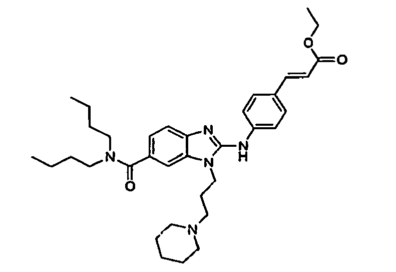 | 588,4 | 8,7 |
| 492 | 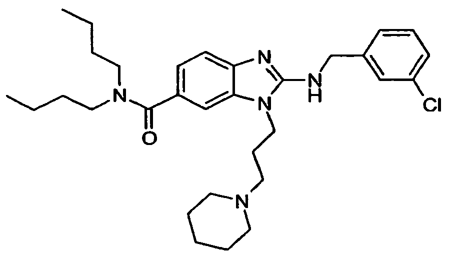 | 538,3 | 8,3 |
| 493 | 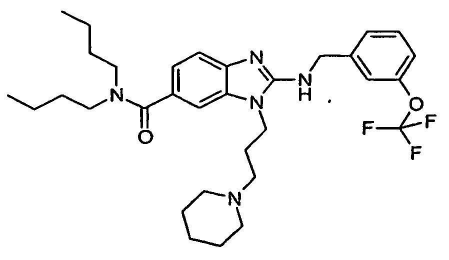 | 588,3 | 8,5 |
| 494 | 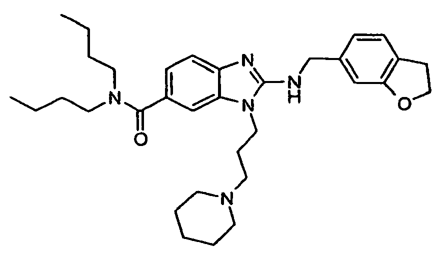 | 546,4 | 8,2 |
| 495 | 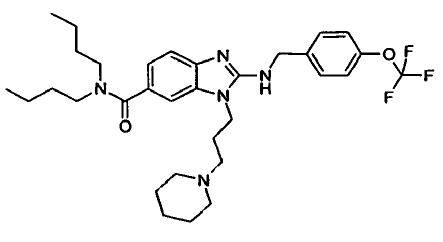 | 588,3 | 8,5 |
| 496 | 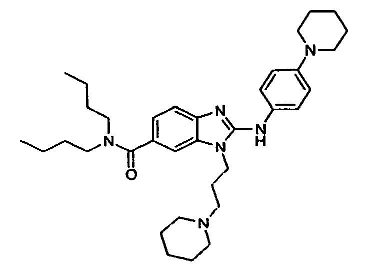 | 573,4 | 7,9 |
| 497 | 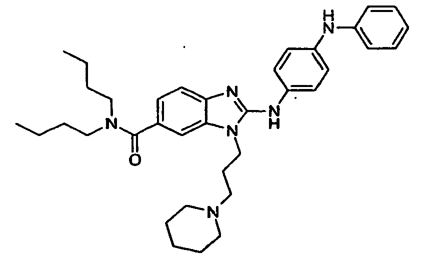 | 581,4 | 8,5 |
| 498 | 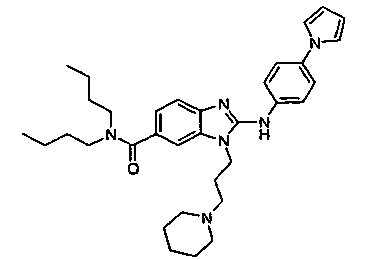 | 555,4 | 8,4 |
| 499 | 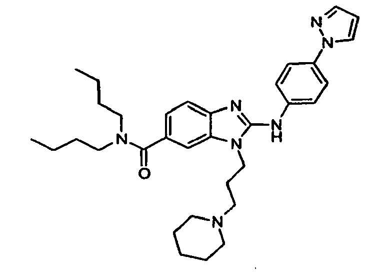 | 556,3 | 8,2 |
| 500 | 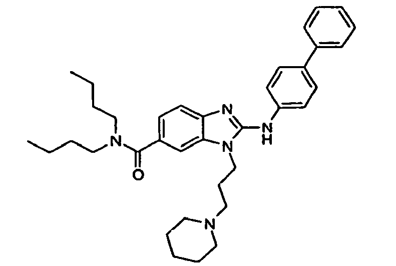 | 566,4 | 8,7 |
| 501 | 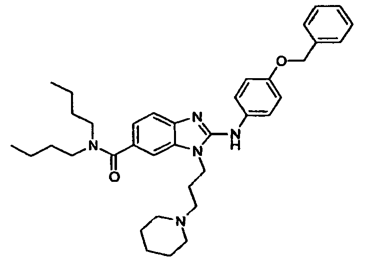 | 596,4 | 8,6 |
| 502 | 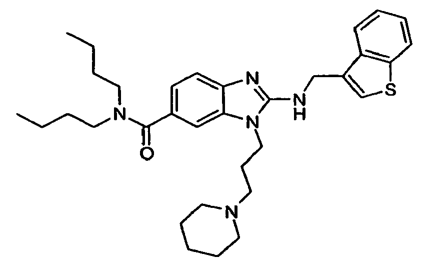 | 560,3 | 8,3 |
| 503 | 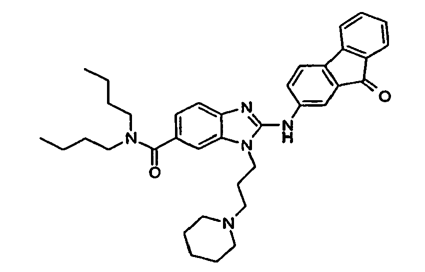 | 592,3 | 8,8 |
| 504 | 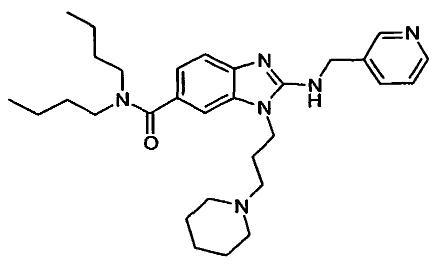 | 505,4 | 7,7 |
| 505 | 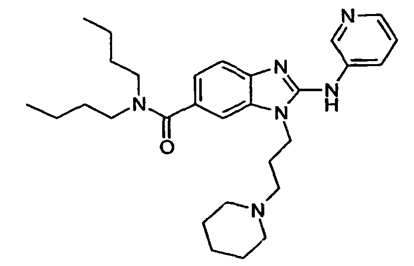 | 491,4 | 7,9 |
| 506 | 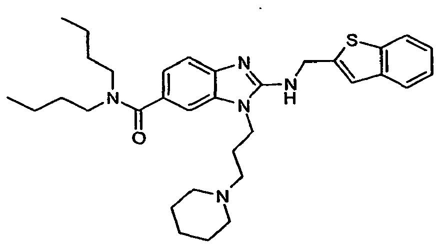 | 560,3 | 8,4 |
| 507 | 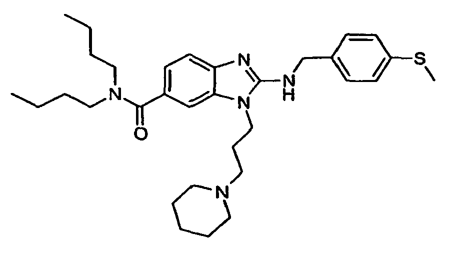 | 550,3 | 8,3 |
| 508 | 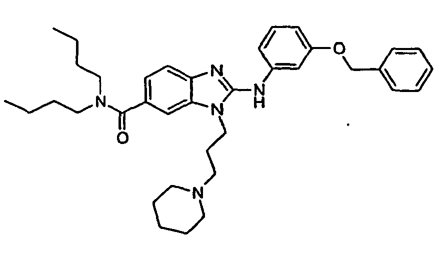 | 596,4 | 8,7 |
| 509 | 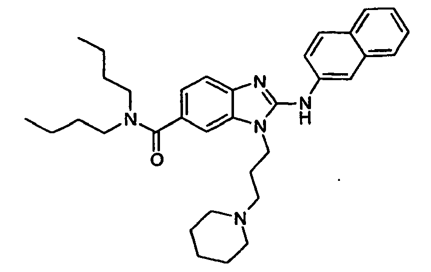 | 540,3 | 8,5 |
| 510 | 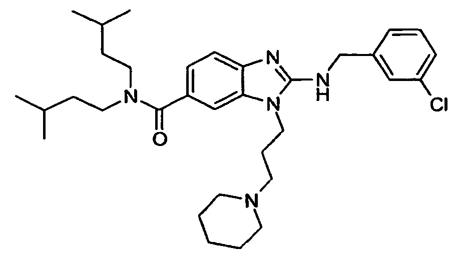 | 566,3 | 8,5 |
| 511 | 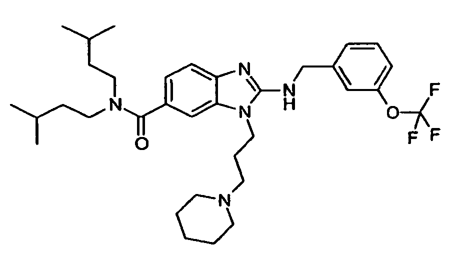 | 616,4 | 8,7 |
| 512 | 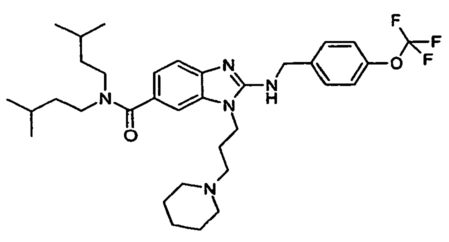 | 616,4 | 8,7 |
| 513 | 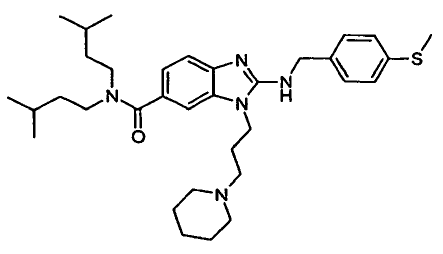 | 578,3 | 8,6 |
| 514 | 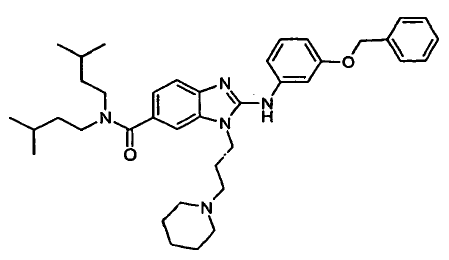 | 624,4 | 9,0 |
| 515 | 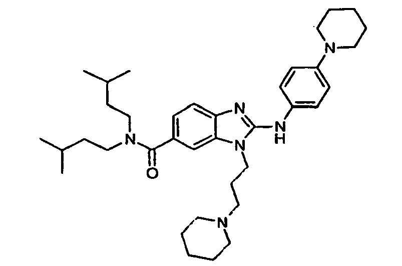 | 601,4 | 8,1 |
| 516 | 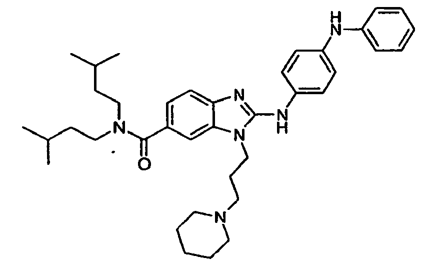 | 609,4 | 8,8 |
| 517 | 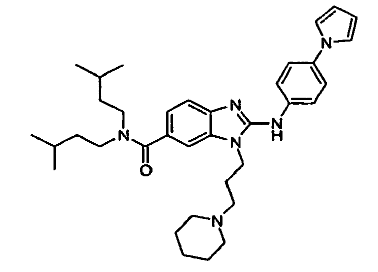 | 583,4 | 8,7 |
| 518 | 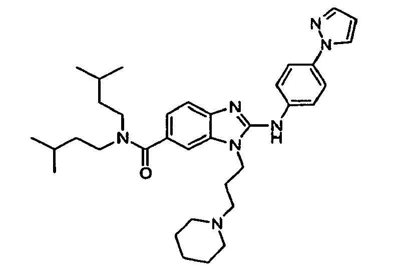 | 584,4 | 8,4 |
| 519 | 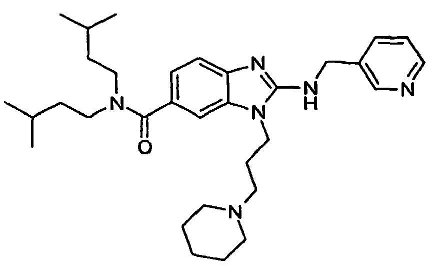 | 533,4 | 7,8 |
| 520 | 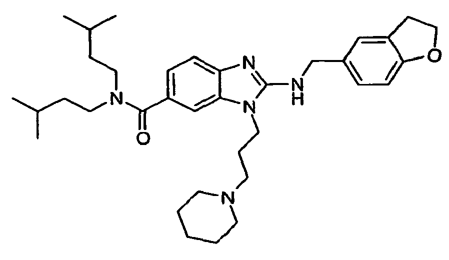 | 574,4 | 8,4 |
| 521 | 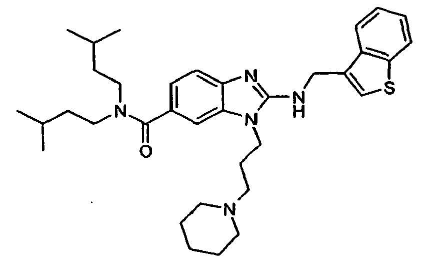 | 588,3 | 8,5 |
| 522 | 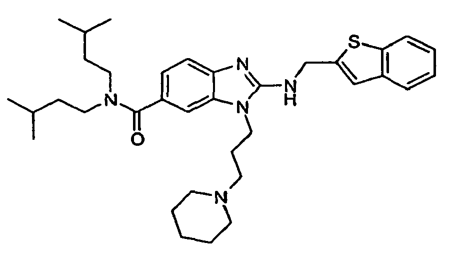 | 588,3 | 8,6 |
| 523 | 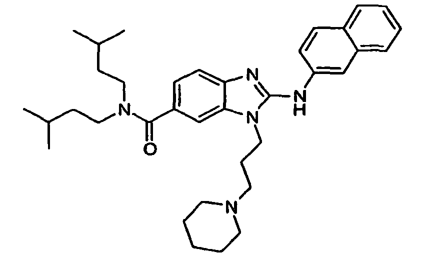 | 568,4 | 8,7 |
| 524 | 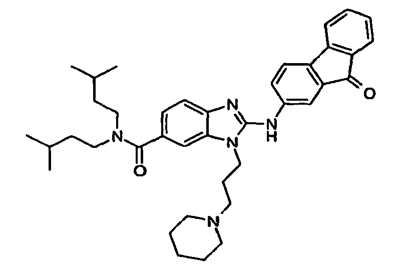 | 620,4 | 9,1 |
| 525 | 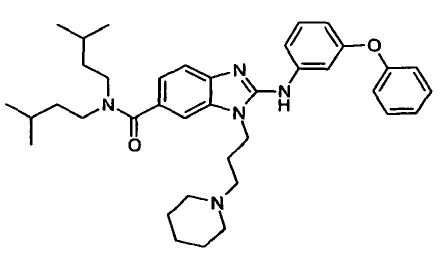 | 610,4 | 9,0 |
| 526 | 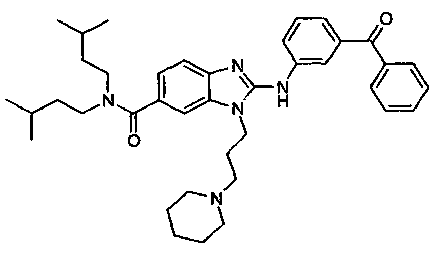 | 622,4 | 9,0 |
| 527 | 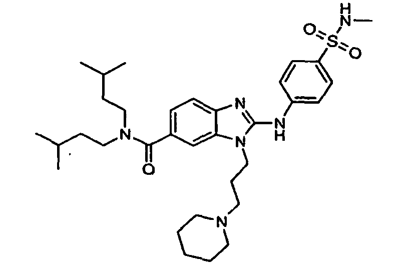 | 611,4 | 8,7 |
| 528 | 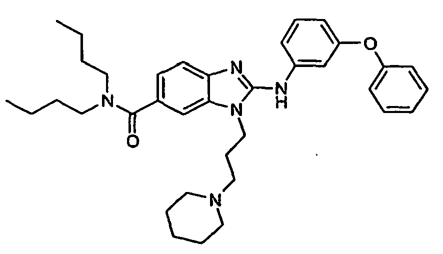 | 582,3 | 8,7 |
| 529 | 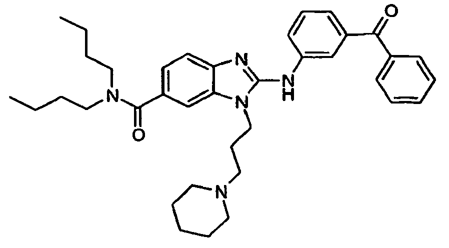 | 594,4 | 8,6 |
| 530 | 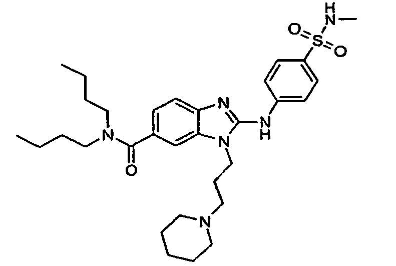 | 583,3 | 8,3 |
| 531 | 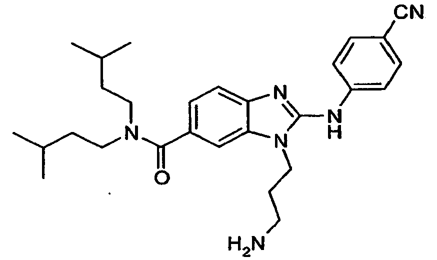 | 475,3 | 8,7 |
| 532 | 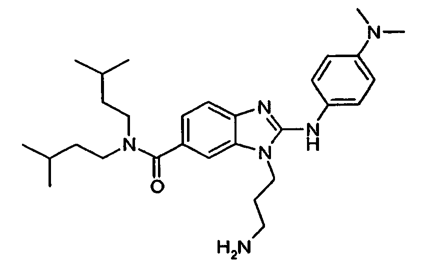 | 493,3 | 8,0 |
| 533 | 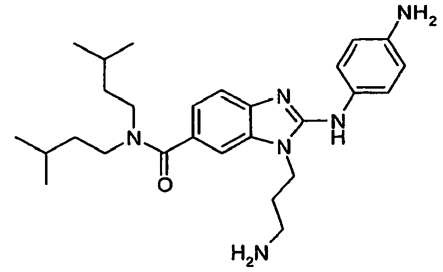 | 465,3 | 7,8 |
| 534 | 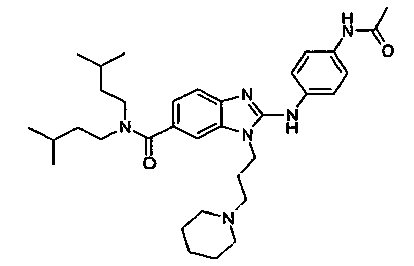 | 575,3 | 8,1 |
| 535 | 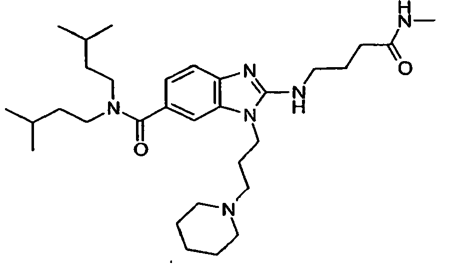 | 541,5 | 8,0 |

### Etude pharmacologique

L'affinité des composés de la présente invention pour les différents sous-types de récepteurs des mélanocortines a été mesurée selon les procédures analogues à celles décrites ci-après pour les récepteurs MC4.

### Etude de l'affinité des composés pour les récepteurs MC4 des mélanocortines :

L'affinité des composés de l'invention pour les récepteurs MC4 est déterminée par la mesure de l'inhibition de la liaison de la [¹²⁵I]-[ Nle⁴, D-Phe⁷]-α-MSH à des préparations membranaines de cellules CHO-K1 transfectées.

Les cellules CHO-K1 exprimant de façon stable les récepteurs MC4 humains sont cultivées dans un milieu RPMI 1640 contenant 10 % de sérum foetal de veau, 2 mM de glutamine, 100 U/ml de pénicilline, 0,1 mg/ml de streptomycine et 0,5 mg/ml de G418. Les cellules sont collectées avec 0,5 mM d'EDTA et centrifugées à 500 g pendant 5 min à 4° C. Le culot est re-suspendu dans un milieu salin avec tampon phosphate (PBS) et centrifugé à 500 g pendant 5 min à 4° C. Le culot est re-suspendu dans un milieu tampon Tris 50 mM à pH 7,4 et centrifugé à 500 g pendant 5 min à 4° C. Les cellules sont lysées par sonication et centrifugées à 39 000 g pendant 10 min à 4° C. Le culot est re-suspendu dans le milieu tampon Tris 50 mM à pH 7,4 et centrifugé à 50 000 g pendant 10 min à 4° C. Les membranes obtenues dans ce dernier culot sont stockées à -80° C.

La mesure de l'inhibition compétitive de la liaison de la [¹²⁵I]-[Nle⁴, D-Phe⁷]-α-MSH sur les récepteurs MC4 est effectuée en duplicats à l'aide de plaques en polypropylène de 96 puits. Les membranes cellulaires (50 µg de protéines/puits) sont incubées avec la [¹²⁵I]-[ Nle⁴, D-Phe⁷]-α-MSH (0,5 nM) pendant 90 min à 37° C dans un milieu tampon Tris-HCl 50 mM, pH 7,4, comprenant 0,2 % d'albumine bovine de sérum (BSA), 5 mM de MgCl₂, et 0,1 mg/ml de bacitracine.

La [¹²⁵I]-[ Nle⁴, D-Phe⁷]-α-MSH liée est séparée de la [¹²⁵I]-[ Nle⁴, D-Phe⁷]-α-MSH libre par filtration à travers des plaques de filtres en fibre de verre GF/C (Unifilter, Packard) pré-imprégné avec 0,1 % de polyéthylènimine (P.E.I.), en utilisant un Filtermate 196 (Packard). Les filtres sont lavés avec du tampon Tris-HCl 50 mM, pH 7,4 à 0-4° C et la radioactivité présente est déterminée à l'aide d'un compteur (Packard Top Count).

La liaison spécifique est obtenue en soustrayant la liaison non spécifique (déterminée en présence de 0,1 µM de Nle⁴, D-Phe⁷-α-MSH) de la liaison totale. Les données sont analysées par régression non-linéaire assistée par ordinateur (MDL) et les valeurs des constantes d'inhibition (Ki) sont déterminées.

L'activité agoniste ou antagoniste des récepteurs MC4 des composés de la présente invention a été déterminée en mesurant la production d'AMP cyclique par les cellules CHO-K1 transfectées par le récepteur MC4.

### Mesure de la production d'AMP cyclique intracellulaire via les récepteurs MC4:

Les cellules CHO-K1 exprimant les récepteurs MC4 des mélanocortines sont cultivées dans des plaques à 384 puits dans un milieu RPMI 1640 avec 10 % de sérum foetal de veau et 0,5 mg/ml de G418. Les cellules sont lavées 2 fois avec 50 µl de milieu RPMI comprenant 0,2 % BSA et 0,5 mM de 3-isobutyl-l-méthylxanthine (IBMX).

Pour mesurer l'effet agoniste d'un composé, les cellules sont incubées pendant 5 min à 37° C en présence de 0,5 mM d' IBMX, puis la stimulation de la production d'AMP cyclique est obtenue en ajoutant le composé à des concentrations comprises entre 1 pM et 10 µM en duplicats pendant 20 min à 37°C. L'effet antagoniste d'un composé est mesuré en inhibant la stimulation de la production d'AMP cyclique induite par la Nle⁴, D-Phe⁷-α-MSH, à des concentrations comprises entre 1 pM et 10 µM, en présence du composé à tester, à des concentrations comprises entre 1 nM et 10 µM, en duplicats pendant 20 min à 37° C.

Le milieu réactionnel est éliminé et 80 µl de tampon de lyse sont ajoutés. Le taux d'AMP cyclique intracellulaire est mesuré par un test de compétition avec de l'AMP cyclique fluorescent (CatchPoint, Molecular Devices).

## Revendications

1. Composés de formule générale (**I**) 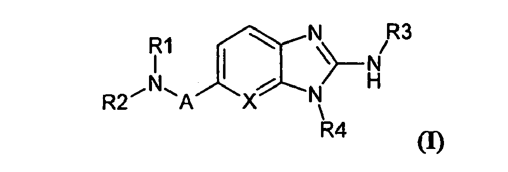 sous forme racémique, d'énantiomère ou toutes combinaisons de ces formes et dans laquelle :
A représente -CH₂- ,-C(O)-, -C(O)-C(Rₐ)(R_{b})- ;
X représente -CH- ou -N- ;
Rₐ et R_{b} représentent, indépendamment, l'atome d'hydrogène ou un radical (C₁-C₆)alkyle ;
R₁ représente l'atome d'hydrogène ; un radical (C₁-C₈)alkyle éventuellement substitué par hydroxy ou un ou plusieurs radicaux halo identiques ou différents ; (C₂-C₆)alkenyle ; ou un radical de formule -(CH₂)ₙ-X₁ ;
R₂ représente un radical (C₁-C₈)alkyle éventuellement substitué par hydroxy ou un ou plusieurs radicaux halo identiques ou différents ; (C₂-C₆)alkenyle ; ou un radical de formule -(CH₂)ₙ-X₁ ;
chaque X₁ représente indépendamment (C₁-C₆)alkoxy, (C₃-C₇)cycloalkyle, adamantyle, hétérocycloalkyle, aryle ou hétéroaryle,
les radicaux (C₃-C₇)cycloalkyle, hétérocycloalkyle, aryle et hétéroaryle étant éventuellement substitués par un ou plusieurs substituants identiques ou différents choisis parmi : -(CH₂)_{n'}-V₁ -Y₁, halo, nitro, cyano et aryle ;
V₁ représente -O-, -S- ou une liaison covalente ;
Y₁ représente un radical (C₁-C₆)alkyle éventuellement substitué par un ou plusieurs radicaux halo identiques ou différents ;
n représente un entier de 0 à 6 et n'un entier de 0 à 2 (étant entendu que lorsque n est égal à 0, alors X₁ ne représente pas le radical alkoxy)
ou bien R₁ et R₂ forment ensemble, avec l'atome d'azote auquel ils sont rattachés, un hétérobicycloalkyle ou un hétérocycloalkyle éventuellement substitué par un ou plusieurs substituants identiques ou différents choisis parmi : hydroxy, (C₁-C₆)alkyle éventuellement substitué par hydroxy, (C₁-C₆)alkoxy-carbonyle, hétérocycloalkyle et -C(O)NV₁'Y'₁ avec V₁' et Y₁' représentant indépendamment l'atome d'hydrogène ou un (C₁-C₆)alkyle, ; ou bien R₁ et R₂ forment ensemble un radical de formule : 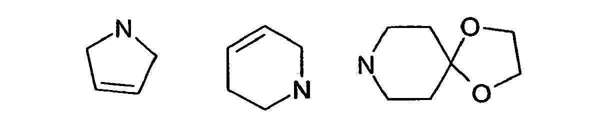
R₃ représente -Z₃, C(R_{Z3})(R'_{Z3})-Z₃, -C(R_{Z3})(R'_{Z3})-(CH₂)ₚ-Z₃ ou -C(O)-Z'₃
R_{Z3} et R'_{Z3} représentent, indépendamment, l'atome d'hydrogène ou un radical (C₁-C₆)alkyle ;
Z₃ représente Z₃ₐ, Z_{3b}, Z_{3c}, Z_{3d}, ou Z₃ₑ ;
Z₃ₐ représente un radical (C₁-C₆)alkyle ;
Z_{3b} représente un radical (C₁-C₆)alkoxy, (C₁-C₆)alkylthio, (C₁-C₆)alkylamino ou di((C₁-C₆)alkyl)amino ;
Z_{3c} représente un radical aryle ou hétéroaryle ;
Z_{3d} représente un radical (C₁-C₆)alkoxy-carbonyle, amino-carbonyle, (C₁-C₆)alkylamino-carbonyle, di((C₁-C₆)alkyl)amino-carbonyle, (C₁-C₆)alkyl-C(O)-NH- , (C₃-C₇)cycloalkyle, hétérocycloalkyle ;
les radicaux (C₃-C₇) cycloalkyle et hétérocycloalkyle étant éventuellement substitués par un ou plusieurs substituants identiques ou différents choisis parmi : halo, nitro, (C₁-C₆)alkoxy éventuellement substitué par un ou plusieurs radicaux halo identiques ou différents, (C₁-C₆)alkyle éventuellement substitué par un ou plusieurs radicaux halo identiques ou différents, (C₁-C₆)alkyl-carbonyle, (C₁-C₆)alkoxy-carbonyle, amino-carbonyle, (C₁-C₆)alkylamino-carbonyle, di((C₁-C₆)alkyl)amino-carbonyle et oxy,
les radicaux aryle et hétéroaryle étant éventuellement substitués par un ou plusieurs substituants identiques ou différents choisis parmi : halo, cyano, nitro, azido, oxy, (C₁-C₆)alkoxy-carbonyl-(C₁-C₆)alkenyle, (C₁-C₆)alkylamino-carbonyl-(C₁-C₆)alkenyle, -SO₂NR₃₁R₃₂, hétérocycloalkyle, hétéroaryle ou -(CH₂)_{p'}-V₃-Y₃;
R₃₁ et R₃₂ forment ensemble, avec l'atome d'azote sur lequel ils sont rattachés, un hétérocycloalkyle ;
V₃ représente -O-, -S-, -C(O)-, -C(O)-O-, -O-C(O)-, -SO₂-, -SO₂NH-, -NR'₃-SO₂-, -NR'₃-, -NR'₃-C(O)-, -C(O)-NR'₃-, -NH-C(O)-NR'₃- ou une liaison covalente ;
Y₃ représente l'atome d'hydrogène ; un radical (C₁-C₆)alkyle éventuellement substitué par un ou plusieurs radicaux halo identiques ou différents ; un radical aryle éventuellement substitué par un ou plusieurs substituants identiques ou différents choisis parmi : halo, nitro, (C₁-C₆)alkyle et (C₁-C₆)alkoxy ; ou un radical aryl-(C₁-C₆)alkyle éventuellement substitué par un ou plusieurs substituants identiques ou différents choisis parmi : halo, nitro, (C₆-C₆)alkyle et (C₁-C₆)alkoxy;
Z₃ₑ représente un radical de formule 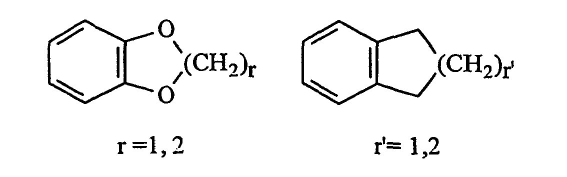 Z'₃ représente un radical aryle éventuellement substitué par un ou plusieurs substituants identiques ou différents choisis parmi : halo, nitro et -(CH₂)_{p"}-V'₃-Y'₃ ; V'₃ représente -O-, -C(O)-, -C(O)-O-, -C(O)-NR'₃, -NH-C(O)-NR'₃ ou une liaison covalente ;
Y'₃ représente l'atome d'hydrogène ou un radical (C₁-C₆)alkyle éventuellement substitué par un ou plusieurs radicaux halo identiques ou différents ;
R'₃ représente l'atome d'hydrogène, un radical (C₁-C₆)alkyle ou (C₁-C₆)alkoxy ;
p représente un entier de 1 à 4 ; p'et p" représentent, indépendamment, un entier de0à4;
R₄ représente un radical de formule-(CH₂)ₛ-R'₄ ;
R'₄ représente le radical guanidino ; un hétérocycloalkyle contenant au moins un atome d'azote et éventuellement substitué par (C₁-C₆)alkyle ou aralkyle ; un hétéroaryle contenant au moins un atome d'azote et éventuellement substitué par (C₁-C₆)alkyle ; ou un radical de formule -NW₄W₄'
W₄ représente l'atome d'hydrogène ou (C₁-C₈)alkyle ;
W'₄ représente un radical de formule -(CH₂)_{s'}-Z₄ ;
Z₄ représente l'atome d'hydrogène, (C₁-C₈)alkyle éventuellement substitué par un ou plusieurs substituants identiques ou différents choisis parmi : (C₁-C₆)alkoxy, (C₁-C₆)allcylthio et hydroxy ; (C₂-C₆)alkényle ; (C₃-C₇)cycloalkyle éventuellement substitué par un ou plusieurs substituants (C₁-C₆)alkyle identiques ou différents ; cyclohexène ; hétéroaryle et aryle ;
les radicaux aryle et hétéroaryle étant éventuellement substitués par un ou plusieurs radicaux identiques ou différents choisis de formule -(CH₂)_{s"}-V₄-Y₄, hydroxy, halo, nitro et cyano ;
V₄ représente -O-, -S-, -NH-C(O)-, -NV'₄ ou une liaison covalente ;
Y₄ représente un atome d'hydrogène ou un radical (C₁-C₆)alkyle éventuellement substitué par un ou plusieurs radicaux halo identiques ou différents ;
V₄' représente un atome d'hydrogène ou un (C₁-C₆)alkyle ;
s" représente un entier de 0 à 4 ;
ou bien Z₄ représente un radical de formule 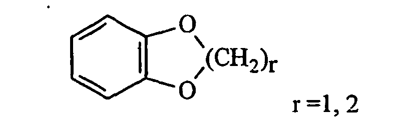
s et s' représentent, indépendamment, un entier de 0 à 6 ;
et i) lorsque R₃ représente -C(O)-Z'₃ et R₄ représente un radical de formule-(CH₂)ₛ-NW₄W'₄ et W₄ et W'₄ représentent, indépendamment, l'atome d'hydrogène ou le radical (C₁-C₆)alkyle, alors -(CH₂)ₛ ne représente ni le radical éthylène ni le radical -(CH₂)-CH((C₁-C₄)alkyle)- et ii) lorsque R₃ représente -Z_{3c} et Z_{3c} représente un radical phényle ou naphtyle, alors phényle et naphtyle ne sont pas susbtitués par cyano ; et étant entendu que lorsque R₃ représente -Z_{3d} alors Z_{3d} ne représente qu'un radical (C₃-C₇)cycloalkyle ou hétérocycloalkyle ;
ou un sel pharmaceutiquement acceptable de ces derniers.

2. Composés selon la revendication 1 **caractérisés en ce que** X représente -CH- ; ou un sel pharmaceutiquement acceptable de ces derniers.

3. Composés selon la revendication 2 **caractérisés en ce que** A représente -CH₂- ; ou un sel pharmaceutiquement acceptable de ces derniers.

4. Composés selon la revendication 3 **caractérisés en ce que**
R₁ et R₂ représentent, indépendamment, un radical (C₁-C₈)alkyle
R₃ représente -Z_{3c}, -C(R_{Z3})(R'_{Z3})-Z_{3c}, -C(R_{Z3})(R'_{Z3})-Z_{3d,}-C(R_{Z3})(R'_{Z3})-(CH₂)ₚ-Z_{3d} ;
R₄ représente un radical de formule-(CH₂)ₛ-R'₄ ;
R'₄ représente un hétérocycloalkyle contenant au moins un atome d'azote et éventuellement substitué par (C₁-C₆)alkyle ; ou un radical de formule -NW₄W'₄
W₄ représente l'atome d'hydrogène ou (C₁-C₈)alkyle ;
W'₄ représente un radical de formule -(CH₂)_{s'}-Z₄ ;
Z₄ représente l'atome d'hydrogène ;
s représente un entier de 2 à 4 ; s' représente un entier de 0 à 4 ;
ou un sel pharmaceutiquement acceptable de ces derniers.

5. Composés selon la revendication 4 **caractérisés en ce que**
l'hétérocycloalkyle que représente R'₄ est le cycle pipéridine ;
R_{Z3} et R'_{Z3} représentent l'atome d'hydrogène ;
Z_{3c} représente le radical thiényle, furyle ou phényle,
le radical phényle étant substitué par un ou plusieurs substituants identiques ou différents choisis parmi : halo et -(CH₂)_{p'}-V₃-Y₃;
V₃ représente -O-, -C(O)-, -C(O)-O-, -C(O)-NR'₃- ou une liaison covalente ;
R'₃ représente l'atome d'hydrogène ou un radical (C₁-C₆)alkyle ;
Y₃ représente l'atome d'hydrogène ; un radical (C₁-C₆)alkyle éventuellement substitué par un ou plusieurs radicaux halo identiques ou différents ;
Z_{3d} représente le radical (C₁-C₆)alkoxy-carbonyle ou hétérocycloalkyle, et de préférence l'hétérocycloalkyle est l'imidazolidine.
ou un sel pharmaceutiquement acceptable de ces derniers.

6. Composés selon la revendication 2 **caractérisés en ce que** A représente -C(O)-C(Rₐ)(R_{b})- avec Rₐ et R_{b} qui représentent le radical méthyle ; ou un sel pharmaceutiquement acceptable de ces derniers.

7. Composés selon la revendication 6 **caractérisés en ce que**
R₁ et R₂ représentent, indépendamment, un radical (C₁-C₈)alkyle ;
R₃ représente -Z_{3c}, -C(R_{Z3})(R'_{Z3})-Z_{3c}, -C(R_{Z3})(R'_{Z3})-Z_{3d} ou -C(R_{Z3})(R'_{Z3})-(CH₂)ₚ-Z_{3d};
R₄ représente un radical de formule-(CH₂)ₛ-R'₄
R'₄ représente un hétérocycloalkyle contenant au moins un atome d'azote et éventuellement substitué par (C₁-C₆)alkyle; ou un radical de formule -NW₄W'₄
W₄ représente l'atome d'hydrogène ou (C₁-C₈)alkyle;
W'₄ représente un radical de formule -(CH₂)_{s'}-Z₄;
Z₄ représente l'atome d'hydrogène, le radical phényle ou un hétéroaryle;
s représente un entier de 2 à 4 ; s' représente un entier de 0 à 4;
ou un sel pharmaceutiquement acceptable de ces derniers.

8. Composés selon la revendication 7 **caractérisés en ce que**
R_{Z3} et R'_{Z3} représentent, indépendamment, l'atome d'hydrogène ;
Z_{3c} représente un radical thiényle éventuellement substitué par (C₁-C₆)alkoxy-carbonyle ; ou phényle substitué par un ou plusieurs substituants identiques ou différents choisis parmi : halo, nitro ou -(CH₂)_{p'}-V₃-Y₃;
V₃ représente -O-, -C(O)-, -C(O)-O-, -C(O)-NR'₃- ou une liaison covalente ;
Y₃ représente l'atome d'hydrogène ; un radical (C₁-C₆)alkyle éventuellement substitué par un ou plusieurs radicaux halo identiques ou différents ;
R'₃ représente l'atome d'hydrogène ;
Z_{3d} représente un radical (C₁-C₆)alkoxy-carbonyle ;
l'hétérocycloalkyle que représente R'₄ est la pipéridine ;
l'hétéroaryle que représente Z₄ est la pyridine ;ou un sel pharmaceutiquement acceptable de ces derniesr.

9. Composés selon la revendication 2 **caractérisés en ce que** A représente -C(O)- ; ou un sel pharmaceutiquement acceptable de ces derniers.

10. Composés selon la revendication 9 **caractérisés en ce que** R₃ représente -C(O)-Z'₃ ;
R₁ et R₂ représentent, indépendamment, un radical (C₁-C₈)alkyle ;
Z'₃ représente un radical phényle éventuellement substitué par un ou plusieurs substituants identiques ou différents choisis parmi : halo, nitro et -(CH₂)_{p"}-V'₃-Y'₃ ;
V'₃ représente -O-, -C(O)-O- ou une liaison covalente ;
Y'₃ représente l'atome d'hydrogène ou un radical (C₁-C₆)alkyle ;
p" représente l'entier 0 ;
R₄ représente un radical de formule-(CH₂)ₛ-R'₄ et R'₄ qui représente un radical de formule -NW₄W'₄
W₄ représente l'atome d'hydrogène ou (C₁-C₈)alkyle
W'₄ représente un radical de formule -(CH₂)_{s'}-Z₄ et Z₄ qui représente l'atome d'hydrogène ;
s représente un entier de 2 à 4 ; s' représente un entier de 0 à 4 ;
ou un sel pharmaceutiquement acceptable de ces derniers.

11. Composés selon la revendication 9 **caractérisés en ce que**
R₁ représente un atome d'hydrogène, un radical (C₁-C₈)alkyle éventuellement substitué par hydroxy, (C₂-C₆)alkenyle ou un radical de formule -(CH₂)ₙ-X₁ ;
R₂ représente un radical (C₁-C₈)alkyle éventuellement substitué par hydroxy, (C₂-C₆)alkenyle ou un radical de formule -(CH₂)ₙ-X₁ ;
chaque X₁ représente indépendamment (C₁-C₆)alkoxy, (C₃-C₇)cycloalkyle , aryle ou hétéroaryle,
le radical aryle étant éventuellement substitué par un ou plusieurs substituants identiques ou différents choisis parmi : -(CH₂)_{n'}-V₁-Y₁, halo ;
V₁ représente -O- ou une liaison covalente ;
Y₁ représente un radical (C₁-C₆)alkyle éventuellement substitué par un ou plusieurs radicaux halo identiques ou différents ; ou aryle ;
ou bien R₁ et R₂ forment ensemble, avec l'atome d'azote auquel ils sont rattachés, un hétérocycloalkyle éventuellement substitué par un ou plusieurs substituants identiques ou différents choisis parmi : hydroxy, (C₁-C₆)alkyle éventuellement substitué par hydroxy, (C₁-C₆)alkoxy-carbonyle, hétérocycloalkyle et -C(O)NV₁'Y₁' avec V₁' et Y₁' représentant indépendamment l'atome d'hydrogène
ou un (C₁-C₆)alkyle,
ou bien R₁ et R₂ forment ensemble un radical de formule : 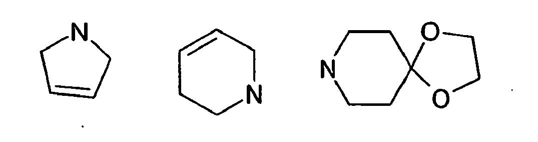 R₃ représente -Z₃, -C(R_{Z3})(R'_{Z3})-Z₃ ou -C(R_{Z3})(R'_{Z3})-(CH₂)ₚ-Z₃ ;
R₄ représente un radical de formule-(CH₂)ₛ-R'₄ ;
R'₄ représente un hétérocycloalkyle contenant au moins un atome d'azote et éventuellement substitué par (C₁-C₆)alkyle ou aralkyle ; un hétéroaryle contenant au moins un atome d'azote et éventuellement substitué par (C₁-C₆)alkyle ; ou un radical de formule -NW₄W'₄
W₄ représente l'atome d'hydrogène ou (C₁-C₈)alkyle ;
W'₄ représente un radical de formule -(CH₂)_{s'}-Z₄;
Z₄ représente l'atome d'hydrogène, (C₃-C₇)cycloalkyle ou aryle ;
s représente un entier de 0 à 5 ; s' représente un entier de 0 à 4 ; ou un sel pharmaceutiquement acceptable de ces derniers.

12. Composés selon la revendication 11 **caractérisés en ce qu'**ils comportent au moins une des caractéristiques suivantes :
■ le radical (C₃-C₇)cycloalkyle que représent X₁ est le cyclopropyle ;
■ le radical aryle que représente X₁ le radical phényle ;
■ le radical hétéroaryle que représente X₁ est la pyridine ;
■ l'hétérocycloalkyle que forment ensemble R₁ et R₂ avec l'atome d'azote auquel ils sont rattachés, est choisi parmi : pyrrolidine, pipéridine, azépane, azacyclooctane, morpholine, pipérazine et décahydroisoquinoline ;
■ le radical hétérocycloalkyle que représente R'₄ , éventuellement substitué par C₁-C₆)alkyle ou benzyle, est choisi parmi : pyrrolidinyle, pipéridinyle, morpholinyle ou pipérazinyle ;
■ le radical hétéroaryle que représente R'₄ est le radical imidazolyle ;
■ le cycloalkyle que représente Z₄ est choisi parmi : cyclopropyle, cyclobutyle, cyclopentyle cyclohexyle ou cycloheptyle ;
■ l'aryle que représente Z₄ est le phényle ; ou un sel pharmaceutiquement acceptable de ces derniers.

13. Composés selon la revendication 11, **caractérisés en ce que** R₄ représente un radical de formule-(CH₂)ₛ-R'₄ avec R'₄ qui représente le radical pyrrolidinyle ou pipéridinyle ; ou un radical de formule -NW₄W'₄
W₄ qui représente l'atome d'hydrogène ou (C₁-C₈)alkyle ;
W'₄ représente un radical de formule -(CH₂)_{s'}-Z₄ avec Z₄ qui représente l'atome d'hydrogène ;
s représente un entier de 2 à 4 ; s' représente un entier de 0 à 4 ; ou un sel pharmaceutiquement acceptable de ce dernier.

14. Composés selon l'une des revendications 11 à 13, **caractérisés en ce que** R₁ et R₂ représentent, indépendamment, un radical (C₁-C₈)alkyle ; ou un sel pharmaceutiquement acceptable de ces derniers.

15. Composés selon l'une des revendications 11 à 14, **caractérisés en ce que**
R₃ représente -Z₃ et Z₃ représente Z_{3c}, Z_{3d} ou Z₃ₑ ;
Z_{3d} représente un radical (C₃-C₇)cycloalkyle ou hétérocycloalkyle ; ou un sel pharmaceutiquement acceptable de ces derniers.

16. Composés selon la revendication 15, **caractérisés en ce que**
Z_{3c} représente un radical hétéroaryle choisi parmi thiényle, furyle, indolyle, dihydroindolyle, pyridyle, benzothiényle et benzofuryle ; ou un radical aryle choisi parmi phényle, napthyle et fluorènyle ;
le radical hétéroaryle étant éventuellement substitué par un ou plusieurs substituants identiques ou différents choisis parmi : (C₁-C₆)alkyl-carbonyle et (C₁-C₆)alkoxy-carbonyle ;
le radical aryle étant éventuellement substitué par un ou plusieurs substituants identiques ou différents choisis parmi : halo, cyano, nitro, azido,(C₁-C₆)alkoxy-carbonyl-(C₁-C₆)alkenyle, oxy, -SO₂-NR₃₁R₃₂, hétérocycloalkyle, hétéroaryle, ou -(CH₂)_{p'}-V₃-Y₃ ;
R₃₁ et R₃₂ forment ensemble, avec l'atome d'azote sur lequel ils sont rattachés, le cycle pipéridine ;
V₃ représente -O-, -S-, -C(O)-, -C(O)-O-, -SO₂-, -SO₂NH-, -NR'₃-, -NR'₃-C(O)-, - C(O)-NR'₃-, -NH-C(O)-NR'₃- ou une liaison covalente ;
Y₃ représente l'atome d'hydrogène ; un radical (C₁-C₆)alkyle éventuellement substitué par un ou plusieurs radicaux halo identiques ou différents ; un radical phényle ; ou un radical benzyle ;
R'₃ représente l'atome d'hydrogène, un radical (C₁-C₆)alkyle ou (C₁-C₆)alkoxy ;
Z_{3d} représente le radical cyclopropyle, cyclohexyle ou pipéridinyle, chacun pouvant être substitué par un radical (C₁-C₆)alkoxy-carbonyle ; ou un sel pharmaceutiquement acceptable de ces derniers.

17. Composés selon la revendication 15, **caractérisés en ce que**
R₃ représente -Z₃ et Z₃ représente Z_{3c}, Z_{3d} ou Z₃ₑ ;
Z₃, représente un radical hétéroaryle choisi parmi thiényle, indolyle et benzothiényle ; ou un radical aryle choisi parmi phényle et napthyle ;
le radical hétéroaryle étant éventuellement substitué par un ou plusieurs radicaux oxy ;
le radical aryle étant éventuellement substitué par un ou plusieurs substituants identiques ou différents choisis parmi : halo, nitro, hétéroaryle ou -(CH₂)_{p'}-V₃-Y₃ ;
V₃ représente -O-, -S-, -C(O)-, -C(O)-O-, -SO₂-, -SO₂NH-, -NR'₃-C(O)-, - C(O)-NR'₃-, -NH-C(O)-NR'₃- ou une liaison covalente ;
Y₃ représente l'atome d'hydrogène ; un radical (C₁-C₆)alkyle éventuellement substitué par un ou plusieurs radicaux halo identiques ou différents; un radical phényle ; ou un radical benzyle ;
R'₃ représente l'atome d'hydrogène, un radical (C₁-C₆)alkyle ou (C₁-C₆)alkoxy ;
Z_{3d} représente le radical cyclopropyle ou pipéridinyle, chacun éventuellement substitué par (C₁-C₆)alkoxy-carbonyle ; ou un sel pharmaceutiquement acceptable de ces derniers.

18. Composés selon la revendication 17, **caractérisés en ce que**
Z₃ représente Z_{3c} ou Z₃ₑ ;
Z₃ₑ représente un radical phényle étant éventuellement substitué par un ou plusieurs substituants identiques ou différents choisis parmi nitro et -(CH₂)_{p'}-V₃-Y₃;
V₃ représente -O-, -S-, -C(O)-, -C(O)-O-, -SO₂-, -SO₂NH-, -NR'₃-C(O)-, - C(O)-NR'₃- ou une liaison covalente ;
Y₃ représente l'atome d'hydrogène ; un radical (C₁-C₆)alkyle ; phényle ; ou benzyle ;
R'₃ représente l'atome d'hydrogène;
Z₃ₑ représente 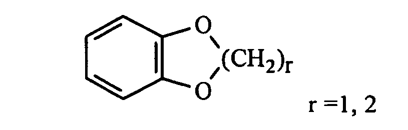 ou un sel pharmaceutiquement acceptable de ces derniers.

19. Composés selon l'une des revendications 11 à 14, **caractérisés en ce que** R₃ représente -C(R_{Z3})(R'_{Z3})-Z₃ et Z₃ représente Z_{3b}, Z_{3c}, Z_{3d} ou Z₃ₑ ; ou un sel pharmaceutiquement acceptable de ces derniers.

20. Composés selon la revendication 19, **caractérisés en ce que**
R₃ représente -C(R_{Z3})(R'_{Z3})-Z₃ et Z₃ représente Z_{3b} ou Z_{3c}
R_{Z3} et R'_{Z3} représentent l'atome d'hydrogène ;
ou un sel pharmaceutiquement acceptable de ces derniesr.

21. Composés selon la revendication 20 **caractérisés en ce que**
Z_{3b} représente un radical (C₁-C₆)alkoxy ;
Z_{3c} représente un radical hétéroaryle choisi parmi thiényle, furyle, pyridyle, benzothiényle et dihydrobenzofuryle ; ou un radical aryle choisi parmi phényle et napthyle,
le radical aryle étant éventuellement substitué par un ou plusieurs substituants identiques ou différents choisis parmi : halo ou -(CH₂)_{p'}-V₃-Y₃;
V₃ représente -O-, -S-, -C(O)-, -C(O)-O-, -SO₂-, -SO₂NH-, -NR'₃-C(O)-, -C(O)-NR'₃-,
Y₃ représente l'atome d'hydrogène; un radical (C₁-C₆)alkyle éventuellement substitué par un ou plusieurs radicaux halo identiques ou différents ;
R'₃ représente l'atome d'hydrogène ;
ou un sel pharmaceutiquement acceptable de ces derniers.

22. Composés selon la revendication 20, **caractérisés en ce que**
R₃ représente -C(R_{Z3})(R'_{Z3})-Z₃ et Z₃ représente Z_{3b} ou Z_{3c} ;
Z_{3b} représente un radical (C₁-C₆)alkoxy ;
Z_{3c} représente un hétéroaryle choisi parmi thiényle, furyle, , dihydrobenzofuryle; ou un radical phényle ;
le radical phényle étant éventuellement substitué par un ou plusieurs substituants identiques ou différents choisis parmi : nitro ou -(CH₂)_{p'}-V₃-Y₃ ;
V₃ représente -O-, -S-, -C(O)-, -C(O)-O-, -SO₂-, -SO₂NH-, -C(O)-NR'₃- ,
Y₃ représente l'atome d'hydrogène ; ou un radical (C₁-C₆)alkyle éventuellement substitué par un ou plusieurs radicaux halo identiques ou différents ;
R'₃ représente l'atome d'hydrogène ;
ou un sel pharmaceutiquement acceptable de ces derniers.

23. Composés selon la revendication 22, **caractérisés en ce que**
Z₃ représente Z_{3c} ;
Z_{3c} représente un radical furyle ou phényle,
le radical phényle étant éventuellement substitué par un ou plusieurs substituants identiques ou différents de formule -(CH₂)_{p'}-V₃-Y₃ ;
V₃ représente -O-, -S-, -C(O)-, -C(O)-O-, -SO₂-, -SO₂NH-, -C(O)-NR'₃- ,
Y₃ représente l'atome d'hydrogène ; ou un radical (C₁-C₆)alkyle éventuellement substitué par un ou plusieurs radicaux halo identiques ou différents ;
R'₃ représente l'atome d'hydrogène ;
ou un sel pharmaceutiquement acceptable de ces derniers.

24. Composés selon la revendication 19, **caractérisés en ce que**
R₃ représente -C(R_{Z3})(R'_{Z3})-Z₃ et Z₃ représente Z_{3d} ou Z₃ₑ ;
R_{Z3} et R'_{Z3} représentent l'atome d'hydrogène ou (C₁-C₆)alkyle ;
Z_{3d} représente un radical (C₁-C₆)alkoxy-carbonyle, (C₃-C₇)cycloalkyle ou hétérocycloalkyle ;
Z₃ₑ représente 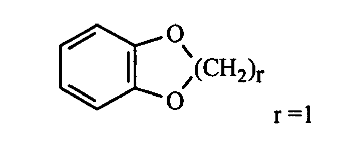 ou un sel pharmaceutiquement acceptable de ces derniers.

25. Composés selon la revendication 24 **caractérisés en ce que**
Z_{3d} représente un radical (C₁-C₆)alkoxy-carbonyle, cyclohexyle ou un tétrahydrofuranyle ;
ou un sel pharmaceutiquement acceptable de ces derniers.

26. Composés selon la revendication 24 **caractérisés en ce que** Z₃ représente Z_{3d} ou Z₃ₑ ;
Z_{3d} représente un radical (C₁-C₆)alkoxy-carbonyle ;
Z₃ₑ représente 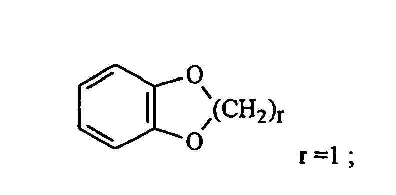 ou un sel pharmaceutiquement acceptable de ces derniers.

27. Composés selon la revendication 26 **caractérisé en ce que** Z₃ représente Z₃ₑ 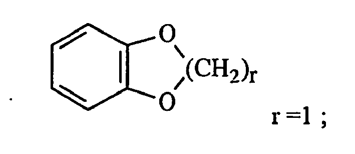 ou un sel pharmaceutiquement acceptable de ces derniers.

28. Composés selon l'une des revendications 11 à 14, **caractérisé en ce que** R₃ représente -C(R_{Z3})(R'_{Z3})-(CH₂)ₚ-Z₃ et Z₃ représente Z_{3b}, Z_{3c} ou Z_{3d} ; ou un sel pharmaceutiquement acceptable de ces derniers.

29. Composés selon la revendication 28, **caractérisé en ce que** R₃ représente -C(R_{Z3})(R'_{Z3})-(CH₂)ₚ-Z₃ et Z₃ représente Z_{3b} ; ou un sel pharmaceutiquement acceptable de ces derniers.

30. Composés selon la revendication 29, **caractérisés en ce que**
R_{Z3} et R'_{Z3} représentent, indépendamment, l'atome d'hydrogène ou un radical (C₁-C₆)alkyle ;
Z_{3b} représente un radical (C₁-C₆)alkoxy, (C₁-C₆)alkylthio ou di((C₁-C₆)alkyl)amino ; ou un sel pharmaceutiquement acceptable de ces derniers.

31. Composés selon la revendication 29, **caractérisés en ce que**
R_{Z3} et R'_{Z3} représentent, indépendamment, l'atome d'hydrogène ou un radical (C₁-C₆)alkyle ;
Z_{3b} représente un radical (C₁-C₆)alkoxy ou (C₁-C₆)alkylthio; ou un sel pharmaceutiquement acceptable de ces derniers.

32. Composés selon la revendication 28, **caractérisés en ce que** R₃ représente - C(R_{Z3})(R'_{Z3})-(CH₂)ₚ-Z₃ et Z₃ représente Z_{3c} ou Z_{3d}; ou un sel pharmaceutiquement acceptable de ces derniesr.

33. Composés selon la revendication 32, **caractérisés en ce que**
R_{Z3} et R'_{Z3} représentent, indépendamment, l'atome d'hydrogène ou un radical (C₁-C₆)alkyle ;
Z_{3c} représente un radical indolyle ou phényle ;
le radical phényle étant éventuellement substitué par un ou plusieurs substituants identiques ou différents choisi parmi : halo et -(CH₂)_{p'}-V₃-Y₃ ;
V₃ représente -SO₂NH-,
Y₃ représente l'atome d'hydrogène ; ou un radical (C₁-C₆)alkyle ;
Z_{3d} représente un radical (C₁-C₆)alkoxy-carbonyle, amino-carbonyle, (C₁-C₆)alkyl-amino-carbonyle, (C₁-C₆)alkyl-C(O)-NH-, ou hétérocycloalkyle éventuellement substitué par oxy, et de préférence pipéridinyle, morpholinyle, pyrrolidine ou imidazolidinyle ; ou un sel pharmaceutiquement acceptable de ces derniers.

34. Composés selon la revendication 32, **caractérisés en ce que**
Z₃ représente Z_{3d} ;
R_{Z3} et R'_{Z3} représentent, indépendamment, l'atome d'hydrogène ou un radical (C₁-C₆)alkyle;
Z_{3d} représente un radical (C₁-C₆)alkoxy-carbonyle, amino-carbonyle, (C₁-C₆)alkylamino-carbonyle, (C₁-C₆)alkyl-C(O)-NH- ou hétérocycloalkyle, et de préférence pyrrolidine ou imidazolidine, éventuellement substitué par oxy ; ou un sel pharmaceutiquement acceptable de ces derniers.

35. Procédé de préparation d'un composé de formule (I) selon l'une des revendications précédentes **caractérisé en ce que** l'on traite le composé de formule générale : 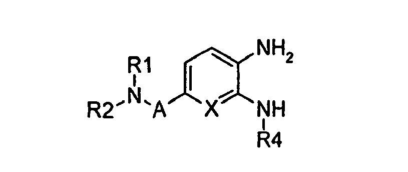 dans laquelle A, X, R₁, R₂, R₄ ont la signification indiquée dans la revendication 1, par un isothiocyanate de formule générale R₃N=C=S dans lequel R₃ a la signification indiquée dans la revendication 1, en présence d'un agent de couplage ou d'oxyde de mercure (II) jaune en présence de soufre, pendant une durée de 3 à 48 heures, dans un solvant protique ou aprotique, à une température de 50 à 80°C.

36. Composition pharmaceutique contenant, à titre de principes actifs, au moins un composé selon l'une des revendications 1 à 34, en association avec un support pharmaceutiquement acceptable.

37. Utilisation d'un composé selon l'une des revendications 1 à 34, pour la préparation d'un médicament pour le traitement des désordres pondéraux, des troubles mentaux , de la douleur, ou des désordres de l'activité sexuelle.

38. Utilisation selon la revendication 37, pour le traitement des désordres pondéraux tels que l'anorexie et la cachexie et plus particulièrement la cachexie des pathologies cancéreuses, cachexie du sida, cachexie de la personne âgée, cachexie cardiaque, cachexie rénale, cachexie de l'arthrite rhumatoïde.

39. Utilisation selon la revendication 37, pour le traitement de la douleur et plus particulièrement la douleur neuropathique.

40. Utilisation selon la revendication 37, pour le traitement des troubles mentaux tels que l'anxiété et la dépression.

41. Utilisation d'un composé de formule générale (**I**') 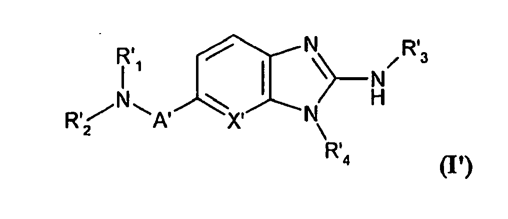 sous forme racémique, d'énantiomère ou toutes combinaisons de ces formes et dans laquelle :
A' représente -CH₂- ,-C(O)-, -C(O)-C(Rₐ)(R_{b})- ;
X' représente -CH- ;
Rₐ et R_{b} représentent, indépendamment, l'atome d'hydrogène ou un radical (C₁-C₆)alkyle ;
R'₁ représente l'atome d'hydrogène ; un radical (C₁-C₈)alkyle éventuellement substitué par hydroxy ou un ou plusieurs radicaux halo identiques ou différents ; (C₂-C₆)alkenyle ; ou un radical de formule -(CH₂)ₙ-X₁ ;
R'₂ représente un radical (C₁-C₈)alkyle éventuellement substitué par hydroxy ou un ou plusieurs radicaux halo identiques ou différents ; (C₂-C₆)alkenyle ; ou un radical de formule -(CH₂)ₙ-X₁ ;
chaque X₁ représente indépendamment (C₁-C₆)alkoxy, (C₃-C₇)cycloalkyle, adamantyle, hétérocycloalkyle, aryle ou hétéroaryle,
les radicaux (C₃-C₇)cycloalkyle, hétérocycloalkyle, aryle et hétéroaryle étant éventuellement substitués par un ou plusieurs substituants identiques ou différents choisis parmi : -(CH₂)_{n'}-V₁-Y₁, halo, nitro, cyano et aryle;
V₁ représente -O-, -S- ou une liaison covalente ;
Y₁ représente un radical (C₁-C₆)alkyle éventuellement substitué par un ou plusieurs radicaux halo identiques ou différents ;
n représente un entier de 0 à 6 et n' un entier de 0 à 2 (étant entendu que lorsque n est égal à 0, alors X₁ ne représente pas le radical alkoxy) ;
ou bien R₁ et R₂ forment ensemble, avec l'atome d'azote auquel ils sont rattachés, un hétérobicycloalkyle ou un hétérocycloalkyle éventuellement substitué par un ou plusieurs substituants identiques ou différents choisis parmi : hydroxy, (C₁-C₆)alkyle éventuellement substitué par hydroxy, (C₁-C₆)alkoxy-carbonyle, hétérocycloalkyle et -C(O)NV₁'Y₁' avec V₁' et Y₁' représentant indépendamment l'atome d'hydrogène ou un (C₁-C₆)alkyle ; ou bien R₁ et R₂ forment ensemble un radical de formule : 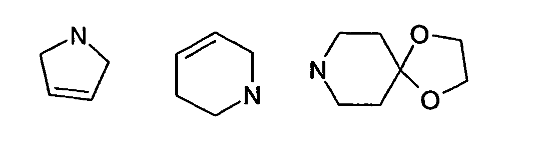
R'₃ représente -Z₃, C(R_{Z3})(R'_{Z3})-Z₃, -C(R_{Z3})(R'_{Z3})-(CH₂)ₚ-Z₃ ou-C(O)-Z'₃
R_{Z3} et R'_{Z3} représentent, indépendamment, l'atome d'hydrogène ou un radical (C₁-C₆)alkyle ;
Z₃ représente Z₃ₐ, Z_{3b}, Z_{3c}, Z_{3d}, ou Z₃ₑ ;
Z₃ₐ représente un radical (C₁-C₆)alkyle ;
Z_{3b} représente un radical (C₁-C₆)alkoxy, (C₁-C₆)alkylthio, (C₁-C₆)alkylamino ou di((C₁-C₆)alkyl)amino ;
Z_{3c} représente un radical aryle ou hétéroaryle ;
Z_{3d} représente un radical (C₁-C₆)alkoxy-carbonyle, amino-carbonyle, (C₁-C₆)alkylamino-carbonyle, di((C₁-C₆)alkyl)amino-carbonyle, (C₁-C₆)alkyl-C(O)-NH- , (C₃-C₇)cycloalkyle, hétérocycloalkyle ;
les radicaux (C₃-C₇) cycloalkyle et hétérocycloalkyle étant éventuellement substitués par un ou plusieurs substituants identiques ou différents choisis parmi : halo, nitro, (C₁-C₆)alkoxy éventuellement substitué par un ou plusieurs radicaux halo identiques ou différents , (C₁-C₆)alkyle éventuellement susbtitué par un ou plusieurs radicaux halo identiques ou différents, (C₁-C₆)alkyl-carbonyle, (C₁-C₆)alkoxy-carbonyle, amino-carbonyle, (C₁-C₆)alkylamino-carbonyle, di((C₁-C₆)alkyl)amino-carbonyle et oxy,
les radicaux aryle et hétéroaryle étant éventuellement substitués par un ou plusieurs substituants identiques ou différents choisis parmi : halo, cyano, nitro, azido, oxy, (C₁-C₆)alkoxy-carbonyl-(C₁-C₆)alkenyle, (C₁-C₆)alkylamino-carbonyl-(C₁-C₆)alkenyle, -SO₂-NR₃₁R₃₂, hétérocycloalkyle, hétéroaryle ou -(CH₂)_{p'}-V₃-Y₃;
R₃₁ et R₃₂ forment ensemble, avec l'atome d'azote sur lequel ils sont rattachés, un hétérocycloalkyle ;
V₃ représente -O-, -S-, -C(O)-, -C(O)-O-, -O-C(O)-, -SO₂-, -SO₂NH-, -NR'₃-SO₂-, -NR'₃-, -NR'₃-C(O)-, -C(O)-NR'₃-, -NH-C(O)-NR'₃- ou une liaison covalente ;
Y₃ représente l'atome d'hydrogène ; un radical (C₁-C₆)alkyle éventuellement substitué par un ou plusieurs radicaux halo identiques ou différents ; un radical aryle éventuellement substitué par un ou plusieurs substituants identiques ou différents choisis parmi : halo, nitro, (C₁-C₆)alkyle et (C₁-C₆)alkoxy ; ou un radical aryl-(C₁-C₆)alkyle éventuellement substitué par un ou plusieurs substituants identiques ou différents choisis parmi : halo, nitro, (C₁-C₆)alkyle et (C₁-C₆)alkoxy ;
Z₃ₑ représente un radical de formule 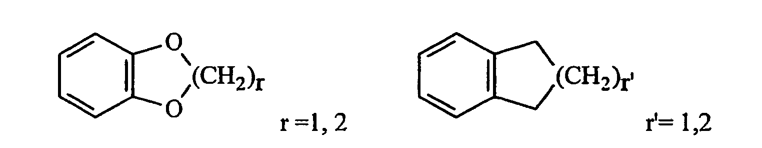 Z'₃ représente un radical aryle éventuellement substitué par un ou plusieurs substituants identiques ou différents choisis parmi : halo, nitro et -(CH₂)_{p"}-V'₃- Y'₃;
V'₃ représente -O-, -C(O)-, -C(O)-O-, -C(O)-NR'₃, -NH-C(O)-NR'₃ ou une liaison covalente ;
Y'₃ représente l'atome d'hydrogène ou un radical (C₁-C₆)alkyle éventuellement substitué par un ou plusieurs radicaux halo identiques ou différents ;
R'₃ représente l'atome d'hydrogène, un radical (C₁-C₆)alkyle ou (C₁-C₆)alkoxy
p représente un entier de 1 à 4 ; p'et p" représentent, indépendamment, un entier de 0 à 4 ;
R₄ représente un radical de formule-(CH₂)ₛ-R'₄ ;
R'₄ représente le radical guanidino ; un hétérocycloalkyle contenant au moins un atome d'azote et éventuellement substitué par (C₁-C₆)alkyle ou aralkyle ; un hétéroaryle contenant au moins un atome d'azote et éventuellement substitué par (C₁-C₆)alkyle ; ou un radical de formule -NW₄W'₄
W₄ représente l'atome d'hydrogène ou (C₁-C₈)alkyle;
W'₄ représente un radical de formule -(CH₂)_{s'}-Z₄ ;
Z₄ représente l'atome d'hydrogène, (C₁-C₈)alkyle éventuellement substitué par un ou plusieurs substituants identiques ou différents choisis parmi : (C₁-C₆)alkoxy, (C₁-C₆)alkylthio et hydroxy ; (C₂-C₆)alkényle ; (C₃-C₇)cycloalkyle éventuellement substitué par un ou plusieurs substituants (C₁-C₆)alkyle identiques ou différents ; cyclohexène ; hétéroaryle et aryle ;
les radicaux aryle et hétéroaryle étant éventuellement substitués par un ou plusieurs radicaux identiques ou différents choisis de formule -(CH₂)_{s"}-V₄-Y₄, hydroxy, halo, nitro et cyano ;
V₄ représente -O-, -S-, -NH-C(O)-, -NV'₄ ou une liaison covalente ;
Y₄ représente un atome d'hydrogène ou un radical (C₁-C₆)alkyle éventuellement substitué par un ou plusieurs radicaux halo identiques ou différents ;
V₄' représente un atome d'hydrogène ou un (C₁-C₆)alkyle
s" représente un entier de 0 à 4 ;
ou bien Z₄ représente un radical de formule 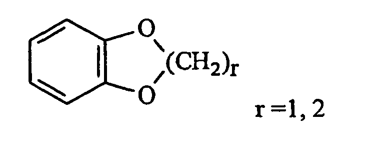
s et s' représentent, indépendamment, un entier de 0 à 6 ; ou un sel pharmaceutiquement acceptable de ce dernier.
pour la préparation d'un médicament pour le traitement des désordres pondéraux, des troubles mentaux , de la douleur, ou des désordres de l'activité sexuelle.

42. Utilisation selon la revendication 41, **caractérisé en ce que**
R₁ et R₂ représentent, indépendamment, un radical (C₁-C₈)alkyle ;
R₃ représente Z_{3c} et Z_{3c} représente un radical phényle ou naphtyle, chacun substitué au moins par cyano ;
R₄ représente un radical de formule-(CH₂)ₛ-R'₄ avec R'₄ qui représente le radical pyrrolidinyle ou pipéridinyle ; ou un radical de formule -NW₄W'₄ ;
W₄ qui représente l'atome d'hydrogène ou (C₁-C₈)alkyle;
W'₄ représente un radical de formule -(CH₂)_{s'}-Z₄ avec Z₄ qui représente l'atome d'hydrogène ;
s représente un entier de 2 à 4 ; s' représente un entier de 0 à 4 ;
ou un sel pharmaceutiquement acceptable de ces derniers.

## Claims

1. Compounds of general formula (I) 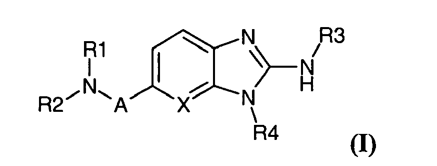 in racemic, enantiomeric form or any combinations of these forms and in which:
A represents -CH₂-,-C(O)-, -C(O)-C(Rₐ)(R_{b})-;
X represents -CH- or -N-;
Rₐ and R_{b} represent, independently, the hydrogen atom or a (C₁-C₆)alkyl radical;
R₁ represents the hydrogen atom, a (C₁-C₈)alkyl radical optionally substituted by hydroxy, or one or more identical or different halo radicals ; (C₂-C₆)alkenyl or a radical of formula-(CH₂)ₙ-X₁;
R₂ represents a (C₁-C₈)alkyl radical optionally substituted by hydroxy or one or more identical or different halo radicals; (C₂-C₆)alkenyl or a radical of formula -(CH₂)ₙ-X₁;
each X₁ independently represents (C₁-C₆)alkoxy, (C₃-C₇)cycloalkyl, adamantyl, heterocycloalkyl, aryl or heteroaryl,
the (C₃-C₇)cycloalkyl, heterocycloalkyl, aryl and heteroaryl radicals being optionally substituted by one or more identical or different substituents chosen from: -(CH₂)_{n'}-V₁-Y₁, halo, nitro, cyano and aryl;
V₁ represents -O -S- or a covalent bond;
Y₁ represents a (C₁-C₆)alkyl radical optionally substituted by one or more identical or different halo radicals;
n represents an integer from 0 to 6 and n' an integer from 0 to 2 (it being understood that when n is equal to 0, then X₁ does not represent the alkoxy radical);
or R₁ and R₂ form together with the nitrogen atom to which they are attached, a heterobicycloalkyl or a heterocycloalkyl optionally substituted by one or more identical or different substituents chosen from: hydroxy, (C₁-C₆)alkyl, optionally substituted by hydroxy, (C₁-C₆)alkoxy-carbonyl, heterocycloalkyl and -C(O)NV₁'Y₁' with V₁' and Y₁' independently representing the hydrogen atom or a (C₁-C₆)alkyl; or R₁ and R₂ together form a radical of formula: 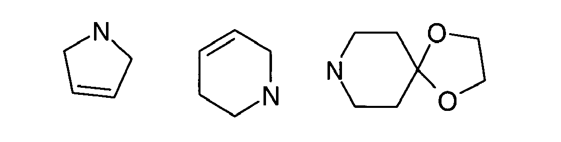
R₃ represents -Z₃, -C(R_{Z3})(R'_{Z3})-Z₃, -C(R_{Z3})(R'_{Z3})-(CH₂)ₚ-Z₃ or -C(O)-Z'₃
R_{Z3} and R'_{Z3} represent, independently, the hydrogen atom or a (C₁-C₆)alkyl radical;
Z₃ represents Z₃ₐ, Z_{3b}, Z_{3c}, Z_{3d}, or Z₃ₑ;
Z₃ₐ represents a (C₁-C₆)alkyl radical;
Z_{3b} represents a (C₁-C₆)alkoxy, (C₁-C₆)alkylthio, (C₁-C₆)alkylamino or di((C₁-C₆)alkyl)amino radical;
Z_{3c} represents an aryl or heteroaryl radical;
Z_{3d} represents a (C₁-C₆) alkoxy-carbonyl, amino-carbonyl, (C₁-C₆)alkylaminocarbonyl, di((C₁-C₆)alkyl)amino-carbonyl, (C₁-C₆)alkyl-C(O)-NH-, (C₃-C₇)cycloalkyl, heterocycloalkyl radical;
the (C₃-C₇)cycloalkyl and heterocycloalkyl radicals being optionally substituted by one or more identical or different substituents chosen from: halo, nitro, (C₁-C₆)alkoxy optionally substituted by one or more identical or different halo radicals, (C₁-C₆)alkyl optionally substituted by one or more identical or different halo radicals, (C₁-C₆)alkyl-carbonyl, (C₁-C₆)alkoxy-carbonyl, amino-carbonyl, (C₁-C₆)alkylamino-carbonyl, di((C-C₆)alkyl)amino-carbonyl and oxy,
the aryl and heteroaryl radicals being optionally substituted by one or more identical or different substituents chosen from: halo, cyano, nitro, azido, oxy, (C₁-C₆)alkoxy-carbonyl-(C₁-C₆)alkenyl, (C₁-C₆)alkylamino-carbonyl-(C₁-C₆)alkenyl, -SO₂-NR₃₁R₃₂, heterocycloalkyl, heteroaryl or-(CH₂)_{p'}-V₃-Y₃;
R₃₁ and R₃₂ form together with the nitrogen atom to which they are attached, a heterocycloalkyl;
V₃ represents -O-, -S-, -C(O)-, -C(O)-O-, -O-C(O)-, -SO₂-, -SO₂NH-, -NR'₃-SO₂-, -NR'₃-, -NR'₃-C(O)-, -C(O)-NR'₃-, -NH-C(O)-NR'₃- or a covalent bond;
Y₃ represents the hydrogen atom; a (C₁-C₆)alkyl radical optionally substituted by one or more identical or different halo radicals; an aryl radical optionally substituted by one or more identical or different substituents chosen from: halo, nitro, (C₁-C₆)alkyl and (C₁-C₆)alkoxy; or an aryl-(C₁-C₆)alkyl radical optionally substituted by one or more identical or different substituents chosen from: halo, nitro, (C₁-C₆)alkyl and (C₁ -C₆)alkoxy;
Z₃ₑ represents a radical of formula 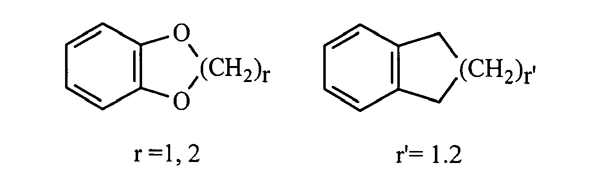 Z'₃ represents an aryl radical optionally substituted by one or more identical or different substituents chosen from: halo, nitro and -(CH₂)_{p"}-V'₃-Y'₃;
V'₃ represents -O- -C(O)-, -C(O)-O-, -C(O)-NR'₃-, -NH-C(O)-NR'₃- or a covalent bond;
Y'₃ represents the hydrogen atom or a (C₁-C₆)alkyl radical optionally substituted by one or more identical or different halo radicals;
R'₃ represents the hydrogen atom, a (C₁-C₆)alkyl or (C₁-C₆)alkoxy radical;
p represents an integer from 1 to 4; p' and p" represent, independently, an integer from 0 to 4;
R₄ represents a radical of formula -(CH₂)ₛ-R'₄
R'₄ represents the guanidino radical; a heterocycloalkyl containing at least one nitrogen atom and optionally substituted by (C₁-C₆)alkyl or aralkyl; a heteroaryl containing at least one nitrogen atom and optionally substituted by (C₁-C₆)alkyl; or a radical of formula -NW₄W'₄
W₄ represents the hydrogen atom or (C₁-C₈)alkyl;
W'₄ represents a radical of formula -(CH₂)_{s'}-Z₄;
Z₄ represents the hydrogen atom, (C₁-C₈)alkyl optionally substituted by one or more identical or different substituents chosen from: (C₁-C₆)alkoxy, (C₁-C₆)alkylthio and hydroxy; (C₂-C₆)alkenyl; (C₃-C₇)cycloalkyl optionally substituted by one or more identical or different (C₁-C₆)alkyl substituents; cyclohexene; heteroaryl and aryl;
the aryl and heteroaryl radicals being optionally substituted by one or more identical or different radicals chosen from formula: -(CH₂)_{s"}-V₄-Y₄, hydroxy, halo, nitro and cyano;
V₄ represents -O-, -S-, -NH-C(O)-, -NV₄'- or a covalent bond;
Y₄ represents a hydrogen atom or a (C₁-C₆)alkyl radical optionally substituted by one or more identical or different halo radicals;
V₄'represents a hydrogen atom or a (C₁-C₆)alkyl;
s" represents an integer from 0 to 4;
or Z₄ represents a radical of formula 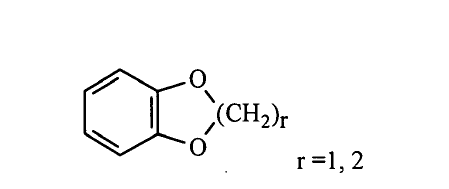
s and s' represent, independently, an integer from 0 to 6;
and i) when R₃ represents -C(O)-Z'₃ and R₄ represents a radical of formula -(CH₂)ₛ-NW₄W'₄ and W₄ and W'₄ represent, independently, the hydrogen atom or the (C₁-C₆)alkyl radical, then -(CH₂)ₛ represents neither the ethylene radical nor the -(CH₂)-CH((C₁-C₄)alkyl)- radical and ii) when R₃ represents -Z_{3c} and Z_{3c} represents a phenyl or naphthyl radical, then phenyl and naphthyl are not substituted by cyano; and it being understood that when R₃ represents -Z_{3d} then Z_{3d} only represents one (C₃-C₇) cycloalkyl or heterocycloalkyl radical;
or a pharmaceutically acceptable salt thereof.

2. Compounds according to claim 1 **characterized in that** X represents -CH-; or a pharmaceutically acceptable salt thereof.

3. Compounds according to claim 2 **characterized in that** A represents -CH₂-; or a pharmaceutically acceptable salt thereof.

4. Compounds according to claim 3 **characterized in that**
R₁ and R₂ represent, independently, a (C₁-C₈)alkyl radical;
R₃ represents -Z_{3c}, -C(R_{Z3})(R'_{Z3})-Z_{3c}, -C(R_{Z3})(R'_{Z3})-Z_{3d}, -C(R_{Z3})(R_{Z3})-(CH₂)ₚ-Z_{3d};
R₄ represents a radical of formula -(CH₂)ₛ-R'₄;
R'₄ represents a heterocycloalkyl containing at least one nitrogen atom and optionally substituted by (C₁-C₆)alkyl; or a radical of formula -NW₄W'₄
W₄ represents the hydrogen atom or (C₁-C₈)alkyl;
W'₄ represents a radical of formula -(CH₂)_{s'}-Z₄;
Z₄ represents the hydrogen atom;
s represents an integer from 2 to 4; s' represents an integer from 0 to 4;
or a pharmaceutically acceptable salt thereof.

5. Compounds according to claim 4 **characterized in that**
the heterocycloalkyl represented by R'₄ is the piperidine ring;
R_{Z3} and R'_{Z3} represent the hydrogen atom;
Z_{3c} represents the thienyl, furyl or phenyl radical,
the phenyl radical being substituted by one or more identical or different substituents chosen from: halo and -(CH₂)_{p'}-V₃-Y₃;
V₃ represents -O-, -C(O)-, -C(O)-O-, -C(O)-NR'₃- or a covalent bond;
R'₃ represents the hydrogen atom or a (C₁-C₆)alkyl radical;
Y₃ represents the hydrogen atom; a (C₁-C₆)alkyl radical optionally substituted by one or more identical or different halo radicals;
Z_{3d} represents the (C₁-C₆)alkoxy-carbonyl or heterocycloalkyl radical, and preferably the heterocycloalkyl is imidazolidine;
or a pharmaceutically acceptable salt thereof.

6. Compounds according to claim 2 **characterized in that** A represents -C(O)-C(Rₐ)(R_{b})-with Rₐ and R_{b} representing the methyl radical; or a pharmaceutically acceptable salt thereof

7. Compounds according to claim 6 **characterized in that**
R₁ and R₂ represent, independently, a (C₁-C₈)alkyl radical;
R₃ represents -Z_{3c}, -C(R_{Z3})(R'_{Z3})-Z_{3c}, -C(R_{Z3})(R'_{Z3})-Z_{3d} or -C(R_{Z3})(R'_{Z3})-(CH₂)ₚ-Z_{3d};
R₄ represents a radical of formula -(CH₂)ₛ-R'_{4;}
R'₄ represents a heterocycloalkyl containing at least one nitrogen atom and optionally substituted by (C₁-C₆)alkyl; or a radical of formula -NW₄W'₄;
W₄ represents the hydrogen atom or (C₁-C₈)alkyl;
W'₄ represents a radical of formula -(CH₂)_{s'}-Z₄;
Z₄ represents the hydrogen atom, the phenyl radical or a heteroaryl;
s represents an integer from 2 to 4; s' represents an integer from 0 to 4;
or a pharmaceutically acceptable salt thereof.

8. Compounds according to claim 7 **characterized in that**
R_{Z3} and R'_{Z3} represent, independently, the hydrogen atom;
Z_{3c} represents a thienyl radical optionally substituted by (C₁-C₆)alkoxy-carbonyl; or phenyl substituted by one or more identical or different substituents chosen from: halo, nitro or -(CH₂)_{p'}-V₃-Y_{3;}
V₃ represents -O-, -C(O)-, -C(O)-O-, -C(O)-NR'₃- or a covalent bond;
Y₃ represents the hydrogen atom; a (C₁-C₆)alkyl radical optionally substituted by one or more identical or different halo radicals;
R'₃ represents the hydrogen atom;
Z_{3d} represents a (C₁-C₆)alkoxy-carbonyl radical;
the heterocycloalkyl represented by R'₄ is piperidine;
the heteroaryl represented by Z₄ is pyridine; or a pharmaceutically acceptable salt thereof.

9. Compounds according to claim 2 **characterized in that** A represents -C(O)-; or a pharmaceutically acceptable salt thereof.

10. Compounds according to claim 9 **characterized in that** R₃ represents -C(O)-Z'₃;
R₁ and R₂ represent, independently, a (C₁-C₈)alkyl radical;
Z'₃ represents a phenyl radical optionally substituted by one or more identical or different substituents chosen from: halo, nitro and -(CH₂)_{p''}-V'₃-Y'₃
V'₃ represents -O-, -C(O)-O- or a covalent bond;
Y'₃ represents the hydrogen atom or a (C₁-C₆)alkyl radical;
p" represents the integer 0;
R₄ represents a radical of formula -(CH₂)ₛ-R'₄ and R'₄ represents a radical of formula -NW₄W'₄
W₄ represents the hydrogen atom or (C₁-C₈)alkyl;
W'₄ represents a radical of formula -(CH₂)ₛ,-Z₄ and Z₄ represents the hydrogen atom;
s represents an integer from 2 to 4; s' represents an integer from 0 to 4;
or a pharmaceutically acceptable salt thereof

11. Compounds according to claim 9 **characterized in that**
R₁ represents a hydrogen atom, a (C₁-C₈)alkyl radical optionally substituted by hydroxy, (C₂-C₆)alkenyl or a radical of formula -(CH₂)ₙ-X₁;
R₂ represents a (C₁-C₈)alkyl radical optionally substituted by hydroxy, (C₂-C₆)alkenyl or a radical of formula -(CH₂)ₙ-X₁;
each X₁ represents, independently, (C₁-C₆)alkoxy, (C₃-C₇)cycloalkyl, aryl or heteroaryl,
the aryl radical being optionally substituted by one or more identical or different substituents chosen from: -(CH₂)_{n'}-V₁-Y₁, halo;
V₁ represents -O- or a covalent bond;
Y₁ represents a (C₁-C₆)alkyl radical optionally substituted by one or more identical or different halo radicals; or aryl;
or R₁ and R₂ form together with the nitrogen atom to which they are attached, a heterocycloalkyl optionally substituted by one or more identical or different substituents chosen from: hydroxy, (C₁-C₆)alkyl optionally substituted by hydroxy, (C₁-C₆)alkoxy-carbonyl, heterocycloalkyl and -C(O)NV₁'Y₁' with V₁' and Y₁' independently representing the hydrogen atom or a (C₁-C₆)alkyl,
or R₁ and R₂ together form a radical of formula: 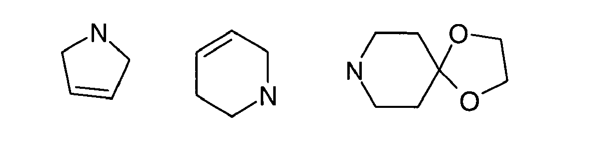 R₃ represents -Z₃, -C(R_{Z3})(R'_{Z3})-Z₃ or -C(R_{Z3})(R'_{Z3})-(-(CH₂)ₚ-Z₃;
R₄ represents a radical of formula -(CH₂)ₛ-R'₄ ;
R'₄ represents a heterocycloalkyl containing at least one nitrogen atom and optionally substituted by (C₁-C₆)alkyl or aralkyl; a heteroaryl containing at least one nitrogen atom and optionally substituted by (C₁-C₆)alkyl; or a radical of formula -NW₄W'₄
W₄ represents the hydrogen atom or (C₁-C₈)alkyl;
W'₄ represents a radical of formula -(CH₂)ₛ,-Z₄;
Z₄ represents the hydrogen atom, (C₃-C₇)cycloalkyl or aryl;
s represents an integer from 0 to 5; s' represents an integer from 0 to 4; or a pharmaceutically acceptable salt thereof.

12. Compounds according to claim 11 **characterized in that** they have at least one of the following characteristics:
■ the (C₃-C₇)cycloalkyl radical represented by X₁ is cyclopropyl;
■ the aryl radical represented by X₁ the phenyl radical;
■ the heteroaryl radical represented by X₁ is pyridine;
■ the heterocycloalkyl that R₁ and R₂ form together with the nitrogen atom to which they are attached is chosen from: pyrrolidine, piperidine, azepane, azacyclooctane, morpholine, piperazine and decahydroisoquinoline;
■the heterocycloalkyl radical represented by R'₄ , optionally substituted by C₁-C₆)alkyl or benzyl, is chosen from: pyrrolidinyl, piperidinyl, morpholinyl or piperazinyl;
■ the heteroaryl radical represented by R'₄ is the imidazolyl radical;
■ the cycloalkyl represented by Z₄ is chosen from: cyclopropyl, cyclobutyl, cyclopentyl, cyclohexyl or cycloheptyl;
■ the aryl represented by Z₄ is phenyl; or a pharmaceutically acceptable salt thereof.

13. Compounds according to claim 11, **characterized in that** R₄ represents a radical of formula -(CH₂)ₛ-R'₄ with R'₄ representing the pyrrolidinyl or piperidinyl radical; or a radical of formula -NW₄W'₄
W₄ represents the hydrogen atom or (C₁-C₈)alkyl;
W'₄ represents a radical of formula -(CH₂)_{s'}-Z₄ with Z₄ representing the hydrogen atom;
s represents an integer from 2 to 4; s' represents an integer from 0 to 4; or a pharmaceutically acceptable salt thereof.

14. Compounds according to one of claims 11 to 13, **characterized in that** R₁ and R₂ represent, independently, a (C₁-C₈)alkyl radical; or a pharmaceutically acceptable salt thereof.

15. Compounds according to one of claims 11 to 14, **characterized in that**
R₃ represents -Z₃ and Z₃ represents Z_{3c}, Z_{3d} or Z₃ₑ;
Z_{3d} represents a (C₃-C₇)cycloalkyl or heterocycloalkyl radical; or a pharmaceutically acceptable salt thereof.

16. Compounds according to claim 15, **characterized in that**
Z_{3c} represents a heteroaryl radical chosen from thienyl, furyl, indolyl, dihydroindolyl, pyridyl, benzothienyl and benzofuryl; or an aryl radical chosen from phenyl, naphthyl and fluorenyl;
the heteroaryl radical being optionally substituted by one or more identical or different substituents chosen from: (C₁-C₆)alkyl-carbonyl and (C₁-C₆)alkoxy-carbonyl;
the aryl radical being optionally substituted by one or more identical or different substituents chosen from: halo, cyano, nitro, azido, (C₁-C₆)alkoxy-carbonyl-(C₁-C₆)alkenyl, oxy, -SO₂-NR₃₁R₃₂, heterocycloalkyl, heteroaryl, or -(CH₂)_{p'}-V₃-Y₃;
R₃₁ and R₃₂ form together with the nitrogen atom to which they are attached, the piperidine ring;
V₃ represents -O-, -S-, -C(O)-, -C(O)-O-, -SO₂-, -SO₂NH-, -NR'₃-, -NR'₃-C(O)-, -C(O)-NR'₃-, -NH-C(O)-NR'₃- or a covalent bond;
Y₃ represents the hydrogen atom; a (C₁-C₆)alkyl radical optionally substituted by one or more identical or different halo radicals; a phenyl radical; or a benzyl radical;
R'₃ represents the hydrogen atom, a (C₁-C₆)alkyl or (C₁-C₆)alkoxy radical;
Z_{3d} represents the cyclopropyl, cyclohexyl or piperidinyl radical, each being able to be substituted by a (C₁-C₆)alkoxy-carbonyl radical; or a pharmaceutically acceptable salt thereof.

17. Compounds according to claim 15, **characterized in that**
R₃ represents -Z₃ and Z₃ represents Z_{3c}, Z_{3d} or Z₃ₑ;
Z_{3c} represents a heteroaryl radical chosen from thienyl, indolyl and benzothienyl; or an aryl radical chosen from phenyl and naphthyl;
the heteroaryl radical being optionally substituted by one or more oxy radicals;
the aryl radical being optionally substituted by one or more identical or different substituents chosen from: halo, nitro, heteroaryl or -(CH₂)_{p'}-V₃-Y₃;
V₃ represents -O-, -S-, -C(O)-, -C(O)-O-, -SO₂-, -SO₂NH-, -NR'₃-C(O)-, -C(O)-NR'₃-, -NH-C(O)-NR'₃- or a covalent bond;
Y₃ represents the hydrogen atom; a (C₁-C₆)alkyl radical optionally substituted by one or more identical or different halo radicals; a phenyl radical; or a benzyl radical;
R'₃ represents the hydrogen atom, a (C₁-C₆)alkyl or (C₁-C₆)alkoxy radical;
Z_{3d} represents the cyclopropyl or piperidinyl radical, each optionally substituted by (C₁-C₆)alkoxy-carbonyl; or a pharmaceutically acceptable salt thereof.

18. Compounds according to claim 17, **characterized in that**
Z₃ represents Z_{3c} or Z₃ₑ;
Z_{3c} represents a phenyl radical being optionally substituted by one or more identical or different substituents chosen from nitro and -(CH₂)_{p'}-V₃-Y₃;
V₃ represents -O-, -S-, -C(O)-, -C(O)-O-, -SO₂-, -SO₂NH-, -NR'₃-C(O)-, -C(O)-NR'₃- or a covalent bond;
Y₃ represents the hydrogen atom; a (C₁-C₆)alkyl radical; phenyl; or benzyl;
R'₃ represents the hydrogen atom;
Z₃ₑ represents 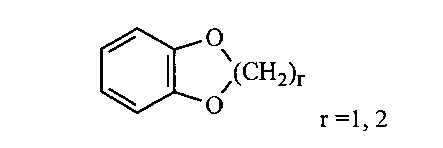 or a pharmaceutically acceptable salt thereof.

19. Compounds according to one of claims 11 to 14, **characterized in that** R₃ represents -C(R_{Z3})(R'_{Z3})-Z₃ and Z₃ represents Z_{3b}, Z_{3c}, Z_{3d} or Z₃ₑ; or a pharmaceutically acceptable salt thereof.

20. Compounds according to claim 19, **characterized in that**
R₃ represents -C(R_{Z3})(R'_{Z3})-Z₃ and Z₃ represents Z_{3b} or Z_{3c};
R_{Z3} and R'_{Z3} represent the hydrogen atom; or a pharmaceutically acceptable salt thereof.

21. Compounds according to claim 20 **characterized in that**
Z_{3b} represents a (C₁-C₆)alkoxy radical;
Z_{3c} represents a heteroaryl radical chosen from thienyl, furyl, pyridyl, benzothienyl and dihydrobenzofuryl; or an aryl radical chosen from phenyl and naphthyl,
the aryl radical being optionally substituted by one or more identical or different substituents chosen from: halo or -(CH₂)_{p'}-V₃-Y_{3;}
V₃ represents -O-, -S-, -C(O)-, -C(O)-O-, -SO₂- -SO₂N -NR'₃-C(O)-, -C(O)-NR'₃-,
Y₃ represents the hydrogen atom; a (C₁-C₆)alkyl radical optionally substituted by one or more identical or different halo radicals;
R'₃ represents the hydrogen atom; or a pharmaceutically acceptable salt thereof

22. Compounds according to claim 20, **characterized in that**
R₃ represents -C(R_{Z3})(R'_{Z3})-Z₃ and Z₃ represents Z_{3b} or Z_{3c};
Z_{3b} represents a (C₁-C₆)alkoxy radical;
Z_{3c} represents a heteroaryl radical chosen from thienyl, furyl, dihydrobenzofuryl; or a phenyl radical;
the phenyl radical being optionally substituted by one or more identical or different substituents chosen from: nitro or -(CH₂)_{p'}-V₃-Y₃;
V₃ represents -O-, -S-, -C(O)-, -C(O)-O-, -SO₂-, -SO₂NH-, -C(O)-NR'₃-,
Y₃ represents the hydrogen atom; or a (C₁-C₆)alkyl radical optionally substituted by one or more identical or different halo radicals;
R'₃ represents the hydrogen atom; or a pharmaceutically acceptable salt thereof.

23. Compounds according to claim 22, **characterized in that**
Z₃ represents Z_{3c};
Z_{3c} represents a furyl or phenyl radical,
the phenyl radical being optionally substituted by one or more identical or different substituents of formula -(CH₂)ₚ'-V₃-Y₃;
V₃ represents -O-, -S-, -C(O)-, -C(O)-O-, -SO₂-, -SO₂NH-, -C(O)-NR'₃-,
Y₃ represents the hydrogen atom; or a (C₁-C₆)alkyl radical optionally substituted by one or more identical or different halo radicals;
R'₃ represents the hydrogen atom; or a pharmaceutically acceptable salt thereof.

24. Compounds according to claim 19, **characterized in that**
R₃ represents -C(R_{Z3})(R'_{Z3})-Z₃ and Z₃ represents Z_{3d} or Z₃ₑ;
R_{Z3} and R'_{Z3} represent the hydrogen atom or (C₁-C₆)alkyl;
Z_{3d} represents a (C₁-C₆)alkoxy-carbonyl, (C₃-C₇)cycloalkyl or heterocycloalkyl radical;
Z₃ₑ represents 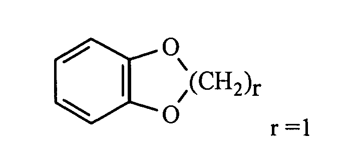 or a pharmaceutically acceptable salt thereof.

25. Compounds according to claim 24 **characterized in that**
Z_{3d} represents a (C₁-C₆)alkoxy-carbonyl, cyclohexyl or a tetrahydrofuranyl radical; or a pharmaceutically acceptable salt thereof.

26. Compounds according to claim 24 **characterized in that** Z₃ represents Z_{3d} or Z₃ₑ;
Z_{3d} represents a (C₁-C₆)alkoxy-carbonyl radical;
Z₃ₑ represents 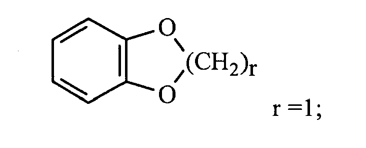 or a pharmaceutically acceptable salt thereof.

27. Compounds according to claim 26 **characterized in that** Z₃ represents Z₃ₑ 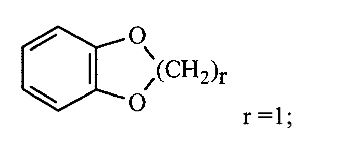 or a pharmaceutically acceptable salt thereof.

28. Compounds according to one of claims 11 to 14, **characterized in that** R₃ represents -C(R_{Z3})(R'_{Z3})-(CH₂)ₚ-Z₃ and Z₃ represents Z_{3b}, Z_{3c} or Z_{3d}; or a pharmaceutically acceptable salt thereof.

29. Compounds according to claim 28, **characterized in that** R₃ represents -C(R_{Z3})(R'_{Z3})-(CH₂)ₚ-Z₃ and Z₃ represents Z_{3b}; or a pharmaceutically acceptable salt thereof.

30. Compounds according to claim 29, **characterized in that**
R_{Z3} and R'_{Z3} represent, independently, the hydrogen atom or a (C₁-C₆)alkyl radical;
Z_{3b} represents a (C₁-C₆)alkoxy, (C₁-C₆)alkylthio or di((C₁-C₆)alkyl)amino radical; or a pharmaceutically acceptable salt thereof.

31. Compounds according to claim 29, **characterized in that**
R_{Z3} and R'_{Z3} represent, independently, the hydrogen atom or a (C₁-C₆)alkyl radical;
Z_{3b} represents a (C₁-C₆)alkoxy or (C₁-C₆)alkylthio radical; or a pharmaceutically acceptable salt thereof.

32. Compounds according to claim 28, **characterized in that** R₃ represents -C(R_{Z3})(R'_{Z3})-(CH₂)ₚ-Z₃ and Z₃ represents Z_{3c} or Z_{3d}; or a pharmaceutically acceptable salt thereof.

33. Compounds according to claim 32, **characterized in that**
R_{Z3} and R'_{Z3} represent, independently, the hydrogen atom or a (C₁-C₆)alkyl radical;
Z_{3c} represents an indolyl or phenyl radical;
the phenyl radical being optionally substituted by one or more identical or different substituents chosen from: halo and -(CH₂)_{p'}-V₃-Y₃;
V₃ represents -SO₂NH- ,
Y₃ represents the hydrogen atom; or a (C₁-C₆)alkyl radical;
Z_{3d} represents a (C₁-C₆)alkoxy-carbonyl, amino-carbonyl, (C₁-C₆)alkyl-amino-carbonyl, (C₁-C₆)alkyl-C(O)-NH-, or heterocycloalkyl radical optionally substituted by oxy, and preferably piperidinyl, morpholinyl, pyrrolidine or imidazolidinyl; or a pharmaceutically acceptable salt thereof.

34. Compounds according to claim 32, **characterized in that**
Z₃ represents Z_{3d};
R_{Z3} and R'_{Z3} represent, independently, the hydrogen atom or a (C₁-C₆)alkyl radical;
Z_{3d} represents a (C₁-C₆)alkoxy-carbonyl, amino-carbonyl, (C₁-C₆)alkylaminocarbonyl, (C₁-C₆)alkyl-C(O)-NH- or heterocycloalkyl radical, and preferably pyrrolidine or imidazolidine, optionally substituted by oxy; or a pharmaceutically acceptable salt thereof.

35. Process for the preparation of a compound of formula (I) according to one of the preceding claims **characterized in that** the compound of general formula: 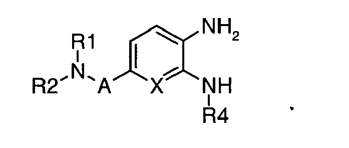 in which A, X, R₁, R₂, R₄ have the meaning indicated in claim 1, is treated with an isothiocyanate of general formula R₃N=C=S in which R₃ has the meaning indicated in claim 1, in the presence of a coupling agent or yellow mercury (II) oxide in the presence of sulphur, for a period of 3 to 48 hours, in a protic or aprotic solvent, at a temperature of 50 to 80°C.

36. Pharmaceutical composition containing, as active ingredients, at least one compound according to one of claims 1 to 34, in combination with a pharmaceutically acceptable support.

37. Use of a compound according to one of claims 1 to 34, for the preparation of a medicament for the treatment of weight disorders, mental health problems or sexual activity disorders.

38. Use according to claim 37, for the treatment of weight disorders such as anorexia and cachexia and more particularly cancer cachexia, AIDS cachexia, old-age cachexia, cardiac cachexia, renal cachexia, cachexia in rheumatoid arthritis.

39. Use according to claim 37, for the treatment of pain and more particularly neuropathic pain.

40. Use according to claim 37, for the treatment of mental health problems such as anxiety and depression.

41. Use of a compound of general formula (I') 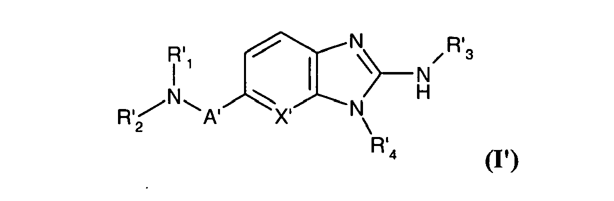 in racemic or enantiomeric form or any combinations of these forms and in which:
A' represents -CH₂- ,-C(O)-, -C(O)-C(Rₐ)(R_{b})-;
X' represents -CH-;
Rₐ and R_{b} represent, independently, the hydrogen atom or a (C₁-C₆)alkyl radical;
R'₁ represents the hydrogen atom; a (C₁-C₈)alkyl radical optionally substituted by hydroxy or one or more identical or different halo radicals; (C₂-C₆)alkenyl; or a radical of formula-(CH₂)ₙ-X₁;
R'₂ represents a (C₁-C₈)alkyl radical optionally substituted by hydroxy or one or more identical or different halo radicals; (C₂-C₆)alkenyl; or a radical of formula -(CH₂)ₙ-X₁;
each X₁ independently represents (C₁-C₆)alkoxy, (C₃-C₇)cycloalkyl, adamantyl, heterocycloalkyl, aryl or heteroaryl,
the (C₃-C₇)cycloalkyl, heterocycloalkyl, aryl and heteroaryl radicals being optionally substituted by one or more identical or different substituents chosen from: -(CH₂)_{n'}-V₁-Y₁, halo, nitro, cyano and aryl;
V₁ represents -0-, -S- or a covalent bond;
Y₁ represents a (C₁-C₆)alkyl radical optionally substituted by one or more identical or different halo radicals;
n represents an integer from 0 to 6 and n' an integer from 0 to 2 (it being understood that when n is equal to 0, then X₁ does not represent the alkoxy radical)
or R₁ and R₂ form together with the nitrogen atom to which they are attached, a heterobicycloalkyl or a heterocycloalkyl optionally substituted by one or more identical or different substituents chosen from: hydroxy, (C₁-C₆)alkyl optionally substituted by hydroxy, (C₁-C₆)alkoxy-carbonyl, heterocycloalkyl and -C(O)NV₁'Y₁' with V₁' and Y₁' independently representing the hydrogen atom or a (C₁-C₆)alkyl; or R₁ and R₂ form together a radical of formula: 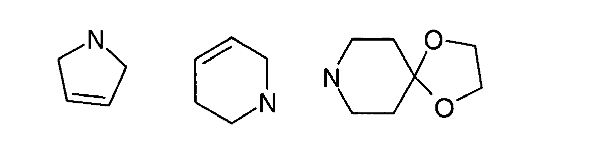
R'₃ represents -Z₃, C(R_{Z3})(R'_{Z3})-Z₃, -C(R_{Z3})(R'_{Z3})-(CH₂)ₚ-Z₃ or -C(O)-Z'₃
R_{Z3} and R'_{Z3} represent, independently, the hydrogen atom or a (C₁-C₆)alkyl radical;
Z₃ represents Z₃ₐ, Z_{3b}, Z_{3c}, Z_{3d}, or Z₃ₑ;
Z₃ₐ represents a (C₁-C₆)alkyl radical;
Z_{3b} represents a (C₁-C₆)alkoxy, (C₁-C₆)alkylthio, (C₁-C₆)alkylamino or di((C₁-C₆)alkyl)amino radical;
Z_{3c} represents an aryl or heteroaryl radical;
Z_{3d} represents a (C₁-C₆)alkoxy-carbonyl, amino-carbonyl, (C₁-C₆)alkylaminocarbonyl, di((C₁-C₆)alkyl)amino-carbonyl, (C₁-C₆)alkyl-C(O)-NH-, (C₃-C₇)cycloalkyl, heterocycloalkyl radical;
the (C₃-C₇) cycloalkyl and heterocycloalkyl radicals being optionally substituted by one or more identical or different substituents chosen from: halo, nitro, (C₁)-C₆)alkoxy optionally substituted by one or more identical or different halo radicals, (C₁-C₆)alkyl optionally substituted by one or more identical or different halo radicals, (C₁-C₆)alkyl-carbonyl, (C₁-C₆)alkoxy-carbonyl, amino-carbonyl, (C₁-C₆)alkylamino-carbonyl, di((C₁-C₆)alkyl)amino-carbonyl and oxy,
the aryl and heteroaryl radicals being optionally substituted by one or more identical or different substituents chosen from: halo, cyano, nitro, azido, oxy, (C₁-C₆)alkoxy-carbonyl-(C₁-C₆)alkenyl, (C₁-C₆)alkylamino-carbonyl-(C₁-C₆)alkenyl, -SO₂-NR₃₁R₃₂, heterocycloalkyl, heteroaryl or -(CH₂)_{p'}-V₃-Y₃;
R₃₁ and R₃₂ form together with the nitrogen atom to which they are attached, a heterocycloalkyl;
V₃ represents -O-, -S-, -C(O)-, -C(O)-O-, -O-C(O)-, -SO₂-, -SO₂NH-, -NR'₃-SO₂-, -NR'₃-, -NR'₃-C(O)-, -C(O)-NR'₃-, -NH-C(O)-NR'₃- or a covalent bond;
Y₃ represents the hydrogen atom; a (C₁-C₆)alkyl radical optionally substituted by one or more identical or different halo radicals; an aryl radical optionally substituted by one or more identical or different substituents chosen from: halo, nitro, (C₁-C₆)alkyl and (C₁-C₆)alkoxy; or an aryl-(C₁-C₆)alkyl radical optionally substituted by one or more identical or different substituents chosen from: halo, nitro, (C₁-C₆)alkyl and (C₁-C₆)alkoxy;
Z₃ₑ represents a radical of formula 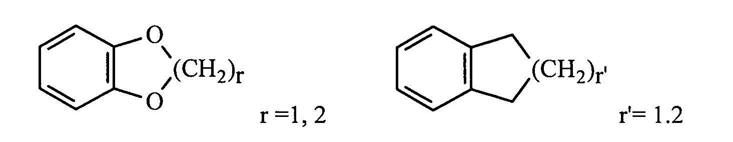 Z'₃ represents an aryl radical optionally substituted by one or more identical or different substituents chosen from: halo, nitro and -(CH₂)_{p"}-V'₃-Y'_{3;}
V'₃ represents -O-, -C(O)-, -C(O)-O-, -C(O)-NR'₃, -NH-C(O)-NR'₃ or a covalent bond;
Y'₃ represents the hydrogen atom or a (C₁-C₆)alkyl radical optionally substituted by one or more identical or different halo radicals;
R'₃ represents the hydrogen atom, a (C₁-C₆)alkyl or (C₁-C₆)alkoxy radical;
p represents an integer from 1 to 4; p' and p" represent, independently, an integer from 0 to 4;
R₄ represents a radical of formula -(CH₂)ₛ-R'₄;
R'₄ represents the guanidino radical; a heterocycloalkyl containing at least one nitrogen atom and optionally substituted by (C₁-C₆)alkyl or aralkyl; a heteroaryl containing at least one nitrogen atom and optionally substituted by (C₁-C₆)alkyl; or a radical of formula -NW₄W'₄
W₄ represents the hydrogen atom or (C₁-C₈)alkyl;
W'₄ represents a radical of formula -(CH₂)_{s'}-Z₄;
Z₄ represents the hydrogen atom, (C₁-C₈)alkyl optionally substituted by one or more identical or different substituents chosen from: (C₁-C₆)alkoxy, (C₁-C₆)alkylthio and hydroxy; (C₂-C₆)alkenyl; (C₃-C₇)cycloalkyl optionally substituted by one or more identical or different (C₁-C₆)alkyl substituents; cyclohexene; heteroaryl and aryl;
the aryl and heteroaryl radicals being optionally substituted by one or more identical or different radicals chosen from formula -(CH₂)_{s"}-V₄-Y₄, hydroxy,
halo, nitro and cyano;
V₄ represents -O-, -S-, -NH-C(O)-, -NV'₄ or a covalent bond;
Y₄ represents a hydrogen atom or a (C₁-C₆)alkyl radical optionally substituted by one or more identical or different halo radicals;
V₄' represents a hydrogen atom or a (C₁-C₆)alkyl;
s" represents an integer from 0 to 4;
or Z₄ represents a radical of formula 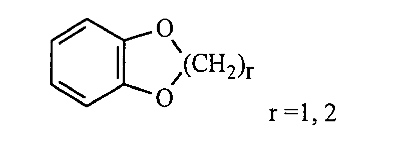
s and s' represent, independently, an integer from 0 to 6; or a pharmaceutically acceptable salt thereof;
for the preparation of a medicament for the treatment of weight disorders, mental health problems, pain, or sexual activity disorders.

42. Use according to claim 41, **characterized in that**
R₁ and R₂ represent, independently, a (C₁-C₈)alkyl radical;
R₃ represents Z_{3c} and Z_{3c} represents a phenyl or naphthyl radical, each substituted at least by cyano;
R₄ represents a radical of formula -(CH₂)ₛ-R'₄ with R'₄ representing the pyrrolidinyl or piperidinyl radical; or a radical of formula -NW₄W'₄;
W₄ represents the hydrogen atom or (C₁-C₈)alkyl;
W'₄ represents a radical of formula -(CH₂)_{s'}-Z₄ with Z₄ representing the hydrogen atom;
s represents an integer from 2 to 4; s' represents an integer from 0 to 4;
or a pharmaceutically acceptable salt thereof.

## Patentansprüche

1. Verbindungen der allgemeinen Formel (I) 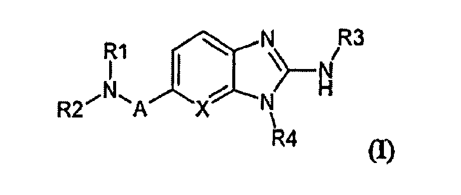 in racemischer Form, Enantiomerenform oder allen Kombinationen dieser Formen, in der:
A -CH₂-, -C(O)-, -C(O)-C(Rₐ)(R_{b})- darstellt;
X -CH- oder -N- darstellt;
Rₐ und R_{b} unabhängig voneinander ein Wasserstoffatom oder einen (C₁-C₆)-Alkylrest darstellen;
R₁ darstellt: ein Wasserstoffatom; einen (C₁-C₈)-Alkylrest, der gegebenenfalls mit Hydroxy oder einem oder mehreren gleichen oder verschiedenen Halogenresten substituiert ist; (C₂-C₆)-Alkenyl; oder einen Rest der Formel -(CH₂)ₙ-X₁;
R₂ darstellt: einen (C₁-C₈)-Alkylrest, der gegebenenfalls mit Hydroxy oder einem oder mehreren gleichen oder verschiedenen Halogenresten substituiert ist; (C₂-C₆)-Alkenyl; oder einen Rest der Formel -(CH₂)ₙ-X₁;
jedes X₁ unabhängig voneinander (C₁-C₆)-Alkoxy, (C₃-C₇)-Cycloalkyl, Adamantyl, Heterocycloalkyl, Aryl oder Heteroaryl darstellt;
wobei die Reste (C₃-C₇)-Cycloalkyl, Heterocycloalkyl, Aryl und Heteroaryl gegebenenfalls mit einem oder mehreren gleichen oder verschiedenen Substituenten substituiert sind, die ausgewählt sind aus: -(CH₂)ₙ-V₁-Y₁, Halogen, Nitro, Cyano und Aryl;
V₁ -O-, -S- oder eine kovalente Bindung darstellt;
Y₁ einen (C₁-C₆)-Alkylrest darstellt, der gegebenenfalls mit einem oder mehreren gleichen oder verschiedenen Halogenresten substituiert ist;
n eine ganze Zahl von 0 bis 6 und n' eine ganze Zahl von 0 bis 2 darstellt (wobei gilt, dass, wenn n gleich 0 ist, dann X₁ nicht den Alkoxyrest darstellt)
oder R₁ und R₂ zusammen mit dem Stickstoffatom, an das sie gebunden sind, ein Heterobicycloalkyl oder ein Heterocycloalkyl bilden, das gegebenenfalls mit einem oder mehreren gleichen oder verschiedenen Substituenten substituiert ist, die ausgewählt sind aus: Hydroxy, (C₁-C₆)-Alkyl, das gegebenenfalls mit Hydroxy substituiert ist, (C₁-C₆)-Alkoxycarbonyl, Heterocycloalkyl und -C(O)NV₁'Y₁', wobei V₁' und Y₁' unabhängig voneinander ein Wasserstoffatom oder ein (C₁-C₆)-Alkyl darstellen; oder R₁ und R₂ zusammen einen Rest der Formel bilden: 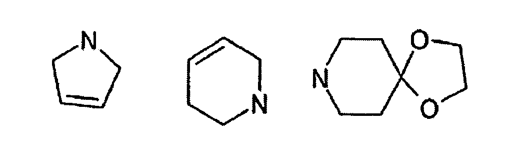
R₃ -Z₃, C(R_{Z3})(R'_{Z3})-Z₃, -C(R_{Z3})(R'_{Z3})-(CH₂)ₚ-Z₃ oder -C(O)-Z'₃ darstellt
R_{Z3} und R'_{Z3} unabhängig voneinander ein Wasserstoffatom oder einen (C₁-C₆)-Alkylrest darstellen;
Z₃ Z₃ₐ, Z_{3b}, Z_{3c}, Z_{3d} oder Z₃ₑ darstellt;
Z₃ₐ einen (C₁-C₆)-Alkylrest darstellt;
Z_{3b} einen der Reste (C₁-C₆)-Alkoxy, (C₁-C₆)-Alkylthio, (C₁-C₆)-Alkylamino oder Di((C₁-C₆)-alkyl)amino darstellt;
Z_{3c} einen Aryl- oder Heteroarylrest darstellt;
Z_{3d} einen der Reste (C₁-C₆)-Alkoxycarbonyl, Aminocarbonyl, (C₁-C₆)-Alkylaminocarbonyl, Di((C₁-C₆)-alkyl)aminocarbonyl, (C₁-C₆)-Alkyl-C(O)-NH-, (C₃-C₇)-Cycloalkyl, Heterocycloalkyl darstellt;
wobei die Reste (C₃-C₇)-Cycloalkyl und Heterocycloalkyl gegebenenfalls mit einem oder mehreren gleichen oder verschiedenen Substituenten substituiert sind, die ausgewählt sind aus: Halogen, Nitro, (C₁-C₆)-Alkoxy, das gegebenenfalls mit einem oder mehreren gleichen oder verschiedenen Halogenresten substituiert ist, (C₁-C₆)-Alkyl, das gegebenenfalls mit einem oder mehreren gleichen oder verschiedenen Halogenresten substituiert ist, (C₁-C₆)-Alkylcarbonyl, (C₁-C₆)-Alkoxycarbonyl, Aminocarbonyl, (C₁-C₆)-Alkylaminocarbonyl, Di((C₁-C₆)-alkyl)amino-carbonyl und Oxy,
wobei die Aryl- und Heteroarylreste gegebenenfalls mit einem oder mehreren gleichen oder verschiedenen Substituenten substituiert sind, die ausgewählt sind aus: Halogen, Cyano, Nitro, Azido, Oxy, (C₁-C₆)-Alkoxycarbonyl-(C₁-C₆)-alkenyl, (C₁-C₆)-Alkylaminocarbonyl-(C₁-C₆)-alkenyl, -SO₂-NR₃₁R₃₂, Heterocycloalkyl, Heteroaryl oder -(CH₂)ₚ'-V₃-Y₃;
R₃₁ und R₃₂ zusammen mit dem Stickstoffatom, an das sie gebunden sind, ein Heterocycloalkyl bilden;
V₃ -O-, -S-, -C(O)-, -C(O)-O-, -O-C(O)-, -SO₂-, -SO₂NH-, -NR'₃-SO₂-, -NR'₃-, -NR'₃-C(O)-, -C(O)-NR'₃-, -NH-C(O)-NR'₃ oder eine kovalente Bindung darstellt;
Y₃ darstellt: ein Wasserstoffatom; einen (C₁-C₆)-Alkylrest, der gegebenenfalls mit einem oder mehreren gleichen oder verschiedenen Halogenresten substituiert ist; einen Arylrest, der gegebenenfalls mit einem oder mehreren gleichen oder verschiedenen Substituenten substituiert ist, die ausgewählt sind aus: Halogen, Nitro, (C₁-C₆)-Alkyl und (C₁-C₆)-Alkoxy; oder einen Aryl-(C₁-C₆)-alkylrest, der gegebenenfalls mit einem oder mehreren gleichen oder verschiedenen Substituenten substituiert ist, die ausgewählt sind aus: Halogen, Nitro, (C₁-C₆)-Alkyl und (C₁-C₆)-Alkoxy;
Z₃ₑ einen Rest der Formel darstellt: 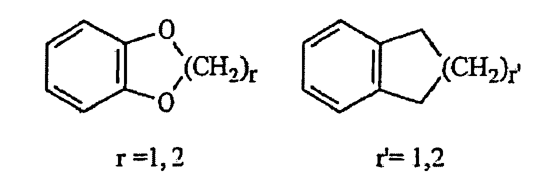 Z'₃ einen Arylrest darstellt, der gegebenenfalls mit einem oder mehreren gleichen oder verschiedenen Substituenten substituiert ist, die ausgewählt sind aus: Halogen, Nitro und -(CH₂)_{p"}-V'₃-Y'₃;
V'₃ -O-, -C(O)-, -C(O)-O-, -C(O)-NR'₃, -NH-C(O)-NR'₃ oder eine kovalente Bindung darstellt;
Y'₃ ein Wasserstoffatom oder einen (C₁-C₆)-Alkylrest darstellt, der gegebenenfalls mit einem oder mehreren gleichen oder verschiedenen Halogenresten substituiert ist;
R'₃ ein Wasserstoffatom, einen Rest (C₁-C₆)-Alkyl oder (C₁-C₆)-Alkoxy darstellt;
p eine ganze Zahl von 1 bis 4 darstellt; p' und p" unabhängig voneinander eine ganze Zahl von 0 bis 4 darstellen;
R₄ einen Rest der Formel -(CH₂)ₛ-R'₄ darstellt;
R'₄ darstellt: den Guanidinorest; ein Heterocycloalkyl, das mindestens ein Stickstoffatom enthält und gegebenenfalls mit (C₁-C₆)-Alkyl oder Aralkyl substituiert ist; ein Heteroaryl, das mindestens ein Stickstoffatom enthält und gegebenenfalls mit (C₁-C₆)-Alkyl substituiert ist; oder einen Rest der Formel -NW₄W'₄;
W₄ ein Wasserstoffatom oder (C₁-C₈)-Alkyl darstellt;
W'₄ einen Rest der Formel -(CH₂)_{s'}-Z₄ darstellt;
Z₄ darstellt: ein Wasserstoffatom, (C₁-C₈)-Alkyl, das gegebenenfalls mit einem oder mehreren gleichen oder verschiedenen Substituenten substituiert ist, die ausgewählt sind aus: (C₁-C₆)-Alkoxy, (C₁-C₆)-Alkylthio und Hydroxy; (C₂-C₆)-Alkenyl; (C₃-C₇)-Cycloalkyl, das gegebenenfalls mit einem oder mehreren gleichen oder verschiedenen (C₁-C₆)-Alkylsubstituenten substituiert ist; Cyclohexen; Heteroaryl und Aryl;
wobei die Reste Aryl und Heteroaryl gegebenenfalls mit einem oder mehreren gleichen oder verschiedenen Resten substituiert sind, die ausgewählt sind aus der Formel -(CH₂)_{s"}-V₄-Y₄, Hydroxy, Halogen, Nitro und Cyano;
V₄ -O-, -S-, -NH-C(O)-, -NV'₄ oder eine kovalente Bindung darstellt;
Y₄ ein Wasserstoffatom oder einen Rest (C₁-C₆)-Alkyl darstellt, der gegebenenfalls mit einem oder mehreren gleichen oder verschiedenen Halogenresten substituiert ist;
V₄' ein Wasserstoffatom oder ein (C₁-C₆)-Alkyl darstellt;
s" eine ganze Zahl von 0 bis 4 darstellt;
oder Z₄ einen Rest der Formel darstellt: 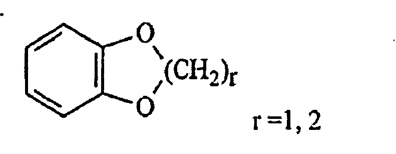
s und s' unabhängig voneinander eine ganze Zahl von 0 bis 6 darstellen;
und i) wenn R₃ -C(O)-Z'₃ darstellt und R₄ einen Rest der Formel -(CH₂)ₛ NW₄W'₄ darstellt und W₄ und W'₄ unabhängig voneinander ein Wasserstoffatom oder den (C₁-C₆)-Alkylrest darstellen, dann stellt -(CH₂)ₛ weder den Ethylenrest-noch-den Rest -(CH₂)-CH((C₁-C₄)-Alkyl)- dar und ii) wenn R₃ -Z_{3c} darstellt und Z_{3c} einen Phenyl- oder Naphthylrest darstellt, dann sind Phenyl und Naphthyl nicht mit Cyano substituiert; wobei gilt, dass wenn R₃ -Z_{3d} darstellt, dann Z_{3d} nur einen (C₃-C₇)-Cycloalkyl- oder Heterocycloalkylrest darstellt;
oder ein pharmazeutisch annehmbares Salz dieser Verbindungen.

2. Verbindungen nach Anspruch 1, **dadurch gekennzeichnet, dass** X -CH- darstellt; oder ein pharmazeutisch annehmbares Salz dieser Verbindungen.

3. Verbindungen nach Anspruch 2, **dadurch gekennzeichnet, dass** A -CH₂- darstellt; oder ein pharmazeutisch annehmbares Salz dieser Verbindungen.

4. Verbindungen nach Anspruch 3, **dadurch gekennzeichnet, dass**
R₁ und R₂ unabhängig voneinander einen (C₁-C₈)-Alkylrest darstellen;
R₃ -Z_{3c}, -C(R_{Z3})(R'_{Z3})-Z_{3c}, -C(R_{Z3})(R'_{Z3})-Z_{3d}, -C(R_{Z3})(R'_{Z3})-(CH₂)ₚ-Z_{3d} darstellt;
R₄ einen Rest der Formel -(CH₂)ₛ-R'₄ darstellt;
R'₄ ein Heterocycloalkyl darstellt, das mindestens ein Stickstoffatom enthält und gegebenenfalls mit (C₁-C₆)-Alkyl substituiert ist; oder einen Rest der Formel -NW₄W'₄
W₄ ein Wasserstoffatom oder (C₁-C₈)-Alkyl darstellt;
W'₄ einen Rest der Formel -(CH₂)_{s'}-Z₄ darstellt;
Z₄ ein Wasserstoffatom darstellt;
s eine ganze Zahl von 2 bis 4 darstellt; s' eine ganze Zahl von 0 bis 4 darstellt;
oder ein pharmazeutisch annehmbares Salz dieser Verbindungen.

5. Verbindungen nach Anspruch 4, **dadurch gekennzeichnet, dass**
das Heterocycloalkyl, das R'₄ darstellt, der Piperidinring ist;
R_{Z3} und R'_{Z3} ein Wasserstoffatom darstellen;
Z_{3c} den-Thienyl-, Furyl-oder-Phenylrest-darstellt;
der Phenylrest mit einem oder mehreren gleichen oder verschiedenen Substituenten substituiert ist, die ausgewählt sind aus: Halogen und -(CH₂)_{p'}-V₃-Y₃;
V₃ -O-, -C(O)-, -C(O)-O-, -C(O)-NR'₃- oder eine kovalente Bindung darstellt;
R'₃ ein Wasserstoffatom oder einen (C₁-C₆)-Alklyrest darstellt;
Y₃ darstellt: ein Wasserstoffatom; einen (C₁-C₆)-Alkylrest, der gegebenenfalls mit einem oder mehreren gleichen oder verschiedenen Halogenresten substituiert ist;
Z_{3d} den Rest (C₁-C₆)-Alkoxycarbonyl oder Heterocycloalkyl darstellt und vorzugsweise das Heterocycloalkyl das Imidazolidin ist;
oder ein pharmazeutisch annehmbares Salz dieser Verbindungen.

6. Verbindungen nach Anspruch 2, **dadurch gekennzeichnet, dass** A -C(O)-C(Rₐ)(R_{b})- darstellt, wobei Rₐ und R_{b} den Methylrest darstellen; oder ein pharmazeutisch annehmbares Salz dieser Verbindungen.

7. Verbindungen nach Anspruch 6, **dadurch gekennzeichnet, dass**
R₁ und R₂ unabhängig voneinander einen (C₁-C₈)-Alkylrest darstellen;
R₃ -Z_{3c}, -C(R_{Z3})(R'_{Z3})-Z_{3c}, -C(R_{Z3})(R'_{Z3})-Z_{3d} oder -C(R_{Z3})(R'_{Z3})-(CH₂)ₚ-Z_{3d} darstellt;
R₄ einen Rest der Formel -(CH₂)ₛ-R'₄ darstellt;
R'₄ ein Heterocycloalkyl darstellt, das mindestens ein Stickstoffatom enthält und gegebenenfalls mit (C₁-C₆)-Alkyl substituiert ist; oder einen Rest der Formel -NW₄W'₄;
W₄ ein Wasserstoffatom oder (C₁-C₈)-Alkyl darstellt;
W'₄ einen Rest der Formel -(CH₂)_{s'}-Z₄ darstellt;
Z₄ ein Wasserstoffatom, den Phenylrest oder ein Heteroaryl darstellt;
s eine ganze Zahl von 2 bis 4 darstellt; s' eine ganze Zahl von 0 bis 4 darstellt;
oder ein pharmazeutisch annehmbares Salz dieser Verbindungen.

8. Verbindungen nach Anspruch 7, **dadurch gekennzeichnet, dass**
R_{Z3} und R'_{Z3} unabhängig voneinander ein Wasserstoffatom darstellen;
Z_{3c} darstellt: einen Thienylrest, der gegebenenfalls mit (C₁-C₆)-Alkoxycarbonyl substituiert ist; oder Phenyl, das mit einem oder mehreren gleichen oder verschiedenen Substituenten substituiert ist, die ausgewählt sind aus: Halogen, Nitro oder -(CH₂)_{p'}-V₃-Y₃;
V₃ -O-, -C(O)-, -C(O)-O-, -C(O)-NR'₃- oder eine kovalente Bindung darstellt;
Y₃ darstellt: ein Wasserstoffatom; einen (C₁-C₆)-Alkylrest, der gegebenenfalls mit einem oder mehreren gleichen oder verschiedenen Halogenresten substituiert ist;
R'₃ ein Wasserstoffatom darstellt;
Z_{3d} einen (C₁-C₆)-Alkoxycarbonylrest darstellt;
das Heterocycloalkyl, das R'₄ darstellt, Piperidin ist;
das Heteroaryl, das Z₄ darstellt, Pyridin ist; oder ein pharmazeutisch annehmbares Salz dieser Verbindungen.

9. Verbindungen nach Anspruch 2, **dadurch gekennzeichnet, dass** A -C(O)- darstellt; oder ein pharmazeutisch annehmbares Salz dieser Verbindungen.

10. Verbindungen nach Anspruch 9, **dadurch gekennzeichnet, dass** R₃ -C(O)-Z'₃ darstellt;
R₁ und R₂ unabhängig voneinander einen (C₁-C₈)-Alkylrest darstellen;
Z'₃ einen Phenylrest darstellt, der gegebenenfalls mit einem oder mehreren gleichen oder verschiedenen Substituenten substituiert ist, die ausgewählt sind aus: Halogen, Nitro und -(CH₂)_{p"}-V'₃-Y'₃;
V'₃ -O-, -C(O)-O- oder eine kovalente Bindung darstellt;
Y'₃ ein Wasserstoffatom oder einen (C₁-C₆)-Alkylrest darstellt;
p" die ganze Zahl 0 darstellt;
R₄ einen Rest der Formel -(CH₂)ₛ-R'₄ darstellt und R'₄ einen Rest der Formel -NW₄W'₄ darstellt
W₄ ein Wasserstoffatom oder (C₁-C₈)-Alkyl darstellt;
W'₄ einen Rest der Formel -(CH₂)_{s'}-Z₄ darstellt und Z₄ ein Wasserstoffatom darstellt;
s eine ganze Zahl von 2 bis 4 darstellt; s' eine ganze Zahl von 0 bis 4 darstellt;
oder ein pharmazeutisch annehmbares Salz dieser Verbindungen.

11. Verbindungen nach Anspruch 9, **dadurch gekennzeichnet, dass**
R₁ ein Wasserstoffatom, einen (C₁-C₈)-Alkylrest, der gegebenenfalls mit Hydroxy substituiert ist, (C₂-C₆)-Alkenyl oder einen Rest der Formel -(CH₂)ₙ-X₁ darstellt;
R₂ einen (C₁-C₈)-Alkylrest, der gegebenenfalls mit Hydroxy substituiert ist, (C₂-C₆)-Alkenyl oder einen Rest der Formel -(CH₂)ₙ-X₁ darstellt;
jedes X₁ unabhängig voneinander (C₁-C₆)-Alkoxy, (C₃-C₇)-Cycloalkyl, Aryl oder Heteroaryl darstellt,
wobei der Arylrest gegebenenfalls mit einem oder mehreren gleichen oder verschiedenen Substituenten substituiert ist, die ausgewählt sind aus: -(CH₂)_{n'}-V₁-Y₁, Halogen;
V₁ -O- oder eine kovalente Bindung darstellt;
Y₁ einen (C₁-C₆)-Alkylrest, der gegebenenfalls mit einem oder mehreren gleichen oder verschiedenen Halogenresten substituiert ist, oder Aryl darstellt;
oder R₁ und R₂ zusammen mit dem Stickstoffatom, an das sie gebunden sind, ein Heterocycloalkyl bilden, das gegebenenfalls mit einem oder mehreren gleichen oder verschiedenen Substituenten substituiert ist, die ausgewählt sind aus: Hydroxy, (C₁-C₆)-Alkyl, das gegebenenfalls mit Hydroxy substituiert ist, (C₁-C₆)-Alkoxycarbonyl, Heterocyloalkyl und -C(O)NV₁'Y₁', wobei V₁' und Y₁' unabhängig voneinander ein Wasserstoffatom oder ein (C₁-C₆)-Alkyl darstellen,
oder R₁ und R₂ zusammen einen Rest der Formel bilden: 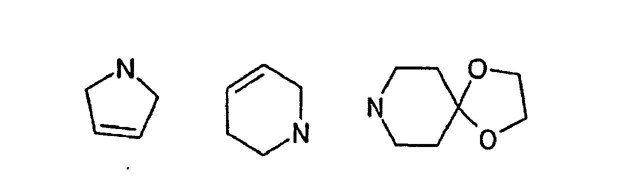 R₃ -Z_{3,}-C(R_{Z3})(R'_{Z3})-Z₃, oder-C(R_{Z3})(R'_{Z3})-(CH₂)ₚ-Z₃-darstellt:
R₄ einen Rest der Formel -(CH₂)ₛ-R'₄ darstellt;
R'₄ darstellt: ein Heterocycloalkyl, das mindestens ein Stickstoffatom enthält und gegebenenfalls mit (C₁-C₆)-Alkyl oder Aralkyl substituiert ist; ein Heteroaryl, das mindestens ein Stickstoffatom enthält und gegebenenfalls mit (C₁-C₆)-Alkyl substituiert ist; oder einen Rest der Formel -NW₄W'₄
W₄ ein Wasserstoffatom oder (C₁-C₈)-Alkyl darstellt;
W'₄ einen Rest der Formel -(CH₂)ₛ-Z₄ darstellt;
Z₄ ein Wasserstoffatom, (C₃-C₇)-Cycloalkyl oder Aryl darstellt;
s eine ganze Zahl von 0 bis 5 darstellt; s' eine ganze Zahl von 0 bis 4 darstellt; oder ein pharmazeutisch annehmbares Salz dieser Verbindungen.

12. Verbindungen nach Anspruch 11, **dadurch gekennzeichnet, dass** sie mindestens eines der folgenden Merkmale umfassen:
• der (C₃-C₇)-Cycloalkylrest, den X₁ darstellt, ist Cyclopropyl;
**•** der Arylrest, den X₁ darstellt, ist der Phenylrest;
**•** der Heteroarylrest, den X₁ darstellt, ist Pyridin;
**•** das Heterocycloalkyl, das R₁ und R₂ zusammen mit dem Stickstoffatom, an das sie gebunden sind, bilden, ist ausgewählt aus: Pyrrolidin, Piperidin, Azepan, Azacyclooctan, Morpholin, Piperazin und Decahydroisochinolin;
**•** der Heterocycloalkylrest, den R'₄ darstellt, der gegebenenfalls mit (C₁-C₆)-Alkyl oder Benzyl substituiert ist, ist ausgewählt aus: Pyrrolidinyl, Piperidinyl, Morpholinyl oder Piperazinyl;
● der Heteroarylrest, den R'₄ darstellt, ist der Imidazolylrest;
● das Cycloalkyl, das Z₄ darstellt, ist ausgewählt aus: Cyclopropyl, Cyclobutyl, Cyclopentyl, Cyclohexyl oder Cycloheptyl;
● das Aryl, das Z₄ darstellt, ist Phenyl; oder ein pharmazeutisch annehmbares Salz dieser Verbindungen.

13. Verbindungen nach Anspruch 11, **dadurch gekennzeichnet, dass** R₄ darstellt: einen Rest der Formel -(CH₂)ₛ-R'₄, wobei R'₄ den Pyrrolidinyl- oder Piperidinylrest darstellt; oder einen Rest der Formel -NW₄W'₄
W₄ ein Wasserstoffatom oder (C₁-C₈)-Alkyl darstellt;
W'₄ einen Rest der Formel -(CH₂)_{s'}-Z₄ darstellt, wobei Z₄ ein Wasserstoffatom darstellt;
s eine ganze Zahl von 2 bis 4 darstellt; s' eine ganze Zahl von 0 bis 4 darstellt; oder ein pharmazeutisch annehmbares Salz dieser Verbindung.

14. Verbindungen nach einem der Ansprüche 11 bis 13, **dadurch gekennzeichnet, dass** R₁ und R₂ unabhängig voneinander einen (C₁-C₈)-Alkylrest darstellen; oder ein pharmazeutisch annehmbares Salz dieser Verbindungen.

15. Verbindungen nach einem der Ansprüche 11 bis 14, **dadurch gekennzeichnet, dass**
R₃ -Z₃ darstellt und Z₃ Z_{3c}, Z_{3d} oder Z₃ₑ darstellt;
Z_{3d} einen Rest (C₃-C₇)-Cycloalkyl oder Heterocycloalkyl darstellt; oder ein pharmazeutisch annehmbares Salz dieser Verbindungen.

16. Verbindungen nach Anspruch 15, **dadurch gekennzeichnet, dass**
Z_{3c} darstellt: einen Heteroarylrest, der aus Thienyl, Furyl, Indolyl, Dihydroindolyl, Pyridyl, Benzothienyl und Benzofuryl ausgewählt ist; oder einen Arylrest, der aus Phenyl, Naphthyl und Fluorenyl ausgewählt ist;
wobei der Heteroarylrest gegebenenfalls mit einem oder mehreren gleichen oder verschiedenen Substituenten substituiert ist, die ausgewählt sind aus: (C₁-C₆)-Alkylcarbonyl und (C₁-C₆)-Alkoxycarbonyl;
wobei der Arylrest gegebenenfalls mit einem oder mehreren gleichen oder verschiedenen Substituenten substituiert ist, die ausgewählt sind aus: Halogen, Cyano, Nitro, Azido, (C₁-C₆)-Alkoxycarbonyl-(C₁-C₆)-Alkenyl, Oxy, -SO₂-NR₃₁R₃₂, Heterocycloalkyl, Heteroaryl oder-(CH₂)ₚ-V₃-Y₃;
R₃₁ und R₃₂ zusammen-mit dem-Stickstoffatom, an das sie gebunden sind, den Piperidinring bilden;
V₃ -O-, -S-, -C(O)-, -C(O)-O-, -SO₂-, -SO₂NH-, -NR'₃-, -NR'₃-C(O)-, -C(O)-NR'₃, -NH-C(O)-NR'₃- oder eine kovalente Bindung darstellt;
Y₃ darstellt: ein Wasserstoffatom; einen (C₁-C₆)-Alkylrest, der gegebenenfalls mit einem oder mehreren gleichen oder verschiedenen Halogenresten substituiert ist; einen Phenylrest; oder einen Benzylrest;
R'₃ ein Wasserstoffatom, einen Rest (C₁-C₆)-Alkyl oder (C₁-C₆)-_{A}lkoxy darstellt;
Z_{3d} den Cyclopropyl-, Cyclohexyl- oder Piperidinylrest darstellt, wobei jeder mit einem (C₁-C₆)-Alkoxycarbonylrest substituiert sein kann; oder ein pharmazeutisch annehmbares Salz dieser Verbindungen.

17. Verbindungen nach Anspruch 15, **dadurch gekennzeichnet, dass**
R₃ -Z₃ darstellt und Z₃ Z_{3c}, Z_{3d} oder Z₃ₑ darstellt;
Z_{3c} darstellt: einen Heteroarylrest, der aus Thienyl, Indolyl und Benzothienyl ausgewählt ist; oder einen Arylrest, der aus Phenyl und Naphthyl ausgewählt ist;
wobei der Heteroarylrest gegebenenfalls mit einem oder mehreren Oxyresten substituiert ist;
wobei der Arylrest gegebenenfalls mit einem oder mehreren gleichen oder verschiedenen Substituenten substituiert ist, die ausgewählt sind aus: Halogen, Nitro, Heteroaryl oder -(CH₂)_{p'}-V₃-Y₃;
V₃ -O-, -S-, -C(O)-, -C(O)-O-, -SO₂-, -SO₂NH-, -NR'₃-C(O)-, -C(O)-NR'₃-, -NH-C(O)-NR'₃- oder eine kovalente Bindung darstellt;
Y₃ darstellt: ein Wasserstoffatom; einen (C₁-C₆)-Alkylrest, der gegebenenfalls mit einem oder mehreren gleichen oder verschiedenen Halogenresten substituiert ist; einen Phenylrest; oder einen Benzylrest;
R'₃ ein Wasserstoffatom, einen (C₁-C₆)-Alkyl- oder (C₁-C₆)-Alkoxyrest darstellt;
Z_{3d} den Cyclopropyl- oder Piperidinylrest darstellt, wobei jeder gegebenenfalls mit (C₁-C₆)-Alkoxycarbonyl substituiert ist; oder ein pharmazeutisch annehmbares Salz dieser Verbindungen.

18. Verbindungen nach Anspruch 17, **dadurch gekennzeichnet, dass**
Z₃ Z₃, oder Z₃ₑ darstellt;
Z_{3c} einen Phenylrest darstellt, der gegebenenfalls mit einem oder mehreren gleichen oder verschiedenen Substituenten substituiert ist, die ausgewählt sind aus Nitro und -(CH₂)_{p'}-V₃-Y₃;
V₃ -O-, -S-, -C(O)-, -C(O)-O-, -SO₂-, -SO₂NH-, -NR'₃-C(O)-, -C(O)-NR'₃- oder eine kovalente Bindung darstellt;
Y₃ darstellt: ein Wasserstoffatom; einen Rest (C₁-C₆)-Alkyl; Phenyl; oder Benzyl;
R'₃ ein Wasserstoffatom darstellt;
Z_{3c} darstellt: 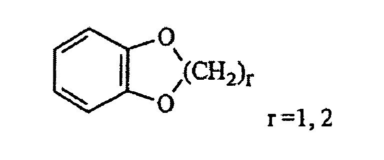 oder ein pharmazeutisch annehmbares Salz dieser Verbindungen.

19. Verbindungen nach einem der Ansprüche 11 bis 14, **dadurch gekennzeichnet, dass** R₃ -C(R_{Z3})(R'_{Z3})-Z₃ darstellt und Z₃ Z_{3b}, Z_{3c}, Z_{3d} oder Z₃ₑ darstellt; oder ein pharmazeutisch annehmbares Salz dieser Verbindungen.

20. Verbindungen nach Anspruch 19, **dadurch gekennzeichnet, dass**
R₃ -C(R_{Z3})(R'_{Z3})-Z₃ darstellt und Z₃ Z_{3b} oder Z_{3c} darstellt;
R_{Z3} und R'_{Z3} ein Wasserstoffatom darstellen;
oder ein pharmazeutisch annehmbares Salz dieser Verbindungen.

21. Verbindungen nach Anspruch 20, **dadurch gekennzeichnet, dass**
Z_{3b} einen (C₁-C₆)-Alkoxyrest darstellt;
Z_{3c} darstellt: einen Heteroarylrest, der aus Thienyl, Furyl, Pyridyl, Benzothienyl und Dihydrobenzofuryl ausgewählt ist; oder einen Arylrest; der aus Phenyl und Naphthyl ausgewählt ist,
wobei der Arylrest gegebenenfalls mit einem oder mehreren gleichen oder verschiedenen Substituenten substituiert ist, die ausgewählt sind aus: Halogen oder -(CH₂)_{p'}-V₃-Y₃;
V₃ -O-, -S-, -C(O)-, -C(O)-O-, -SO₂-, -SO₂NH-, -NR'₃-C(O)-, -C(O)-NR'₃- darstellt,
Y₃ darstellt: ein Wasserstoffatom, einen (C₁-C₆)-Alkylrest, der gegebenenfalls mit einem oder mehreren gleichen oder verschiedenen Halogenresten substituiert ist;
R'₃ ein Wasserstoffatom darstellt;
oder ein pharmazeutisch annehmbares Salz dieser Verbindungen.

22. Verbindungen nach Anspruch 20, **dadurch gekennzeichnet, dass**
R₃ -C(R_{Z3})(R'_{Z3})-Z₃ darstellt und Z₃ Z_{3b} oder Z_{3c} darstellt;
Z_{3b} einen (C₁-C₆)-Alkoxyrest darstellt;
Z_{3c} darstellt: ein Heteroaryl, das aus Thienyl, Furyl, Dihydrobenzofuryl ausgewählt ist; oder einen Phenylrest;
wobei der Phenylrest gegebenenfalls mit einem oder mehreren gleichen oder verschiedenen Substituenten substituiert ist, die ausgewählt sind aus: Nitro oder -(CH₂)_{p'}-V₃-Y₃;
V₃ -O-, -S-, -C(O)-, -C(O)-O-, -SO₂-, -SO₂NH-, -C(O)-NR'₃ darstellt;
Y₃ ein Wasserstoffatom oder einen (C₁-C₆)-Alkylrest darstellt, der gegebenenfalls mit einem oder mehreren gleichen oder verschiedenen Halogenresten substituiert ist;
R'₃ ein Wasserstoffatom darstellt;
oder ein pharmazeutisch annehmbares Salz dieser Verbindungen.

23. Verbindungen nach Anspruch 22, **dadurch gekennzeichnet, dass**
Z₃ Z_{3c} darstellt;
Z_{3c} einen Furyl- oder Phenylrest darstellt,
wobei der Phenylrest gegebenenfalls mit einem oder mehreren gleichen oder verschiedenen Substituenten der Formel -(CH₂)_{p'}-V₃-Y₃ substituiert ist;
V₃ -O-, -S-, -C(O)-, -C(O)-O-, -SO₂-, -SO₂NH-, -C(O)-NR'₃- darstellt,
Y₃ ein Wasserstoffatom oder einen (C₁-C₆)-Alkylrest darstellt, der gegebenenfalls mit einem oder mehreren gleichen oder verschiedenen Halogenresten substituiert ist;
R'₃ ein Wasserstoffatom darstellt;
oder ein pharmazeutisch annehmbares Salz dieser Verbindungen.

24. Verbindungen nach Anspruch 19, **dadurch gekennzeichnet, dass**
R₃ -C(R_{Z3})(R'_{Z3})-Z₃ darstellt und Z₃ Z_{3d} oder Z₃ₑ darstellt;
R_{Z3} und R'_{Z3} ein Wasserstoffatom oder (C₁-C₆)-Alkyl darstellen;
Z_{3d} einen der Reste (C₁-C₆)-Alkoxycarbonyl, (C₃-C₇)-Cycloalkyl oder Heterocycloalkyl darstellt;
Z₃ₑ darstellt: 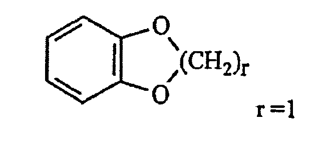 oder ein pharmazeutisch annehmbares Salz dieser Verbindungen.

25. Verbindungen nach Anspruch 24, **dadurch gekennzeichnet, dass**
Z_{3d} einen Rest (C₁-C₆)-Alkoxycarbonyl, Cyclohexyl oder ein Tetrahydrofuranyl darstellt;
oder ein pharmazeutisch annehmbares Salz dieser Verbindungen.

26. Verbindungen nach Anspruch 24, **dadurch gekennzeichnet, dass** Z₃ Z_{3d} oder Z₃ₑ darstellt;
Z_{3d} einen (C₁-C₆)-Alkoxycarbonylrest darstellt;
Z₃ₑ darstellt: 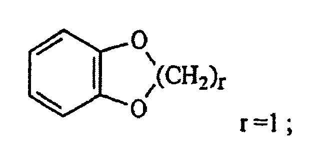 oder ein pharmazeutisch annehmbares Salz dieser Verbindungen.

27. Verbindungen nach Anspruch 26, **dadurch gekennzeichnet, dass** Z₃ Z₃ₑ darstellt 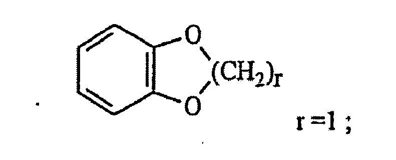 oder ein pharmazeutisch annehmbares Salz dieser Verbindungen.

28. Verbindungen nach einem der Ansprüche 11 bis 14, **dadurch gekennzeichnet, dass** R₃-C(R_{Z3})(R'_{Z3})-(CH₂)ₚ-Z₃ darstellt und Z₃ Z_{3b}, Z_{3c} oder Z_{3d} darstellt; oder ein pharmazeutisch annehmbares Salz dieser Verbindungen.

29. Verbindungen nach Anspruch 28, **dadurch gekennzeichnet, dass** R₃-C(R_{Z3})(R'_{Z3})-(CH₂)ₚ-Z₃darstellt und Z₃Z_{3b} darstellt; oder ein pharmazeutisch annehmbares Salz dieser Verbindungen.

30. Verbindungen nach Anspruch 29, **dadurch gekennzeichnet, dass**
R_{Z3} und R'_{Z3} unabhängig voneinander ein Wasserstoffatom oder einen (C₁-C₆)-Alkylrest darstellen;
Z_{3b} einen der Reste (C₁-C₆)-Alkoxy, (C₁-C₆)-Alkylthio oder Di((C₁-C₆)-alkyl)amino darstellt; oder ein pharmazeutisch annehmbares Salz dieser Verbindungen.

31. Verbindungen nach Anspruch 29, **dadurch gekennzeichnet, dass**
R_{Z3} und R'_{Z3} unabhängig voneinander ein Wasserstoffatom oder einen (C₁-C₆)-Alkylrest darstellen;
Z_{3b} einen-(C₁-C₆)-Alkoxy-oder-(C₁-C₆)-Alkylthiorest darstellt; oder ein pharmazeutisch annehmbares Salz dieser Verbindungen.

32. Verbindungen nach Anspruch 28, **dadurch gekennzeichnet, dass** R₃-C(R_{Z3})(R'_{Z3})-(CH₂)ₚ-Z₃ darstellt und Z₃ Z_{3c} oder Z_{3d} darstellt; oder ein pharmazeutisch annehmbares Salz dieser Verbindungen.

33. Verbindungen nach Anspruch 32, **dadurch gekennzeichnet, dass**
R_{Z3} und R'_{Z3} unabhängig voneinander ein Wasserstoffatom oder einen (C₁-C₆)-Alkylrest darstellen;
Z_{3c} einen Indolyl- oder Phenylrest darstellt;
wobei der Phenylrest gegebenenfalls mit einem oder mehreren gleichen oder verschiedenen Substituenten substituiert ist, die ausgewählt sind aus: Halogen und -(CH₂)_{p'}-V₃-Y₃;
V₃ -SO₂NH- darstellt,
Y₃ ein Wasserstoffatom oder einen (C₁-C₆)-Alkylrest darstellt;
Z_{3d} einen Rest (C₁-C₆)-Alkoxycarbonyl, Aminocarbonyl, (C₁-C₆)-Alkylaminocarbonyl, (C₁-C₆)-Alkyl-C(O)-NH- oder Heterocycloalkyl, der gegebenenfalls mit Oxy substituiert ist, und vorzugsweise Piperidinyl, Morpholinyl, Pyrrolidin oder Imidazolidinyl darstellt; oder ein pharmazeutisch annehmbares Salz dieser Verbindungen.

34. Verbindungen nach Anspruch 32, **dadurch gekennzeichnet, dass**
Z₃ Z_{3d} darstellt;
R_{Z3} und R'_{Z3} unabhängig voneinander ein Wasserstoffatom oder einen (C₁-C₆)-Alkylrest darstellen;
Z_{3d} einen Rest (C₁-C₆)-Alkoxycarbonyl, Aminocarbonyl, (C₁-C₆)-Alkylaminocarbonyl, (C₁-C₆)-Alkyl-C(O)-NH- oder Heterocycloalkyl und vorzugsweise Pyrrolidin oder imidazolidin darstellt, gegebenenfalls mit Oxy substituiert; oder ein pharmazeutisch annehmbares Salz dieser Verbindungen.

35. Verfahren zur Herstellung einer Verbindung der Formel (I) nach einem der vorhergehenden Ansprüche, **dadurch gekennzeichnet, dass** man die Verbindung der allgemeinen Formel: 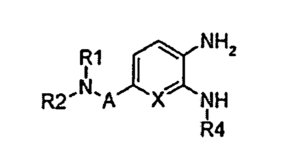 in der A, X, R₁, R₂, R₄ die in Anspruch 1 angegebene Bedeutung haben, mit einem Isothiocyanat der allgemeinen Formel R₃N=C=S, in der R₃ die in Anspruch 1 angegebene Bedeutung hat, in Gegenwart eines Kopplungsmittels oder von gelbem Quecksilber(II)-Oxid in Gegenwart von Schwefel während einer Dauer von 3 bis 48 Stunden in einem protischen oder aprotischen Lösungsmittel bei einer Temperatur von 50 bis 80°C behandelt.

36. Pharmazeutische Zusammensetzung, die als Wirkstoffe mindestens eine Verbindung nach einem der Ansprüche 1 bis 34 in Kombination mit einem pharmazeutisch annehmbaren Träger enthält.

37. Verwendung einer Verbindung nach einem der Ansprüche 1 bis 34 für die Herstellung eines Medikaments für die Behandlung von Gewichtsstörungen, mentalen Störungen, Schmerz oder Störungen der sexuellen Aktivität.

38. Verwendung nach Anspruch 37 für die Behandlung von Gewichtsstörungen, wie Anorexie und Kachexie und insbesondere Kachexie bei Krebserkrankungen, Kachexie bei Aids, Kachexie älterer Menschen, Herzkachexie, Nierenkachexie, Kachexie bei rheumatoider Arthritis.

39. Verwendung nach Anspruch 37 für die Behandlung von Schmerzen und insbesondere von neuropathischen Schmerzen.

40. Verwendung nach Anspruch 37 für die Behandlung von mentalen Störungen wie Angst und Depression.

41. Verwendung einer Verbindung der allgemeinen Formel (I') 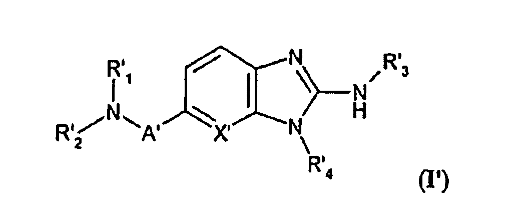 in racemischer Form, Enantiomerenform oder allen Kombinationen dieser Formen, in der:
A' -CH₂-, -C(O)-, -C(O)-C(Rₐ)(R_{b})- darstellt;
X'-CH- darstellt;
Rₐ und R_{b} unabhängig voneinander ein Wasserstoffatom oder einen (C₁-C₆)-Alkylrest darstellen;
R'₁ darstellt: ein Wasserstoffatom; einen (C₁-C₈)-Alkylrest, der gegebenenfalls mit Hydroxy oder einem oder mehreren gleichen oder verschiedenen Halogenresten substituiert ist; (C₂-C₆)-Alkenyl; oder einen Rest der Formel -(CH₂)ₙ-X₁;
R'₂ darstellt: einen (C₁-C₈)-Alkylrest, der gegebenenfalls mit Hydroxy oder einem oder mehreren gleichen oder verschiedenen Halogenresten substituiert ist; (C₂-C₆)-Alkenyl; oder einen Rest der Formel -(CH₂)ₙ-X₁;
jedes X₁ unabhängig voneinander (C₁-C₆)-Alkoxy, (C₃-C₇)-Cycloalkyl, Adamantyl, Heterocycloalkyl, Aryl oder Heteroaryl darstellt,
wobei die Reste (C₃-C₇)-Cycloalkyl, Heterocycloalkyl, Aryl und Heteroaryl gegebenenfalls mit einem oder mehreren gleichen oder verschiedenen Substituenten substituiert sind, die ausgewählt sind aus: -(CH₂)_{n'}-V₁-Y₁, Halogen, Nitro, Cyano und Aryl;
V₁ -O-, -S- oder eine kovalente Bindung darstellt;
Y₁ einen (C₁-C₆)-Alkylrest darstellt, der gegebenenfalls mit einem oder mehreren gleichen oder verschiedenen Halogenresten substituiert ist;
n eine ganze Zahl von 0 bis 6 und n' eine ganze Zahl von 0 bis 2 darstellt (wobei gilt, dass, wenn n gleich 0 ist, dann X₁ nicht den Alkoxyrest darstellt);
oder R₁ und R₂ zusammen mit dem Stickstoffatom, an das sie gebunden sind, ein Heterobicycloalkyl oder ein Heterocycloalkyl bilden, das gegebenenfalls mit einem oder mehreren gleichen oder verschiedenen Substituenten substituiert ist, die ausgewählt sind aus: Hydroxy, (C₁-C₆)-Alkyl, das gegebenenfalls mit Hydroxy substituiert ist, (C₁-C₆)-Alkoxycarbonyl, Heterocycloalkyl und -C(O)NV₁'Y₁', wobei V₁' und Y₁' unabhängig voneinander ein Wasserstoffatom oder ein (C₁-C₆)-Alkyl darstellen; oder R₁ und R₂ zusammen einen Rest der Formel bilden: 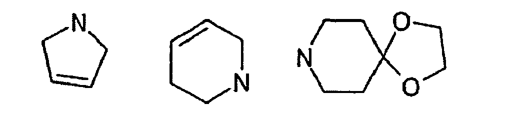
R'₃ -Z₃, -C(R_{Z3})(R'_{Z3})-Z₃, -C(R_{Z3})(R'_{Z3})-(CH₂)ₚ-Z₃ oder -C(O)-Z'₃ darstellt;
R_{Z3} und R'_{Z3} unabhängig voneinander ein Wasserstoffatom oder einen (C₁-C₆)-Alkylrest darstellen;
Z₃ Z₃ₐ, Z_{3b}, Z_{3c}, Z_{3d} oder Z₃ₑ darstellt;
Z₃ₐ einen (C₁-C₆)-Alkylrest darstellt;
Z_{3b} einen der Reste (C₁-C₆)-Alkoxy, (C₁-C₆)-Alkylthio, (C₁-C₆)-Alkylamino oder Di((C₁-C₆)-alkyl)amino darstellt;
Z_{3c} einen Aryl- oder Heteroarylrest darstellt;
Z_{3d} einen der Reste (C₁-C₆)-Alkoxycarbonyl, Aminocarbonyl, (C₁-C₆)-Alkylaminocarbonyl, Di((C₁-C₆)-alkyl)aminocarbonyl, (C₁-C₆)-Alkyl-C(O)-NH-, (C₃-C₇)-Cycloalkyl, Heterocycloalkyl darstellt;
wobei die Reste (C₃-C₇)-Cycloalkyl und Heterocycloalkyl gegebenenfalls mit einem oder mehreren gleichen oder verschiedenen Substituenten substituiert sind, die ausgewählt sind aus: Halogen, Nitro, (C₁-C₆)-Alkoxy, das gegebenenfalls mit einem oder mehreren gleichen oder verschiedenen Halogenresten substituiert ist, (C₁-C₆)-Alkyl, das gegebenenfalls mit einem oder mehreren gleichen oder verschiedenen Halogenresten substituiert ist, (C₁-C₆)-Alkylcarbonyl, (C₁-C₆)-Alkoxycarbonyl, Aminocarbonyl, (C₁-C₆)-Alkylaminocarbonyl, Di((C₁-C₆)-alkyl)aminocarbonyl und Oxy,
wobei die Reste Aryl und Heteroaryl gegebenenfalls mit einem oder mehreren gleichen oder verschiedenen Substituenten substituiert sind, die ausgewählt sind aus: Halogen, Cyano, Nitro, Azido, Oxy, (C₁-C₆)-Alkoxycarbonyl-(C₁-C₆)-Alkenyl, (C₁-C₆)-Alkenylaminocarbonyl-(C₁-C₆)-alkenyl, -SO₂-NR₃₁R₃₂, Heterocycloalkyl, Heteroaryl oder-(CH₂)_{p'}-V₃-Y₃;
R₃₁ und R₃₂ zusammen mit dem Stickstoffatom, an das sie gebunden sind, ein Heterocycloalkyl bilden;
V₃ -O-, -S-, -C(O)-, -C(O)-O-, -O-C(O)-, -SO₂-, -SO₂NH-, -NR'₃-SO₂-, -NR'₃-, -NR'₃-C(O)-, -C(O)-NR'₃-, -NH-C(O)-NR'₃- oder eine kovalente Bindung darstellt;
Y₃ darstellt: ein Wasserstoffatom; einen Rest (C₁-C₆)-Alkyl, der gegebenenfalls mit einem oder mehreren gleichen oder verschiedenen Halogenresten substituiert ist; einen Arylrest, der gegebenenfalls mit einem oder mehreren gleichen oder verschiedenen Substituenten substituiert ist, die ausgewählt sind aus: Halogen, Nitro, (C₁-C₆)-Alkyl und (C₁-C₆)-Alkoxy-, oder einen Rest Aryl-(C₁-C₆)-Alkyl, der gegebenenfalls mit einem oder mehreren gleichen oder verschiedenen Substituenten substituiert ist, die ausgewählt sind aus: Halogen, Nitro, (C₁-C₆)-Alkyl und (C₁-C₆)-Alkoxy;
Z₃ₑ einen Rest der Formel darstellt: 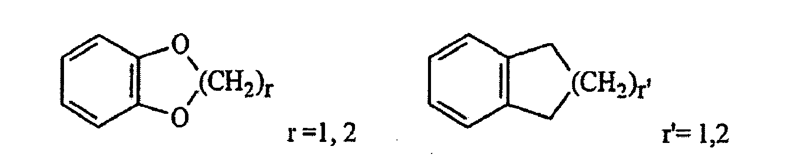 Z'₃ einen Arylrest darstellt, der gegebenenfalls mit einem oder mehreren gleichen oder verschiedenen Substituenten substituiert ist, die ausgewählt sind aus: Halogen, Nitro und -(CH₂)_{p'}-V'₃-Y'₃;
V'₃ -O-, -C(O)-, -C(O)-O-, -C(O)-NR'₃, -NH-C(O)-NR'₃ oder eine kovalente Bindung darstellt;
Y'₃ ein Wasserstoffatom oder einen (C₁-C₆)-Alkylrest darstellt, der gegebenenfalls mit einem oder mehreren gleichen oder verschiedenen Halogenresten substituiert ist;
R'₃ ein Wasserstoffatom, einen (C₁-C₆)-Alkyl- oder (C₁-C₆)-Alkoxyrest darstellt;
p eine ganze Zahl von 1 bis 4 darstellt; p' und p" unabhängig voneinander eine ganze Zahl von 0 bis 4 darstellen;
R₄ einen Rest der Formel -(CH₂)ₛ-R'₄ darstellt;
R'₄ darstellt: den Guanidinorest; ein Heterocycloalkyl, das mindestens ein Stickstoffatom enthält und gegebenenfalls mit (C₁-C₆)-Alkyl oder Aralkyl substituiert ist; ein Heteroaryl, das mindestens ein Stickstoffatom enthält und gegebenenfalls mit (C₁-C₆)-Alkyl substituiert ist; oder einen Rest der Forme) -NW₄W'₄
W₄ ein Wasserstoffatom oder (C₁-C₈)-Alkyl darstellt;
W'₄ einen Rest der Formel -(CH₂)ₛ-Z₄ darstellt;
Z₄ darstellt: ein Wasserstoffatom, (C₁-C₈)-Alkyl-, das-gegebenenfalls mit einem oder mehreren gleichen oder verschiedenen Substituenten substituiert ist, die ausgewählt sind aus: (C₁-C₆)-Alkoxy, (C₁-C₆)-Alkylthio und Hydroxy; (C₂-C₆)-Alkenyl; (C₃-C₇)-Cycloalkyl, das gegebenenfalls mit einem oder mehreren gleichen oder verschiedenen Substituenten (C₁-C₆)-Alkyl substituiert ist; Cyclohexen; Heteroaryl und Aryl;
wobei die Reste Aryl und Heteroaryl gegebenenfalls mit einem oder mehreren gleichen oder verschiedenen Resten substituiert sind, die ausgewählt sind aus der Formel -(CH₂)_{s"}-V₄-Y₄, Hydroxy, Halogen, Nitro und Cyano;
V₄ -O-, -S-, -NH-C(O)-, -NV'₄ oder eine kovalente Bindung darstellt;
Y₄ ein Wasserstoffatom oder einen (C₁-C₆)-Alkylrest darstellt, der gegebenenfalls mit einem oder mehreren gleichen oder verschiedenen Halogenresten substituiert ist;
V₄' ein Wasserstoffatom oder (C₁-C₆)-Alkyl darstellt;
s" eine ganze Zahl von 0 bis 4 darstellt;
oder Z₄ einen Rest der Formel darstellt: 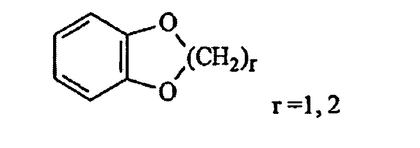
s und s' unabhängig voneinander eine ganze Zahl von 0 bis 6 darstellen; oder ein pharmazeutisch annehmbares Salz dieser Verbindung,
für die Herstellung eines Medikaments zur Behandlung von Gewichtsstörungen, mentalen Störungen, Schmerz oder Störungen der sexuellen Aktivität.

42. Verwendung nach Anspruch 41, **dadurch gekennzeichnet, dass**
R₁ und R₂ unabhängig voneinander einen (C₁-C₈)-Alkylrest darstellen;
R₃ Z_{3c} darstellt und Z_{3c} einen Rest Phenyl oder Naphthyl darstellt, der jeweils mindestens mit Cyano substituiert ist;
R₄ darstellt: einen Rest der Formel -(CH₂)ₛ R'₄, wobei R'₄ den Rest Pyrrolidinyl oder Piperidinyl darstellt; oder einen Rest der Formel -NW₄W'₄ ;
wobei W₄ ein Wasserstoffatom oder (C₁-C₈)-Alkyl darstellt;
W'₄ einen Rest der Formel -(CH₂)_{s'}-Z₄ darstellt, wobei Z₄ ein Wasserstoffatom darstellt;
s eine ganze Zahl von 2 bis 4 darstellt; s' eine ganze Zahl von 0 bis 4 darstellt;
oder ein pharmazeutisch annehmbares Salz dieser Verbindungen.
